(19) Europäisches Patentamt
European Patent Office
Office européen des brevets

(11) EP 2 520 573 A1

(12) EUROPEAN PATENT APPLICATION

(43) Date of publication:
07.11.2012 Bulletin 2012/45

(21) Application number: 12166980.8

(22) Date of filing: 07.05.2012

(51) Int Cl.:
C07D 309/12 (2006.01) C07D 213/42 (2006.01)
C07C 311/29 (2006.01) C07B 59/00 (2006.01)
A61K 31/255 (2006.01) A61K 31/4409 (2006.01)
A61P 9/00 (2006.01) A61P 29/00 (2006.01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 08.06.2011 US 201113155558
06.05.2011 EP 11165157

(71) Applicants:
- Westfälische Wilhelms-Universität Münster 48149 Münster (DE)
- Siemens Medical Solutions USA, Inc. Malvern, PA 19355 (US)

(72) Inventors:
- Haufe, Guenter 48163 Muenster (DE)
- Behrends, Malte 49808 Lingen (DE)
- Kopka, Klaus 48159 Muenster (DE)
- Wagner, Stefan 48163 Muenster (DE)
- Hugenberg, Verena 49593 Bersenbrueck (DE)
- Breyholz, Hans-Joerg 48151 Muenster (DE)
- Hermann, Sven 48161 Muenster (DE)
- Schaefers, Michael 48329 Havixbeck (DE)
- Kolb, Hartmuth Playa del Rey, CA California 90293 (US)

(74) Representative: Schiweck, Weinzierl & Koch
European Patent Attorneys
Landsberger Straße 98
80339 München (DE)

# (54) Compounds with matrix-metalloproteinase inhibitory activity

(57) The present invention relates to the field of therapeutic and diagnostic agents and more specifically to compounds of formula (I) that are inhibitors of matrix-metalloproteinases (MMPs) and are useful in the treatment of diseases related thereto such as cardiovascular diseases, inflammatory diseases and malignant diseases. One embodiment of the invention is a compound of formula (I) labeled with a 18-fluorine atom having matrix metalloproteinase inhibitory activity suitable for diagnostic imaging. Also disclosed in the present invention is a pharmaceutical composition comprising the inhibitors of matrix-metalloproteinases (MMPs) of the invention or the corresponding labeled compounds useful as diagnostic imaging agents of the invention in a form suitable for mammalian administration. The invention furthermore discloses intermediates in the synthesis of the inhibitors of matrix-metalloproteinases (MMPs) of the invention and of the diagnostic imaging agents of the invention and kits for the preparation of the pharmaceutical composition of the invention.

EP 2 520 573 A1

Printed by Jouve, 75001 PARIS (FR)

## Description

### Technical Field of the Invention

The present invention relates to the field of therapeutic and diagnostic agents and more specifically to compounds that are inhibitors of matrix-metalloproteinases (MMPs) and are useful in the treatment of diseases related thereto such as cardiovascular diseases, inflammatory diseases and malignant diseases. The compounds are useful in the field of *in vivo* diagnostic imaging and in particular in (Positron Emission Tomography) PET imaging, as well.

### Description of Related Art

The matrix-metalloproteinases (MMPs) are a family of at least 20 zinc-dependent endo-peptidases which mediate degradation, or remodeling of the extracellular matrix (ECM) [Massova et al., FASEB J. 1998, 12, 1075-1095]. Together, the members of the MMP family can degrade e.g. components of the blood vessel wall and play a major role in both physiological and pathological events that involve the degradation of components of the ECM. Since the MMPs can interfere with the cell-matrix interactions that control cell behavior, their activity affects processes as diverse as cellular differentiation, migration, proliferation and apoptosis [Nagase and Woessner, J. Biol. Chem. 1999, 274, 21491-21494]. The negative regulatory controls that finely regulate MMP activity in physiological situations do not always function as they should. Inappropriate expression of MMP activity is thought to constitute part of the pathological mechanism in several disease states. MMPs are therefore targets for therapeutic inhibitors in many inflammatory, malignant and degenerative diseases [Whittaker et al., Chem. Rev. 1999, 99, 2735-2776].

Consequently, it is believed that synthetic inhibitors of MMPs may be useful in the treatment of many inflammatory, malignant and degenerative diseases. Furthermore, it has been suggested that inhibitors of MMPs may be useful in the diagnosis of these diseases. WO 01/60416 discloses compounds, which are proposed to be useful in the diagnosis of cardiovascular pathologies associated with extracellular matrix degradation such as atherosclerosis, heart failure and restenosis. The compounds disclosed therein comprise MMP inhibitors linked, via an optional linker, to a chelator capable of conjugating to a diagnostic metal. Preferred MMP inhibitors, chelators and linkers are described therein. A report by Zheng et al. [Nuc. Med. Biol. 2002, 29, 761-770] documented the synthesis of MMP inhibitors labeled with the positron emission tomography (PET) tracers $^{11}C$ and $^{18}F$. These compounds described therein are useful in the non-invasive imaging of breast cancer.

### Summary of the Invention

Novel compounds having MMP inhibitory activity are disclosed, which have been found to be particularly useful in the prevention, treatment and diagnostic imaging of diseases associated with an unpaired activity of MMP, amongst others MMP-2, MMP-8, MMP-9 and/or MMP-13.

Another aspect of the present invention relates to pharmaceutical compositions useful in prevention, treatment and diagnostic imaging of diseases associated with an unpaired activity of MMP.

The compounds of the present invention are useful for the prevention, the treatment and the *in vivo* diagnostic imaging of a range of disease states (inflammatory, malignant and degenerative diseases) where specific matrix metalloproteinases are known to be involved. These include:

(a) atherosclerosis, where various MMPs are overexpressed. Elevated levels of MMP-1, 3, 7, 9, 11, 12, 13 and MT1-MMP have been detected in human atherosclerotic plaques [George, Exp. Opin. Invest. Drugs 2000, 9, 993-1007 and references therein]. Expression of MMP-2 [Li et al. Am. J. Pathol. 1996, 148, 121-128] and MMP-8 [Herman et al., Circulation 2001, 104, 1899-1904] in human atheroma has also been reported;

(b) CHF [Peterson et al., Matrix metalloproteinase inhibitor development for the treatment of heart failure, Drug Dev. Res. 2002, 55, 29-44] report that MMP-1, MMP-2, MMP-3, MMP-8, MMP-9, MMP-13 and MMP-14 are upregulated in heart failure;

(c) cancer [Vihinen et al., Int. J. Cancer 2002, 99, 157-186] reviews MMP involvement in cancers, and particularly highlights MMP-2, MMP-3, MMP-7, and MMP-9];

(d) arthritis [Jacson et al., Inflamm. Res. 2001, 50, 183-186 "Selective matrix metalloproteinase inhibition in rheumatoid arthritis-targeting gelatinase A activation"], MMP-2 is particularly discussed;

(e) amyotrophic lateral sclerosis [Lim et al., J. Neurochem. 1996, 67, 251-259] where MMP-2 and MMP-9 are involved;

(f) brain metastases, where MMP-2, MMP-9 and MMP-13 have been reported to be implicated [Spinale, Circul. Res. 2002, 90, 520-530];

(g) cerebrovascular diseases, where MMP-2 and MMP-9 have been reported to be involved [Lukes et al., Mol. Neurobiol. 1999, 19, 267-284];

(h) Alzheimer's disease, where MMP-2 and MMP-9 have been identified in diseased tissue [Backstrom et al., J. Neurochem. 1992, 58, 983-992];

(i) neuroinflammatory diseases, where MMP-2, MMP-3 and MMP-9 are involved [Mun-Bryce et al., Brain. Res. 2002, 933, 42-49];

(j) COPD (i. e. chronic obstructive pulmonary disease) where MMP-1, MMP-2, MMP-8 and MMP-9 have been reported to be upregulated [Segura-Valdez et al., Chest. 2000, 117, 684-694];

(k) eye pathology [Kurpakus-Wheater et al., Prog. Histo. Cytochem. 2001, 36, 179-259];

(l) skin diseases [Herouy, Int. J. Mol. Med. 2001, 7, 3-12].

**Detailed Description of the Invention**

It has been surprisingly found that compounds of below general formula (I) wherein the group $R^3$ is an alkenyl optionally substituted with a fluorine or an alkyl substituted with a fluorine act as inhibitors of MMP and in particular of MMP-2 and MMP-9. It has been surprisingly found that the presence of the group $R^3$ as defined below in formula (I) provide these compounds with a very good inhibitory activity. Additionally, it has been surprisingly found that the inhibition is remarkable also in the (S)-enantiomer of the compounds of formula (I). This is particularly surprising because the (*R*)-enantiomer of the prior art analog compounds (e.g. hydroxamate derivatives such as CGS 27023A) are far more active than the corresponding (*S*)-enantiomers. For example, it is known that the (*S*)-enantiomer of compound CGS 27023A is far less potent then the corresponding (*R*)-enantiomer in the inhibition of MMP-2 and MMP-9 (see table 1). As a consequence, the use of the (*S*)-enantiomers or the racemates of the compounds of formula (I) as MMP inhibitors as well as the use of the (*R*)-enantiomers as MMP inhibitors is rendered possible by the introduction of the $R^3$ group.

A first aspect of the present invention relates to compounds of formula (I)

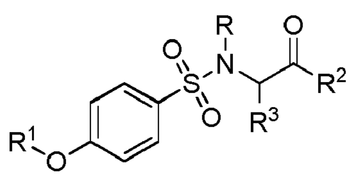

formula (I)

wherein

R is selected from the group of optionally substituted arylalkyl and optionally substituted heteroarylalkyl;

$R^1$ is $C_1$-$C_6$ alkyl, $C_1$-$C_6$ alkenyl, $C_1$-$C_7$ alkynyl wherein the $C_1$-$C_6$ alkyl, $C_1$-$C_6$ alkenyl, $C_1$-$C_7$ alkynyl are optionally substituted with one or more substituents selected from halogen, OH or OTs, or $R^1$ is $(CH_2$-$CH_2$-$O$-$)_n$- $CH_2$-$CH_2$-$R^5$;

$R^2$ is $C_1$-$C_6$ alkyl, $C_1$-$C_6$ alkoxy or-NH-$OR^4$;

$R^3$ is $C_1$-$C_6$ fluoro-alkyl or optionally substituted $C_2$-$C_6$ alkenyl wherein the substituent is one or more F; preferably F is a fluorine atom on one of the carbons forming the double bond, preferably when $R^3$ is a $C_2$-$C_6$ alkenyl substituted with F then $R^3$ is a $CH_2$-C(F)=$CH_2$. Preferably, the F atom is on the carbon atom which is the second carbon atom of the alkyl or alkenyl chain of $R^3$ counted starting from the first carbon atom of R3 attached to the rest of the molecule (e.g. -$CH_2$-C(F)=$CH_2$);

$R^4$ is H, $C_1$-$C_6$ alkyl, $C_3$-$C_6$ cycloalkyl or $C_5$-$C_6$ heterocycloalkyl;

$R^5$ is F, Cl, OH, OTs; and

n is 0, 1, 2, 3, 4, 5 or 6, preferably n is 3.

and enantiomers and pharmaceutically acceptable salts and ester thereof.

Preferably, in the compounds of formula (I)

R is pyrid-3-yl-$CH_2$- or phenyl-$CH_2$-;

$R^1$ is -$CH_3$, -$CH_2$-$CH_2$Cl, -$CH_2$-$CH_2$F, -$CH_2$-$CH_2$-OTs or -$CH_2$-$CH_2$OH;

$R^2$ is -NH-$OR^4$;

$R^3$ is $-CH_2-CH_2F$, $-CH_2-C(F)=CH_2$ or $CH_2-CH=CH_2$;
$R^4$ is H, methyl, t-butyl or tetrahydropyranyl.

The pyrid-3-yl-$CH_2$- or the phenyl-$CH_2$- may be optionally substituted. Preferably, the substituent(s) when present is/are on the ring.

The compounds according to formula (I) have the carbon atom bearing radical $R^3$ with a (*S*) configuration or the compounds according to formula (I) have the carbon atom bearing radical $R^3$ with a (*R*) configuration.

In another aspect, the present invention relates to compounds of formula (I) wherein R denotes benzyl, 2-picolyl, and in particular 3-picolyl.

In another aspect, the present invention relates to compounds of formula (I) wherein $R^1$ is - $CH_3$, $-CH_2-CH_2F$, $-CH_2-CH_2-^{18}F$, $CH_2-CH_2-Cl$, $-CH_2-CH_2-OH$ or $-CH_2-CH_2-OTs$.

In another aspect, the present invention relates to compounds of formula (I) wherein $R^2$ is - NH-OH, -O-*tert*-butyl, or $-OCH_3$.

In another aspect, the present invention relates to compounds of formula (I) wherein $R^3$ is selected from $-CH_2-CH_2F$, $-CH_2-CH_2-^{18}F$, $CH_2-C(F)=CH_2$, $-CH_2-C(^{18}F)=CH_2$ and $CH_2-CH=CH_2$.

In another aspect, the present invention relates to compounds wherein $R^4$ is selected from H, *tert*-butyl and methyl.

In another aspect the present invention relates to compounds of formula (I) selected from:

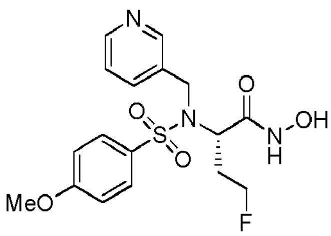,
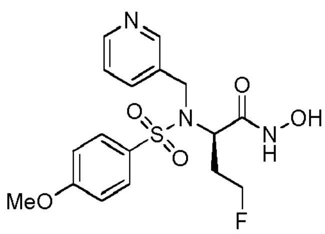,
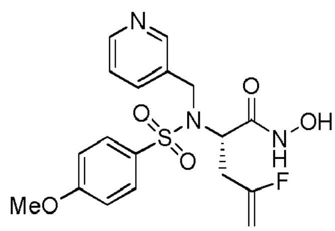,
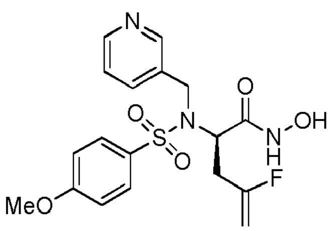,
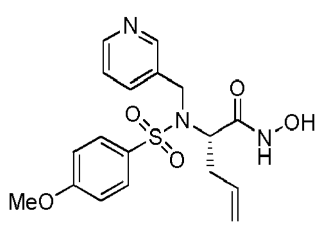,
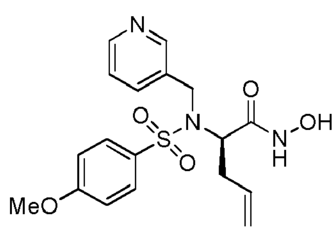,
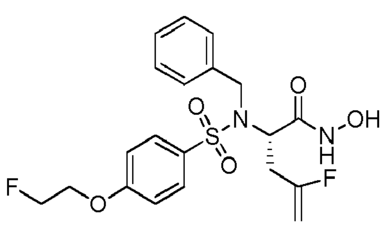,
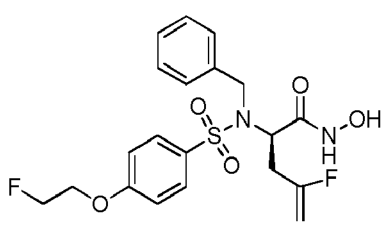

and the corresponding racemates, ester and pharmaceutically acceptable salts thereof.

In another aspect, the present invention relates to compounds of formula (I) which are substituted with $^{18}F$.

In another aspect, the present invention relates to compounds of formula (I) wherein $R^3$ is selected from $C_1$-$C_6$($^{18}F$) alkyl, $C_2$-$C_6$($^{18}F$) alkenyl. More preferably, $R^3$ is $CH_2CH_2-^{18}F$ or $-CH_2-C(^{18}F)=CH_2$.

In another aspect, the present invention relates to compounds of formula (I) wherein $R^1$ is $C_1$-$C_6$ alkyl substituted with a $^{18}F$. More preferably, $R^1$ is $CH_2CH_2-^{18}F$.

In another aspect, the present invention relates to compounds of formula (I) wherein radical R is substituted with a $^{18}F$. Preferably, $^{18}F$ is on the aryl or heteroaryl moiety of radical R or on a substituent of the aryl or heteroaryl moiety of radical R.

$R^5$ in the compounds of formula (I) may be $^{18}F$.

Preferably, the compounds of formula (I) bear one $^{18}F$. In this case, $^{18}F$ may be on a carbon atom of radical R or $R^1$ or $R^3$, preferably as specified above or $R^5$ can be $^{18}F$.

The compounds according to formula (I) as described above have the carbon atom bearing radical $R^3$ with a

(S) configuration or a (R) configuration.

In another aspect, the present invention relates to compounds of formula (I) for use as a medicament.

In another aspect, the present invention relates to compounds of formula (I) which are labeled with a $^{18}F$ atom for use as a diagnostic agent, in particular as an *in vivo* diagnostic agent and more in particular in Positron Emission Tomography (PET).

In a further aspect, the present invention related to compounds for use in the prevention and/or treatment of pathological conditions associated with unpaired expression of matrix-metalloproteases in human and animal, in particular mammals.

In another aspect, the present invention relates to compounds of formula (I) labeled with a $^{18}F$ for use in the diagnosis of pathological conditions associated with unpaired expression of metalloproteases in human and animal, in particular mammals. In particular, the compounds are used in the *in vivo* diagnostic imaging, more in particular in PET.

The pathological conditions are selected from the group consisting of cardiovascular diseases, inflammatory diseases and malignant diseases. More in particular, the cardiovascular diseases are selected from atherosclerosis and congestive heart failure, the inflammatory disease is a chronic obstructive pulmonary disease, the malignant diseases are cancers.

The use of the diagnostic imaging compound of the invention permits the identification of active plaque burden, which allows risk stratification of patients with known or suspected coronary artery disease, i. e. patients with pain or a history of pain, or identified as high risk but asymptomatic. In addition, the diagnostic imaging agents of the invention permit identification of vulnerable plaques in symptomatic patients, which allows identification of high risk of acute myocardial infarction or stroke irrespective of stenosis and permits immediate risk stratification when the patient presents with chest pain. Furthermore, angioplasty of vulnerable plaques is high risk, and may lead to embolism of the artery tree post surgery. Thus imaging of this subtype of plaques may help reduce post-surgical complication.

In a further aspect the present invention relates to pharmaceutical compositions comprising a compound of formula (I).

Suitable preparations include for example tablets, capsules, suppositories, solutions, - particularly solutions for injection (s.c, i.v., i.m.) and infusion - syrups, elixirs, solution for inhalation.

The invention relates to pharmaceutical compositions comprising an effective amount, especially an amount effective in the treatment of one of the above-mentioned disorders, of the active ingredient together with pharmaceutically acceptable carriers that are suitable for topical, enteral, for example oral or rectal, or intravenous or parenteral administration and that may be inorganic or organic, solid or liquid. They are used for oral administration, especially tablets or gelatin capsules that comprise the active ingredient together with diluents, for example lactose, dextrose, sucrose, mannitol, sorbitol, cellulose and/or glycerol, and/or lubricants, for example silica, talc, stearic acid or salts thereof, such as magnesium or calcium stearate, and/or polyethylene glycol. Tablets may also comprise binders, for example magnesium aluminum silicate, starches, such as corn, wheat or rice starch, gelatin, methylcellulose, sodium carboxymethylcellulose and/or polyvinylpyrrolidone, and, if desired, disintegrators, for example starches, agar, alginic acid or a salt thereof, such as sodium alginate, and/or effervescent mixtures, or adsorbents, dyes, flavorings and sweeteners.

It is also possible to use the pharmacologically active compounds of the present invention in the form of intravenously and parentally administrable compositions or in the form of infusion solutions. Such solutions are preferably isotonic aqueous solutions or suspensions which, for example in the case of lyophilized compositions that comprise the active ingredient alone or together with a carrier, for example mannitol, can be made up prior to use. The pharmaceutical compositions may be sterilized and/or may comprise excipients, for example preservatives, stabilizers, wetting agents and/or emulsifiers, solubilizes, salts for regulating the osmotic pressure and/or buffers. The present pharmaceutical compositions are prepared in a manner known per se, for example by means of conventional mixing, granulating, confectioning, dissolving or lyophilizing processes, and comprise approximately from 1 % to 95%, especially from approximately 1 % to approximately 20%, active ingredient(s).

The present invention further provides a pharmaceutical composition which comprises a compound of formula (I) labeled with a $^{18}F$ together with a biocompatible carrier, in a form suitable for mammalian, more in particular human administration.

In a further aspect, the present invention relates to a precursor useful in the preparation of the compounds of formula (I) labeled with a $^{18}F$ atom. The precursor comprises a group suitable for reaction with $^{18}F$ to give said compound of formula (I). Suitable precursors of the invention for preparation of imaging compounds of formula (I) are compounds of formula (I) which comprise a non-radioactive group (e.g. tosyl group) to permit $^{18}F$ exchange, preferably via nucleophile substitution reaction. Preferably, the radiofluorination with $^{18}F$-fluoride occurs via nucleophile substitution reaction of a tosyl group on a precursor compound of the compounds of formula (I) with [$^{18}F$](Kryptofix222)KF.

Preferably the precursors are compounds of formula (II)

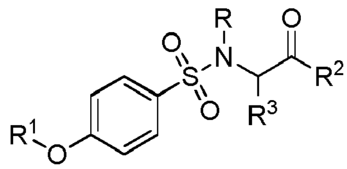

formula (II)

wherein R and $R^2$ are as defined above in formula (I); $R^1$ is an optionally substituted $C_1$-$C_6$ alkyl (preferably a $C_2$ alkyl), wherein the substituent is a OH, OTs or another leaving group for nucleophile substitution; $R^3$ is an optionally substituted $C_1$-$C_6$ alkyl or an optionally substituted $C_2$-$C_6$ alkenyl wherein the substituent is a OH, OTs or suitable leaving group for nucleophile substitution, with the proviso that at least one of $R^1$ and $R^3$ is substituted with a OH, OTs or another leaving group for nucleophile substitution and /or with the proviso that when $R^1$ is substituted with a OH, OTs or another leaving group for nucleophile substitution then $R^3$ is $C_1$-$C_6$ fluoro alkyl or $C_2$-$C_6$ alkenyl optionally substituted with F. Preferably, in formula (II): $R^1$ is $CH_2$-$CH_2$-OTs and $R^3$ is $CH_2$-$CH_2$ F or $CH_2$-C(F)=$CH_2$ or $CH_2$-C(H)=$CH_2$ ; or $R^1$ is $CH_3$ and $R^3$ is $CH_2$-$CH_2$OTs or $CH_2$-C(OTs)=$CH_2$ .

In another aspect, the invention provides a process for preparing compounds of formula (I) having at least one carbon atom substituted with a $^{18}$F. The process comprises reacting a compound of formula (II)

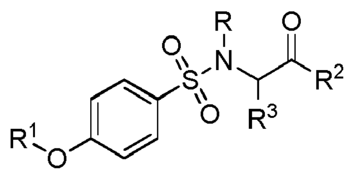

formula (II)

as defined above with a $^{18}$F containing reagent for substitution of a OH, OTs or another leaving group with $^{18}$F, preferably for substitution of OTs. Preferably, in formula (II):
$R^1$ is $CH_2$-$CH_2$-OTs and $R^3$ is $CH_2$-$CH_2$-F or $CH_2$-C(F)=$CH_2$; or $R^1$ is $CH_3$ and $R^3$ is $CH_2$-$CH_2$-OTs. Preferably, the $^{18}$F containing reagent is [$^{18}$F](Kryptofix222)KF.

In a further aspect, the present invention relates to compounds of formula (III) that are intermediate compounds in the synthesis of the compounds of the invention:

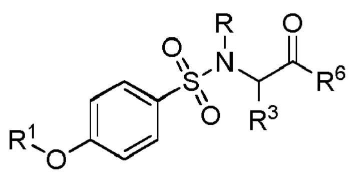

Formula (III)

wherein
R, $R^1$ and $R^3$ are as defined in formula (I) above; and
$R^6$ is OH.

Compounds of formula (I) wherein $R^2$ is $C_1$-$C_6$ alkoxy can also be used as intermediate compounds in the synthesis of compounds of formula (I). In particular, they may be used in the synthesis of compounds of formula (I) wherein $R^2$ is NH-O$R^4$ and $R^4$ is H, $C_1$-$C_6$ alkyl, $C_3$-$C_6$ cycloalkyl or $C_5$-$C_6$ heterocycloalkyl.

Compounds of formula (I) wherein $R^2$ is NH-O$R^4$ and $R^4$ is $C_1$-$C_6$ alkyl, $C_3$-$C_6$ cycloalkyl or $C_5$-$C_6$ heterocycloalkyl can also be used as intermediate compound in the synthesis of compounds of formula (I). In particular, they are used in the synthesis of compounds of formula (I) wherein $R^2$ is NH-O$R^4$ and $R^4$ is H.

Intermediates according to the invention are for example compounds having the following structures:

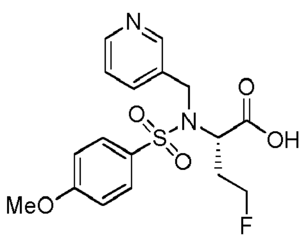
,
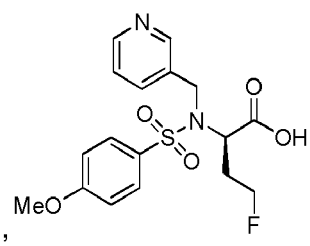
,
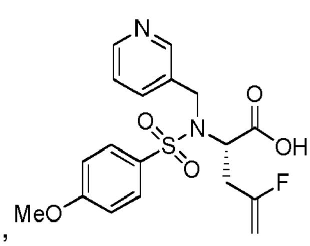
,

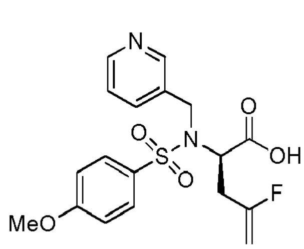, 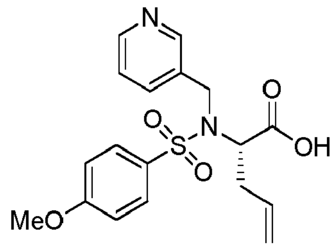, 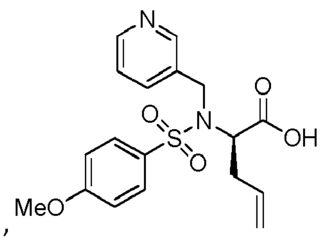,

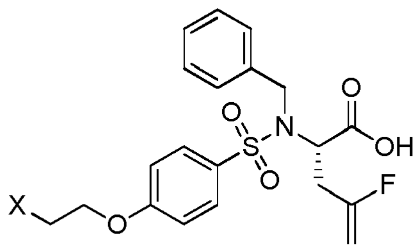, 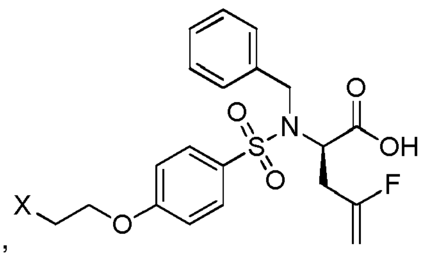

wherein X is selected from F, Cl, OH, OTs and ester thereof.

A further aspect of the present invention is a process for preparing a compound of formula (I)

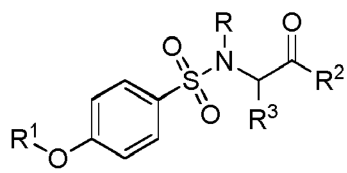

formula (I)

wherein

R, $R^1$ and $R^3$ are as defined in formula (I) above; and

$R^2$ is NH-O$R^4$; and

$R^4$ is H, $C_1$-$C_6$ alkyl, $C_3$-$C_6$ cycloalkyl or $C_5$-$C_6$ heterocycloalkyl comprising

a) reacting a compound of formula (III')

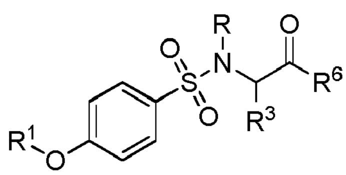

formula (III')

wherein

R, $R^1$ and $R^3$ are as defined in formula (I)

and $R^6$ is OH or $C_1$-$C_6$ alkoxy

with an hydroxylamine derivative of formula (IV)

$R^4$O-$NH_2$ formula (IV)

wherein $R^4$ is as defined above.

A compound obtained in step a) having $R^4$ selected from $C_1$-$C_6$ alkyl, $C_3$-$C_6$ cycloalkyl or $C_5$-$C_6$ heterocycloalkyl may be optionally hydrolyzed to a compound wherein $R^4$ is H.

The compound of formula (III') is prepared by

a') reacting a compound of formula (V)

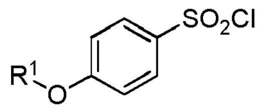

formula (V)

with a compound of formula (VI)

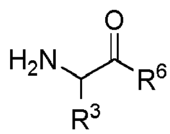

formula (VI)

to obtain a compound of formula (VII)

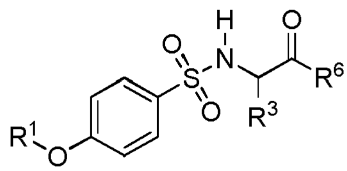

formula (VII)

and

a") reacting the compound of formula (VII) with a compound of formula (VIII)

R-Cl formula (VIII)

to obtain a compound of formula (III')

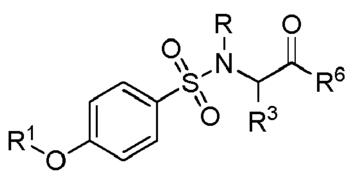

formula (III')

wherein

in any of the above formula (III'), (V), (VI), and (VII) and (VIII), R, $R^1$, $R^2$, $R^3$, and $R^6$ are as defined above.

Preferably, in step a') pyridine is used as a solvent. Preferably, the reaction of step a") is conducted in dimethylformamide (DMF) in the presence of potassium carbonate. Preferably, the reaction of step a) is conducted in the presence of dichloromethane, 1-Hydroxy-benzotriazol (HOBT), N-methyl-morpholin (NMM) and 1-(3-Dimethylaminopropyl)-3-ethylcarbodiimide (EDC) hydrochloride.

In a further aspect, the present invention provides a kit for the preparation of the pharmaceutical composition of the invention. Where the pharmaceutical composition of the invention comprises a diagnostic imaging agent labeled with a $^{18}F$, said kit comprises a precursor which is a compound of formula (I) comprising a group (e.g. tosyl group) suitable for reaction with a $^{18}F$ containing compound (e.g. [$^{18}F$](Kryptofix222)KF) such that reaction of said precursor with said $^{18}F$ containing compound gives said diagnostic imaging agent product of formula (I). Preferably said precursor is a compound of formula (II).

The kits may optionally further comprise additional components such as a radioprotectant, antimicrobial preservative, pH-adjusting agent or filler.

**Definitions**

The term "$C_1$-$C_6$ alkyl", when used either alone or within other terms such as "haloalkyl" and "arylalkyl" or "heteroarylalkyl" embraces linear or branched radicals having $C_1$-$C_6$ carbon atoms. Examples of such radicals include methyl, ethyl, *n*-propyl, isopropyl, *n*-butyl, isobutyl, *tert*-butyl, pentyl, isoamyl, hexyl. The term "alkylen" embraces bridging divalent alkyl radicals such as methylen and ethylen.

The term "$C_2$-$C_6$ alkenyl", when used alone or in combination, embraces linear or branched radicals having at least one carbon-carbon double bond in a moiety having between two and six carbon atoms. Examples of alkenyl radicals include, without limitation, vinyl, propenyl, allyl, , butenyl and 4-methylbutenyl. The term "alkenyl" encompasses radicals having "cis" and *"trans"* orientations, or alternatively, "*E*" and "*Z*" orientations, as appreciated by those of ordinary skill in the art.

The term "$C_1$-$C_6$ alkoxy" when used alone or in combination, embraces linear or branched oxygen-containing radicals each having alkyl portions of one to six carbon atoms. Examples of such radicals include methoxy, ethoxy, propoxy, butoxy and *tert*-butoxy. Alkoxy radicals may be further substituted with one or more halo atoms, such as fluoro, chloro or bromo, to provide "haloalkoxy" radicals. Examples of such radicals include fluoromethoxy, chloromethoxy, trifluoromethoxy, trifluoroethoxy, fluoroethoxy, fluoropropoxy and fluorobutoxy.

The term "halo", when used alone or in combination, means halogens such as fluorine, chlorine, bromine or iodine atoms, preferably fluorine. The term "haloalkyl", when used alone or in combination, embraces radicals wherein any one or more of the alkyl carbon atoms is substituted with halo as defined above. For example, this term includes monohaloalkyl, dihaloalkyl and polyhaloalkyl radicals such as a perhaloalkyl. A monohaloalkyl radical, for example, may have either an iodo, bromo, chloro or fluoro atom within the radical, preferably a fluoro atom. Dihalo and polyhaloalkyl radicals may have two or more of the same halo atoms or a combination of different halo radicals.

The term "$C_1$-$C_6$ haloalkyl" embraces radicals having 1-6 carbon atoms and, for example, haloalkyl radicals having one to three carbon atoms. Examples of haloalkyl radicals include fluoromethyl, difluoromethyl, trifluoromethyl, chloromethyl, dichloromethyl, trichloromethyl, pentafluoroethyl, heptafluoropropyl, chlorodifluoromethyl, dichlorofluoromethyl, fluoroethyl, difluoroethyl, trifluoroethyl, fluoropropyl, difluoropropyl, trifluoropropyl, chloroethyl, dichloroethyl and dichloropropyl.

The term "$C_2$-$C_6$ haloalkenyl", when used alone or in combination, embraces radicals wherein anyone or more of the alkyl carbon atoms is substituted with halo as defined above, preferably a fluorine atom. For example, this term includes monohaloalkenyl, dihaloalkenyl and polyhaloalkenyl radicals. A monohaloalkenyl radical, for example, may have either an iodo, bromo, chloro or fluoro atom within the radical. Dihalo and polyhaloalkenyl radicals may have two or more of the same halo atoms or a combination of different halo radicals. Examples of haloalkenyl radicals include fluorovinyl, fluoropropenyl and fluorobutenyl.

The term "aryl", when used alone or in combination, means a carbocyclic aromatic moiety containing one, two or even three rings wherein such rings may be attached together in a fused manner. Thus, the term "aryl" embraces aromatic radicals such as phenyl, naphthyl, indenyl, tetrahydronaphthyl, dihydrobenzofuranyl, anthracenyl, indanyl, benzodioxazinyl. The "aryl" group may be subsitituted, such as with 1 to 5 substituents including $C_1$-$C_6$ alkyl, hydroxyl, halo, halo-$C_1$-$C_6$-alkyl, nitro, cyano, $C_1$-$C_6$ alkoxy and $C_1$-$C_6$ alkylamino.

The term "heteroaryl", as used herein, either alone or in combination, means a fully unsaturated (aromatic) ring moiety formed from carbon atoms and having one or more heteroatoms selected from nitrogen, oxygen and sulfur. The ring moiety or ring system may contain one ("monocyclic"), two ("bicyclic") or even three ("tricyclic") rings wherein such rings are attached together in a fused manner. Every ring of a "heteroaryl" ring system need not be aromatic, and the ring(s) fused thereto (to the heteroaromatic ring) may be partially or fully saturated and optionally include one or more heteroatoms selected from nitrogen, oxygen and sulfur.

Examples of unsaturated heteroaryl radicals, include unsaturated 5- to 6-membered heteromonocyclyl groups containing 1 to 4 nitrogen atoms, including for example, pyrrolyl, imidazolyl, pyrazolyl, 2-pyridyl, 3-pyridyl, 4-pyridyl, pyrimidyl, pyrazinyl, pyridazinyl and tetrazole; unsaturated 7- to 10-membered heterobicyclyl groups containing 1 to 4 nitrogen atoms, including for example, quinolinyl, isoquinolinyl, quinazolinyl, isoquinazolinyl, aza-quinazolinyl; unsaturated 5- to 6-membered heteromonocyclic groups containing an oxygen atom, for example, pyranyl, 2-furyl, 3- furyl, benzofuryl, etc.; unsaturated 5 to 6-membered heteromonocyclic groups containing a sulfur atom, for example, 2-thienyl, 3-thienyl, benzothienyl, etc.; unsaturated 5- to 6-membered heteromonocyclic groups containing 1 to 2 oxygen atoms and 1 to 3 nitrogen atoms, for example, oxazolyl, isoxazolyl, oxadiazolyl [e.g., 1,2,4-oxadiazolyl, 1,3,4-oxadiazolyl, 1,2,5-oxadiazolyl]; unsaturated 5 to 6-membered heteromonocyclic group containing 1 to 2 sulfur atoms and 1 to 3 nitrogen atoms, for example, thiazolyl, isothiazolyl, thiadiazolyl [e.g. 1,2,4-thiadiazolyl, 1,3,4-thiadiazolyl, 1,2,5-thiadiazolyl].

The term "aryl-alkyl" is any $C_7$-$C_{20}$ group consisting of an alkyl and an aryl group as defined above, preferably a benzyl group. Preferably the alkyl moiety of the "arylalkyl" group is a - $CH_2$- or a -$CH_2$-$CH_2$- group. Preferably the aryl moiety of the "arylalkyl" group is a phenyl. The "arylalkyl" may be optionally substituted with one or more substituents independently selected from OH, OTs, amino, halo in particular F and Cl, $C_1$-$C_6$ alkyl optionally substituted with OH, OTs, amino, halo in particular F and Cl or the "arylalkyl" may be optionally substituted with $-O(CH_2-CH_2-O)_nCH_2-CH_2-R^5$ wherein n is 1, 2, 3, 4, 5, 6, preferably 3, and $R^5$ can be F, $^{18}F$, Cl, OH, OTs; or $-(CH_2-CH_2-O)_nCH_2-CH_2-R^5$ wherein n is 1, 2, 3, 4, 5, 6, preferably 3, and $R^5$ can be F, $^{18}F$, Cl, OH, OTs. Preferably the substituent(s) are on the "aryl" moiety.

The term "heteroaryl-alkyl" is any $C_7$-$C_{20}$ group consisting of an alkyl and a heteroaryl group as defined above, preferably a picolyl group, more preferably a 3-picolyl group. Preferably, the alkyl moiety of the "heteroaryl-alkyl" group is a -$CH_2$- or a -$CH_2$-$CH_2$- group. Preferably, the heteroaryl moiety of the "heteroaryl-alkyl" group is a pyridyl, more

preferably a 3-pyridyl. The "heteroaryl-alkyl" may be optionally substituted with one or more substituents independently selected from OH, OTs, amino, halo in particular F and Cl, $C_1$-$C_6$ alkyl optionally substituted with OH, OTs, amino, halo in particular F and Cl; the "heteroaryl-alkyl" may be optionally substitued with $-O(CH_2-CH_2-O)_nCH_2-CH_2-R^5$ wherein n is 1, 2, 3, 4, 5, 6, preferably 3, and $R^5$ can be F, $^{18}F$, Cl, OH, OTs; or $-(CH_2-CH_2-O)_nCH_2-CH_2-R^5$ wherein n is 1, 2, 3, 4, 5, 6, preferably 3, and $R^5$ can be F, $^{18}F$, Cl, OH, OTs. Preferably the substituent(s) are on the "aryl" moiety.

The term "$C_5$-$C_6$-heterocycloalkyl" relates to saturated or unsaturated mono ring comprising 5 or 6 carbon atoms, which carry heteroatoms such as N, O, S instead of one or more carbon atoms. Examples of such heterocyclic group are tetrahydrofuranyl, tetrahydropyranyl, pyrrolidinyl, pyrrolidino, piperidinyl, piperidino, piperazinyl, piperazino, morpholinyl, morpholino, thiomorpholinyl, thiomorpholino, tetrahydrothiofuranyl, and pyranyl

"Leavings group for nucleophile substitution" are known to the skilled person in the art. They include e.g., Cl, Br, I etc..

The phrase "labeled with a $^{18}F$" used herein means that a compounds of the invention comprises a $^{18}F$ either as an artificially enriched level of an atom intrinsic to the substructure, or as an additional essential feature that has been chemically attached via a functionality suitable for coupling said $^{18}F$.

By the term "biocompatible carrier" is meant a fluid, especially a liquid, in which the imaging agent can be suspended or dissolved, such that the composition is physiologically tolerable, i. e. it can be administered to the mammalian body without toxicity or undue discomfort. The biocompatible carrier is suitably an injectable carrier liquid such as sterile, pyrogen-free water for injection; an aqueous solution such as saline (which may advantageously be balanced so that the final product for injection is either isotonic or not hypotonic); an aqueous solution of one or more tonicity-adjusting substances (e. g. salts of plasma cations with biocompatible counter-ions), sugars (e. g. glucose or sucrose), sugar alcohols (e. g. sorbitol or mannitol), glycols (e. g. glycerol), or other non-ionic polyol materials (e. g. polyethyleneglycols, propylene glycols and the like).

By the term "biocompatible cation" is meant a positively charged counter-ion which forms a salt with an ionized, negatively charged group, where said positively charged counter-ion is also non-toxic and hence suitable for administration to the mammalian body, especially the human body. Examples of suitable biocompatible cations include the alkali metals sodium or potassium; the alkaline earth metals calcium and magnesium; and the ammonium ion. Preferred biocompatible cations are sodium and potassium, most preferably sodium.

By the term "radioprotectant" is meant a compound which inhibits degradation reactions, such as redox processes, by trapping highly-reactive free radicals, such as oxygen-containing free radicals arising from the radiolysis of water. The radioprotectants of the present invention are suitably chosen from: ascorbic acid, para-aminobenzoic acid (i. e. 4-aminobenzoic acid), gentisic acid (i. e. 2,5-dihydroxybenzoic acid) and salts thereof with a biocompatible cation as described above.

By the term "antimicrobial preservative" is meant an agent which inhibits the growth of potentially harmful microorganisms such as bacteria, yeasts or moulds. The antimicrobial preservative may also exhibit some bactericidal properties, depending on the dose. The main role of the antimicrobial preservative(s) of the present invention is to inhibit the growth of any such micro-organism in the pharmaceutical composition post-reconstitution, i. e. in the radioactive diagnostic product itself. The antimicrobial preservative may, however, also optionally be used to inhibit the growth of potentially harmful microorganisms in one or more components of the kit of the present invention prior to reconstitution. Suitable antimicrobial preservatives include: the parabens, i. e. methyl, ethyl, propyl or butyl paraben or mixtures thereof; benzyl alcohol; phenol; cresol; cetrimide and thiomersal. Preferred antimicrobial preservative(s) are the parabens.

The term "pH-adjusting agent" means a compound or mixture of compounds useful to ensure that the pH of the reconstituted kit is within acceptable limits (approximately pH 4.0 to 10.5) for human or mammalian administration. Suitable such pH-adjusting agents include pharmaceutically acceptable buffers, such as tricine, phosphate or TRIS [i. e. tris(hydroxymethyl)aminomethane], and pharmaceutical acceptable bases such as sodium carbonate, sodium bicarbonate or mixtures thereof. When the ligand conjugate is employed in acid salt form, the pH-adjusting agent may optionally be provided in a separate vial or container, so that the user of the kit can adjust the pH as part of a multi-step procedure.

By the term "filler" is meant a pharmaceutically acceptable bulking agent which may facilitate material handling during production and lyophilisation. Suitable fillers include inorganic salts such as sodium chloride, and water soluble sugars or sugar alcohols such as sucrose, maltose, mannitol or trehalose.

**Brief Description of the Figures**

Figure 1 illustrates the synthetic route that was used to prepare precursor compounds bearing a fluorine atom on the moiety corresponding to radical $R^3$ of formula (I) in the synthesis of the compounds of the invention. These syntheses were executed according to the following references:

D. M. Shendage, R. Fröhlich, K. Bergander, G. Haufe, Eur. J. Org. Chem. 2005, 719-727.
K.-W. Laue, S. Kröger, E. Wegelius, G. Haufe, Eur. J. Org. Chem. 2000, 3737-3743.
K.-W. Laue, C. Mück-Lichtenfeld, G. Haufe, Tetrahedron 1999, 55, 10413-10424.
K.-W. Laue, G. Haufe, Synthesis 1998, 1453-1456.
S. Kröger, G. Haufe, Amino Acids 1997, 12, 363-372.

Figure 2a illustrates the synthetic route that was used to prepare compounds 1 a, 1 b, 3a, 3b, 4a, 4b, 5a and 5b, respectively.

Figure 2b illustrates the synthetic route that was used to prepare compounds 6a, 6b, 8a, 8b, 9a, 9b, 10a and 10b, respectively.

Figure 2c illustrates the synthetic route that was used to prepare compounds 16a, 16b, 17a, 17b, 18a, 18b, 19a and 19b, respectively.

Figure 3 illustrates the synthetic route that was used to prepare compounds 20a, 20b, 21 a, 21 b, 22a, 22b, 23a and 23b, respectively.

Figures 4a-c illustrate the synthetic route that was used to prepare the compounds of formula (I) wherein $R^1$ is F-$CH_2$-$CH_2$, TsO-$CH_2$-$CH_2$, HO-$CH_2$-$CH_2$, Cl-$CH_2$-$CH_2$, respectively.

Figure 5 illustrates the synthetic route that was used to prepare compounds 30a (MAB 254), 30b (MAB 255), 31 a (MAB 258) and 31 b (MAB 259).

Figure 6 illustrates the synthetic route that was used to prepare e.g. compounds 33a (X = F), (HUG 78), and 33b (X = F), (HUG 74) or compound wherein X is OTs, OH, Cl.

The syntheses disclosed in Figures 1 to 6 are useful for the preparation of the enantiomers of the compound of the invention. They are also useful in the preparation of the racemate of the compounds of the invention. In this case, the starting compounds of the respective synthesis are non-chiral molecules or they are in the form of racemate.

Figure 7 illustrates the in vivo biodistribution of radioactivity in an adult C57/Bl6 mouse after intravenous injection of **[$^{18}$F]HUG 74.** Time-activity curves illustrate tracer dynamics in selected regions of interests (ROI). %ID: percentage injected dose.

## Examples

### Example 1.

**Example 1a.** *Tert*-butyl (*S*)-*N*-(4-methoxyphenylsulfonyl)aminobutanoate

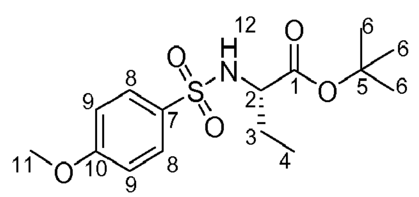

According to the general procedure tert-butyl (S)-aminobutanoate (630 mg, 3.96 mmol) in pyridin (15 mL) was treated with p-methoxyphenylsulfonyl chloride (818 mg, 3.96 mmol). The crude product was purified by column chromatography to get a white solid. **Yield**: 467 mg (36 %). **M.p**. 82 ˚C. **$^1$H NMR** (300 MHz, $CDCl_3$): δ 0.92 (t, $^3J_{H,H}$ = 7.4 Hz, 3 H, 4-$CH_3$), 1.27 (s, 9 H, 6-$CH_3$), 1.61 (m, 1 H, 3-$CH_A$), 1.77 (m, 1 H, 3-$CH_B$), 3.72 (ddd, $^3J_{H,H}$ = 9.1 Hz, $^3J_{H,H}$ = 6.9 Hz, $^3J_{H,H}$ = 5.2 Hz, 1 H, 2-CH), 3.85 (s, 3 H, 11-$CH_3$), 5.19 (d, $^3J_{H,H}$ = 9.1 Hz, 1 H, 12-NH), 6.95 (dm, $^3J_{H,H}$ = 9.0 Hz, 2 H, 8-CH), 7.78 (dm, $^3J_{H,H}$ = 9.0 Hz, 2 H, 9-CH). **$^{13}$C NMR** (75 MHz, $CDCl_3$): δ 9.2 (q, C-4), 26.8 (q, C-3), 27.7 (t, C-6), 55.6 (q, C-11), 57.1 (d, C-2), 82.2 (s, C-5), 114.1 (d, C-8), 129.4 (d, C-9), 131.4 (s, C-7), 162.9 (s, C-10), 170.8

(s, C-1). **Exact mass** ($ESI^+$): $C_{15}H_{23}NO_5S + Na^+$, calcd. 352.1195, found 352.1192, $(C_{15}H_{23}NO_5S)_2 + Na^+$, calcd. 681.2492, found 681.2483. **MS** (GC/MS, 70 eV): *m/z* (%) = 329 (1) [$M^+$], 228 (88) [$M^+ - C_5H_9O_2$], 171 (100) [$M^+ - C_7H_7O_3S$], 155 (5) [$C_6H_5NO_2S^+$], 123 (18), 107 (21) [$C_7H_7O^+$], 77 (13) [$C_6H_5^+$], 57 (15) [$C_4H_9^+$]. **Optical rotation:**

$$[\alpha]_{589}^{20} = +21.3, [\alpha]_{578}^{20} = +22.3, [\alpha]_{546}^{20} = +26.2, [\alpha]_{436}^{20} = +54.4, [\alpha]_{365}^{20} = +110.5$$ (c = 1.009, $CHCl_3$).

**Example 1b.** *Tert*-butyl (*R*)-*N*-(4-methoxyphenylsulfonyl)aminobutanoate

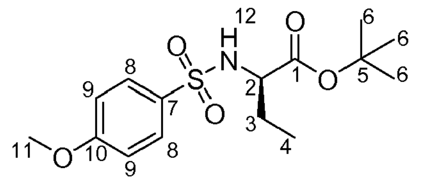

According to the general procedure *tert*-butyl (*R*)-aminobutanoic acid (551 mg, 3.46 mmol) was dissolved in pyridin (15 mL) and treated with *p*-methoxyphenylsulfonyl chloride (715 mg, 3.46 mmol). The crude product was purified by column chromatography to get a white solid. **Yield**: 446 mg (39 %). **M.p**. 81 °C. **$^1$H NMR** (400 MHz, $CDCl_3$): δ 0.92 (t, $^3J_{H,H}$ = 7.4 Hz, 3 H, 4-$CH_3$), 1.27 (s, 9 H, 6-$CH_3$), 1.64 (m, 1 H, 3-$CH_A$), 1.76 (ddd, $^2J_{H,H}$ = 14.7 Hz, $^3J_{H,H}$ = 7.4 Hz, $^3J_{H,H}$ = 5.2 Hz, 1 H, 3-$CH_B$), 3.72 (ddd, $^3J_{H,H}$ = 9.1 Hz, $^3J_{H,H}$ = 7.0 Hz, $^3J_{H,H}$ = 5.2 Hz, 1 H, 2-CH), 3.85 (s, 3 H, 11-$CH_3$), 5.18 (d, $^3J_{H,H}$ = 9.1 Hz, 1 H, 12-NH), 6.95 (dm, $^3J_{H,H}$ = 9.0 Hz, 2 H, 8-CH), 7.78 (dm, $^3J_{H,H}$ = 9.0 Hz, 2 H, 9-$CH_3$)- **$^{13}$C NMR** (100 MHz, $CDCl_3$): δ 9.2 (q, C-4), 26.8 (t, C-3), 27.7 (q, C-6), 55.6 (q, C-11), 57.1 (d, C-2), 82.3 (s, C-5), 114.1 (d, C-8), 129.4 (d, C-9), 131.4 (s, C-7), 162.9 (s, C-10), 170.8 (s, C-1). **Exact mass** ($ESI^+$): $C_{15}H_{23}NO_5S + Na^+$, calcd. 352.1195, found 352.1189; $(C_{15}H_{23}NO_5S)_2 + Na^+$, calcd. 681.2492, found 681.2483. **MS** (GC/MS, 70 eV): *m/z* (%), 29 (3) [$M^+$], 273 (1) [$M^+ - C_4H_8$], 228 (100) [$M^+ - C_5H_9O_2$], 171 (90) [$C_7H_7O_3S^+$] 155 (4) [$C_6H_5NO_2S^+$], 123 (22), 107 (29) [$C_7H_7O^+$], 92 (10) [$C_6H_4O^+$], 77 (15) [$C_6H_5^+$], 57 (15) [$C_4H_9^+$], 56 (48) [$C_4H_8^+$] **Optical rotation:**

$$[\alpha]_{589}^{20} = -20.6, [\alpha]_{578}^{20} = -22.0, [\alpha]_{546}^{20} = -26.8, [\alpha]_{436}^{20} = -54.1, [\alpha]_{365}^{20} = -109.9$$ (c = 1.008, $CHCl_3$).

$CHCl_3$).

**Example 2**

**Example 2a.** Methyl (*S*)-*N*-(4-methoxyphenylsulfonyl)aminobutanoate

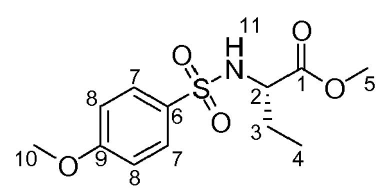

According to the general procedure 558 mg (3.63 mmol) methyl (*S*)-aminobutanoate hydrochloride was dissolved in pyridin (30 mL) and treated with *p*-methoxyphenylsulfonyl chloride (751 mg, 3.63 mmol). The crude product was purified by column chromatography to get a white solid. **Yield:** 450 mg (43 %). **M.p.** 64 °C. **$^1$H NMR** (300 MHz, $CDCl_3$): δ 0.91 (t, $^3J_{H,H}$ = 7.4 Hz, 3 H, 4-$CH_3$), 1.60 - 1.86 (m, 2 H, 3-$CH_2$), 3.52 (s, 3 H, 5-$CH_3$), 3.86 (s, 3 H, 10-$CH_3$), 3.85 (m 1 H, 2-CH), 5.30 (d, 3$J_{H,H}$ = 9.2 Hz, 1 H, 11-NH), 6.93 - 6.99 (m, 2 H, 7-CH), 7.75 - 7.81 (m, 2 H, 8-CH). **$^{13}$C NMR** (75 MHz, $CDCl_3$): δ 9.3 (q, C-4), 26.6 (t, C-3), 52.4 (q, C-5), 55.6 (q, C-10), 56.7 (d, C-2), 114.0 (d, C-7), 129.3 (d,

C-8), 131.2 (s, C-6), 162.9 (s, C-9), 172.1 (s, C-1). **Elemental analysis:** $C_{12}H_{17}NO_5S$ (M = 287.332 g/mol), calcd. C 50.16, H 5.96, N 4.87, found C 50.16, H 5.91, N 4.91 %. **Exact mass** (ESI$^+$): $C_{12}H_{17}NO_5S + H^+$, calcd. 288.0906, found 288.0891. $C_{12}H_{17}NO_5S + Na^+$, calcd. 310.0725, found 310.0718; $(C_{12}H_{17}NO_5S)_2 + Na^+$, 597.1553, found 597.1546. **MS** (GC/MS, 70 eV): *m/z* (%) 287 (5) [$M^+$], 228 (70) [$M^+ - CO_2CH_3$], 171 (100) [$C_7H_7O_3S^+$], 155 (5) [$C_7H_7O_2S^+$], 123 (23) [$C_7H_7O_2^+$], 107 (38) [$C_7H_7O^+$], 92 (9), 77 (12) [$C_6H_5^+$], 64 (6) [$SO_2^+$], 59 (3) [$CO_2CH_3^+$]. **Optical rotation**:

$$[\alpha]_{589}^{20} = +8.4, [\alpha]_{578}^{20} = +8.7, [\alpha]_{546}^{20} = +10.7, [\alpha]_{436}^{20} = +27.0, [\alpha]_{365}^{20} = +64.0 \ (c = 1.001, CHCl_3).$$

**Example 2b**. Methyl (*R*)-*N*-(4-methoxyphenylsulfonyl)aminobutanoate

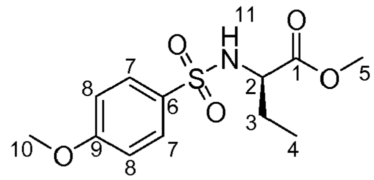

According to the general procedure methyl (*R*)-aminobutanoate hydrochloride (1000 mg, 6.51 mmol) was dissolved in pyridin (30 mL) and treated with p-methoxyphenylsulfonyl chloride (1350 mg, 6.51 mmol). The crude product was purified by column chromatography to get a white solid. **Yield**: 879 mg (47 %). **M.p**. 63-64 °C. **$^1$H NMR** (300 MHz, $CDCl_3$): δ 0.91 (t, $^3J_{H,H}$ = 7.4 Hz, 3 H, 4-$CH_3$), 1.60 - 1.84 (m, 2 H, 3-$CH_2$), 3.52 (s, 3 H, 5-$CH_3$), 3.86 (s, 3 H, 10-$CH_3$), 3.85 (m 1 H, 2-CH), 5.24 (d, $^3J_{H,H}$ = 9.2 Hz, 1 H, 11-NH), 6.92 - 6.99 (m, 2 H, 7-CH), 7.73 - 7.83 (m, 2 H, 8-CH). **$^{13}$C NMR** (75 MHz, $CDCl_3$): δ 9.3 (q, C-4), 26.6 (t, C-3), 52.4 (q, C-5), 55.6 (q, C-10), 56.7 (d, C-2), 114.1 (d, C-7), 129.3 (d, C-8), 131.2 (s, C-6), 162.9 (s, C-9), 172.1 (s, C-1). **Exact mass** (ESI$^+$): $C_{12}H_{17}NO_5S + H^+$, calcd. 288.0906, found 288.0913; $C_{12}H_{17}NO_5S + Na^+$, calcd. 310.0725, found 310.0733; $(C_{12}H_{17}NO_5S)_2 + Na^+$, calcd. 597.1553, found 597.1550. **MS** (ESI$^+$, daughter ion experiment): *m/z* (%) = 288 (9) [$M^+ + H^+$], 228 (100) [$M^+ - C_2H_3O_2$], 171 (32) [$C_7H_7O_3S^+$].

**Example 3**

**Example 3a.** *Tert*-butyl (*S*)-*N*-(4-methoxyphenylsulfonyl)aminopent-4-enoate

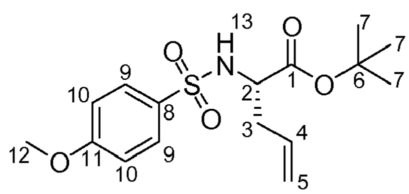

According to the general procedure *tert*-butyl (*S*)-aminopent-4-enoate (1200 mg, 7.02 mmol) was dissolved in pyridin (20 mL) and treated with *p*-methoxyphenylsulfonyl chloride (1454 mg, 7.02 mmol). The crude product was purified by column chromatography to get a white solid. **Yield:** 1150 mg (48 %). **M.p.** 58-59 °C. **$^1$H NMR** (300 MHz, $CDCl_3$): δ 1.29 (s, 9 H, 7-$CH_3$), 2.40 - 2.48 (m, 2 H, 3-$CH_2$), 3.85 (s, 3 H, 12-$CH_3$), 3.88 (dt, $^3J_{H,H}$ = 9.0 Hz, $^3J_{H,H}$ = 5.8 Hz, 1 H, 13-NH), 5.05 - 5.13 (m, 2 H, 5-$CH_2$), 5.20 (d, $^3J_{H,H}$ = 8.9 Hz, 1 H, 2-CH), 5.66 (ddt, $^3J_{H,H}$ = 15.9 Hz, $^3J_{H,H}$ = 11.2 Hz, $^3J_{H,H}$ = 7.1 Hz, 1 H, 4-CH), 6.95 (dm, $^3J_{H,H}$ = 9.0 Hz, 2 H, 9-CH), 7.78 (dm, $^3J_{H,H}$ = 9.0 Hz, 2 H, 10-CH). **$^{13}$C NMR** (75 MHz, $CDCl_3$): δ 27.8 (q, C-7), 37.9 (t, C-3), 55.5 (d, C-2), 55.6 (q, C-12), 82.6 (s, C-6), 114.2 (d, C-9), 119.3 (t, C-5), 129.4 (d, C-10), 131.6 (d, C-4 and C-8), 163.0 (s, C-11), 170.0 (s, C-1).

**Example 3b**. *tert*-Butyl (*R*)-*N*-(4-methoxyphenylsulfonyl)aminopent-4-enoate

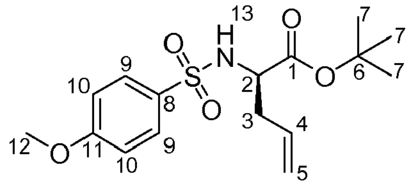

According to the general procedure *tert*-butyl (*R*)-aminopent-4-enoate (2140 mg, 12.48 mmol) was dissolved in pyridin (25 mL) and treated with *p*-methoxyphenylsulfonyl chloride (2570 mg, 12.48 mmol). The crude product was purified by column chromatography to get a white solid. **Yield**: 2375 mg (56 %). **M.p**. 59 °C. **$^1$H NMR** (300 MHz, $CDCl_3$): δ 1.27 (s, 9 H, 7-$CH_3$), 2.39 - 2.50 (m, 2 H, 3-$CH_2$), 3.85 (s, 3 H, 12-$CH_3$), 3.88 (dt, $^3J_{H,H}$ = 8.6 Hz, $^3J_{H,H}$ = 5.5 Hz, 1 H, 13-NH), 5.04 - 5.13 (m, 2 H, 5-$CH_2$), 5.20 (d, $^3J_{H,H}$ = 9.0 Hz, 1 H, 2-CH), 5.67 (m, 1 H, 4-CH), 6.95 (dm, $^3J_{H,H}$ = 9.0 Hz, 2 H, 9-CH), 7.78 (dm, $^3J_{H,H}$ = 9.0 Hz, 2 H, 10-CH). **$^{13}$C NMR** (75 MHz, $CDCl_3$): δ 27.7 (q, C-7), 37.9 (t, C-3), 55.4 (d, C-2), 55.6 (q, C-12), 82.5 (s, C-6), 114.1 (d, C-9), 119.4 (t, C-5), 129.4 (d, C-10), 131.4 (s, C-8), 131.5 (d, C-4), 162.9 (s, C-11), 169.9 (s, C-1). **Exact mass** (ESI$^+$): $C_{16}H_{23}NO_5S$ + $Na^+$, calcd. 364.1195, found 364.1187; $(C_{16}H_{23}NO_5S)_2$ + $Na^+$, calcd. 705.2492, found 705.2488. **MS** (GC/MS, 70 eV): m/z (%) 364 (0) [$M^+$], 300 (10) [$M^+$ - $C_3H_5$], 285 (4) [$M^+$ - $C_4H_8$], 240 (52) [$M^+$ - $C_5H_9O_2$], 171 (100) [$C_7H_7O_3S^+$], 155 (5) [$C_6H_5NO_2S^+$], 123 (36), 107 (50) [$C_7H_7O^+$], 92 (13) [$C_6H_4O^+$], 77 (24) [$C_6H_5^+$], 64 (8) [$SO_2^+$], 57 (42) [$C_4H_9^+$], 56 (48) [$C_4H_8^+$], 41 (40) [$C_3H_5^+$]. **Optical rotation:**

$$[\alpha]_{589}^{20} = -17.1, [\alpha]_{578}^{20} = -18.4, [\alpha]_{546}^{20} = -20.4, [\alpha]_{436}^{20} = -43.8, [\alpha]_{365}^{20} = -88.5 \text{ (c = 1.010, CHCl}_3\text{)}.$$

## Example 4

**Example 4a.** *Tert*-butyl (*S*)-*N*-(4-methoxyphenylsulfonyl)-γ-fluoro-a-aminobutanoate

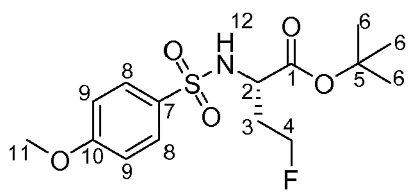

According to the general procedure *tert*-butyl (*S*)-2-amino-4-fluorobutanoate (1600 mg, 9.03 mmol) was dissolved in pyridin (15 mL) and treated with *p*-methoxyphenylsulfonyl chloride (1860 mg, 9.03 mmol). The crude product was purified by column chromatography to get a white solid. **Yield:** 2200 mg (70 %). **M.p.** 89-90 °C. $^1$H **NMR** (300 MHz, $CDCl_3$): δ 1.28 (s, 9 H, 6-$CH_3$), 1.87 - 2.25 (m, $^3J_{H,F}$ = 26.2 Hz, $^3J_{H,H}$ = 5.5 Hz, 2 H, 3-$CH_2$), 3.85 (s, 3 H, 11-$CH_3$), 3.89 (m, $^3J_{H,H}$ = 8.0 Hz, 1 H, 2-CH), 4.58 (dt, $^2J_{H,F}$ = 46.8 Hz, $^3J_{H,H}$ = 5.5 Hz, 2 H, 4-$CH_2F$), 5.34 (d, $^3J_{H,H}$ = 8.0 Hz, 1 H, 12-NH), 6.96 (dm, $^3J_{H,H}$ = 8.8 Hz, 2 H, 8-CH), 7.79 (dm, $^3J_{H,H}$ = 8.8 Hz, 2 H, 9-CH). **$^{13}$C NMR** (75 MHz, $CDCl_3$): δ 27.6 (q, C-6), 34.1 (dt, $^2J_{C,F}$ = 20.3 Hz, C-3), 52.7 (dd, $^3J_{C,F}$ = 3.7 Hz, C-2), 55.6 (q, C-11), 79.7 (dt, $^1J_{C,F}$ = 165.9 Hz, C-4), 82.9 (s, C-5), 114.2 (d, C-8), 129.5 (d, C-9), 130.9 (s, C-7), 163.1 (s, C-10), 170.2 (s, C-1). **$^{19}$F NMR** (282 MHz, $CDCl_3$): δ -221.6 (tt, $^2J_{H,F}$ = 46.8 Hz, $^3J_{H,F}$ = 26.0 Hz, 4-$CH_2F$). **Elemental analysis:** $C_{15}H_{22}FNO_5S$ (M = 347.402 g/mol), calcd. C 51.86, H 6.38, N 4.03, found C 51.92, H 6.24, N 3.96 %. **Exact mass** (ESI$^+$): $C_{15}H_{22}FNO_5S$ + $Na^+$, cacld. 370.1100, found 370.1094. $(C_{15}H_{22}FNO_5S)_2$ + $Na^+$, calcd. 717.2303, found 717.2298. **Optical rotation**:

$$[\alpha]_{589}^{20} = -23.5, [\alpha]_{578}^{20} = -25.1, [\alpha]_{546}^{20} = -29.3, [\alpha]_{436}^{20} = -58.4, [\alpha]_{365}^{20} = -114.1 \text{ (c = 1.021, CHCl}_3\text{)}.$$

**Example 4b**. *Tert*-butyl (*R*)-*N*-(4-methoxyphenylsulfonyl)-γ-fluoro-α-aminobutanoate

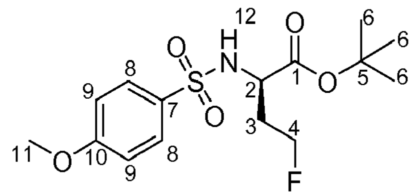

According to the general procedure *tert*-butyl (*R*)-2-amino-4-fluorobutanoate (1480 mg, 8.35 mmol) was dissolved in pyridin (15 mL) and treated with *p*-methoxyphenylsulfonyl chloride (1730 mg, 8.35 mmol). The crude product was purified by column chromatography to get a white solid. **Yield**: 1190 mg (41 %). **M.p**. 89 ˚C. **$^1$H NMR** (400 MHz, $CDCl_3$): δ 1.28 (s, 9 H, 6-$CH_3$), 1.90 - 2.23 (m, 2 H, 3-$CH_2$), 3.85 (s, 3 H, 11-$CH_3$), 3.89 (m, 1 H, 12-NH), 4.58 (dt, $^2J_{H,F}$ = 46.8 Hz, $^3J_{H,H}$ = 5.5 Hz, 2 H, 4-$CH_2$F), 5.38 (d, $^3J_{H,H}$ = 8.6, 1 H, 2-CH), 6.87 - 7.07 (m, 2 H, 8-CH), 7.75 - 7.82 (m, 2 H, 9-CH). **$^{13}$C NMR** (100 MHz, $CDCl_3$): δ 27.6 (q, C-6), 34.1 (dt , $^2J_{C,F}$ = 20.3 Hz, C-3), 52.7 (dt, $^3J_{C,F}$ = 3.7 Hz, C-2), 55.6 (q, C-11), 79.7 (dt, $^1J_{C,F}$ = 165.9 Hz, C-4), 82.9 (s, C-5), 114.2 (s, C-8), 129.4 (d, C-9), 130.9 (s, C-7), 163.0 (s, C-10), 170.2 (s, C-1). **$^{19}$F NMR** (282 MHz, $CDCl_3$): δ -221.6 (tt, $^2J_{H,F}$ = 46.8 Hz, $^3J_{H,F}$ = 26.0 Hz, 4-$CH_2$F). **Exact mass** (ESI$^+$): $C_{15}H_{22}FNO_5S + Na^+$, calcd. 370.1100, found 370.1094. **MS** (GC/MS, 70 eV): *m/z* (%) 347 (2) [M$^+$], 246 (82) [M$^+$ - $CO_2C(CH_3)_3$], 171 (100) [$C_7H_7O_3S^+$], 155 (5) [177 - $CH_3$], 123 (19) [$C_6H_4OS^+$], 107 (38) [$C_7H_7O^+$], 92 (18), 77 (27) [$C_6H_5^+$], 64 (10) [$SO_2^+$], 57 (52) [$C_4H_9^+$], 41 (35). **Optical rotation**:

$$[\alpha]_{589}^{20} = +24.7, [\alpha]_{578}^{20} = +26.1, [\alpha]_{546}^{20} = +30.4, [\alpha]_{436}^{20} = +61.2, [\alpha]_{365}^{20} = +118.4 \ (c = 1.015, CHCl_3).$$

## Example 5

**Example 5a**. *Tert*-butyl (*S*)-*N*-(4-methoxyphenylsulfonyl)-γ-fluoro-a-aminopent-4-enoate

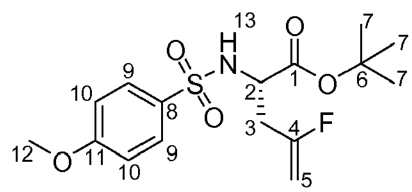

According to the general procedure *tert*-butyl (*S*)-2-amino-4-fluoropent-4-enoate (1360 mg, 7.19 mmol) was dissolved in pyridin (20 mL) and treated with *p*-methoxyphenylsulfonyl chloride (1490 mg, 7.19 mmol). The crude product was purified by column chromatography to get a greenish viscose liquid. **Yield**: 1.27 g (49 %). **$^1$H NMR** (300 MHz, $CDCl_3$): δ 1.30 (s, 9 H, 7-$CH_3$), 2.58 (dd, $^3J_{H,H}$ = 5.7 Hz, $^3J_{H,H}$ = 3.6 Hz, 1 H, 3-$CH_A$), 2.64 (d, $^3J_{H,H}$ = 5.9 Hz, 1 H, 3-$CH_B$), 3.85 (s, 3 H, 12-$CH_3$), 3.99 (t, $^3J_{H,H}$ = 5.6 Hz, 1 H, 2-CH), 4.34 (dd, $^3J_{H,F}$ = 49.3 Hz, $^2J_{H,H}$ = 3.0 Hz, 1 H, 5-$CH_{trans}$), 4.63 (dd, $^3J_{H,F}$ = 17.1 Hz, $^2J_{H,H}$ = 3.0 Hz, 1 H, 5-$CH_{cis}$), 5.41 (s, 1 H, 13-NH), 6.96 (dm, $^3J_{H,H}$ = 9.0 Hz, 2 H, 9-CH), 7.79 (dm, $^3J_{H,H}$ = 9.0 Hz, 2 H, 10-CH). **$^{13}$C NMR** (75 MHz, $CDCl_3$): δ 27.6 (q, C-7), 36.4 (dt, $^2J_{C,F}$ = 27.5 Hz, C-3), 53.3 (d, C-2), 55.5 (q, C-12), 83.0 (s, C-6), 93.9 (dt, $^2J_{C,F}$ = 18.7 Hz, C-5), 114.1 (d, C-9), 129.4 (d, C-10), 131.3 (s, C-8), 161.0 (ds, $^1J_{C,F}$ = 257.1 Hz, C-4), 163.0 (s, C-11), 169.2 (s, C-1). **$^{19}$F NMR** (282 MHz, $CDCl_3$): δ -96.0 (ddt, $^3J_{H,F}$ = 49.5 Hz, $^3J_{H,F}$ = 19.5 Hz, $^3J_{H,F}$ = 17.5 Hz, 1 H). **MS** (ESI$^+$, daughter ion experiment): *m/z* (%) 360 (0) [M$^+$ + H], 304 (8) [M$^+$ - $C_4H_8$], 284 (20) [304 - F], 244 (10) [304 - $C_3H_4F$], 214 (10) [M$^+$ - $C_5H_9O_2$ - $C_2H_2F$], 188 (15) [M$^+$ - $C_7H_7O_3S$], 171 (100) [$C_7H_7O_3S^+$], 107 (1) [$C_7H_7O^+$], 88 (60), 77 (2) [$C_6H_5^+$]. **Exact mass** (ESI$^+$): $C_{16}H_{22}FNO_5S + Na^+$, calcd. 382.1100,

found 382.1096. **Optical rotation:**

$$[\alpha]_{589}^{20} = +3.0, [\alpha]_{578}^{20} = +3.1, [\alpha]_{546}^{20} = +4.1, [\alpha]_{436}^{20} = +13.2, [\alpha]_{365}^{20} = +36.8 \text{ (c = 1.005, CHCl}_3\text{).}$$

**Example 5b**. *Tert*-butyl (*R*)-*N*-(4-methoxyphenylsulfonyl)-γ-fluoro-a-aminopent-4-enoate

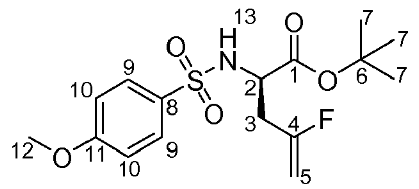

According to the general procedure tert-butyl (*R*)-2-amino-4-fluoro-pent-4-enoate (1362 mg, 7.20 mmol) was dissolved in pyridin (20 mL) and treated with *p*-methoxyphenylsulfonyl chloride (1490 mg, 7.20 mmol). The crude product was purified by column chromatography to get a greenish viscose liquid. **Yield:** 1.01 g (39 %). $^1$**H NMR** (400 MHz, $CDCl_3$): δ 1.30 (s, 9 H, 7-$CH_3$), 2.45 - 2.69 (m, $^3J_{H,F}$ = 20.5 Hz, 2 H, 3-$CH_2$), 3.85 (s, 3 H, 12-$CH_3$), 3.98 (dt, $^3J_{H,H}$ = 8.9 Hz, $^3J_{H,H}$ = 5.8 Hz, 1 H, 2-CH), 4.35 (dd, $^3J_{H,F}$ = 49.3, $^2J_{H,H}$ = 3.0 Hz, 1 H, 5-$CH_{trans}$), 4.64 (dd, $^3J_{H,F}$ = 17.1 Hz, $^2J_{H,H}$ = 3.0 Hz, 1 H, 5-$CH_{cis}$), 5.33 (d, $^3J_{H,H}$ = 8.9 Hz, 1 H, 13-NH), 6.96 (dm, $^3J_{H,H}$ = 9.0 Hz, 2 H, 9-CH), 7.79 (dm, $^3J_{H,H}$ = 9.0 Hz, 2 H, 10-CH). $^{13}$**C NMR** (100 MHz, $CDCl_3$): δ 27.6 (q, C-7), 36.5 (dt, $^2J_{C,F}$ = 27.5 Hz, C-3), 53.2 (d, C-2), 55.6 (q, C-12), 83.1 (s, C-6), 94.1 (dt, $^2J_{C,F}$ = 18.7 Hz, C-5), 114.2 (d, C-9), 129.4 (d, C-10), 131.2 (s, C-8), 161.0 (ds, $^1J_{C,F}$ = 257.1 Hz, C-4), 163.0 (s, C-11), 169.2 (s, C-1). $^{19}$**F NMR** (282 MHz, $CDCl_3$): δ -96.1 (ddt, $^3J_{H,F}$ = 49.3 Hz, $^3J_{H,F}$ = 20.1 Hz, $^3J_{H,F}$ = 17.2 Hz, 4-CF). **Elemental analysis:** $C_{16}H_{22}FNO_5S$ (M = 359.413 g/mol), calcd. C 53.47, H 6.17, N 3.90, found C 53.44, H 6.01, N 3.83 %. **MS** (GC/MS, 70 eV): *m/z* (%) 359 (0) [$M^+$], 303 (7) [$M^+$ - $C_4H_8$], 300 (5) [$M^+$ - $C_3H_4F$], 258 (52) [$M^+$ - $CO_2C(CH_3)_3$], 244 (21) [258 - $CH_2$], 187 (1) [$C_7H_7O_3SNH_2^+$], 173 (9) [$M^+$ - $C_7H_8O_3SNH$ / $C_9H_{14}FO_2^+$], 171 (100) [$C_7H_7O_3S^+$], 155 (8) [$C_6H_5NO_2S^+$] / $C_9H_{15}O_2^+$], 123 (21), 107 (28) [$C_7H_7O^+$], 92 (11), 78 (3) [$NO_2S^+$], 77 (19) [$C_6H_5^+$], 64 (7) [$SO_2^+$], 59 (4) [$C_3H_4F^+$], 57 (24) [$C_4H_9^+$], 41 (6). **Optical rotation:**

$$[\alpha]_{589}^{20} = -0.8, [\alpha]_{578}^{20} = -1.0, [\alpha]_{546}^{20} = -1.2, [\alpha]_{436}^{20} = -3.9, [\alpha]_{365}^{20} = -10.2 \text{ (c = 0.998, CHCl}_3\text{).}$$

**Example 6**

**Example 6a.** *Tert*-butyl (*S*)-*N*-(4-methoxyphenylsulfonyl)-*N*-(3-pyridylmethyl)-amino-butanoate

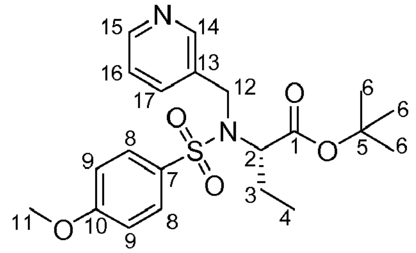

According to the general procedure *tert*-butyl (*S*)-*N*-(4-methoxyphenyl-sulfonyl)amino butanoate (450 mg, 1.37 mmol) was treated with potassium carbonate (1890 mg, 13.70 mmol) and picolyl chloride (225 mg, 1.37 mmol) in DMF (30 mL). After column chromatography (column Ø 3 cm × 16 cm) the product was isolated as a white solid. **Yield:** 505 mg (88 %). **M.p**. 170 °C. $^1$**H NMR** (400 MHz, $CDCl_3$): δ 0.80 (t, $^3J_{H,H}$ = 7.4 Hz, 3 H, 4-$CH_3$), 1.31 (s, 9 H, 6-$CH_3$), 1.49 (ddd, $^2J_{H,H}$ = 14.1 Hz, $^3J_{H,H}$ = 11.5 Hz, $^3J_{H,H}$ = 6.1 Hz, 1 H, 3-$CH_A$), 1.77 (m, 1H, 3-$CH_B$), 3.84 (s, 3 H , 11-$CH_3$), 4.31 (dd, $^3J_{H,H}$ = 8.4 Hz, $^3J_{H,H}$ = 6.7 Hz, 1 H, 2-CH), 4.50 (d, $^2J_{H,H}$ = 16.5 Hz, 1 H, 12-$CH_A$), 4.71 (d, $^2J_{H,H}$ = 16.5 Hz, 1 H, 12-$CH_B$), 6.94 (dm, $^3J_{H,H}$ = 9.0 Hz, 2 H, 8-CH), 7.24 (dd, $^3J_{H,H}$ = 7.8 Hz, $^3J_{H,H}$ = 4.8 Hz, 1 H, 16-CH), 7.72 (dm, $^3J_{H,H}$

= 8.9 Hz, 2 H, 9-CH), 7.88 (dd, $^3J_{H,H}$ = 7.9 Hz, $^4J_{H,H}$ = 1.7 Hz, 1 H, 17-CH), 8.49 (dd, $^3J_{H,H}$ = 4.8 Hz, $^4J_{H,H}$ = 1.5 Hz, 1 H, 15-CH), 8.56 (d, $^4J_{H,H}$ = 1.9 Hz, 1 H, 14-CH). **$^{13}$C NMR** (100 MHz, $CDCl_3$): δ 10.7 (q, C-4), 24.2 (t, C-3), 27.6 (q, C-6), 46.2 (t, C-12), 55.4 (q, C-11), 61.9 (d, C-2), 81.7 (s, C-5), 113.9 (d, C-8), 123.0 (d, C-16), 129.3 (d, C-9), 131.4 (s, C-7), 133.7 (s, C-13), 136.0 (d, C-17), 148.5 (d, C-15), 149.2 (d, C-14), 162.7 (s, C-10), 169.7 (s, C-1).

**Optical rotation**: $[\alpha]_{589}^{20} = -43.3, [\alpha]_{578}^{20} = -45.5, [\alpha]_{546}^{20} = -52.5, [\alpha]_{436}^{20} = -96.2, [\alpha]_{365}^{20} = -169.7$ (c = 1.013, $CHCl_3$):

**Example 6b**. Tert-butyl (R)-*N*-(4-methoxyphenylsulfonyl)-N-(3-pyridylmethyl)amino-butanoate

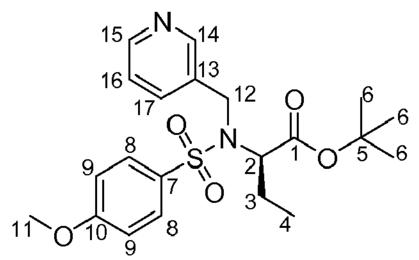

According to the general procedure *tert*-butyl (R)-N-(4-methoxyphenylsulfonyl)amino butanoate (338 mg, 1.03 mmol), potassium carbonate (1420 mg, 10.30 mmol) and picolyl chloride (169 mg, 1.03 mmol) in DMF (30 mL). After column chromatography (column Ø 3 cm × 16 cm) the product was isolated as a white solid. **Yield**: 394 mg (91 %). **M.p.** 171 °C. **$^1$H NMR** (400 MHz, $CDCl_3$): δ 0.81 (t, $^3J_{H,H}$ = 7.4 Hz, 3 H, 4-$CH_3$), 1.31 (s, 9 H, 6-$CH_3$), 1.50 (m, $^2J_{H,H}$ = 14.5 Hz, 1 H, 3-$CH_A$), 1.77 (m, $^2J_{H,H}$ = 14.5 Hz, $^3J_{H,H}$ = 7.3 Hz, 1 H, 3-$CH_B$), 3.86 (s, 3 H, 11-$CH_3$), 4.32 (dd, $^3J_{H,H}$ = 8.2 Hz, $^3J_{H,H}$ = 6.9 Hz, 1 H, 2-CH), 4.51 (d, $^3J_{H,H}$ = 16.6 Hz, 1 H, 12-$CH_A$), 4.71 (d, $^3J_{H,H}$ = 16.6 Hz, 1 H, 12-$CH_B$), 6.94 (dm, $^3J_{H,H}$ = 9.0 Hz, 2 H, 8-CH), 7.27 (dd, $^3J_{H,H}$ = 7.6 Hz, $^3J_{H,H}$ = 4.7 Hz, 1 H, 16-CH), 7.72 (dm, $^3J_{H,H}$ = 9.0 Hz, 2 H, 9-CH), 7.92 (dd, $^3J_{H,H}$ = 8.2 Hz, $^4J_{H,H}$ = 1.4 Hz, 1 H, 17-CH), 8.51 (d, $^3J_{H,H}$ = 4.7 Hz, 1 H, 15-CH), 8.55 (d, $^4J_{H,H}$ = 1.5 Hz, 1 H, 14-CH). **$^{13}$C NMR** (100 MHz, $CDCl_3$). δ 11.0 (q, C-4), 24.5 (t, C-3), 27.8 (q, C-6), 46.4 (t, C-12), 55.6 (q, C-11), 62.1 (d, C-2), 82.9 (s, C-5), 114.1 (d, C-8), 123.4 (d, C-16), 129.5 (d, C-9), 131.6 (s, C-7), 134.1 (s, C-13), 136.6 (d, C-17), 148.5 (d, C-15), 149.0 (d, C-14), 162.9 (s, C-10), 167.0 (s, C-1). **Exact mass** (ESI$^+$): $C_{21}H_{28}N_2O_5S + H^+$, calcd. 421.1797, found 421.1788; $C_{21}H_{28}N_2O_5S + Na^+$, calcd. 443.1617, found 443.1609; $(C_{21}H_{28}N_2O_5S)_2 + H^+$, calcd. 841.3516, found 841.3514; $(C_{21}H_{28}N_2O_5S)_2 + Na^+$, calcd. 863.3336, found 863.3341. Optical rotation:

**Optical rotation**: $[\alpha]_{589}^{20} = +44.8, [\alpha]_{578}^{20} = +47.0, [\alpha]_{546}^{20} = +54.0, [\alpha]_{436}^{20} = +98.7, [\alpha]_{365}^{20} = +173.6$ (c = 1.011, $CHCl_3$).

**Example 7**

**Example 7a**. Methyl (*S*)-*N*-(4-methoxyphenylsulfonyl)-N-(3-pyridylmethyl)amino-butanoate

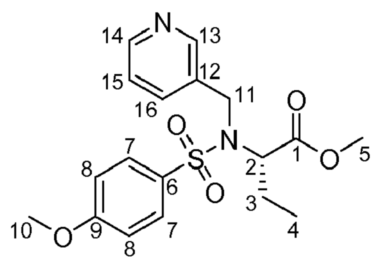

According to the general procedure methyl (S)-N-(4-methoxyphenylsulfonyl)-aminobutanoate (386 mg, 1.34 mmol) was treated with potassium carbonate (1850 mg, 12.40 mmol) and picolyl chloride (222 mg, 1.34 mmol) in DMF (15 mL). After column chromatography (column Ø 2 cm × 12 cm) the product was isolated as colorless highly viscose liquid. **Yield:** 384 mg (76 %). **$^1$H NMR** (300 MHz, $CDCl_3$). δ 0.80 (t, $^3J_{H,H}$ = 7.4 Hz, 3 H, 4-$CH_3$), 1.54 (ddq, $^2J_{H,H}$ = 14.2 Hz, $^3J_{H,H}$ = 7.3 Hz, $^3J_{H,H}$ = 7.0 Hz, 1 H, 3-$CH_A$), 1.81 (ddq, $^2J_{H,H}$ = 14.4 Hz, $^3J_{H,H}$ = 7.3 Hz, $^3J_{H,H}$ = 7.0 Hz, 1 H, 3-$CH_B$), 3.43 (s, 3 H, 5-$CH_3$), 3.86 (s, 3 H, 10-$CH_3$), 4.43 (dd, $^3J_{H,H}$ = 8.1 Hz, $^3J_{H,H}$ = 7.0 Hz, 1 H, 2-CH), 4.48 (d, $^2J_{H,H}$ = 16.5 Hz, 1 H, 11-$CH_A$), 4.61 (d, $^2J_{H,H}$ = 16.4 Hz, 1 H, 11-$CH_B$), 6.96 (d, $^3J_{H,H}$ = 9.0 Hz, 2 H, 7-CH), 7.25 (dd, $^3J_{H,H}$ = 7.8 Hz, $^3J_{H,H}$ = 4.9 Hz, 1 H, 15-CH), 7.72 (d, $^3J_{H,H}$ = 9.0 Hz, 2 H, 8-CH), 7.84 (d, $^3J_{H,H}$ = 7.9 Hz, 1 H, 16-CH), 8.50 (d, $^3J_{H,H}$ = 4.5 Hz, 1 H, 14-CH), 8.54 (s, 1 H, 13-CH). $^{13}$C NMR (75.48 MHz, $CDCl_3$): δ 10.8 (q, C-4), 23.9 (t, C-3), 46.4 (t, C-11), 51.9 (q, C-5), 55.6 (q, C-10), 61.2 (d, C-2), 114.0 (d, C-7), 123.3 (d, C-15), 129.5 (d, C-8), 131.2 (s, C-6), 133.5 (s, C-12), 136.4 (d, C-16), 148.7 (d, C-14), 149.1 (d, C-13), 163.0 (s, C-9), 171.2 (s, C-1). Exact mass (ESI$^+$): $C_{18}H_{22}N_2O_5S$ + $H^+$, calcd. 379.1328, found 379.1325; $C_{18}H_{22}N_2O_5S$ + $Na^+$, calcd. 401.1147, found 401.1142; $(C_{18}H_{22}N_2O_5S)_2$ + $H^+$, calcd. 757.2577, found 757.2583; $(C_{18}H_{22}N_2O_5S)_2$ + $Na^+$, calcd. 779.2397, found 779.2397. MS (GC-MS-EI): m/z (%) 378 (0) [$M^+$], 349 (3) [$M^+$ - $C_2H_5$], 319 (100) [$M^+$ - $CO_2CH_3$], 207 (22) [$M^+$ - $C_7H_7O_3S$], 171 (24) [$C_7H_7O_3S^+$], 147 (13) [$C_5H_9NO_2S^+$], 123 (16) [$C_7H_7O_2$]$^+$, 107 (32) [$C_7H_7O^+$], 92 (65) [$C_6H_6N^+$], 77 (14) [$C_6H_5^+$], 64 (5) [$SO_2^+$], 59 (3) [$CO_2CH_3^+$].

Optical rotation: $[\alpha]^{20}_{589} = -52.5, [\alpha]^{20}_{578} = -55.0, [\alpha]^{20}_{546} = -63.4, [\alpha]^{20}_{436} = -115.8, [\alpha]^{20}_{365} = -203.8$

(c = 1.010, $CHCl_3$).

Example 7b. Methyl (R)-N-(4-methoxyphenylsulfonyl)-N-(3-pyridylmethyl)amino-butanoate

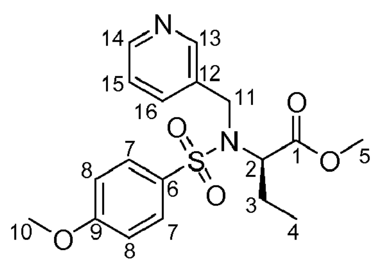

According to the general procedure methyl (R)-N-(4-methoxyphenylsulfonyl)-aminobutanoate (920 mg, 3.2 mmol), potassium carbonate (4770 mg, 32.0 mmol) and picolyl chloride (539 mg, 3.2 mmol) in DMF (15 mL). After column chromatography (column Ø 3 cm × 19 cm) the produkt was isolated as a colorless highly viscose liquid. Yield: 1160 mg (96 %). $^1$H NMR (400 MHz, $CDCl_3$): δ 0.80 (t, $^3J_{H,H}$ = 7.4 Hz, 3 H, 4-$CH_3$), 1.51 (m, $^2J_{H,H}$ = 21.5 Hz, $^3J_{H,H}$ = 7.3 Hz, 1 H, 3-$CH_A$), 1.81 (dddd, $^2J_{H,H}$ = 21.5 Hz, $^3J_{H,H}$ = 14.4 Hz, $^3J_{H,H}$ = 7.3 Hz, $^3J_{H,H}$ = 7.0 Hz, 1 H, 3-$CH_B$), 3.44 (s, 3 H, 5-$CH_3$), 3.86 (s, 3 H, 10-$CH_3$), 4.44 (dd, $^3J_{H,H}$ = 8.4 Hz, $^3J_{H,H}$ = 7.0 Hz, 1 H, 2-CH), 4.48 (d, $^2J_{H,H}$ = 16.6 Hz, 1 H, 11-$CH_A$), 4.61 (d, $^2J_{H,H}$ = 16.5 Hz, 1 H, 11-$CH_B$), 6.96 (dm, $^3J_{H,H}$ = 9.0 Hz, 2 H, 7-CH), 7.25 (ddd, $^3J_{H,H}$ = 7.9 Hz, $^3J_{H,H}$ = 4.8 Hz, $^4J_{H,H}$ = 0.5 Hz, 1 H, 15-CH),7.72 (dm, $^3J_{H,H}$ = 9.0 Hz, 2 H, 8-CH), 7.85 (dt, $^3J_{H,H}$ = 7.9 Hz, $^4J_{H,H}$ = 1.9 Hz, 1 H, 16-CH), 8.50 (dd, $^3J_{H,H}$ = 4.8 Hz, $^4J_{H,H}$ = 1.5 Hz, 1 H, 14-CH), 8.53 (d, $^4J_{H,H}$ = 1.9 Hz, 1 H, 13-CH). **$^{13}$C NMR** (100.62 MHz, $CDCl_3$): δ 10.6 (q, C-4), 23.7 (t, C-3), 46.3 (t, C-11), 51.7 (q, C-5), 55.4 (q, C-10), 61.1 (d, C-2), 113.8 (d, C-7),

123.1 (d, C-15), 129.3 (d, C-8), 131.0 (s, C-6), 133.2 (s, C-12), 136.1 (d, C-16), 148.6 (d, C-14), 149.1 (d, C-13), 162.8 (s, C-9), 170.9 (s, C-1). **Exact mass** ($ESI^+$): $C_{18}H_{22}N_2O_5S + H^+$, calcd. 379.1322, found 379.1314; $C_{18}H_{22}N_2O_5S + Na^+$, calcd. 401.1142, found 401.1135; $(C_{18}H_{22}N_2O_5S)_2 + H^+$, cacld. 757.2572, found 757.2559; $(C_{18}H_{22}N_2O_5S)_2 + Na^+$, calcd 779.2391, found 779.2383. **MS** ($ESI^+$, daughter ion experiment): *m/z* (%) 379 (85) [$M^+ + H^+$], 319 (100) [$M^+ - CO_2CH_3$], 264 (8), 200 (30) [$C_8H_{10}NO_3S^+$], 171 (22) [$C_7H_7O_3S^+$], 123 (3) [$C_7H_7O_2^+$].

Example 8

Example 8a. Tert-butyl (S)-N-(4-methoxyphenylsulfonyl)-N-(3-pyridylmethyl)-2-amino-pent-4-enoate

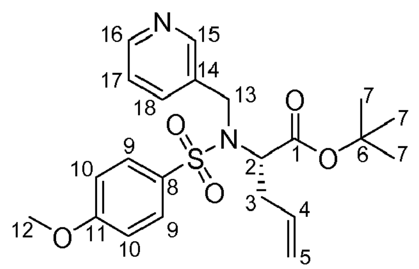

According to the general procedure tert-butyl (S)-N-(4-methoxyphenylsulfonyl)-aminopent-4-enoate (1084 mg, 3.17 mmol), potassium carbonate (4380 mg 31.70 mmol) and picolyl chloride (520 mg, 3.17 mmol) in DMF (25 mL). After column chromatography (column Ø 4 cm × 16 cm) the product was isolated as a white solid. Yield: 750 mg (55 %). M.p. 176-177 °C. **$^1$H NMR** (300 MHz, $CDCl_3$): δ 1.31 (s, 9 H, 7-$CH_3$), 2.27 (ddd, $^2J_{H,H}$ = 14.7 Hz, $^3J_{H,H}$ = 8.0 Hz, $^3J_{H,H}$ = 6.9 Hz, 1 H, 3-$CH_A$), 2.49 (dt, $^3J_{H,H}$ = 13.8 Hz, $^3J_{H,H}$ = 6.9 Hz, 1 H, 3-$CH_B$), 3.86 (s, 3 H, 12-$CH_3$), 4.49 (d, $^2J_{H,H}$ = 16.1 Hz, 1 H, 13-$CH_A$), 4.51 (t, $^3J_{H,H}$ = 7.5 Hz, 1 H, 2-CH), 4.69 (d, $^2J_{H,H}$ = 16.6 Hz, 1 H, 13-$CH_B$), 4.89 (dd, $^3J_{H,H}$ = 17.1 Hz, $^2J_{H,H}$ = 1.5 Hz, 1 H, 5-$CH_{trans}$), 4.99 (dd, $^3J_{H,H}$ = 10.2 Hz, $^2J_{H,H}$ = 1.5 Hz, 1 H, 5-$CH_{cis}$), 5.60 (ddt, $^3J_{H,H}$ = 17.0 Hz, $^3J_{H,H}$ = 10.3 Hz, $^3J_{H,H}$ = 6.7 Hz, 1 H, 4-CH), 6.94 (dm, $^3J_{H,H}$ = 9.0 Hz, 2 H, 9-CH), 7.25 (dd, $^3J_{H,H}$ = 7.9 Hz, $^3J_{H,H}$ = 4.9 Hz, 1 H, 17-CH), 7.73 (dm, $^3J_{H,H}$ = 9.0 Hz, 2 H, 10-CH), 7.86 (d, $^3J_{H,H}$ = 7.9 Hz, 1 H, 18-CH), 8.50 (d, $^3J_{H,H}$ = 4.0 Hz, 1 H, 16-CH), 8.54 (d, $^4J_{H,H}$ = 1.7 Hz, 1 H, 15-CH). $^{13}$C NMR (75 MHz, $CDCl_3$): δ 27.8 (q, C-7), 35.5 (t, C-3), 46.6 (t, C-13), 55.6 (q, C-12), 60.2 (d, C-2), 82.3 (s, C-6), 114.1 (d, C-9), 118.4 (t, C-5), 123.3 (d, C-17), 129.6 (d, C-10), 131.5 (s, C-8), 133.0 (d, C-4), 133.7 (s, C-14), 136.6 (d, C-18), 148.5 (d, C-16), 149.0 (d, C-15), 163.0 (s, C-11), 169.3 (s, C-1). Exact mass ($ESI^+$): $C_{22}H_{28}N_2O_5S + H^+$, calcd. 433.1797, found 433.1812; $C_{22}H_{28}N_2O_5S + Na^+$, calcd. 455.1617, found 455.1630, $(C_{22}H_{28}N_2O_5S)_2 + H^+$, calcd. 865.3516, found 865.3532; $(C_{22}H_{28}N_2O_5S)_2 + Na^+$, calcd. 887.3336, found 887.3345. Optical rotation:

$$[\alpha]_{589}^{20} = -26.4, [\alpha]_{578}^{20} = -27.3, [\alpha]_{546}^{20} = -31.2, [\alpha]_{436}^{20} = -56.6, [\alpha]_{365}^{20} = -97.7 \text{ (c = 1.001, CHCl}_3\text{):}$$

**Example 8b**. *Tert*-butyl (*R*)-*N*-(4-methoxyphenylsulfonyl)-N-(3-pyridylmethyl)-2-aminopent-4-enoate

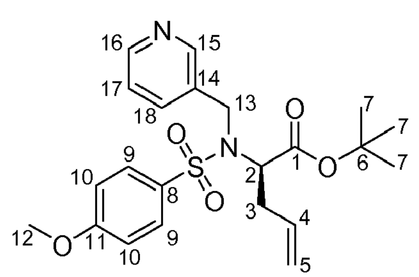

According to the general procedure *tert*-butyl (*R*)-*N*-(4-methoxyphenylsulfonyl)-aminopent-4-enoate (2375 mg, 6.96 mmol), was treated with potassium carbonate (9600 mg 69.60 mmol) and picolyl chloride (1140 mg 6.96 mmol) in DMF (45 mL). After column chromatography (column Ø 5 cm × 20 cm) the product was isolated as a white solid. **Yield:** 2010 mg (67 %). **M.p.** 177 ˚C. **$^1$H NMR** (300 MHz, $CDCl_3$): δ 1.31 (s, 9 H, 7-$CH_3$), 2.27 (m, 1 H, 3-$CH_A$), 2.49 (m, 1H, 3-$CH_B$), 3.86 (s, 3 H, 12-$CH_3$), 4.49 (d, $^2J_{H,H}$ = 16.4 Hz, 1 H, 13-$CH_A$), 4.52 (t, $^3J_{H,H}$ = 7.5 Hz, 1 H, 2-CH), 4.69 (d, $^2J_{H,H}$ = 16.6 Hz, 1 H, 13-$CH_B$), 4.89 (dq, $^3J_{H,H}$ = 17.1 Hz, $^2J_{H,H}$ = 1.4 Hz, 1 H, 5-$CH_{cis}$), 4.99 (dd, $^3J_{H,H}$ = 10.3 Hz, $^2J_{H,H}$ = 1.4 Hz, 1 H, 5-$CH_{trans}$), 5.60 (ddt, $^3J_{H,H}$ = 17.0 Hz, $^3J_{H,H}$ = 10.3 Hz, $^3J_{H,H}$ = 6.7 Hz, 1 H, 4-CH), 6.94 (dm, $^3J_{H,H}$ = 9.0 Hz, 2 H, 9-CH), 7.26 (ddd, $^3J_{H,H}$ = 4.8 Hz, $^3J_{H,H}$ = 3.1 Hz, $^4J_{H,H}$ = 0.6 Hz, 1 H, 17-CH), 7.73 (dm, $^3J_{H,H}$ = 9.0 Hz, 2 H, 10-CH), 7.87 (dt, $^3J_{H,H}$ = 7.9 Hz, $^4J_{H,H}$ = 1.8 Hz, 1 H, 18-CH), 8.50 (dd, $^3J_{H,H}$ = 4.8 Hz, $^4J_{H,H}$ = 1.5 Hz, 1 H, 16-CH), 8.54 (d, $^4J_{H,H}$ = 1.9 Hz, 1 H, 15-CH). $^{13}$C NMR (75 MHz, $CDCl_3$): δ 27.8 (q, C-7), 35.5 (t, C-3), 46.6 (t, C-13), 55.6 (q, C-12), 60.2 (d, C-2), 82.3 (s, C-6), 114.1 (d, C-9), 118.4 (t, C-5), 123.3 (d, C-17), 129.6 (d, C-10), 131.5 (s, C-8), 133.0 (d, C-4), 133.7 (s, C-14), 136.7 (d, C-18), 148.4 (d, C-16), 149.0 (d, C-15), 163.0 (s, C-11), 169.3 (s, C-1). Exact mass (ESI$^+$): $C_{22}H_{28}N_2O_5S$ + $H^+$, calcd. 433.1797, found 433.1782; $C_{22}H_{28}N_2O_5S$ + $Na^+$, calcd. 455.1617, found 455.1599; $(C_{22}H_{28}N_2O_5S)_2$ + $H^+$, calcd. 865.3516, found 865.3505; $(C_{22}H_{28}N_2O_5S)_2$ + $Na^+$, calcd. 887.3336, found 887.3318. MS (ESI$^+$, daughter ion experiment): m/z (%) 433 (0) [$M^+$ + $H^+$], 377 (12) [433 - $C_4H_8$], 279 (4) [$C_{13}H_{15}N_2O_3S^+$], 171 (15) [$C_7H_7O_3S^+$], 165 (100) [$C_8H_9N_2O_2^+$], 121 (24) [$C_7H_9N_2^+$], 92 (18) [$C_6H_6N^+$]. Optical rotation: (c=1.009, $CHCl_3$).

Example 9

Example 9a. Tert-butyl (S)-N-(4-methoxyphenylsulfonyl)-N-(3-pyridylmethyl)-2-amino-4-fluorobutanoate

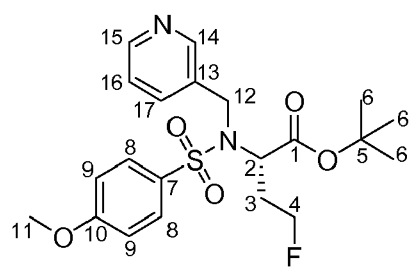

According to the general procedure tert-butyl (S)-N-(4-methoxyphenylsulfonyl)-2-amino-4-fluorobutanoate (1415 mg, 4.07 mmol), was reacted with potassium carbonate (5500 mg, 40.00 mmol) and picolyl chloride (673 mg 4.1 mmol) in DMF (20 mL). After column chromatographie (column Ø 4 cm × 16 cm) the product was isolated as a greenish-white, viscose liquid. *Yield*: 1512 mg (85 %). **$^1$H NMR** (300 MHz, $CDCl_3$): δ 1.35 (s, 9 H, 6-$CH_3$), 1.86 (m, 1 H, 3-$CH_A$), 2.22 (ddt, $^2J_{H,H}$ = 13.6 Hz, $^3J_{H,H}$ = 9.7 Hz, $^3J_{H,H}$ = 6.0 Hz, 1 H, 3-$CH_B$), 3.86 (s, 3 H, 11-$CH_3$), 4.19 - 4.45 (m, 2 H, 4-$CH_2$F), 4.36 (d, $^2J_{H,H}$ = 16.1 Hz, 1 H, 12-$CH_A$), 4.49 (dd, $^3J_{H,H}$ = 7.7 Hz, $^3J_{H,H}$ = 6.6 Hz, 1 H, 2-CH), 4.69 (d, $^2J_{H,H}$ = 16.2 Hz, 1 H, 12-$CH_B$), 6.96 (dm, $^3J_{H,H}$ = 8.9 Hz, 2 H, 8-CH), 7.25 (dd, $^3J_{H,H}$ = 7.9 Hz, $^3J_{H,H}$ = 4.8 Hz, 1 H, 16-CH), 7.77 (dm, $^3J_{H,H}$ = 8.9 Hz, 2 H, 9-CH), 7.82 (dm, $^3J_{H,H}$ = 8.4 Hz, 1 H, 17-CH), 8.51 (s, 1 H, 15-CH), 8.53 (s, 1 H, 14-CH). **$^{13}$C NMR** (75 MHz, $CDCl_3$): δ 27.7 (q, C-6), 31.8 (dt, $^2J_{C,F}$ = 20.5 Hz, C-3), 47.3 (t, C-12), 55.6 (q, C-11), 57.0 (dd, $^3J_{C,F}$ = 4.3 Hz, C-2), 80.1 (dt, $^1J_{C,F}$ = 166.3 Hz, C-4), 82.5 (s, C-5), 114.1 (d, C-8), 123.4 (d, C-16), 129.6 (d, C-9), 131.3 (s, C-7), 132.9 (s, C-13), 136.3 (d, C-17), 149.1 (d, C-15), 149.4 (d, C-14), 163.0 (s, C-10), 169.0 (s, C-1). $^{19}$F NMR (282 MHz, $CDCl_3$): δ -221.4 (ddt, $^2J_{H,F}$ = 46.9 Hz, $^3J_{H,F}$ = 28.3 Hz, $^3J_{H,F}$ = 22.8 Hz, 4-$CH_2$F). Exact mass (ESI$^+$): $C_{21}H_{27}N_2O_5S$ + $H^+$, calcd. 439.1703, found 439.1701; $C_{21}H_{27}N_2O_5S$ + $Na^+$, calcd. 461.1522, found 431.1526; $(C_{21}H_{27}N_2O_5S)_2$ + $H^+$, calcd. 877.3328, found 877.3320; $(C_{21}H_{27}N_2O_5S)_2$ + $Na^+$, calcd. 899.3147, found 899.3146. MS (ESI$^+$, daughter ion experiment): m/z (%) 439 (15) [$M^+$ + $H^+$], 383 (100) [439 - $C_4H_8$], 171 (5) [$C_7H_7O_3S^+$], 167 (10) [383 - $C_7H_7O_3S$ - $CO_2H$], 92 (3) [$C_6H_6N^+$]. Optical rotation:

$$[\alpha]_{589}^{20} = +34.1, [\alpha]_{578}^{20} = +35.5, [\alpha]_{546}^{20} = +41.1, [\alpha]_{436}^{20} = +74.5, [\alpha]_{365}^{20} = \text{n.d.}\ (c = 1.001, CHCl_3).$$

Example 9b. Tert-butyl (R)-N-(4-methoxyphenylsulfonyl)-N-(3-pyridylmethyl)-2-amino-4-fluorobutanoate

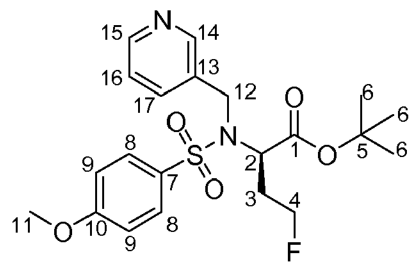

According to the general procedure tert-butyl (S)-N-(4-methoxyphenyl-sulfonyl)-2-amino-4-fluorobutanoate (500 mg, 1.44 mmol) was reacted with potassium carbonate (1990 mg 14.40 mmol) and picolyl chloride (237 mg, 1.44 mmol) in DMF (20 mL). After column chromatography (column Ø 2 cm × 15 cm) the product was isolated as greenish-white, viscose liquid. Yield: 587 mg (93 %). $^1$H NMR (400 MHz, $CDCl_3$): δ 1.35 (s, 9 H, 6-$CH_3$), 1.86 (m, $^3J_{H,F}$ = 28.3 Hz, $^2J_{H,H}$ = 14.2 Hz, 1 H, 3-$CH_B$), 2.22 (dddt, $^3J_{H,F}$ = 22.7 Hz, $^2J_{H,H}$ = 14.2 Hz, $^3J_{H,H}$ = 7.5 Hz, $^3J_{H,H}$ = 6.6 Hz, 1 H, 3-$CH_A$), 3.87 (s, 3 H, 11-$CH_3$), 4.37 (d, $^2J_{H,H}$ = 16.2 Hz, 1 H, 12-$CH_A$), 4.22 - 4.46 (m, $^2J_{H,F}$ = 47.1 Hz, 2 H, 4-$CH_2$F), 4.48 (dd, $^3J_{H,H}$ = 7.9 Hz, $^3J_{H,H}$ = 6.7 Hz, 1 H, 2-CH), 4.69 (d, $^2J_{H,H}$ = 16.2 Hz, 1 H, 12-$CH_B$), 6.96 (dm, $^3J_{H,H}$ = 9.0 Hz, 2 H, 8-CH), 7.25 (dd, $^3J_{H,H}$ = 7.9 Hz, $^3J_{H,H}$ = 4.8 Hz, 1 H, 16-CH), 7.77 (dm, $^3J_{H,H}$ = 8.9 Hz, 2 H, 9-CH), 7.82 (dt, $^3J_{H,H}$ = 7.9 Hz, $^4J_{H,H}$ = 1.8 Hz, 1 H, 17-CH), 8.50 (d, $^4J_{H,H}$ = 2.0 Hz, 1 H, 14-CH), 8.52 (dd, $^3J_{H,H}$ = 4.8 Hz, $^4J_{H,H}$ = 1.6 Hz, 1 H, 15-CH). **$^{13}$C NMR** (100 MHz, $CDCl_3$): δ 27.8 (q, C-6), 31.9 (dt, $^2J_{C,F}$ = 20.5 Hz, C-3), 47.3 (t, C-12), 55.6 (q, C-11), 57.1 (dd, $^3J_{C,F}$ = 4.3 Hz, C-2), 80.2 (dt, $^1J_{C,F}$ = 166.4 Hz, C-4), 82.6 (s, C-5), 114.2 (d, C-8), 123.4 (d, C-16), 129.7 (d, C-9), 131.4 (s, C-7), 132.9 (s, C-13), 136.3 (d, C-17), 149.2 (d, C-15), 149.5 (d, C-14), 163.1 (s, C-10), 169.1 (s, C-1). **$^{19}$F NMR** (282 MHz, $CDCl_3$): δ 221.4 (tdd , $^2J_{H,F}$ = 46.9 Hz, $^3J_{H,F}$ = 28.3 Hz, $^3J_{H,F}$ = 22.8 Hz, 4-$CH_2$F). **Elemental analysis:** $C_{21}H_{27}FN_2O_5S$ (M = 438.513 g/mol); calcd. C: 57.52, H 6.21, N 6.39, found C 57.82, H 6.00, N 6.38 %. Exact mass (ESI$^+$): $C_{21}H_{27}FN_2O_5S + H^+$, calcd. 439.1703, found 439.1697; $C_{21}H_{27}FN_2O_5S + Na^+$, calcd. 461.1522, found 461.1513; $(C_{21}H_{27}FN_2O_5S)_2 + H^+$, calcd. 877.3328, found 877.3321; $(C_{21}H_{27}FN_2O_5S)_2 + Na^+$, calcd. 899.3147, found 899.3145. **MS** (GC/MS, 70 eV): m/z (%) 438 (0) [M$^+$], 362 (1), 277 (3) [M$^+$ - $C_8H_{14}FO_2$], 191 (100), 171 (22) [$C_7H_7O_3S^+$], 147 (75), 123 (12) [$C_6H_4OS^+$], 107 (22) [$C_7H_7O^+$], 92 (32) [$C_6H_6N^+$], 77 (10) [$C_6H_5^+$]. **Optical rotation**:

$$[\alpha]_{589}^{20} = -33.2, [\alpha]_{578}^{20} = -34.5, [\alpha]_{546}^{20} = -39.9, [\alpha]_{436}^{20} = -72.6, [\alpha]_{365}^{20} = -124.9 \text{ (c = 1.001, CHCl}_3\text{).}$$

**Example 10**

**Example 10a**. *Tert*-butyl (*S*)-*N*-(4-methoxyphenylsulfonyl)-*N*-(3-pyridylmethyl)-2-amino-4-fluoropent-4-enoate

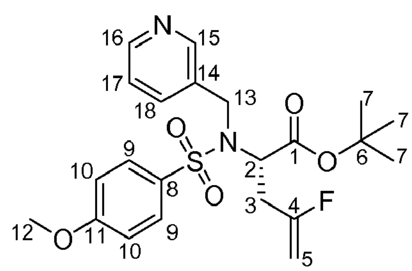

According to the general procedure *t*ert-butyl (*S*)-*N*-(4-methoxyphenyl-sulfonyl)-2-amino-4-fluoropent-4-enoate (1500 mg, 4.17 mmol) was reacred with potassium carbonate (5760 mg 41.7 mmol) and picolyl chloride (685 mg, 4.17 mmol) in DMF (20 mL). After column chromatography (column Ø 4 cm × 16 cm) the product was isolated as yellow,

viscose liquid. Yield: 1428 mg (76 %). $^1$H NMR (300 MHz, $CDCl_3$): δ 1.35 (s, 9 H, 7-$CH_3$), 2.48 (m, $^3J_{H,F}$ = 19.2 Hz, 1 H, 3-$CH_A$), 2.73 (td, $^3J_{H,F}$ = 15.0 Hz, $^3J_{H,H}$ = 6.8 Hz, 1 H, 3-$CH_B$), 3.86 (s, 3 H, 12-$CH_3$), 4.14 (dd, $^3J_{H,F}$ = 49.7 Hz, $^2J_{H,H}$ = 3.1 Hz, 1 H, *5-$CH_{trans}$),* 4.39 (d, $^2J_{H,H}$ = 16.3 Hz, 1 H, 13-$CH_A$), 4.55 (dd, $^3J_{H,F}$ = 17.2 Hz, $^2J_{H,H}$ = 3.0 Hz, 1 H, 5-$CH_{cis}$), 4.58 (t, $^3J_{H,H}$ = 7.3 Hz, 1 H, 2-CH), 4.65 (d, $^2J_{H,H}$ = 16.3 Hz, 1 H, 13-$CH_B$), 6.95 (d, $^3J_{H,H}$ = 9.0 Hz, 2 H, 9-CH), 7.23 (ddd, $^3J_{H,H}$ = 7.9 Hz, $^3J_{H,H}$ = 4.8 Hz, $^4J_{H,H}$ = 0.7 Hz, 1 H, 17-CH), 7.78 (m, 1 H, 18-CH), 7.78 (d, $^3J_{H,H}$ = 9.0 Hz, 2 H, 10-CH), 8.51 (dd, $^3J_{H,H}$ = 4.6 Hz, $^4J_{H,H}$ = 1.7 Hz, 2 H, 15-CH and 16-CH). $^{13}$C NMR (75 MHz, $CDCl_3$): δ 27.8 (s, C-7), 34.0 (d, $^2J_{C,F}$ = 28.1 Hz, C-3), 47.4 (s, C-13), 55.6 (s, C-12), 57.6 (s, C-2), 82.8 (s, C-6), 93.4 (d, $^2J_{C,F}$ = 19.1 Hz, C-5), 114.2 (s, C-9), 123.3 (s, C-17), 129.8 (s, C-10), 131.4 (s, C-8), 132.8 (s, C-14), 136.4 (s, C-18), 149.1 (s, C-16), 149.5 (s, C-15), 161.5 (d, $^1J_{C,F}$ = 256.5 Hz, C-4), 163.1 (s, C-11), 168.4 (s, C-1). $^{19}$F NMR (282 MHz, $CDCl_3$): δ -97.6 (dddd, $^3J_{H,F}$ = 50.0 Hz, $^3J_{H,F}$ = 19.2 Hz, $^3J_{H,F}$ = 17.2 Hz, $^3J_{H,F}$ = 15.0 Hz, 4-CF). Exact mass (ESI$^+$): $C_{22}H_{27}FN_2O_5S + H^+$, cacld. 451.1703, found 451.1701; $C_{22}H_{27}FN_2O_5S + Na^+$, calcd. 473.1522, found 473.1521; $C_{22}H_{27}FN_2O_5S)_2 + H^+$, calcd. 901.3328, found 901.3320; $(C_{22}H_{27}FN_2O_5S)_2 + Na^+$, calcd. 923.3147, found 923.3144. Optical rotation:

**Example 10b**. *Tert*-butyl (*R*)-*N*-(4-methoxyphenylsulfonyl)-*N*-(3-pyridylmethyl)-2-amino-4-fluoropent-4-enoate

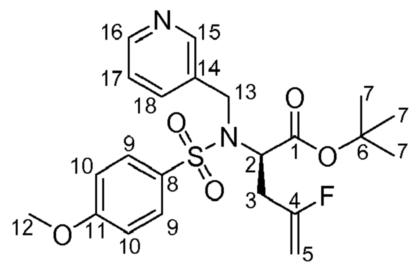

According to the general procedure *tert*-butyl (*R*)-*N*-(4-methoxyphenyl-sulfonyl)-2-amino-4-fluoropent-4-enoate(720 mg, 2.00 mmol) was reacted with potassium carbonate (2760 mg, 20.00 mmol) and picolyl chloride (329 mg, 2.00 mmol) in DMF (20 mL). After column chromatography the product was isolated as yellow, viscose oil. **Yield:** 523 g (58 %). **$^1$H NMR** (500 MHz, $CDCl_3$): δ 1.37 (s, 9 H, 7-$CH_3$), 2.48 (ddd, $^3J_{H,F}$ = 19.2 Hz, $^2J_{H,H}$ = 15.1 Hz, $^3J_{H,H}$ = 7.8 Hz, 1 H, 3-$CH_A$), 2.74 (dt, $^2J_{H,H}$ = 15.2 Hz, $^3J_{H,H}$ = 6.9 Hz, 1 H, 3-$CH_B$), 3.88 (s, 3 H, 12-$CH_3$), 4.17 (dd, $^3J_{H,F}$ = 49.7 Hz, $^2J_{H,H}$ = 3.1 Hz, 1 H, 5-$CH_{trans}$*),* 4.42 (d, $^2J_{H,H}$ = 16.3 Hz, 1 H, 13-$CH_A$), 4.57 (dd, $^3J_{H,F}$ = 17.2 Hz, $^2J_{H,H}$ = 3.1 Hz, 1 H, 5-$CH_{cis}$), 4.60 (t, $^3J_{H,H}$ = 7.3 Hz, 1 H, 2-CH), 4.67 (d, $^2J_{H,H}$ = 16.3 Hz, 1 H, 13-$CH_B$), 6.97 (dm, $^3J_{H,H}$ = 9.0 Hz, 2 H, 9-CH), 7.29 (dd, $^3J_{H,H}$ = 7.9 Hz, $^3J_{H,H}$ = 4.9 Hz, 1 H, 17-CH), 7.79 (dm, $^3J_{H,H}$ = 9.0 Hz, 2 H, 10-CH), 7.86 (dd, $^3J_{H,H}$ = 7.8 Hz, $^4J_{H,H}$ = 1.4 Hz, 1 H, 18-CH), 8.52 - 8.54 (m, $^4J_{H,H}$ = 1.5 Hz, 2 H, 15-CH and 16-CH). **$^{13}$C NMR** (100.62 MHz, $CDCl_3$): δ 27.8 (q, C-7), 34.0 (dt, $^2J_{C,F}$ = 28.1 Hz, C-3), 47.3 (t, C-13), 55.6 q, C-12), 57.6 (d, C-2), 82.9 (s, C-6), 93.4 (dt, $^2J_{C,F}$ = 19.0 Hz, C-5), 114.2 (d, C-9), 123.4 (d, C-17), 129.8 (d, C-10), 131.4 (s, C-8), 132.9 (s, C-14), 136.6 (d, C-18), 148.9 (d, C-16), 149.3 (d, C-15), 161.5 (ds, $^1J_{C,F}$ = 256.5 Hz, C-4), 163.1 (s, C-11), 168.5 (s, C-1). **$^{19}$F NMR** (470 MHz, $CDCl_3$): δ -97.6 (dddd, $^3J_{H,F}$ = 49.7 Hz, $^3J_{H,F}$ = 19.2 Hz, $^3J_{H,F}$ = 17.2 Hz, $^3J_{H,F}$ = 15.0 Hz, 4-CF).

**Optical rotation**: $[\alpha]_{589}^{20} = +7.7$, $[\alpha]_{578}^{20} = +8.1$, $[\alpha]_{546}^{20} = +9.3$, $[\alpha]_{436}^{20} = +16.3$, $[\alpha]_{365}^{20} = +28.4$ (c = 1.004, $CHCl_3$).

General procedure for hydrolysis of amino acid tert-butylesters

In a dried YOUNG-tube trifluoroacetic acid (20 mL/mmol) is added under argon to the corresponding amino acid tert-butylester dissolved in dry dichloromethane (20 mL/mmol). After flushing with argon the YOUNG-tube is sealed and the mixture is stirred at r.t. for 3-4 h. Then the reaction mixture was evaporated to dryness. The residue is dissolved in chloroform (100 mL/mmol) and washed with an aqueous solution of citric acid and bicarbonate (pH ≈ 4) (25 mL/mmol). The aqueous phase is extracted with chloroform (4 × 30 mL/mmol) and the combined organic layer is dried with magnesium sulfate. The solvent is evaporated to get the crude product.

Scheme 1

3

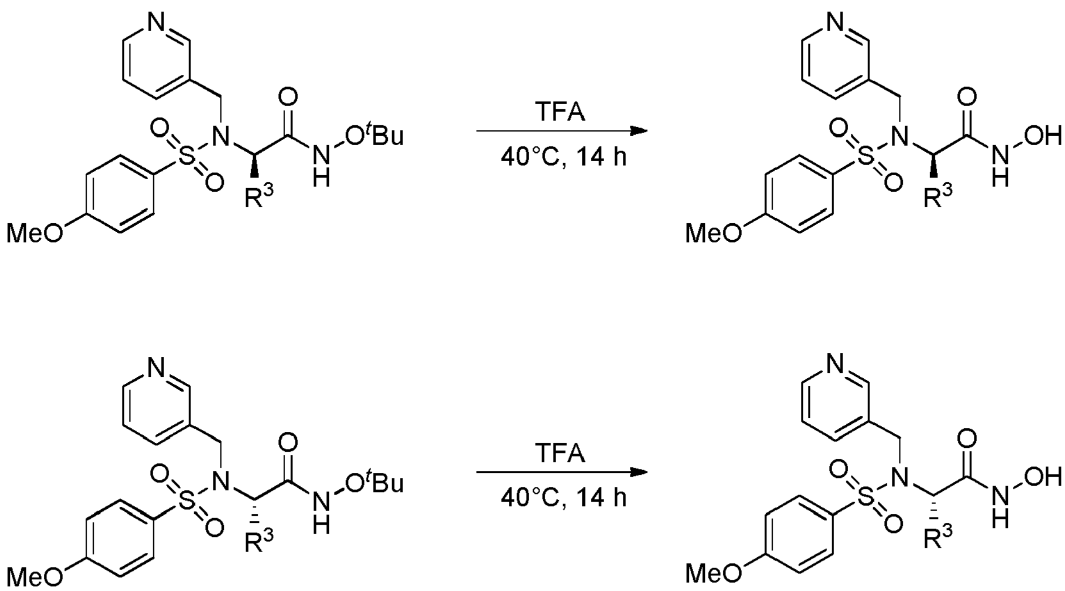


wherein $R^3$ is $C_1$-$C_6$ -fluoro-alkyl or optionally substituted $C_2$-$C_6$ alkenyl wherein the substituent is a F.

**Example 11**

**Example 11a**. (*S*)-*N*-(4-Methoxyphenylsulfonyl)-*N*-(3-pyridylmethyl)-aminobutanoic acid

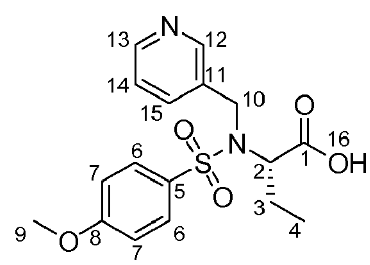

According to the general procedure *tert*-butyl (*S*)-*N*-(4-methoxyphenylsulfonyl)-N-(3-pyridylmethyl)-aminobutanoate (243 mg, 0.58 mmol) was dissolved in dry dichloromethane (12 mL) and treated with trifluoroacetic acid (12 mL). The product was isolated as colorless, highly viscos oil. **Yield**: 194 mg (91 %). **$^1$H NMR** (300 MHz, DMSO-$d_6$): δ 0.70 (t, $^3J_{H,H}$ = 7.3 $H_z$, 3 H, 4-$CH_3$), 1.45 (ddd, $^2J_{H,H}$ = 14.2 Hz, $^3J_{H,H}$ = 8.7 Hz, $^3J_{H,H}$ = 7.3 Hz, 1 H, 3-$CH_A$), 1.75 (dt, $^2J_{H,H}$ = 14.0 Hz, $^3J_{H,H}$ = 6.8 Hz, 1 H, 3-$CH_B$), 3.83 (s, 3 H, 9-$CH_3$), 4.23 (dd, $^3J_{H,H}$ = 8.9 Hz, $^3J_{H,H}$ = 6.0 Hz, 1 H, 2-CH), 4.43 (d, $^2J_{H,H}$ = 16.9 Hz, 1 H, 10-$CH_A$), 4.61 (d, $^2J_{H,H}$ = 16.9 Hz, 1 H, 10-$CH_B$), 7.07 (dm, $^3J_{H,H}$ = 9.0 Hz, 2 H, 6-CH), 7.38 (dd, $^3J_{H,H}$ = 7.8 Hz, $^3J_{H,H}$ = 4.9 Hz, 1 H, 14-CH), 7.74 (dm, $^3J_{H,H}$ = 9.0 Hz, 2 H, 7-CH), 7.84 (d, $^3J_{H,H}$ = 7.9 Hz, 1 H, 15-CH), 8.47 (d, $^3J_{H,H}$ = $_{4\cdot 1}$ Hz, 1 H, 13-CH), 8.57 (s, 1 H, 12-CH). **$^{13}$C NMR** (75 MHz, DMSO-$d_6$): δ 11.0 (q, C-4), 23.2 (t, C-3), 46.4 (t, C-10), 55.7 (q, C-9), 61.5 (d, C-2), 114.3 (d, C-6), 123.4 (d, C-14), 129.5 (d, C-7), 131.0 (s, C-5), 134.3 (s, C-11), 136.3 (d, C-15), 148.0 (d, C-13), 148.9 (d, C-12), 162.6 (s, C-8), 171.7 (s, C-1). **Exact mass** ($ESI^+$): $C_{17}H_{20}N_2O_5S$ + $H^+$, calcd. 365.1171, found 365.1169; $C_{17}H_{20}N_2O_5S$ + $Na^+$, calcd. 387.0991, found 387.0988; $(C_{17}H_{20}N_2O_5S)_2$ + $H^+$, calcd. 729.2264, found 729.2267; $(C_{17}H_{20}N_2O_5S)_2$ + $Na^+$, calcd. 751.2084, found 751.2085. **MS** ($ESI^+$, daughter ion experiment): *m/z* (%) 365 (90) [$M^+$ + $H^+$], 319 (100) [365 - $H^+$ - $CO_2H$], 280 (65) [$C_{13}H_{16}N_2O_3S^+$], 228 (15) [319 - $C_6H_6N$], 200 (30) [228 - $C_2H_5$], 171 (55) [$C_7H_7O_3S^+$].

**Example 11b**. (*R*)-*N*-(4-Methoxyphenylsulfonyl)-N-(3-pyridylmethyl)-aminobutanoic acid

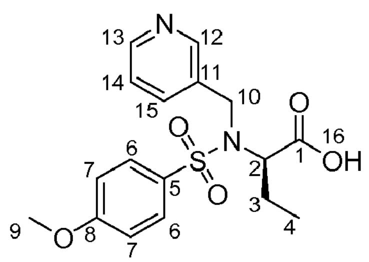

According to the general procedure *tert*-butyl (*R*)-*N*-(4-methoxyphenylsulfonyl)-*N*-(3-pyridylmethyl)-aminobutanoate (133 mg, 0.32 mmol) dissolved in dry dichloromethane (8 mL) and treated with trifluoroacetic (8 mL) acid. The product was isolated as colorless, highly viscos oil. **Yield**: 112 mg (96 %). $^1$**H NMR** (300 MHz, DMSO-$d_6$): δ 0.71 (t, $^3J_{H,H}$ = 7.3 Hz, 3 H, 4-$CH_3$), 1.59 - 1.87 (m, 2 H, 3-$CH_2$), 3.84 (s, 3 H, 9-$CH_3$), 4.25 (dd, $^3J_{H,H}$ = 8.8 Hz, $^3J_{H,H}$ = 6.0 Hz, 1 H, 2-CH), 4.52 (d, $^2J_{H,H}$ = 17.1 Hz, 1 H, 10-$CH_A$), 4.66 (d, $^2J_{H,H}$ = 17.1 Hz, 1 H, 10-$CH_B$), 7.08 (dm, $^3J_{H,H}$ = 8.9 Hz, 2 H, 6-CH), 7.65 (m, 1 H, 14-CH), 7.76 (dm, $^3J_{H,H}$ = 8.9 Hz, 2 H, 7-CH), 8.10 (d, $^3J_{H,H}$ = 7.8 Hz, 1 H, 15-CH), 8.45 - 8.95 (m, 2 H, 12-CH & 13-CH), 12.44 (s br, 1 H, 16-OH). $^{13}$**C-NMR** (75 MHz, DMSO-$d_6$): δ 11.0 (q, C-4), 23.2 (t, C-3), 46.2 (t, C-10), 55.7 (q, C-9), 61.5 (d, C-2), 114.3 (d, C-6), 124.6 (d, C-14), 129.5 (d, C-7), 130.4 (s, C-11), 130.8 (s, C-5), 139.0 (d, C-15), 145.5 (d, C-13), 146.2 (d, C-12), 162.7 (s, C-8), 171.7 (s, C-1). **Exact mass** (ESI$^+$): $C_{17}H_{20}N_2O_5S + H^+$, calcd. 365.1171, 365.1166; $C_{17}H_{20}N_2O_5S + Na^+$, calcd. 387.0991, found 387.0986; $(C_{17}H_{20}N_2O_5S)_2 + H^+$, calcd. 729.2264, found 729.2267; $(C_{17}H_{20}N_2O_5S)_2 + Na^+$, calcd. 751.2084, found 751.2085. **MS** (ESI$^+$, daughter ion experiment): *m*/*z* (%) 365 (100) [$M^+ + H^+$], 319 (32) [365 - $H^+$ - $CO_2H$], 280 (20) [$C_{13}H_{16}N_2O_3S^+$], 228 (5) [319 - $C_6H_6N$], 200 (10) [228 - $C_2H_5$], 171 (18) [$C_7HO_3S^+$], 149 (38) [319 - $C_7H_7O_3S$].

**Example 12**

**Example 12a**. (*S*)-*N*-(4-Methoxyphenylsulfonyl)-*N*-(3-pyridylmethyl)-aminopent-4-enoic acid

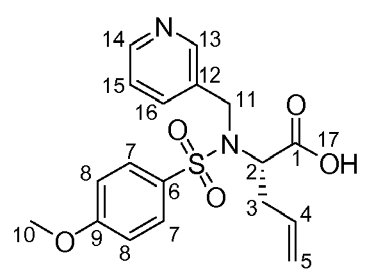

According to the general procedure *tert*-butyl (*S*)-*N*-(4-methoxyphenylsulfonyl)-*N*-(3-pyridylmethyl)-2-aminopent-4-enoate (400 mg, 0.93 mmol) was dissolved in dry dichloromethane (20 mL) and treated with trifluoroacetic acid (20 mL). The product was isolated as colorless, highly viscos oil. **Yield**: 312 mg (89 %). $^1$**H NMR** (400 MHz, DMSO-$d_6$): δ 2.16 (ddd, $^2J_{H,H}$ = 15.0 Hz, $^3J_{H,H}$ = 8.4 Hz, $^3J_{H,H}$ = 7.4 Hz, 1 H, 3-$CH_A$), 2.42 (m, 1 H, 3-$CH_B$), 3.76 (s, 3 H, 10-$CH_3$), 4.35 (dd, $^3J_{H,H}$ = 8.7 Hz, $^2J_{H,H}$ = 6.2 Hz, 1 H, 2-CH), 4.36 (d, $^2J_{H,H}$ = 17.3 Hz, 1 H, 11-$CH_A$), 4.51 (d, $^2J_{H,H}$ = 16.9 Hz, 1 H, 11-$CH_B$), 4.80 (dd, $^3J_{H,H}$ = 17.2 Hz, $^2J_{H,H}$ = 1.6 Hz, 1 H, 5-$CH_{trans}$), 4.84 (dd, $^3J_{H,H}$ = 10.3 Hz, $^2J_{H,H}$ = 1.5 Hz, 1 H, 5-$CH_{cis}$), 5.49 (ddt, $^3J_{H,H}$ = 17.0 Hz, $^3J_{H,H}$ = 10.3 Hz, $^3J_{H,H}$ = 6.7 Hz, 1 H, 4-CH), 6.99 (dm, $^3J_{H,H}$ = 9.0 Hz, 2 H, 7-CH), 7.25 (dd, $^3J_{H,H}$ = 7.8 Hz, $^3J_{H,H}$ = 4.8 Hz, 1 H, 15-CH), 7.66 (dm, $^3J_{H,H}$ = 8.9 Hz, 2 H, 8-CH), 7.70 (m, 1 H, 16-CH), 8.36 (d, $^3J_{H,H}$ = 4.1 Hz, 1 H, 14-CH), 8.46 (s, 1 H, 13-CH). $^{13}$**C NMR** (100 MHz, DMSO-$d_6$): δ 34.1 (t, C-3), 46.5 (t, C-11), 55.7 (q, C-10), 59.7 (d, C-2), 114.3 (d, C-7), 117.8 (t, C-5), 123.1 (d, C-15), 129.5 (d, C-8), 131.0 (s, C-6), 133.8 (d, C-4), 134.0 (s, C-12), 135.9 (d, C-16), 148.1 (d, C-14), 149.2 (d, C-13), 162.6 (s, C-9), 171.2 (s, C-1). **Exact mass** (ESI$^+$): $C_{18}H_{20}N_2O_5S + H^+$, calcd. 377.1171, found 377.1186; $C_{18}H_{20}N_2O_5S + Na^+$, calcd. 399.0991, found 399.1004; $(C_{18}H_{20}N_2O_5S)_2 + H^+$, calcd. 753.2264, found 753.2259; $(C_{18}H_{20}N_2O_5S)_2 + Na^+$, calcd. 775.2084, found 775.2094. **Exact mass** (ESI$^-$): $C_{18}H_{20}N_2O_5S-H^+$, calcd. 375.1015, found 375.1035; $(C_{18}H_{20}N_2O_5S)_2 - H^+$, calcd. 751.2108, found

751.2141. **MS** ($ESI^+$, daughter ion experiment): *m/z* (%) 377 (75) [$M^+ + H^+$], 331 (75) [377 - $H^+$ - $CO_2H$], 280 (42) [$C_{13}H_{16}N_2O_3S^+$], 240 (8) [331 - $C_6H_6N$], 200 (20) [$C_8H_{10}NO_3S^+$], 171 (50) [$C_7H_7O_3S^+$], 161 (100) [331 - $C_7H_7O_3S$], 123 (8) [$C_7H_7O_2{}^1$].

**Example 12b**.(*R*)-*N*-(4-Methoxyphenylsulfonyl)-*N*-(3-pyridylmethyl)-aminopent-4-enoic acid

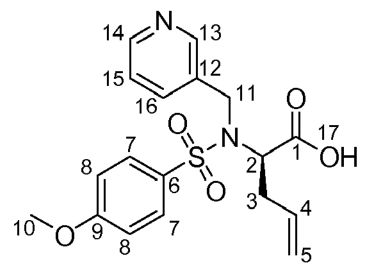

According to the general procedure *tert*-butyl (*R*)-*N*-(4-methoxyphenylsulfonyl)-*N*-(3-pyridylmethyl)-2-aminopent-4-enoate (247 mg, 0.57 mmol) was dissolved in dry dichloromethane (12 mL) and treated with trifluoroacetic acid (12 mL). The product was isolated as white highly viscos oil. **Yield**: 196 mg (92 %). **$^1$H NMR** (400 MHz, DMSO-$d_6$): δ 2.29 (ddd, $^2J_{H,H}$ = 15.0 Hz, $^3J_{H,H}$ = 8.6 Hz, $^3J_{H,H}$ = 7.6 Hz, 1 H, 3-$CH_A$), 2.54 (m, $^2J_{H,H}$ = 15.0 Hz, $^3J_{H,H}$ = 6.1 Hz, 1 H, 3-$CH_B$), 3.84 (s, 3 H, 10-$CH_3$), 4.48 (dd, $^3J_{H,H}$ = 8.9 Hz, $^3J_{H,H}$ = 6.0 Hz, 1 H, 2-CH), 4.53 (d, $^2J_{H,H}$ = 17.3 Hz, 1 H, 11-$CH_A$), 4.65 (d, $^2J_{H,H}$ = 17.2 Hz, 1 H, 11-$CH_B$), 4.89 (dd, $^3J_{H,H}$ = 17.2 Hz, $^2J_{H,H}$ = 1.7 Hz, 1 H, 5-$CH_{trans}$), 4.93 (dd, $^3J_{H,H}$ = 10.5 Hz, $^2J_{H,H}$ = 1.6 Hz, 1 H, 5-$CH_{cis}$), 5.58 (ddt, $^3J_{H,H}$ = 17.1 Hz, $^3J_{H,H}$ = 10.3 Hz, $^3J_{H,H}$ = 6.7 Hz, 1 H, 4-CH), 7.09 (d, $^3J_{H,H}$ = 9.0 Hz, 2 H, 7-CH), 7.58 (dd, $^3J_{H,H}$ = 7.9 Hz, $^3J_{H,H}$ = 5.1 Hz, 1 H, 15-CH), 7.75 (d, $^3J_{H,H}$ = 9.0 Hz, 2 H, 8-CH), 8.07 (d, $^3J_{H,H}$ = 8.0 Hz, 1 H, 16-CH), 8.59 (d, $^3J_{H,H}$ = 4.4 Hz, 1 H, 14-CH), 8.67 (s, 1 H, 13-CH), 12.82 (s br, 1 H, 17-OH). **$^{13}$C NMR** (100 MHz, DMSO-$d_6$): δ 34.0 (t, C-3), 46.3 (t, C-11), 55.7 (q, C-10), 59.7 (d, C-2), 114.3 (d, C-7), 117.8 (t, C-5), 124.3 (d, C-15), 129.6 (d, C-8), 130.7 (s, C-6), 134.0 (d, C-4), 135.7 (s, C-12), 139.1 (d, C-16), 145.6 (d, C-14), 146.4 (d, C-13), 162.7 (s, C-9), 171.2 (s, C-1).

**Example 13**

**Example 13a.** (*S*)-*N*-(4-Methoxyphenylsulfonyl)-*N*-(3-pyridylmethyl)-2-amino-4-fluorobutanoic acid

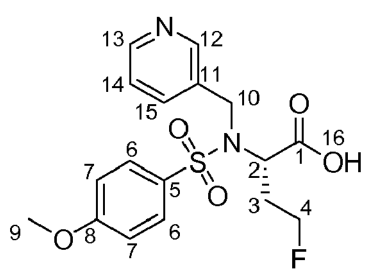

According to the general procedure *tert*-butyl (*S*)-*N*-(4-methoxyphenylsulfonyl)-*N*-(3-pyridylmethyl)-2-amino-4-fluorobutanoate (562 mg, 1.28 mmol) was dissolved in dry dichloromethane (5 mL) and treated with trifluoroacetic acid (25 mL). The product was isolated as white solid. **Yield**: 472 mg (96 %). **M.p**. 156 ˚C. $^1$**H NMR** (300 MHz, DMSO-$d_6$): δ 1.85 (m, $^3J_{H,F}$ = 22.3 Hz, 1 H, 3-$CH_A$), 2.19 (m, $^3J_{H,F}$ = 27.9 Hz, 1 H, 3-$CH_B$), 3.84 (s, 3 H, 9-$CH_3$), 4.32 (dt, $^2J_{H,F}$ = 47.1 Hz, $^3J_{H,H}$ = 5.7 Hz, 2 H, 4-$CH_2$), 4.43 (d, $^2J_{H,H}$ = 17.2 Hz, 1 H, 10-$CH_A$), 4.49 (m, 1 H, 2-CH), 4.62 (d, $^2J_{H,H}$ = 16.8 Hz, 1 H, 10-$CH_B$), 7.09 (dm, $^3J_{H,H}$ = 8.9 Hz, 2 H, 6-CH), 7.39 (dd, $^3J_{H,H}$ = 7.8 Hz, $^3J_{H,H}$ = 4.8 Hz, 1 H, 14-CH), 7.75 (dm, $^3J_{H,H}$ = 8.9 Hz, 2 H, 7-CH), 7.82 (m, 1 H, 15-CH), 8.49 (d, $^3J_{H,H}$ = 3.7 Hz, 1 H, 13-CH), 8.55 (s, 1 H, 12-CH), 13.02 (s br, 1 H, 16-OH). **$^{13}$C NMR** (75 MHz, DMSO-$d_6$): δ 30.9 (dt, $^2J_{C,F}$ = 20.2 Hz, C-3), 46.8 (t, C-10), 55.7 (q, C-9), 56.3 (dd, $^3J_{C,F}$ = 5.0 Hz, C-2), 80.4 (dt, $^1J_{C,F}$ = 163.1 Hz, C-4), 114.4 (d, C-6), 123.5 (d, C-14), 129.6 (d, C-7), 130.8 (s, C-5), 133.9 (s, C-11), 136.4 (d, C-15), 148.0 (d, C-13), 148.8 (d, C-12), 162.7 (s, C-8), 171.2 (s, C-1). **$^{19}$F NMR** (282 MHz, DMSO-$d_6$): δ - 219.3 (ddt, $^2J_{H,F}$ = 47.1 Hz, $^3J_{H,F}$ = 27.8 Hz, $^3J_{H,F}$ = 22.3 Hz, 4-$CH_2F$). **Exact mass** ($ESI^+$): $C_{17}H_{19}FN_2O_5S$

$+ H^+$, calcd. 383.1077, found 383.1073; $C_{17}H_{19}FN_2O_5S + Na^+$, found 405.0896, found 405.0892; $(C_{17}H_{19}FN_2O_5S)_2 + H^+$, calcd. 765.2076, found 765.2071; $(C_{17}H_{19}FN_2O_5S)_2 + Na^+$, calcd. 787.1895, found 787.1887. **Exact mass** ($ESI^-$): $C_{17}H_{19}FN_2O_5S - H^+$, calcd. 381.0926, found 381.0930; $(C_{17}H_{19}FN_2O_5S - H^+)_2 + H^+$, calcd. 763.1925, found 763.1930; $(C_{17}H_{19}FN_2O_5S - H^+)_2 + Na^+$, calcd. 785.1744, found 785.1758.

**Example 13b**.(*R*)-*N*-(4-Methoxyphenylsulfonyl)-*N*-(3-pyridylmethyl)-2-amino-4-fluorobutanoic acid

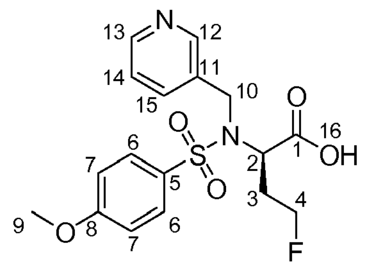

According to the general procedure *tert*-butyl (*R*)-*N*-(4-methoxyphenylsulfonyl)-N-(3-pyridylmethyl)-2-amino-4-fluorobutanoate (448 mg, 1.02 mmol) was dissolved in dry dichloromethane (25 mL) and treated with trifluoroacetic acid (25 mL). The product was isolated as white solid. **Yield**: 352 mg (90 %). **M.p**. 155-156 °C. **$^1$H NMR** (400 MHz, DMSO-$d_6$): δ 1.79 (m, $^3J_{H,F}$ = 22.8 Hz, 1 H, 3-$CH_A$), 2.19 (m, $^3J_{H,F}$ = 27.6 Hz, 1 H, 3-$CH_B$), 3.84 (s, 3 H, 9-$CH_3$), 4.32 (t, $^2J_{H,H}$ = 5.7 Hz, 1 H, 4-$CH_A$), 4.37 (t, $^2J_{H,H}$ = 5.7 Hz, 1 H, 4-$CH_B$), 4.41 (d, $^2J_{H,H}$ = 17.1 Hz, 1 H, 10-$CH_A$), 4.48 (t, $^3J_{H,H}$ = 7.2 Hz, 1 H, 2-CH), 4.61 (d, $^2J_{H,H}$ = 16.7 Hz, 1 H, 10-$CH_B$), 7.09 (dm, $^3J_{H,H}$ = 8.5 Hz, 2 H, 6-CH), 7.34 (dd, $^3J_{H,H}$ = 7.6 Hz, $^3J_{H,H}$ = 4.9 Hz, 1 H, 14-CH), 7.72 - 7.80 (m, 3 H, 7-CH and 15-CH), 8.46 (d, $^3J_{H,H}$ = 4.7 Hz, 1 H, 13-CH), 8.52 (s, 1 H, 12-CH), 13.11 (s br, 1 H, 16-OH). **$^{13}$C NMR** (100 MHz, DMSO-$d_6$): δ 31.0 (dt, $^2J_{C,F}$ = 20.2 Hz, C-3), 46.8 (t, C-10), 55.7 (q, C-9), 56.2 (dd, $^3J_{C,F}$ = 5.0 Hz, C-2), 80.4 (dt, $^1J_{C,F}$ = 163.1 Hz, C-4), 114.3 (d, C-6), 123.2 (d, C-14), 129.5 (d, C-7), 130.9 (s, C-5), 133.6 (s, C-11), 135.8 (d, C-15), 148.5 (d, C-13), 149.3 (d, C-12), 162.7 (s, C-8), 171.2 (s, C-1). **$^{19}$F NMR** (282 MHz, DMSO-$d_6$): δ -221.8 (ddt, $^2J_{H,F}$ = 47.1 Hz, $^2J_{H,F}$ = 27.5 Hz, $^2J_{H,F}$ = 22.5 Hz, 4-$CH_2F$). **Exact mass** ($ESI^+$): $C_{17}H_{19}FN_2O_5S + H^+$, calcd. 383.1077, found 383.1079; $C_{17}H_{19}FN_2O_5S + Na^+$, calcd. 405.0896, found 405.0900; $(C_{17}H_{19}FN_2O_5S)_2 + H^+$, calcd. 765.2076, found 765.2071; $(C_{17}H_{19}FN_2O_5S)_2 + Na^+$, calcd. 787.1895, found 787.1884. **MS** ($ESI^+$, daughter ion experiment): *m/z* (%) 383 (75) [$M^+ + H^+$], 363 (4) [$M^+$ - HF], 337 (22) [383 - $H^+$ - $CO_2H$], 280 (40) [$C_{13}H_{16}N_2O_3S^+$], 211 (5) [383 - $C_7H_7O_3S$], 200 (13) [$C_8H_{10}NO_3S^+$], 171 (18) [$C_7H_7O_3S^+$], 167 (100) [211 - $CO_2H$], 123 (8) [$C_7H_7O_2^+$].

**Example 14**

**Example 14a.** (*S*)-*N*-(4-Methoxyphenylsulfonyl)-N-(3-pyridylmethyl)-2-amino-4-fluoropent-4-enoic acid

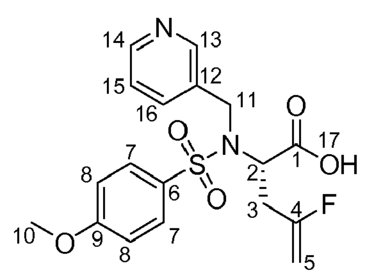

According to the general procedure *tert*-butyl (*S*)-*N*-(4-Methoxyphenylsulfonyl)-N-(3-pyridylmethyl)-2-amino-4-fluoropent-4-enoate (272 mg, 0.60 mmol) was dissolved in dry dichloromethane (20 mL) and treated with 20 mL trifluoroacetic acid. The product was isolated as yellowish-white solid. **Yield:** 200 mg (85 %). **M.p**. 172 °C. **$^1$H NMR** (300 MHz, DMSO-$d_6$): δ 2.59 (m, 1 H, 3-$CH_A$), 2.84 (ddd, $^3J_{H,F}$ = 15.7 Hz, $^2J_{H,H}$ = 12.6 Hz, $^3J_{H,H}$ = 5.5 Hz, 1 H, 3-$CH_B$), 3.91 (s, 3 H, 10-$CH_3$), 4.30 (dd, $^3J_{H,F}$ = 51.6 Hz, $^2J_{H,H}$ = 3.1 Hz, 1 H, 5-$CH_{trans}$), 4.53 (d, $^2J_{H,H}$ = 16.9 Hz, 1 H, 11-$CH_A$), 4.62 (dd, $^3J_{H,F}$ = 17.8 Hz, $^2J_{H,H}$ = 3.0 Hz, 1 H, 5-$CH_{cis}$), 4.66 (d, $^2J_{H,H}$ = 16.8 Hz, 1 H, 11-$CH_B$), 4.69 (dd, $^3J_{H,H}$ = 5.7 Hz, $^3J_{H,H}$

= 3.0 Hz, 1 H, 2-CH), 7.14 (d, $^3J_{H,H}$ = 9.1 Hz, 2 H, 7-CH), 7.45 (m, 1 H, 15-CH), 7.80 (d, $^3J_{H,H}$ = 9.0 Hz, 2 H, 8-CH), 7.88 (m, 1 H, 16-CH), 8.55 (s, 1 H, 14-CH), 8.63 (s, 1 H, 13-CH), 12.94 (s, 1 H, 17-OH). $^{13}$**C NMR** (75 MHz, DMSO-$d_6$): δ 32.6 (dt, $^2J_{C,F}$ = 28.0 Hz, C-3), 46.7 (t, C-11), 55.7 (q, C-10), 57.0 (d, C-2), 93.2 (dt, $^2J_{C,F}$ = 18.4 Hz, C-5), 114.3 (d, C-7), 123.4 (d, C-15), 129.4 (d, C-8), 129.6 (s, C-6), 130.7 (s, C-12), 136.6 (d, C-16), 147.7 (d, C-14), 148.7 (d, C-13), 161.7 (ds, $^1J_{C,F}$ = 255.3 Hz, C-4), 162.7 (s, C-9), 169.9 (s, C-1). $^{19}$**F NMR** (282 MHz, DMSO-$d_6$): δ -96.2 (m, $^3J_{H,F}$ = 51.5 Hz, $^3J_{H,F}$ = 17.8 Hz, $^3J_{H,F}$ = 15.7 Hz, 4-CF). **Exact mass** (ESI$^+$): $C_{18}H_{19}FN_2O_5S$ + $H^+$, calcd. 395.1077, found 395.1088; $C_{18}H_{19}FN_2O_5S$ + $Na^+$, calcd. 417.0896, found 417.0905; $(C_{18}H_{19}FN_2O_5S)_2$ + $H^+$, calcd. 789.2076, found 789.2096; $(C_{18}H_{19}FN_2O_5S)_2$ + $Na^+$, calcd. 811.1895, found 811.1897. **Exact mass** (ESI$^-$): $C_{18}H_{19}FN_2O_5S$ - $H^+$, calcd. 393.0920, found 393.0940; $(C_{18}H_{19}FN_2O_5S - H^+)_2$ + $H^+$, calcd. 787.1919, found 787.1955; $(C_{18}H_{19}FN_2O_5S - H^+)_2$ + $Na^+$, calcd. 809.1739, found 809.1766. **MS** (ESI$^+$, daughter ion experiment): *m*/*z* (%) 395 (12) [$M^+$ + $H^+$], 179 (22) [$M^+$ - $C_2H_2F$ - $C_7H_7O_3S$], 171 (31) [$C_7H_7O_3S^+$], 165 (100) [$M^+$ - $C_3H_4F$ - $C_7H_7O_3S$], 147 (10), 123 (8), 107 (18) [$C_7H_7O^+$], 92 (20) [$C_6H_7N^+$]. **MS** (ESI$^-$, daughter ion experiment): *m*/*z* (%) 393 (2) [$M^-$ - $H^+$], 277 (27) [393 - $C_5H_5FO2^-$], 171 (100) [$C_7H_7O_3S^-$], 157 (4) [$C_6H_5O_3S^-$], 115 (18) [$C_5H_4FO_2^-$].

**Example 14b**.(*R*)-*N*-(4-Methoxyphenylsulfonyl)-*N*-(3-pyridylmethyl)-2-amino-4-fluoropent-4-enoic acid

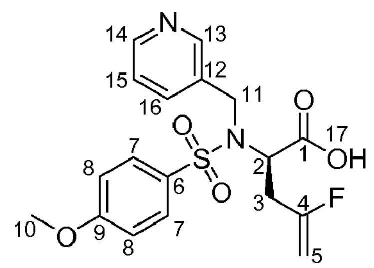

According to the general procedure *tert*-butyl (*R*)-*N*-(4-methoxyphenylsulfonyl)-N-(3-pyridylmethyl)-2-amino-4-fluoropent-4-enoate (256 mg, 0.57 mmol) was dissolved in dry dichloromethane (15 mL) and treated with trifluoroacetic acid (15 mL). The product was isolated as yellowish-white solid. **Yield**: 204 mg (91 %). **M.p**. 172-173 ˚C. $^1$**H NMR** (300 MHz, DMSO-$d_6$): δ 2.51 (m, 1 H, 3-$CH_A$), 2.77 (ddd, $^3J_{H,F}$ = 21.2 Hz, $^2J_{H,H}$ = 12.7 Hz, $^3J_{H,H}$ = 5.8 Hz, 1 H, 3-$CH_B$), 4.22 (dd, $^3J_{H,F}$ = 51.5 Hz, $^2J_{H,H}$ = 3.0 Hz, 1 H, 5-$CH_{trans}$), 4.45 (d, $^2J_{H,H}$ = 16.8 Hz, 1 H, 11-$CH_A$), 4.55 (dd, $^3J_{H,F}$ = 17.8 Hz, $^2J_{H,H}$ = 3.0 Hz, 1 H, 5-$CH_{cis}$), 4.59 (d, $^2J_{H,H}$ = 16.5 Hz, 1 H, 11-$CH_B$), 4.62 (t, $^3J_{H,H}$ = 4.3 Hz, 1 H, 2-CH), 7.07 (d, $^3J_{H,H}$ = 9.0 Hz, 2 H, 7-CH), 7.33 (dd, $^3J_{H,H}$ = 7.7 Hz, $^3J_{H,H}$ = 4.8 Hz, 1 H, 15-CH), 7.73 (d, $^3J_{H,H}$ = 9.0 Hz, 2 H, 8-CH), 7.79 (m, 1 H, 16-CH), 8.45 (d, $^3J_{H,H}$ = 4.6 Hz, 1 H, 14-CH), 8.54 (s, 1 H, 13-CH), 12.89 (s br, 1 H, 17-OH). $^{13}$**C NMR** (75.48 MHz, DMSO-$d_6$): δ 32.7 (dt, $^2J_{C,F}$ = 27.6 Hz, C-3), 46.7 (t, C-11), 55.7 (q, C-10), 56.9 (d, C-2), 93.2 (dt, $^2J_{C,F}$ = 18.1 Hz, C-5), 114.3 (d, C-7), 123.2 (d, C-15), 129.6 (d, C-8), 130.8 (s, C-6), 132.8 (s, C-12), 136.1 (d, C-16), 148.1 (d, C-14), 149.1 (d, C-13), 161.7 (ds, $^1J_{C,F}$ = 255.3 Hz, C-4), 162.7 (s, C-9), 170.5 (s, C-1). $^{19}$**F NMR** (282.37 MHz, DMSO-$d_6$): δ -96.2 (dddd, $^3J_{H,F}$ = 51.5 Hz, $^3J_{H,F}$ = 21.1 Hz, $^3J_{H,F}$ = 17.9 Hz, $^3J_{H,F}$ = 12.6 Hz, 4-CF). **Exac**t **mass** (ESI$^+$): $C_{18}H_{19}FN_2O_5S$ + $H^+$, calcd. 395.1077, found 395.1080; $C_{18}H_{19}FN_2O_5S$ + $Na^+$, calcd. 417.0896, found 417.0896. **Exact mass** (ESI$^-$): $C_{18}H_{19}FN_2O_5S$ - $H^+$, calcd. 393.0920, found 393.0948; $(C_{18}H_{19}FN_2O_5S)_2$ - $H^+$, calcd. 787.1919, found 787.1940. **MS** (ESI$^+$, daughter ion experiment): *m*/*z* (%)395 (45) [$M^+$ + $H^+$], 375 (5) [395 - HF], 349 (22) [395 - $H^+$ - $CO_2H$], 305 (20) [349 - $C_2H_2F$], 280 (30) [$C_{13}H_{16}N_2O_3S^+$], 200 (10) [305 - $C_7H_7O$], 179 (100) [$C_6H_{10}FNO_2S^+$], 171 (75) [$C_7H_7O_3S^+$].

**General procedure for hydrolysis of amino acid methylesters**

The aminoacid methylester (1 equivalent) dissolved in a mixture of THF, methanol and water (ratio 3:1:1; 3.3 mL/mmol) is treated with lithium hydroxide (5 equivalents). This solution is stirred at r.t. over night. The reaction mixture is extracted with ethyl acetate (2 × 20 mL). This extract is discharged. Then the aqueous phase is acidified with 0.5 N hydrochloric acid and extracted again with ethyl acetate (4 × 20 mL). The combined organic phases are dried with magnesium sulfate and the solvent is evaporated.

**Example 15**

**Example 15a.** (*S*)-*N*-(4-Methoxyphenylsulfonyl)-N-(3-pyridylmethyl)aminobutanoic acid

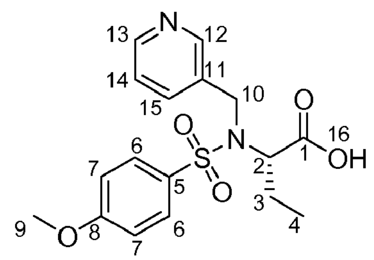

According to the general procedure methyl (*S*)-*N*-(4-methoxyphenylsulfonyl)-N-(3-pyridylmethyl)-aminobutanoate (306 mg, 0.81 mmol) was dissolved in the above-mentioned solvent mixture (10 mL) and treated with lithium hydroxide (169 mg, 4.04 mmol). The product was isolated as white, highly viscos liquid. **Yield**: 174 mg (59 %). The spectroscopic data agree with those given above.

**Example 15b**. (*R*)-*N*-(4-Methoxyphenylsulfonyl)-*N*-(3-pyridylmethyl)aminobutanoic acid

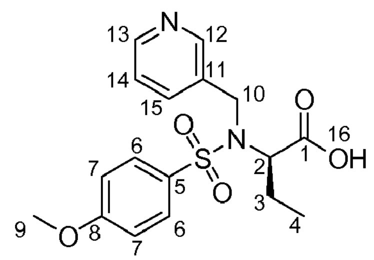

According to the general procedure methyl (*R*)-*N*-(4-methoxyphenylsulfonyl)-*N*-(3-pyridylmethyl)-aminobutanoate (420 mg, 1.11 mmol) was dissolved in the above-mentioned solvent mixture (10 mL) and treated with lithium hydroxide (230 mg, 5.55 mmol). The product was isolated as white, highly viscos liquid. **Yield:** 251 mg (62 %). The spectroscopic data agree with those given above.

**Synthesis of the hydroxamic acids**

**General procedure for synthesis of the hydroxamates**

The N,N-disubstituted $\alpha$-amino acid (1 equivalent) is suspended in dichloromethane (15 mL/mmol). Then 1-hydroxy-benzotriazole (HOBT, 1 equivalent) is added and stirred for a short time. After addition of *N*-methylmorpholin (NMM) (5 equiv.) the acid is dissolved. Subsequently, *O*-*tert*-butylhydroxylamine (3 equiv.) was added and the solution is stirred for 5 min. 1-(3-Dimethylaminopropyl)-3-ethylcarbodiimide (EDC) hydrochloride (1.3 equiv.) is added and the reaction mixture is stirred overnight. The solution is washed with water (20 mL) and the aqueous phase is extracted with dichloromethane (3 $\times$ 20 mL). The combined organic phases are washed with brine (1 $\times$ 20 mL) and dried with magnesium sulfate. After evaporation of the solvent the crude product is purified by column chromatography (silica gel, Cy/EtOAc, 1:3). The silica gel was inactivated by flushing with the eluent containing 2 % triethylamine. The product is obtained as highly viscose liquid, which on drying in high vacuum becomes solid.

Scheme 2

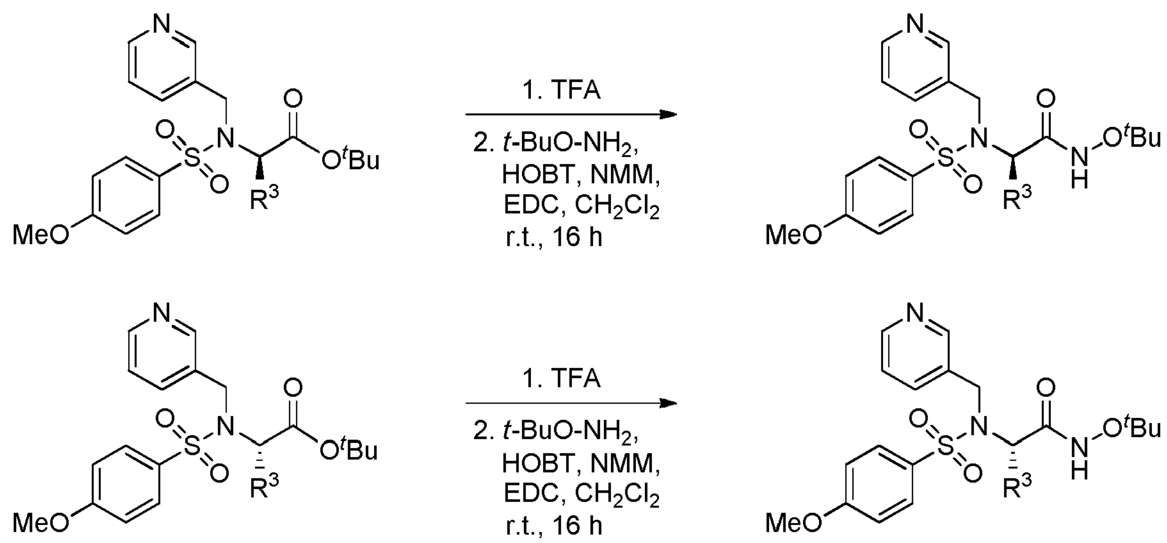


**Example 16**

**Example 16a.** *Tert*-butyl (*S*)-*N*-(4-methoxyphenylsulfonyl)-*N*-(3-pyridylmethyl)-2-aminobutan-hydroxamate

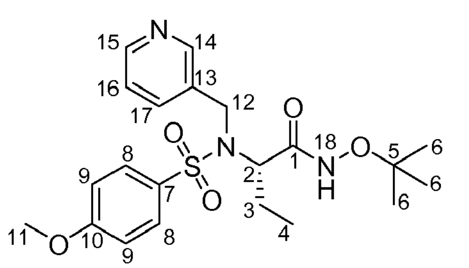

According to the general procedure (*S*)-*N*-(4-methoxyphenylsulfonyl)-*N*-(3-pyridylmethyl)aminobutanoic acid (185 mg, 0.51 mmol) was reacted with HOBT (71 mg, 0.51 mmol), NMM (259 mg, 2.55 mmol), *O-tert*-butylhydroxylamine (194 mg, 1.53 mmol) and EDC (134 mg, 0.69 mmol) in dichloromethane (15 mL). After purification by column chromatography (column Ø 2 cm × 15) the product was isolated as white solid. **Yield**: 173 mg (78 %). **M.p.** 66 °C. **$^1$H NMR** (300 MHz, DMSO-$d_6$): δ 0.69 (t, $^3J_{H,H}$ = 7.2 Hz, 3 H, 4-$CH_3$), 1.23 (s, 9 H, 6-$CH_3$), 1.44 (tq, $^2J_{H,H}$ = 14.8 Hz, $^3J_{H,H}$ = 7.4 Hz, 1 H, 3-$CH_A$), 1.90 (m, 1 H, 3-$CH_B$), 3.85 (s, 3 H, 11-$CH_3$), 4.11 (m, 1 H, 2-CH), 4.56 (s, 2 H, 12-$CH_2$), 6.91 (d, $^3J_{H,H}$ = 8.9 Hz, 2 H, 8-CH), 7.20 (dd, $^3J_{H,H}$ = 7.8 Hz, $^3J_{H,H}$ = 4.9 Hz, 1 H, 16-CH), 7.61 (d, $^3J_{H,H}$ = 8.9 Hz, 2 H, 9-CH), 7.71 (d, $^3J_{H,H}$ = 7.8 Hz, 1 H, 17-CH), 8.48 (d, $^3J_{H,H}$ = 3.7 Hz, 1 H, 15-CH), 8.54 (d, $^4J_{H,H}$ = 1.5 Hz, 1 H, 14-CH), 8.59 (s, 1 H, 18-NH). **$^{13}$C NMR** (75 MHz, DMSO-$d_6$): δ 10.6 (q, C-4), 22.9 (t, C-3), 26.2 (q, C-6), 45.7 (t, C-12), 55.6 (q, C-11), 59.5 (d, C-2), 82.2 (s, C-5), 114.4 (d, C-8), 123.2 (d, C-16), 129.2 (d, C-9), 131.3 (s, C-7), 132.9 (s, C-13), 136.5 (d, C-17), 148.8 (d, C-15), 149.9 (d, C-14), 163.2 (s, C-10), 168.2 (s, C-1). **Exact mass** (ESI$^+$): $C_{21}H_{29}N_3O_5S + H^+$, calcd. 436.1906, found 436.1898; $C_{21}H_{29}N_3O_5S + Na^+$, calcd. 458.1726, found 458.1716; $(C_{21}H_{29}N_3O_5S)_2 + H^+$, calcd. 871.3734, found 871.3717; $(C_{21}H_{29}N_3O_5S)_2 + Na^+$, calcd. 893.3554, found 893.3534. **MS** (ESI$^+$, daughter ion experiment): *m/z* (%) = 436 (50) [$M^+ + H^+$], 380 (100) [436 - $C_4H_8$], 319 (18) [436 - $H^+$ - $CONHOC(CH_3)_3$], 264 (6) [436 - $H^+$ - $C_7H_7O_3S$], 228 (4) [319 - $C_6H_6N$], 171 (12) [$C_7H_7O_3S^+$]. **Optical rotation**:

$$[\alpha]_{589}^{20} = -37.2, [\alpha]_{578}^{20} = -39.3, [\alpha]_{546}^{20} = -45.2, [\alpha]_{436}^{20} = -84.7, [\alpha]_{365}^{20} = -156.2 \text{ (c = 1.007, CHCl}_3\text{).}$$

**Example 16b.** *Tert*-butyl (*R*)-*N*-(4-methoxyphenylsulfonyl)-*N*-(3-pyridylmethyl)-2-aminobutanhydroxamate

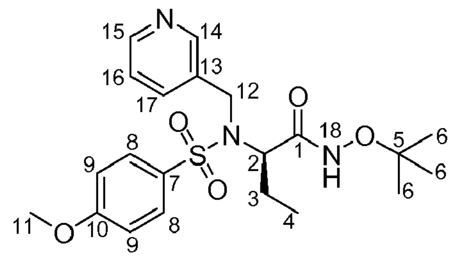

According to the general procedure (*R*)-*N*-(4-methoxyphenylsulfonyl)-N-(3-pyridylmethyl)-aminobutanoic acid (105 mg, 0.29 mmol) was reacted with HOBT (40 mg, 0.29 mmol), NMM (147 mg, 1.45 mmol), O-*tert*-butylhydroxylamine (110 mg, 0.87 mmol) and EDC (74 mg, 0.38 mmol) in dichloromethane (15 mL). After column chromatography (column Ø 2 cm × 15) the product was isolated as white solid. **Yield**: 101 mg (83 %). **M.p**. 65-66 ˚C. **$^1$H NMR** (300.13 MHz, DMSO-$d_6$): δ 0.69 (t, $^3J_{H,H}$ = 7.3 Hz, 3 H, 4-$CH_3$), 1.25 (s, 9 H, 6-$CH_3$), 1.44 (m, 1 H, 3-$CH_A$), 1.90 (m, 1 H, 3-$CH_B$), 3.85 (s, 3 H, 11-$CH_3$), 4.09 (m, 1 H, 2-CH), 4.56 (s, 2 H, 12-$CH_2$), 6.91 (dm, $^3J_{H,H}$ = 8.9 Hz, 2 H, 8-CH), 7.20 (dd, $^3J_{H,H}$ = 7.8 Hz, $^3J_{H,H}$ = 4.8 Hz, 1 H, 16-CH), 7.61 (dm, $^3J_{H,H}$ = 8.9 Hz, 2 H, 9-CH), 7.70 (d, $^3J_{H,H}$ = 7.8 Hz, 1 H, 17-CH), 8.49 (d, $^3J_{H,H}$ = 3.7 Hz, 1 H, 15-CH), 8.53 - 8.56 (m, 2 H, 14-CH and 18-NH). **$^{13}$C NMR** (75.48 MHz, DMSO-$d_6$): δ 10.6 (q, C-4), 22.9 (t, C-3), 26.2 (q, C-6), 45.7 (t, C-12), 55.6 (q, C-11), 59.5 (d, C-2), 82.2 (s, C-5), 114.4 (d, C-8), 123.2 (d, C-16), 129.2 (d, C-9), 131.4 (s, C-7), 132.9 (s, C-13), 136.5 (d, C-17), 148.9 (d, C-15), 150.0 (d, C-14), 163.2 (s, C-10), 168.2 (s, C-1). **Exact mass** (ESI$^+$): $C_{21}H_{29}N_3O_5S + H^+$, calcd. 436.1906, found 436.1897; $C_{21}H_{29}N_3O_5S + Na^+$, calcd. 458.1726, found 458.1715; $(C_{21}H_{29}N_3O_5S)_2 + H^+$, calcd. 871.3734, found 871.3702; $(C_{21}H_{29}N_3O_5S)_2 + Na^+$, calcd. 893.3554, found 893.3521. **MS** (ESI$^+$, daughter ion experiment): *m/z* (%) 436 (65) [$M^+ + H^+$], 380 (100) [436 - $C_4H_8$], 319 (12) [436 - $H^+$ - $CONHOC(CH_3)_3$], 264 (15) [436 - $H^+$ - $C_7H_7O_3S$], 228 (5) [319 - $C_6H_6N$]. **Optical rotation**:

**Example 17**

**Example 17a**. *Tert*-butyl (*S*)-*N*-(4-methoxyphenylsulfonyl)-*N*-(3-pyridylmethyl)-2-aminopent-4-enhydroxamate

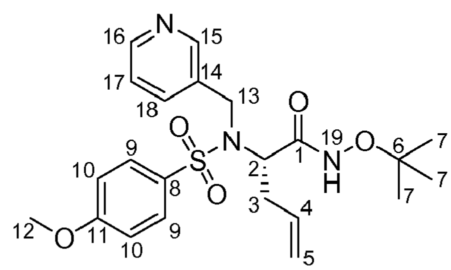

According to the general procedure (S)-N-(4-methoxyphenylsulfonyl)-N-(3-pyridylmethyl)-aminopent-4-enoic acid (280 mg, 0.744 mmol) was reacted with HOBT (101 mg, 0.744 mmol), NMM (377 mg, 3.72 mmol), O-tert-butylhydroxylamine (280 mg, 2.23 mmol) and EDC (186 mg, 0.97 mmol) in dichloromethane (15 mL). After column chromatography (column Ø 2 cm × 15 cm) the product was isolated as white solid. Yield: 306 mg (92 %). M.p. 65-66 ˚C. **$^1$H** NMR (300 MHz, $CDCl_3$): δ 1.21 (s, 9 H, 7-$CH_3$), 2.16 (dt, $^2J_{H,H}$ = 13.8 Hz, $^3J_{H,H}$ = 6.8 Hz, 1 H, 3-$CH_A$), 2.60 (ddd, $^2J_{H,H}$ = 14.0 Hz, $^3J_{H,H}$ = 8.1 Hz, $^3J_{H,H}$ = 7.0 Hz, 1 H, 3-$CH_B$), 3.86 (s, 3 H, 12-$CH_3$), 4.29 (dd, $^3J_{H,H}$ = 8.3 Hz, $^3J_{H,H}$ = 6.9 Hz, 1 H, 2-CH), 4.58 (s, 2 H, 13-$CH_2$), 4.94 (d, $^2J_{H,H}$ = 2.9 Hz, 1 H, 5-$CH_A$), 4.97 (d, $^2J_{H,H}$ = 3.1 Hz, 1 H, 5-$CH_B$), 5.39 (ddt, $^3J_{H,H}$ = 14.1 Hz, $^3J_{H,H}$ = 9.6 Hz, $^3J_{H,H}$ = 6.9 Hz, 1 H, 4-CH), 6.93 (dm, $^3J_{H,H}$ = 8.9 Hz, 2 H, 9-CH), 7.21 (dd, $^3J_{H,H}$ = 7.7 Hz, $^3J_{H,H}$ = 4.9 Hz, 1 H, 17-CH), 7.64 (dm, $^3J_{H,H}$ = 9.0 Hz, 2 H, 10-CH), 7.72 (d, $^3J_{H,H}$ = 7.9 Hz, 1 H, 18-CH), 8.49 (d, $^3J_{H,H}$ = 3.7 Hz, 1 H, 16-CH), 8.56 (d, $^4J_{H,H}$ = 1.5 Hz, 1 H, 15-CH), 8.70 (s, 1 H, 19-NH). **$^{13}$C NMR** (75 MHz, $CDCl_3$): δ 26.2 (q, C-7), 33.9 (t, C-3), 45.8 (t, C-13), 55.6 (q, C-12), 57.6 (d, C-2), 82.2 (s, C-6), 114.4 (d, C-9), 119.0 (t, C-5), 123.3 (d, C-17), 129.3 (d, C-10), 131.0 (s, C-8), 132.5 (d, C-4), 132.8 (s, C-14), 136.4 (d, C-18), 148.8 (d, C-16), 149.8 (d, C-15), 163.2 (s, C-11), 167.7 (s, C-1). **Elemental analysis**: $C_{22}H_{29}N_3O_5S$ (M = 447.18 g/mol), calcd. C 59.04, H

6.53, N 9.39; found C 59.11, H 6.57, N 9.10 %. **Exact mass** ($ESI^+$): $C_{22}H_{29}N_3O_5S + H^+$, calcd. 448.1906, found 448.1907; $C_{22}H_{29}N_3O_5S + Na^+$, calcd. 895.3734, found 895.3730; $(C_{22}H_{29}N_3O_5S)_2 + H^+$, calcd. 470.1726, found 470.1722; $(C_{22}H_{29}N_3O_5S)_2 + Na^+$, calcd. 917.3554, found 917.3552. **MS** ($ESI^+$, daughter ion experiment): *m*/*z* (%) 448 (10) [$M^+ + H^+$], 392 (100) [448 - $C_4H_8$], 331 (32) [448 - $C_5H_{11}NO_2$], 279 (8) [331 - $C_4H_6$], 240 (7), 171 (30) [$C_7H_7O_3S^+$], 161 (20) [331 - $C_7H_7O_3S$], 125 (11), 107 (2) [$C_7H_7O^+$], 92 (8) [$C_6H_6N^+$]. **Optical rotation:** .

**Example 17b.** *Tert*-butyl (*R*)-*N*-(4-methoxyphenylsulfonyl)-*N*-(3-pyridylmethyl)-2-aminopent-4-enhydroxamate

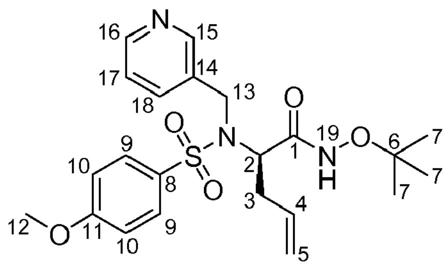

According to the general procedure (*R*)-*N*-(4-methoxyphenylsulfonyl)-N-(3-pyridylmethyl)-aminopent-4-enoic acid (150 mg, 0.40 mmol) was reacted with HOBT (54 mg, 0.40 mmol), NMM (202 mg, 1.99 mmol), *O*-*tert*-butylhydroxylamine (151 mg 1.20 mmol) and EDC (100 mg 0.52 mmol) in dichloromethane (15 mL). After column chromatography (column Ø 2 cm × 15 cm) the product was isolated as white solid. **Yield**: 130 mg (73 %). **M.p**. 65-66 ˚C. **$^1$H NMR** (300 MHz, $CDCl_3$): δ 1.21 (s, 9 H, 7-$CH_3$), 2.17 (dt, $^2J_{H,H}$ = 14.0 Hz, $^3J_{H,H}$ = 6.9 Hz, 1 H, 3-$CH_A$), 2.60 (ddd, $^2J_{H,H}$ = 14.8 Hz, $^3J_{H,H}$ = 8.3 Hz, $^3J_{H,H}$ = 6.8 Hz, 1 H, 3-$CH_B$), 3.86 (s, 3 H, 12-$CH_3$), 4.31 (t, $^3J_{H,H}$ = 7.6 Hz, 1 H, 2-CH), 4.60 (s, 2 H, 13-$CH_2$), 4.95 (dd, $^3J_{H,H}$ = 13.7 Hz, $^2J_{H,H}$ = 1.9 Hz, 2 H, 5-$CH_2$), 5.40 (td, $^3J_{H,H}$ = 16.8 Hz, $^3J_{H,H}$ = 6.9 Hz, 1 H, 4-CH), 6.93 (dm, $^3J_{H,H}$ = 8.9 Hz, 2 H, 9-CH), 7.23 (dd, $^3J_{H,H}$ = 7.8 Hz, $^3J_{H,H}$ = 4.9 Hz, 1 H, 17-CH), 7.65 (dm, $^3J_{H,H}$ = 8.9 Hz, 2 H, 10-CH), 7.75 (d, $^3J_{H,H}$ = 7.9 Hz, 1 H, 18-CH), 8.50 (d, $^3J_{H,H}$ = 3.8 Hz, 1 H, 16-CH), 8.58 (s, 1 H, 15-CH), 8.75 (s, 1 H, 19-NH). **$^{13}$C NMR** (75 MHz, $CDCl_3$): δ 26.2 (q, C-7), 34.0 (t, C-3), 45.7 (t, C-13), 55.6 (q, C-12), 57.6 (d, C-2), 82.2 (s, C-6), 114.4 (d, C-9), 118.9 (t, C-5), 123.3 (d, C-17), 129.3 (d, C-10), 131.0 (s, C-8), 132.5 (d, C-4), 133.1 (s, C-14), 136.8 (d, C-18), 148.5 (d, C-16), 149.5 (d, C-15), 163.2 (s, C-11), 167.7 (s, C-1). **Exact mass** ($ESI^+$): $C_{22}H_{29}N_3O_5S + H^+$, calcd. 448.1906, found 448.1900; $C_{22}H_{29}N_3O_5S + Na^+$, calcd. 470.1726, found 470.1722; $(C_{22}H_{29}N_3O_5S)_2 + H^+$, calcd. 895.3734, found 895.3730; $(C_{22}H_{29}N_3O_5S)_2 + Na^+$, calcd. 917.3554, found 917.3546. **MS** ($ESI^+$, daughter ion experiment): *m*/*z* (%) 448 (50) [$M^+ + H^+$], 392 (100) [448 - $C_4H_8$], 331 (13) [448 - $H^+$ - $CONHOC(CH_3)_3$], 240 (8) [331 - $C_6H_6N$], 171 (12) [$C_7H_7O_3S^+$]. **Optical rotation**:

$$[\alpha]_{589}^{20} = +29.6, [\alpha]_{578}^{20} = +31.2, [\alpha]_{546}^{20} = +36.0, [\alpha]_{436}^{20} = +66.9, [\alpha]_{365}^{20} = +121.9 \ (c = 1.010, CHCl_3).$$

**Example 18**

**Example 18a**. *Tert*-butyl (*S*)-*N*-(4-methoxyphenylsulfonyl)-*N*-(3-pyridylmethyl)-2-amino-4-fluorobutanhydroxamate

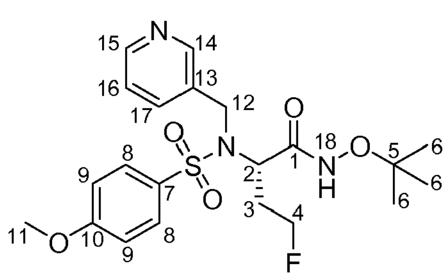

According to the general procedure (*S*)-*N*-(4-methoxyphenylsulfonyl)-N-(3-pyridylmethyl)-2-amino-4-fluorobutanoic acid (395 mg, 1.03 mmol) was reacted with HOBT (140 mg, 1.03 mmol), NMM (522 mg, 5.17 mmol), *O*-*tert*-butylhydroxylamine (390 mg, 3.10 mmol) and EDC (257 mg, 1.34 mmol) in dichloromethane (15 mL). After column chromatography (column Ø 2 cm × 15 cm) the product was isolated as white solid. **Yield**: 355 mg (75 %). **M.p**. 59-60 °C. **$^1$H NMR** (300 MHz, $CDCl_3$): δ 1.24 (s, 9 H, 6-$CH_3$), 1.72 (m, $^3J_{H,F}$ = 22.5 Hz, 1 H, 3-$CH_A$), 2.22 (m, $^3J_{H,F}$ = 31.6 Hz, 1 H 3-$CH_B$), 3.86 (s, 3 H, 11-$CH_3$), 4.25 (m, $^2J_{H,F}$ = 47.6 Hz, 2 H, 4-$CH_2F$), 4.49 (d, $^3J_{H,H}$ = 16.2 Hz, 1 H, 12-$CH_A$), 4.55 (m, 1 H, 2-CH), 4.61 (d, $^3J_{H,H}$ = 16.0 Hz, 1 H, 12-$CH_B$), 6.94 (d, $^3J_{H,H}$ = 8.8 Hz, 2 H, 8-CH), 7.22 (dd, $^3J_{H,H}$ = 7.8 Hz, $^3J_{H,H}$ = 4.8 Hz, 1 H, 16-CH), 7.62 - 7.71 (m, $^3J_{H,H}$ = 8.9 Hz, 3 H, 9-CH & 17-CH), 8.49 - 8.53 (m, 2 H, 14-CH & 15-CH), 8.81 (m, 1 H, 18-NH). **$^{13}$C NMR** (75 MHz, $CDCl_3$): δ 26.9 (s, C-6), 30.6 (d, $^2J_{C,F}$ = 19.5 Hz, C-3), 45.8 (s, C-12), 53.9 (d, $^3J_{C,F}$ = 3.0 Hz, C-2), 55.6 (s, C-11), 80.0 (d, $^1J_{C,F}$ = 165.6 Hz, C-4), 82.4 (s, C-5), 114.5 (s, C-8), 123.4 (s, C-16), 129.2 (s, C-9), 130.8 (s, C-7), 132.6 (s, C-13), 136.4 (s, C-17), 149.1 (s, C-15), 149.8 (s, C-14), 163.3 (s, C-10), 167.4 (s, C-1). **$^{19}$F NMR** (282 MHz, $CDCl_3$): δ -222.2 (ddt, $^2J_{H,F}$ = 47.5 Hz, $^3J_{H,F}$ = 31.5 Hz, $^3J_{H,F}$ = 22.6 Hz, 4-$CH_2F$). **Exact mass** (ESI$^+$): $C_{21}H_{28}FN_3O_5S + H^+$, calcd. 454.1812, found 454.1804; $C_{21}H_{28}FN_3O_5S + Na^+$, calcd. 476.1631, found 476.1623; $(C_{21}H_{28}FN_3O_5S)_2 + H^+$, calcd. 907.3546, found 907.3544; $(C_{21}H_{28}FN_3O_5S)_2 + Na^+$, calcd. 929.3365, found 929.3370. **MS** (GC/MS, 70 eV): *m*/*z* (%) 454 (100) [$M^+ + H^+$], 398 (97) [$M^+ - C_4H_8$], 337 (15) [$M^+ - CONHOC(CH_3)_3$], 317 (4) [337 - HF], 246 (3) [337 - $C_6H_6N$], 171 (15) [$C_7H_7O_3S^+$]. **Optical rotation:**

$$[\alpha]_{589}^{20} = +17.9, [\alpha]_{578}^{20} = +18.7, [\alpha]_{546}^{20} = +21.7, [\alpha]_{436}^{20} = +40.7, [\alpha]_{365}^{20} = +75.1 \text{ (c = 1.005, CHCl}_3\text{).}$$

**Example 18b**. *Tert*-butyl (*R*)-*N*-(4-methoxyphenylsulfonyl)-*N*-(3-pyridylmethyl)-2-amino-4-fluorobutanhydroxamate

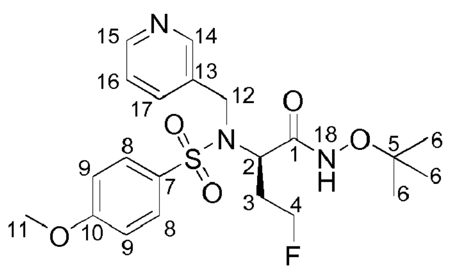

According to the general procedure (*R*)-*N*-(4-methoxyphenylsulfonyl)-*N*-(3-pyridylmethyl)-2-amino-4-fluorobutanoic acid (325 mg, 0.85 mmol) with HOBT (115 mg, 0.85 mmol), NMM (430 mg, 4.25 mmol), O-*tert*-butylhydroxylamine (320 mg, 2.55 mmol) and EDC (213 mg, 1.11 mmol) in dichloromethane (15 mL). After column chromatography (column Ø 2 cm × 10 cm) the product as white solid. **Yield**: 353 mg (91 %). **M.p.** 60 °C. **$^1$H NMR** (400 MHz, $CDCl_3$): δ 1.24 (s, 9 H, 6-$CH_3$), 1.71 (m, $^3J_{H,F}$ = 22.7 Hz, 1 H, 3-$CH_A$), 2.22 (m, $^3J_{H,F}$ = 28.2 Hz, 1 H, 3-$CH_B$), 3.87 (s, 3 H, 11-$CH_3$), 4.17 - 4.37 (m, $^2J_{H,F}$ = 46.9 Hz, 2 H, 4-$CH_2F$), 4.50 (d, $^2J_{H,H}$ = 16.0 Hz, 1 H, 12-$CH_A$), 4.55 (dd, $^3J_{H,H}$ = 8.8 Hz, $^3J_{H,H}$ = 5.9 Hz, 1 H, 2-CH), 4.61 (d, $^2J_{H,H}$ = 16.0 Hz, 1 H, 12-$CH_B$), 6.95 (dm, $^3J_{H,H}$ = 8.9 Hz, 2 H, 8-CH), 7.24 (dd, $^3J_{H,H}$ = 7.8 Hz, $^3J_{H,H}$ = 4.8 Hz, 1 H, 16-CH), 7.66 (dm, $^3J_{H,H}$ = 8.9 Hz, 2 H, 9-CH), 7.71 (d, $^3J_{H,H}$ = 7.9 Hz, 1 H, 17-CH), 8.51 (d, $^3J_{H,H}$ = 4.9 Hz, $^4J_{H,H}$ = 1.2 Hz, 1 H, 15-CH), 8.53 (d, $^4J_{H,H}$ = 1.7 Hz, 1 H, 14-CH), 8.76 (s, 1 H, 18-NH). **$^{13}$C NMR** (100 MHz, $CDCl_3$): δ 26.2 (s, C-6), 30.6 (d, $^2J_{C,F}$ = 19.9 Hz, C-3), 55.6 (s, C-11), 80.0 (d, $^1J_{C,F}$ = 165.9 Hz, C-4), 45.8 (s, C-12), 53.9 (d, $^3J_{C,F}$ = 2.5 Hz, C-2), 82.4 (s, C-5), 114.5 (s, C-8), 123.4 (s, C-16), 129.3 (s, C-9), 130.8 (s, C-7), 132.7 (s, C-13), 136.6 (s, C-17), 148.8 (s, C-15), 149.6 (s, C-14), 163.4 (s, C-10), 167.4 (s, C-1). **$^{19}$F NMR** (282 MHz, $CDCl_3$): δ -221.4 (ddt, $^2J_{H,F}$ = 46.9 Hz, $^3J_{H,F}$ = 28.4 Hz, $^3J_{H,F}$ = 22.8 Hz, 4-$CH_2F$). **Elemental analysis**: $C_{21}H_{30}FN_3O_5S$ (M = 455.54 g/mol): calcd. C 55.61, H 6.22, N 9.27 %, found C 55.24, H 6.47, N 9.22 %. **Exact mass** (ESI$^+$): $C_{21}H_{28}FN_3O_5S + H^+$, calcd. 454.1812, found 454.1817; $C_{21}H_{28}FN_3O_5S + Na^+$, calcd. 476.1631, found 476.1635; $(C_{21}H_{28}FN_3O_5S)_2 + H^+$, calcd. 907.3546, found 907.3567; $(C_{21}H_{28}FN_3O_5S)_2 + Na^+$, calcd. 929.3365, found 929.3387. **MS** (GC/MS, 70 eV): *m*/*z* (%)453 (0) [$M^+$], 433 (2) [$M^+$ - HF], 377 (10) [433 - $C_4H_8$], 347 (13), 277 (4) [$M^+ - C_8H_{15}FNO_2$], 262 (47) [277 - $CH_3$],

213 (3) [$C_9H_{11}NO_3S^+$], 206 (100), 171 (20) [$C_7H_7O_3S^+$], 155 (14), 107 (40) [$C_7H_7O^+$], 92 (34) [$C_6H_6N^+$], 77 (8) [$C_6H_5^+$], 57 (15) [$C_4H_9^+$], 56 (3) [$C_4H_8^+$]. **Optical rotation**:

$$[\alpha]_{589}^{20} = -14.8, [\alpha]_{578}^{20} = -15.7, [\alpha]_{546}^{20} = -18.1, [\alpha]_{436}^{20} = -34.3, [\alpha]_{365}^{20} = -62.8 \text{ (c = 1.004, CHCl}_3\text{)}.$$

**Example 19**

**Example 19a**. *Tert*-butyl (*S*)-*N*-(4-methoxyphenylsulfonyl)-*N*-(3-pyridylmethyl)-2-amino-4-fluoropent-4-enhydroxamate

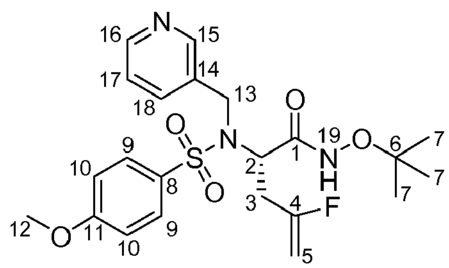

According to the general procedure (*S*)-*N*-(4-methoxyphenylsulfonyl)-*N*-(3-pyridylmethyl)-2-amino-4-fluoropent-4-enoic acid (150 mg, 0.38 mmol) with HOBT (52 mg, 0.38 mmol), NMM (193 mg, 1.90 mmol), O-*tert*-butylhydroxylamine (144 mg, 1.14 mmol) and EDC (95 mg, 0.50 mmol) in dichloromethane (15 mL). After column chromatography (column: Ø 2 cm × 10 cm) was isolated as white solid. **Yield**: 107 mg (61 %). **M.p.** 60-61 °C. $^1$**H NMR** (300 MHz, $CDCl_3$): δ 1.23 (s, 9 H, 7-$CH_3$), 2.26 (ddd, $^3J_{H,F}$ = 17.5 Hz, $^2J_{H,H}$ = 14.7 Hz, $^3J_{H,H}$ = 6.2 Hz, 1 H, 3-$CH_A$), 2.80 (ddd, $^3J_{H,F}$ = 19.6 Hz, $^2J_{H,H}$ = 14.7 Hz, $^3J_{H,H}$ = 8.2 Hz, 1 H, 3-$CH_B$), 3.86 (s, 3 H, 12-$CH_3$), 4.15 (dd, $^3J_{H,F}$ = 49.7 Hz, $^2J_{H,H}$ = 3.1 Hz, 1 H, 5-$CH_{trans}$), 4.48 (dd, $^3J_{H,F}$ = 17.1 Hz, $^2J_{H,H}$ = 3.1 Hz, 1 H, 5-$CH_{cis}$), 4.50 (d, $^2J_{H,H}$ = 16.2 Hz, 1 H, 13-$CH_A$), 4.59 (m, 1 H, 2-CH), 4.62 (d, $^2J_{H,H}$ = 16.0 Hz, 1 H, 13-$CH_B$), 6.93 (d, $^3J_{H,H}$ = 8.9 Hz, 2 H, 9-CH), 7.20 (dd, $^3J_{H,H}$ = 7.8 Hz, $^3J_{H,H}$ = 4.8 Hz, 1 H, 17-CH), 7.67 (t, $^3J_{H,H}$ = 8.9 Hz, 3 H, 10-CH and 18-CH), 8.49 (d, $^3J_{H,H}$ = 3.8 Hz, 1 H, 16-CH), 8.53 (d, $^4J_{H,H}$ = 1.7 Hz, 1 H, 15-CH), 8.69 (s, 1 H, 19-NH). $^{13}$**C NMR** (75.48 MHz, $CDCl_3$): δ 26.2 (q, C-7), 32.8 (dt, $^2J_{C,F}$ = 27.9 Hz, C-3), 45.9 (t, C-13), 54.9 (d, C-2), 55.7 (q, C-12), 82.5 (s, C-6), 93.9 (dt, $^2J_{C,F}$ = 18.5 Hz, C-5), 114.4 (d, C-9), 123.3 (d, C-17), 129.5 (d, C-10), 130.9 (s, C-8), 132.5 (s, C-14), 136.4 (d, C-18), 149.1 (d, C-16), 145.0 (d, C-15), 161.1 (ds, $^1J_{C,F}$ = 256.4 Hz, C-4), 163.4 (s, C-11), 166.9 (s, C-1). $^{19}$**F NMR** (282 MHz, $CDCl_3$): δ -98.4 (ddd, $^3J_{H,F}$ = 49.6 Hz, $^3J_{H,F}$ = 17.9 Hz, $^3J_{H,F}$ = 17.6 Hz, 4-CF). **Exact mass** (ESI$^+$): $C_{22}H_{28}FN_3O_5S$ + $H^+$, calcd. 466.1812, found 466.1819; $C_{22}H_{28}FN_3O_5S$ + $Na^+$, calcd. 488.1631, found 488.1634; $(C_{22}H_{28}FN_3O_5S)_2$ + $H^+$, calcd. 931.3546, found 931.3565; $(C_{22}H_{28}FN_3O_5S)_2$ + $Na^+$, calcd. 953.3384, found 953.3384. **MS** (ESI$^+$, daughter ion experiment): *m/z* (%) 466 (18) [$M^+$ + $H^+$], 410 (100) [466 - $C_4H_8$], 349 (13) [466 - $H^+$ - CONHOC$(CH_3)_3$], 179 (11) [410 - $C_7H_7O_3S^+$ - $C_3H_4F$], 171 (30) [$C_7H_7O_3S^+$], 92 (8) [$C_6H_6N^+$]. **Optical rotation**:

$$[\alpha]_{589}^{20} = -6.5, [\alpha]_{578}^{20} = -6.8, [\alpha]_{546}^{20} = -7.9, [\alpha]_{436}^{20} = -14.3, [\alpha]_{365}^{20} = -24.9 \quad \text{(c = 0.996, CHCl}_3\text{)}.$$

**Example 19b**. *Tert*-butyl (*R*)-*N*-(4-methoxyphenylsulfonyl)-*N*-(3-pyridylmethyl)-2-amino-4-fluoropent-4-enhydroxamate

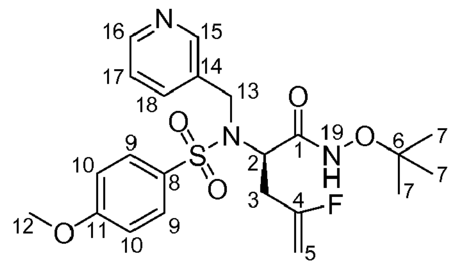

According to the general procedure (*R*)-*N*-(4-methoxyphenylsulfonyl)-*N*-(3-pyridylmethyl)-2-amino-4-fluoro-pent-4-enoic acid (150 mg, 0.38 mmol) was reacted with HOBT (52 mg, 0.38 mmol), NMM (193 mg, 1.90 mmol), *O*-*tert*-butylhydroxylamine (144 mg, 1.14 mmol) and EDC (95 mg, 0.50 mmol) in dichloromethane (15 mL). After column chromatography (column: Ø 2 cm × 10 cm) the product was isolated as white solid. **Yield**: 112 mg (63 %). **M.p**. 59-60 ˚C. $^1$**H NMR** (300 MHz, $CDCl_3$): δ 1.24 (s, 9 H, 7-$CH_3$), 2.27 (m, 1 H, 3-$CH_A$), 2.79 (ddd, $^3J_{H,F}$ = 19.8 Hz, $^2J_{H,H}$ = 14.7 Hz, $^3J_{H,H}$ = 8.3 Hz, 1 H, 3-$CH_B$), 3.86 (s, 3 H, 12-$CH_3$), 4.15 (dd, $^3J_{H,F}$ = 49.8 Hz, $^2J_{H,H}$ = 3.1 Hz, 1 H, 5-$CH_{trans}$), 4.48 (dd, $^3J_{H,F}$ = 17.0 Hz, $^2J_{H,H}$ = 3.2 Hz, 1 H, 5-$CH_{cis}$), 4.50 (d, $^2J_{H,H}$ = 16.1 Hz, 1 H, 13-$CH_A$), 4.56 - 4.66 (m, 2 H, 2-CH and 13-$CH_B$), 6.93 (d, $^3J_{H,H}$ = 9.0 Hz, 2 H, 9-CH), 7.21 (dd, $^3J_{H,H}$ = 7.8 Hz, $^3J_{H,H}$ = 4.9 Hz, 1 H, 17-CH), 7.62 - 7.73 (m, 3 H, 10-CH & 18-CH), 8.50 (dd, $^3J_{H,H}$ = 5.3 Hz, $^2J_{H,H}$ = 1.3 Hz, 1 H, 16-CH), 8.53 (d, $^2J_{H,H}$ = 1.3 Hz, 1 H, 15-CH), 8.72 (s, 1 H, 19-NH). $^{13}$**C NMR** (75 MHz, $CDCl_3$) δ 26.2 (q, C-7), 32.8 (dt, $^2J_{C,F}$ = 27.7 Hz, C-3), 45.8 (t, C-13), 54.8 (d, C-2), 55.7 (q, C-12), 82.5 (s, C-6), 94.0 (dt, $^2J_{C,F}$ = 18.2 Hz, C-5), 114.4 (d, C-9), 123.3 (d, C-17), 129.5 (d, C-10), 130.8 (s, C-8), 132.5 (s, C-14), 136.5 (d, C-18), 149.0 (d, C-16), 149.9 (d, C-15), 161.0 (ds, $^1J_{C,F}$ = 256.9 Hz, C-4), 163.4 (s, C-11), 166.9 (s, C-1). $^{19}$**F NMR** (282 MHz, $CDCl_3$): δ -98.4 (ddd, $^3J_{H,F}$ = 49.8 Hz, $^3J_{H,F}$ = 19.1 Hz, $^3J_{H,F}$ = 17.4 Hz, 4-CF). **Exact mass** ($ESI^+$): $C_{22}H_{28}FN_3O_5S + H^+$, calcd. 466.1812, found 466.1806; $C_{22}H_{28}FN_3O_5S + Na^+$,calcd. 488.1631, found 488.1626; $(C_{22}H_{28}FN_30_5S)_2 + H^+$, calcd. 931.3546, found 931.3519; **MS** ($ESI^+$, daughter ion experiment): *m*/*z* (%) 466 (40) [$M^+ + H^+$], 410 (100) [466 - $C_4H_8$], 349 (10) [466 - $H^+$ - $CONHOC(CH_3)_3$], 171 (5) [$C_7H_7O_3S^+$]. **Optical rotation**:

$[\alpha]^{20}_{589} = +8.5, [\alpha]^{20}_{578} = +8.7, [\alpha]^{20}_{546} = +10.1, [\alpha]^{20}_{436} = +18.2, [\alpha]^{20}_{365} = +31.8$ (c = 1.023, $CHCl_3$).

**General procedure for the hydrolysis of hydroxamates to hydroxamic acids**

In a dried YOUNG-tube, the corresponding hydroxamate is dissolved in fresh trifluoroacetic acid (50 mL/mmol) under argon. The YOUNG-tube is flashed with argon and closed. The solution is stirred at 40 ˚C for 12 h. Then the reaction mixture is evaporated to dryness in vacuum. The residue is dissolved in chloroform (100 mL/mmol) and washed with aqueous citric acid (pH ≈ 4) (25 mL/mmol) and bicarbonate. The aqueous phase is extracted with chloroform and the combined organic phases are dried with magnesium sulfate. After evaporation of the solvent the product is isolated as viscos oil.

**Example 20**

**Example 20a**. (*S*)-*N*-(4-Methoxyphenylsulfonyl)-*N*-(3-pyridylmethyl)-2-amino-butanhydroxamic acid

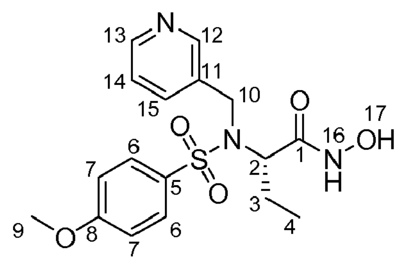

According to the general procedure *tert*-butyl (*S*)-*N*-(4-methoxyphenyl-sulfonyl)-*N*-(3-pyridylmethyl)-2-aminobutanhydroxamate (173 mg, 0.40 mmol) was hydrolyzed in trifluoroacetic acid (25 mL). After work up, the product was isolated as a cloudy, highly viscos liquid. After purification with preparative HPLC the product was 96 % pure. **Yield**: 60 mg (40 %). $^{1}$**H NMR** (300.13 MHz, $CD_3CN$): δ 0.71 (t, $^3J_{H,H}$ = 7.4 Hz, 3 H, 4-$CH_3$), 1.40 (m, 1 H, 3-$CH_A$), 1.66 (m, 1 H, 3-$CH_B$), 3.86 (s, 3 H, 9-$CH_3$), 4.18 (dd, $^3J_{H,H}$ = 8.7 Hz, $^3J_{H,H}$ = 6.6 Hz, 1 H, 2-CH), 4.74 (d, $^2J_{H,H}$ = 17.4 Hz, 1 H, 10-CHA), 4.86 (d, $^2J_{H,H}$ = 17.4 Hz, 1 H, 10-$CH_B$), 7.03 (d, $^3J_{H,H}$ = 9.0 Hz, 2 H, 6-CH), 7.71 (d, $^3J_{H,H}$ = 9.1 Hz, 2 H, 7-CH), 7.91 (dd, $^3J_{H,H}$ = 8.1 Hz, $^3J_{H,H}$ = 5.8 Hz, 1 H, 14-CH), 8.50 (d, $^3J_{H,H}$ = 8.1 Hz, 1 H, 15-CH), 8.58 (d, $^3J_{H,H}$ = 5.7 Hz, 1 H, 13-CH), 8.77 (s, 1 H, 12-CH). $^{13}$**C NMR** (75.48 MHz, $CD_3CN$): □ 10.7 (q, C-4), 23.8 (t, C-3), 46.0 (t, C-10), 56.7 (q, C-9), 59.7 (d, C-2), 115.6 (d, C-6), 127.7 (d, C-14), 130.5 (d, C-7), 131.6 (s, C-5), 141.0 (d, C-12), 141.1 (s, C-11), 142.1 (d, C-13), 146.4 (d, C-15), 164.6 (s, C-9), 168.3 (s, C-1). **Exact mass** ($ESI^+$): $C_{17}H_{21}N_3O_5S + H^+$, calcd. 380.1280, found 380.1263; $C_{17}H_{21}N_3O_5S + Na^+$, calcd. 402.1100, found 402.1082. **MS** ($ESI^+$, daughter ion experiment): *m*/*z* (%) 380 (100) [$M^+ + H^+$], 319 (25) [$M^+$ - CONHOH], 228 (5) [319 - $C_6H_6N$], 171 (30) [$C_7H_7O_3S^+$], 92 (10) [$C_6H_6N^+$].

**Example 20b**.(*R*)-*N*-(4-Methoxyphenylsulfonyl)-*N*-(3-pyridylmethyl)-2-amino-butanhydroxamic acid

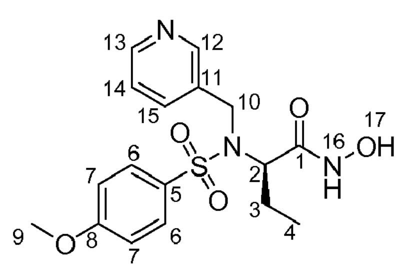

According to the general procedure *tert*-butyl (*R*)-*N*-(4-methoxyphenyl-sulfonyl)-*N*-(3-pyridylmethyl)-2-aminobutanhydroxamate (101 mg, 0.23 mmol) was hydrolyzed in trifluoroacetic acid (25 mL). After workup, the product was isolated as white, highly viscos liquid. HPLC showed 95 % purity of the product. **Yield**: 38 mg (43 %). $^{1}$**H NMR** (300 MHz, $CD_3CN$): δ 0.72 (t, $^3J_{H,H}$ = 7.4 Hz, 3 H, 4-$CH_3$), 1.40 (m, 1 H, 3-$CH_A$), 1.76 (m, 1 H, 3-$CH_B$), 3.85 (s, 3 H, 9-$CH_3$), 4.17 (dd, $^3J_{H,H}$ = 8.7 Hz, $^3J_{H,H}$ = 6.6 Hz, 1 H, 2-CH), 4.75 (d, $^2J_{H,H}$ = 17.4 Hz, 1H, 10-$CH_A$), 4.83 (d, $^2J_{H,H}$ = 17.3 Hz, 1 H, 10-$CH_B$), 7.01 (d, $^3J_{H,H}$ = 9.0 Hz, 2 H, 6-CH), 7.69 (d, $^3J_{H,H}$ = 9.0 Hz, 2 H, 7-CH), 7.88 (dd, $^3J_{H,H}$ = 8.0 Hz, $^3J_{H,H}$ = 5.8 Hz, 1 H, 14-CH), 8.44 (d, $^3J_{H,H}$ = 8.1 Hz, 1 H, 15-CH), 8.59 (d, $^3J_{H,H}$ = 5.5 Hz, 1 H, 13-CH), 8.77 (s, 1 H, 12-CH). $^{13}$**C NMR** (75 MHz, $CD_3CN$): δ 10.6 (q, C-4), 23.7 (t, C-3), 46.9 (t, C-10), 56.6 (q, C-9), 59.7 (d, C-2), 115.6 (d, C-6), 127.5 (d, C-14), 130.4 (d, C-7), 131.7 (s, C-5), 140.8 (d, C-12), 141.1 (s, C-11), 142.4 (d, C-13), 146.0 (d, C-15), 164.6 (s, C-9), 168.2 (s, C-1). **Exact mass** ($ESI^+$): $C_{17}H_{21}N_3O_5S + H^+$, caldc. 380.1280, found 380.1278; $C_{17}H_{21}N_3O_5S + Na^+$, calcd. 402.1100, found 402.1094. **MS** ($ESI^+$, daughter ion experiment): *m*/*z* (%) 759 (100) [$2M^+ + H^+$], 380 (18) [$M^+ + H^+$], 279 (13), 227 (13) [$C_{10}H_{13}NO_3S^+$], 155 (8) [$C_7H_7O_2S^+$].

**Example 21**

**Example 21a**. (*S*)-*N*-(4-Methoxyphenylsulfonyl)-*N*-(3-pyridylmethyl)-2-aminopent-4-enhydroxamic acid

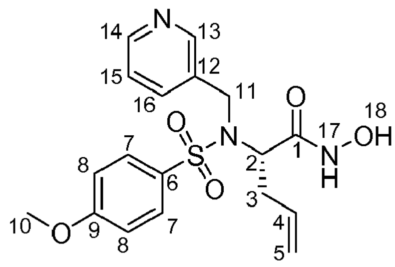

According to the general procedure *tert*-butyl (*S*)-*N*-(4-methoxyphenyl-sulfonyl)-*N*-(3-pyridylmethyl)-2-aminopent-4-enhydroxamate (300 mg, 0.67 mmol) was hydrolyzed in trifluoroacetic acid (25 mL). After workup, the product was isolated as white, highly viscos liquid. HPLC showed 96 % purity of the product. **Yield**: 77 mg (30 %). $^{1}$**H NMR** (400 MHz, $CD_3CN$): δ 2.14 (dt, $^{2}J_{H,H}$ = 14.3 Hz, $^{3}J_{H,H}$ = 6.8 Hz, 1 H, 3-$CH_A$), 2.45 (dt, $^{2}J_{H,H}$ = 14.2 Hz, $^{3}J_{H,H}$ = 7.7 Hz, 1 H, 3-$CH_B$), 3.84 (s, 3 H, 10-$CH_3$), 4.31 (t, $^{3}J_{H,H}$ = 7.6 Hz, 1 H, 2-CH), 4.61 (d, $^{2}J_{H,H}$ = 16.8 Hz, 1 H, 11-$CH_A$), 4.73 (d, $^{2}J_{H,H}$ = 16.8 Hz, 1 H, 11-$CH_B$), 4.92 (s, 1 H, 5-$CH_A$), 4.95 (d, $^{3}J_{H,H}$ = 5.6 Hz, 1 H, 5-$CH_B$), 5.47 (ddt, $^{3}J_{H,H}$ = 17.3 Hz, $^{3}J_{H,H}$ = 10.4 Hz, $^{3}J_{H,H}$ = 6.9 Hz, 1 H, 4-CH), 7.00 (d, $^{3}J_{H,H}$ = 8.8 Hz, 2 H, 7-CH), 7.30 (dd, $^{3}J_{H,H}$ = 7.6 Hz, $^{3}J_{H,H}$ = 4.9 Hz, 1 H, 15-CH), 7.70 (d, $^{3}J_{H,H}$ = 8.9 Hz, 2 H, 8-CH), 7.81 (d, $^{3}J_{H,H}$ = 7.8 Hz, 1 H, 16-CH), 8.41 (s, 1 H, 14-CH), 8.53 (s, 1 H, 13-CH). $^{13}$**C NMR** (100 MHz, $CD_3CN$): δ 35.2 (t, C-3), 46.8 (t, C-11), 56.6 (q, C-10), 58.0 (d, C-2), 115.4 (d, C-7), 119.1 (t, C-5), 124.5 (d, C-15), 130.5 (d, C-8), 132.1 (d, C-4), 133.9 (s, C-12), 135.9 (s, C-6), 137.7 (d, C-16), 148.5 (d, C-14), 149.5 (d, C-13), 164.4 (s, C-9), 167.4 (s, C-1). **Exat mass** ($ESI^+$): $C_{18}H_{21}N_3O_5S + H^+$, calcd. 392.1280, found 392.1271; $C_{18}H_{21}N_30_5S + Na^+$,calcd. 414.1100, found 414.1094. **MS** ($ESI^+$, daughter ion experiment): *m*/*z* (%) 392 (10) [$M^+ + H^+$], 331 (15) [$M^+$ - CONHOH], 279 (4) [$C_{13}H_{15}N_2O_3S^+$], 240 (8) [331 - $C_5H_4N$], 171 (100) [$C_7H_7O_3S^+$], 161 (30) [$C_{10}H_{13}N_2^+$], 92 (45) [$C_6H_6N^+$].

**Example 21b**.(*R*)-*N*-(4-Methoxyphenylsulfonyl)-*N*-(3-pyridylmethyl)-2-aminopent-4-enhydroxamic acid.

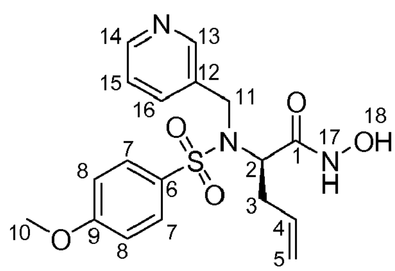

According to the general procedure *tert*-butyl (*R*)-*N*-(4-methoxyphenyl-sulfonyl)-*N*-(3-pyridylmethyl)-2-aminopent-4-enhydroxamate (100 mg, 0.22 mmol) was hydrolyzed in trifluoroacetic acid (25 mL). After workup, the product was isolated as white, highly viscos liquid. HPLC showed 97 % purity of the product. **Yield**: 33 mg (38 %). $^{1}$**H NMR** (400 MHz, $CD_3CN$): δ 2.15 (dt, $^{2}J_{H,H}$ = 14.1 Hz, $^{3}J_{H,H}$ = 6.9 Hz, 1 H, 3-$CH_A$), 2.46 (dt, $^{2}J_{H,H}$ = 14.1 Hz, $^{3}J_{H,H}$ = 7.6 Hz, 1 H, 3-$CH_B$), 3.84 (s, 3 H, 10-$CH_3$), 4.29 (t, $^{3}J_{H,H}$ = 7.6 Hz, 1 H, 2-CH), 4.60 (d, $^{2}J_{H,H}$ = 16.7 Hz, 1 H, 11-$CH_A$), 4.72 (d, $^{2}J_{H,H}$ = 16.7 Hz, 1 H, 11-$CH_B$), 4.93 (s, 1 H, 5-$CH_A$), 4.96 (d, $^{3}J_{H,H}$ = 4.5 Hz, 1 H, 5-$CH_B$), 5.48 (ddt, $^{3}J_{H,H}$ = 16.8 Hz, $^{3}J_{H,H}$ = 9.6 Hz, $^{3}J_{H,H}$ = 6.9 Hz, 1 H, 4-CH), 7.00 (d, $^{3}J_{H,H}$ = 8.7 Hz, 2 H, 7-CH), 7.29 (dd, $^{3}J_{H,H}$ = 7.8 Hz, $^{3}J_{H,H}$ = 4.8 Hz, 1 H, 15-CH), 7.70 (d, $^{3}J_{H,H}$ = 8.9 Hz, 2 H, 8-CH), 7.79 (m, 1 H, 16-CH), 8.41 (d, $^{3}J_{H,H}$ = 4.4 Hz, 1 H, 14-CH), 8.52 (s, 1 H, 13-CH). $^{13}$**C NMR** (100 MHz, $CD_3CN$): □ 35.2 (t, C-3), 46.8 (t, C-11), 56.6 (q, C-10), 58.0 (d, C-2), 115.4 (d, C-7), 119.1 (t, C-5), 124.5 (d, C-15), 130.5 (d, C-8), 132.1 (d, C-4), 134.0 (s, C-12), 135.8 (s, C-6), 137.5 (d, C-16), 148.6 (d, C-14), 149.7 (d, C-13), 164.4 (s, C-9), 167.3 (s, C-1). **Exact mass** ($ESI^+$): $C_{18}H_{21}N_3O_5S + H^+$, calcd. 392.1280, found 392.1275. **MS** ($ESI^+$, daughter ion experiment): *m*/*z* (%) 392 (30) [$M^+ + H^+$], 331 (35) [$M^+$ - CONHOH], 279 (8) [$C_{13}H_{15}N_2O_3S^+$], 240 (20) [331 - $C_5H_4N$], 171 (100) [$C_7H_7O_3S^+$], 161 (55) [$C_{10}H_{13}N_2^+$], 124 (27) [$C_7H_8O_2^+$], 107 (11) [$C_7H_7O^+$], 92 (42) [$C_6H_6N^+$].

**Example 22**

**Example 22a.** (*S*)-*N*-(4-Methoxyphenylsulfonyl)-*N*-(3-pyridylmethyl)-2-amino-4-fluorobutanhydroxamic acid ((*S*)-155)

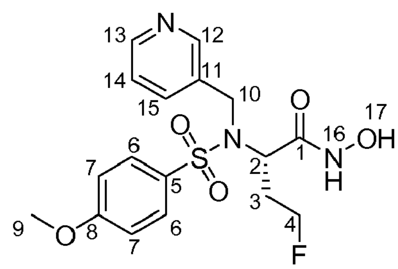

According to the general procedure *tert*-butyl (*S*)-*N*-(4-methoxyphenyl-sulfonyl)-*N*-(3-pyridylmethyl)-2-amino-4-fluorobutanhydroxamate (131 mg, 0.29 mmol) was hydrolyzed in trifluoroacetic acid (25 mL). After workup, the product was isolated as white, highly viscos liquid. Purification by preparative HPLC delivered a product of 95 % purity. **Yield**: 49 mg (42 %). $^1$**H NMR** (600 MHz, $CD_3CN$): δ 1.76 (m, $^3J_{H,F}$ = 23.7 Hz, 1 H, 3-$CH_A$), 2.14 (ddd, $^3J_{H,F}$ = 29.1 Hz, $^2J_{H,H}$ = 11.8 Hz, $^3J_{H,H}$ = 6.1 Hz, 1 H, 3-$CH_B$), 3.85 (s, 3 H, 9-$CH_3$), 4.20 - 4.42 (m, 2 H, 4-$CH_2F$), 4.48 (dd, $^3J_{H,H}$ = 8.9 Hz, $^3J_{H,H}$ = 6.1 Hz, 1 H, 2-CH), 4.76 (d, $^2J_{H,H}$ = 17.1 Hz, 1 H, 10-$CH_A$), 4.81 (d, $^2J_{H,H}$ = 17.1 Hz, 1 H, 10-$CH_B$), 7.01 (d, $^3J_{H,H}$ = 8.9 Hz, 2 H, 6-CH), 7.68 (d, $^3J_{H,H}$ = 8.9 Hz, 2 H, 7-CH), 7.88 (dd, $^3J_{H,H}$ = 8.1 Hz, $^3J_{H,H}$ = 5.7 Hz, 1 H, 14-CH), 8.43 (d, $^3J_{H,H}$ = 7.7 Hz, 1 H, 15-CH), 8.58 (d, $^3J_{H,H}$ = 5.3 Hz, 1 H, 12-CH), 8.74 (s, 1 H, 13-CH). $^{13}$**C NMR** (150 MHz, $CD_3CN$): δ 31.3 (dt, $^2J_{C,F}$ = 20.0 Hz, C-3), 46.1 (t, C-10), 55.0 (dd, $^3J_{C,F}$ = 3.6 Hz, C-2), 56.78 (q, C-9), 81.4 (dt, $^1J_{C,F}$ = 164.2 Hz, C-4), 115.7 (d, C-6), 127.6 (d, C-14), 130.2 (d, C-7), 131.4 (s, C-5), 140.4 (s, C-11), 141.2 (d, C-12), 142.3 (d, C-13), 146.1 (d, C-15), 164.7 (s, C-8), 167.3 (s, C-1). $^{19}$**F NMR** (564 MHz, $CD_3CN$): δ -221.4 (tdd, $^2J_{H,F}$ = 47.1 Hz, $^3J_{H,F}$ = 29.0 Hz, $^3J_{H,F}$ = 23.7 Hz, 4-$CH_2F$). **Exact mass** ($ESI^+$): $C_{17}H_{20}FN_3O_5S^+$ + $H^+$, calcd. 398.1186, found 398.1181. $C_{17}H_{20}FN_3O_5S^+$ + $Na^+$, calcd. 420.1105, found 420.0999; $(C_{17}H_{20}FN_3O_5S^+)_2$ + $H^+$, calcd. 795.2294, found 795.2300; $(C_{17}H_{20}FN_3O_5S^+)_2$ + $Na^+$, calcd. 817.2113, found 817.2116. **MS** ($ESI^+$, daughter ion experiment): *m*/*z* (%)398 (10) [$M^+$ + $H^+$], 337 (16) [$M^+$ - CONHOH], 171 (100) [$C_7H_7O_3S^+$], 167 (30) [337 - $C_7H_7O_3S$], 135 (16) [$C_8H_{11}N_2^+$], 123 (22) [$C_7H_7O2^+$], 107 (5) [$C_7H_7O^+$], 92 (52) [$C_6H_6N^+$].

**Example 22b**.(*R*)-*N*-(4-Methoxyphenylsulfonyl)-*N*-(3-pyridylmethyl)-2-amino-4-fluorobutanhydroxamic acid

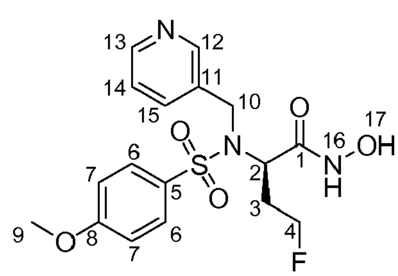

According to the general procedure *tert*-butyl (*R*)-*N*-(4-methoxyphenyl-sulfonyl)-*N*-(3-pyridylmethyl)-2-amino-4-fluorobutanhydroxamate (104 mg, 0.23 mmol) was hydrolyzed in trifluoroacetic acid (25 mL). After workup, the product was isolated as white, highly viscos liquid. Purification by preparative HPLC delivered a product of 95 % purity. **Yield**: 45 mg (49 %). $^1$**H NMR** (400 MHz, $CD_3CN$): δ 1.67 (m, 1 H, 3-$CH_A$), 2.13 (m, 1 H, 3-$CH_B$), 3.86 (s, 3 H, 9-$CH_3$), 4.17 - 4.46 (m, 2 H, 4-$CH_2F$), 4.49 (dd, $^3J_{H,H}$ = 8.8 Hz, $^3J_{H,H}$ = 6.1 Hz, 1 H, 2-CH), 4.75 (d, $^2J_{H,H}$ = 17.3 Hz, 1 H, 10-$CH_A$), 4.86 (d, $^2J_{H,H}$ = 17.3 Hz, 1 H, 10-$CH_B$), 7.04 (d, $^3J_{H,H}$ = 9.0 Hz, 2 H, 6-CH), 7.71 (d, $^3J_{H,H}$ = 9.0 Hz, 2 H, 7-CH), 7.92 (dd, $^3J_{H,H}$ = 8.0 Hz, $^3J_{H,H}$ = 5.9 Hz, 1 H, 14-CH), 8.50 (d, $^3J_{H,H}$ = 8.2 Hz, 1 H, 15-CH), 8.59 (d, $^3J_{H,H}$ = 5.6 Hz, 1 H, 13-CH), 8.75 (s, 1 H, 12-CH). $^{13}$**C NMR** (100 MHz, $CD_3CN$): δ [ppm] 31.5 (dt, $^2J_{C,F}$ = 20.1 Hz, C-3), 46.2 (t, C-10), 55.0 (dd, $^3J_{C,F}$ = 3.4 Hz, C-2), 56.7 (q, C-9), 81.3 (dt, $^1J_{C,F}$ = 164.2 Hz, C-4), 115.7 (d, C-6), 127.9 (d, C-14), 130.6 (d, C-7), 131.3 (s, C-5), 140.8 (s, C-11), 141.2 (s, C-13), 142.1 (s, C-12), 146.7 (s, C-15), 164.8 (s, C-8), 167.3 (s, C-1). $^{19}$**F NMR** (282 MHz, $CD_3CN$): δ [ppm] -222.2 (tdd, $^2J_{H,F}$ = 47.2 Hz, $^3J_{H,F}$ = 31.5 Hz, $^3J_{H,F}$ = 22.3 Hz, 4-$CH_2F$). **Exact mass** ($ESI^+$):

$C_{17}H_{20}N_3O_5S$ + $H^+$, calcd. 398.1186, found 398.1171; $C_{17}H_{20}N_3O_5S$ + $Na^+$, calcd. 420.1005, found 420.0990; $(C_{17}H_{20}N_3O_5S)_2$ + $H^+$, calcd. 795.2294, found 871.3691; $(C_{17}H_{20}N_3O_5S)_2$ + $Na^+$, calcd.817.2113, found 893.3507. **MS** ($ESI^+$, daughter ion experiment): *m/z* (%) 398 (60) [$M^+$ + $H^+$], 337 (100) [$M^+$ - CONHOH], 317 (7) [337 - HF], 246 (6), 171 (20) [$C_7H_7O_3S^+$].

**Example 23**

**Example 23a**. (*S*)-*N*-(4-Methoxyphenylsulfonyl)-*N*-(3-pyridylmethyl)-2-amino-4-fluoropent-4-enhydroxamic acid

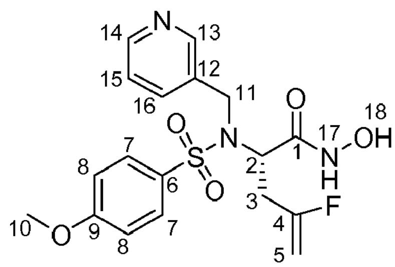

According to the general procedure *tert*-butyl (*S*)-*N*-(4-methoxyphenyl-sulfonyl)-*N*-(3-pyridylmethyl)-2-amino-4-fluoropent-4-enhydroxamate (107 mg, 0.23 mmol) was hydrolyzed in trifluoroacetic acid (25 mL). After workup, the product was isolated as white, highly viscos liquid. Purification by preparative HPLC delivered a product of 97 % purity. **Yield**: 62 mg (66 %).$^1$**H NMR** (400 MHz, $CD_3CN$): δ 2.35 (ddd, $^3J_{H,F}$ = 18.1 Hz, $^2J_{H,H}$ = 14.9 Hz, $^3J_{H,H}$ = 6.6 Hz, 1 H, 3-$CH_A$), 2.74 (ddd, $^3J_{H,F}$ = 18.9 Hz, $^2J_{H,H}$ = 14.8 Hz, $^3J_{H,H}$ = 8.4 Hz, 1 H, 3-$CH_B$), 3.85 (s, 3 H, 10-$CH_3$), 4.34 (dd, $^3J_{H,F}$ = 50.3 Hz, $^2J_{H,H}$ = 3.3 Hz, 1 H, 5-$CH_{trans}$), 4.56 (dd, $^3J_{H,F}$ = 17.3 Hz, $^2J_{H,H}$ = 3.3 Hz, 1 H, 5-$CH_{cis}$), 4.57 (dd, $^3J_{H,H}$ = 8.2 Hz, $^3J_{H,H}$ = 6.5 Hz, 1 H, 2-CH), 4.81 (s, 2 H, 11-$CH_2$), 7.01 (d, $^3J_{H,H}$ = 9.0 Hz, 2 H, 7-CH), 7.66 (d, $^3J_{H,H}$ = 9.0 Hz, 2 H, 8-CH), 7.86 (dd, $^3J_{H,H}$ = 8.0 Hz, $^3J_{H,H}$ = 5.8 Hz, 1 H, 15-CH), 8.41 (d, $^3J_{H,H}$ = 8.2 Hz, 1 H, 16-CH), 8.59 (d, $^3J_{H,H}$ = 5.5 Hz, 1 H, 14-CH), 8.73 (s, 1 H, 13-CH). $^{13}$**C NMR** (100 MHz, $CD_3CN$): δ 33.2 (dt, $^2J_{C,F}$ = 28.0 Hz, C-3), 46.1 (t, C-11), 55.5 (d, C-2), 56.7 (q, C-10), 94.5 (dt, $^2J_{C,F}$ = 18.7 Hz, C-5), 115.7 (d, C-7), 127.5 (d, C-15), 130.5 (d, C-8), 131.5 (s, C-6), 140.1 (s, C-12), 141.4 (d, C-14), 142.6 (d, C-13), 145.7 (d, C-16), 162.5 (ds, $^1J_{C,F}$ = 255.6 Hz, C-4), 164.7 (s, C-9), 166.8 (s, C-1). $^{19}$**F NMR** (282 MHz, $CD_3CN$): δ -97.3 (ddd, $^3J_{H,F}$ = 50.2 Hz, $^3J_{H,F}$ = 18.2 Hz, $^3J_{H,F}$ = 17.7 Hz, 4-CF). **Exact mass** ($ESI^+$): $C_{18}H_{20}FN_3O_5S^+$ + $H^+$, calcd. 410.1186, found 410.1174; $(C_{18}H_{20}FN_3O_5S^+)_2$ + $H^+$, caldc. 819.2294, found 819.2278; $(C_{18}H_{20}FN_3O_5S^+)_2$ + $Na^+$, calcd. 841.2113, found 841.2096. **MS** ($ESI^+$, daughter ion experiment): *m/z* (%) 410 (5) [$M^+$ + $H^+$], 349 (11) [$M^+$ - CONHOH], 179 (23) [349 - $C_7H_7O_3S^+$], 171 (100) [$C_7H_7O_3S^+$], 135 (18) [$C_8H_{11}N_2^+$], 123 (20) [$C_7H_7O_2^+$], 92 (50) [$C_6H_6N^+$].

**Example 23b**.(*R*)-*N*-(4-Methoxyphenylsulfonyl)-*N*-(3-pyridylmethyl)-2-amino-4-fluoropent-4-enhydroxamic acid

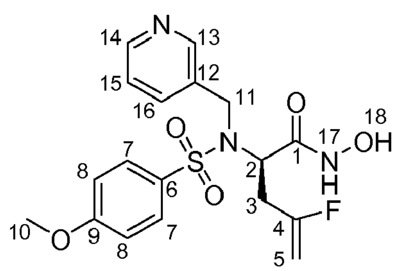

According to the general procedure *tert*-butyl (*R*)-*N*-(4-methoxyphenylsulfonyl)-*N*-(3-pyridylmethyl)-2-amino-4-fluoropent-4-enhydroxamate (112 mg, 0.24 mmol) was hydrolyzed in trifluoroacetic acid (25 mL). After workup, the product was isolated as white, highly viscos liquid. Purification by preparative HPLC delivered a product of 97 % purity. **Yield**: 57 mg (58 %): $^1$**H NMR** (300 MHz, $CD_3CN$): δ 2.35 (ddd, $^3J_{H,F}$ = 18.1 Hz, $^2J_{H,H}$ = 14.9 Hz, $^3J_{H,H}$ = 6.7 Hz, 1 H, 3-$CH_A$), 2.74 (ddd, $^3J_{H,F}$ = 18.6 Hz, $^2J_{H,H}$ = 14.8 Hz, $^3J_{H,H}$ = 8.3 Hz, 1 H, 3-$CH_B$), 3.86 (s, 3 H, 10-$CH_3$), 4.34 (dd, $^3J_{H,F}$ = 50.3 Hz, $^2J_{H,H}$ = 3.2 Hz, 1 H, 5-$CH_{trans}$), 4.56 (dd, $^3J_{H,F}$ = 17.3 Hz, $^2J_{H,H}$ = 3.2 Hz, 1 H, 5-$CH_{cis}$), 4.59 (t, $^3J_{H,H}$ = 7.5

Hz, 1 H, 2-CH), 4.79 (d, $^2J_{H,H}$ = 17.5 Hz, 1 H, 11-$CH_A$), 4.86 (d, $^2J_{H,H}$ = 17.5 Hz, 1 H, 11-$CH_B$), 7.01 (d, $^3J_{H,H}$ = 9.0 Hz, 2 H, 7-CH), 7.68 (d, $^3J_{H,H}$ = 9.0 Hz, 2 H, 8-CH), 7.89 (dd, $^3J_{H,H}$ = 8.0 Hz, $^3J_{H,H}$ = 5.8 Hz, 1 H, 15-CH), 8.45 (d, $^3J_{H,H}$ = 8.1 Hz, 1 H, 16-CH), 8.59 (d, $^3J_{H,H}$ = 5.5 Hz, 1 H, 14-CH), 8.74 (s, 1 H, 13-CH). **$^{13}$C NMR** (75 MHz, $CD_3CN$): δ 33.2 (dt, $^2J_{C,F}$ = 27.9 Hz, C-3), 46.1 (t, C-11), 55.4 (d, C-2), 56.7 (q, C-10), 94.5 (dt, $^2J_{C,F}$ = 18.6 Hz, C-5), 115.7 (d, C-7), 127.6 (d, C-15), 130.6 (d, C-8), 131.3 (s, C-6), 140.4 (s, C-12), 141.2 (d, C-14), 142.2 (d, C-13), 146.1 (d, C-16), 162.4 (ds, $^1J_{C,F}$ = 255.5 Hz, C-4), 164.7 (s, C-9), 166.8 (s, C-1). **$^{19}$F NMR** (282 MHz, $CD_3CN$): δ -97.3 (ddd, $^3J_{H,F}$ = 50.3 Hz, $^3J_{H,F}$ = 18.2 Hz, $^3J_{H,F}$ = 17.5 Hz, 4-CF). **Exact mass** ($ESI^+$): $C_{18}H_{20}FN_3O_5S^+ + H^+$, calcd. 410.1186, found 410.1174; $C_{18}H_{20}FN_3O_5S^+ + Na^+$, calcd. 432.1005, found 432.0991; $(C_{18}H_{20}FN_3O_5S^+)_2 + H^+$:, calcd. 819.2294, found 819.2278; $(C_{18}H_{20}FN_3O_5S^+)_2 + Na^+$, calcd. 841.2113, found 841.2103. **MS** ($ESI^+$, daughter ion experiment): *m/z* (%)410 (6) [$M^+ + H^+$], 349 (10) [$M^+$ - CONHOH], 179 (22) [349 - $C_7H_7O_3S^+$], 171 (100) [$C_7H_7O_3S^+$], 135 (18) [$C_8H_{11}N_2^+$], 123 (22) [$C_7H_7O_2^+$], 92 (50) [$C_6H_6N^+$].

**General procedure for substitution with p-(2-halogenethoxy)-phenylsulfonyl chloride**

The *tert*-butyl 4-fluoro-2-aminopent-4-enecarboxylate is dissolved in pyridine and treated with 1 equivalent of *p*-(2-halogenethoxy)phenylsulfonyl chloride under stirring at 0 °C. The reaction mixture is allowed to warm up to r.t. and stirred for 40 h. Then the mixture is diluted with dichloromethane and the organic solution is washed with 0.5 N HCl, water (2 times each) and saturated aqueous sodium chloride. After drying with magnesium sulfate the solvent was removed in vacuum. The product is passed through a short pad of silica gel and recrystallized from ethyl acetate/ cyclohexane. The products are isolated as white crystalline solids.

**Example 24**

**Example 24a:** 2-Fluoroethyl 4-methylbenzenesulfonate (**KR 2**)

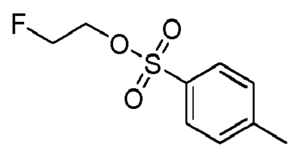

To a cooled solution (0 °C) of 2-fluoroethanol (21.1 mL, 22.99 g, 359 mmol) in pyridine (150 mL) 4-methylbenzene-1-sulfonyl chloride (1.1 eq., 75,3 g, 394 mmol) was added and the reaction mixture was stirred at room temperature for 2 d. Subsequently the mixture was diluted with acidified ice-water (600 mL water, 100 mL concentrated HCl) and extracted with chloroform (3 × 100 mL). The combined organic phases were washed with water and brine (100 mL) and the solvent was removed under reduced pressure. Column chromatographic purification (silica gel, cyclohexane/ ethyl acetate 2:1) gave a yellow oil. **Yield:** 29.46 g (38%). **$^1$H NMR** (300 MHz, $CDCl_3$) δ 7.81 (d, $^3J_{H,H}$= 8.4 Hz, Ph*H*, 2H), 7.36 (d, $^3J_{H,H}$= 8.6 Hz, Ph*H*, 2H), 4.57 (dm, $^2J_{H,F}$ = 47.1 Hz, $CH_2F$, 2H), 4.26 (dm, $^3J_{H,F}$= 27.3 Hz, $CH_2CH_2F$, 2H) 2.45 (s, $SO_2PhCH_3$, 3H). **$^{13}$C NMR** (75 MHz, $CDCl_3$) δ 145.13 ($qPhCCH_3$), 132.51 ($OSO_2CPh$), 129.89 (PhCH), 127.91 (PhCH), 80.50 (d, $^1J_{C,F}$ = 173.6 Hz, $CH_2F$), 68.45 (d, $^2J_{C,F}$ = 20.9 Hz, $CH_2CH_2F$), 21.61 ($SO_2PhCH_3$). **$^{19}$F NMR** (282 MHz, $CDCl_3$) δ -224.68 (tt, $^2J_{H,F}$= 47.1 Hz, $^3J_{H,F}$ = 27.3 Hz, 1 F). **MS-ES(+)-EM**: *m/z* = 241.0293 [$(M+Na)^+$] calcd for $C_9H_{11}FO_3SNa^+$: 241.0305.

**Example 24b:** (2-Fluoroethoxy)benzene (**KR 18**)

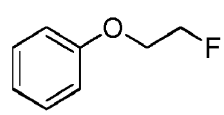

To a solution of phenol (10.75 g, 114 mmol) and 2-fluoroethyl 4-methylbenzenesulfonate (**KR 2**, 24.93 g, 114 mmol) in water (125 mL) NaOH (52.5 g) Tetraoctylammoniumbromid (1.5 g) was added and the mixture was heated to reflux for 16 h. The reaction mixture was diluted with ethyl acetate (100 ml) and the precipitated solid was removed by filtration. The organic layer was washed with 1 N aqueous NaOH, water and brine, dried over $MgSO_4$ and the solvent

was removed under reduced pressure. After column chromatographic purification (silica gel, cyclohexane/ethyl acetate 9:1) the product was obtained as a yellow oil. Yield: 10.04 g (63%). **$^1$H NMR** (300 MHz, $CDCl_3$) δ 7.43 - 7.19 (m, Ph*H*, 2H), 7.09 - 6.85 (m, Ph*H*, 3H), 4.77 (dm, $^2J_{H,F}$= 47.4 Hz, C*H*$_2$F, 2H), 4.23 (dm, $^3J_{H,F}$= 27.9 Hz, C*H*$_2$CH$_2$F, 2H). **$^{13}$C NMR** (75 MHz, $CDCl_3$) δ 158.30 (qPh*C*OCH$_2$CH$_2$), 129.47 (PhCH), 121.20 (PhCH), 114.53 (PhCH), 81.91 (d, $^1J_{C,F}$ = 170.5 Hz, CH$_2$F), 66.93 (d, $^2J_{C,F}$ = 20.5 Hz, CH$_2$CH$_2$F). **$^{19}$F NMR** (282 MHz, $CDCl_3$) δ -223.89 (tt, $^2J_{H,F}$= 47.3 Hz, $^3J_{H,F}$ = 27.6 Hz, 1 F). **MS-ES(+)-EM:** *m*/*z* = 163.0534 [(M+Na)$^+$] calcd for $C_8H_9FONa^+$: 163.0530.

**Example 24c:***p*-(2-Fluoroethoxy)phenylsulfonylchloride (**KR 26**)

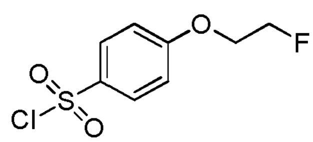

(2-Fluoroethoxy)benzene (**KR 18**, 19 g, 136 mmol) was dissolved in dichloromethane (200 mL) and cooled to 0 ˚C. $HClSO_3$ was added dropwise to the solution. After the gas formation has stopped ice-water (500 mL) was given to the reaction mixture. The aqueous phase was extracted with diethylether (3 × 150 mL) and the combined organic layer was dried over $MgSO_4$. The solvent was removed under reduced pressure and the product was obtained as a yellow oil. **Yield:** 21 g (64%). **1H NMR** (300 MHz, $CDCl_3$) δ 7.99 (d, $^3J_{H,H}$= 9.1 Hz, Ph*H*, 2H), 7.09 (d, $^3J_{H,H}$= 9.1 Hz, Ph*H*, 2H), 4.81 (dm, $^2J_{H,F}$= 47.3 Hz, C*H*$_2$F, 2H), 4.34 (dm, $^3J_{H,F}$= 27.5 Hz, C*H*$_2$CH$_2$F, 2H). **13C NMR** (75 MHz, $CDCl_3$) δ 163.60 (PhCO), 136.60 (OSO$_2$*C*Ph), 129.58 (PhCH), 115,20 (PhCH), 81.29 (d, $^1J_{C,F}$ = 172.2 Hz, *C*H$_2$F), 67.71 (d, $^2J_{C,F}$ = 20.5 Hz, CH$_2$CH$_2$F). **19F NMR** (282 MHz, $CDCl_3$) δ -223.84 (tt, $^2J_{H,F}$= 47.3 Hz, $^3J_{H,F}$ = 27.4 Hz, 1F). **MS- ES (+)-EM:** *m*/*z* = 260.9750 [(M+Na)$^+$] calcd for $C_8H_8FO_3SClNa^+$: 260.9759.

**Example 24d:** Compound MAB 239

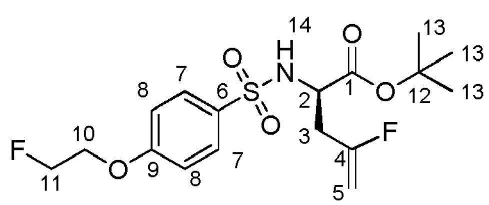

Prepared from *tert*-butyl (*R*)-2-amino-4-fluoropent-4-enecarboxylate (1000 mg, 5.28 mmol) and *p*-(2-fluorethoxy)phenylsulfonyl chloride (1225 mg, 5.28 mmol). **Yield:** 1.32 g (64 %). **M.p.** 102 ˚C (EtOAc/Cy). **$^1$H NMR** (300 MHz, $CDCl_3$): δ = 7.79 (dm, $^3J_{H,H}$= 9.0 Hz, 2H, 8-CH), 6.99 (dm, $^3J_{H,H}$ = 9.0 Hz, 2H, 7-CH), 5.37 (d, $^3J_{H,H}$ = 8.9 Hz, 1 H, 14-NH), 4.78 (dm, $^2J_{H,F}$ = 47.3 Hz, 2H, 11-CH$_2$), 4.64 (dd, $^3J_{H,F}$ = 17.0 Hz, $^2J_{H,H}$ = 3.1 Hz, 1 H, 5-CH$_{cis}$), 4.35 (dd, $^3J_{H,F}$ = 49.3 Hz, $^2J_{H,H}$ = 3.1 Hz, 1 H, 5-CH$_{trans}$), 4.26 (dm, $^3J_{H,F}$ = 27.7 Hz, 2H, 10-CH$_2$), 3.98 (dt, $^3J_{H,H}$ = 9.0 Hz, $^3J_{H,H}$ = 5.9 Hz, 1 H, 2-CH), 2.65 (d, $^3J_{H,H}$ = 5.9 Hz, 1 H, 3-CH$_A$), 2.59 (dd, $^3J_{H,H}$ = 5.7 Hz, $^3J_{H,H}$ = 4.2 Hz, 1 H, 3-CH$_B$), 1.30 (s, 9H, 13-CH$_3$). **$^{13}$C NMR** (75 MHz, $CDCl_3$): δ = 169.2 (s, C-1), 161.8 (s, C-9), 160.9 (ds, $^1J_{C,F}$ = 257.1 Hz, C-4), 131.9 (s, C-6), 129.5 (d, C-8), 114.7 (d, C-7), 94.1 (dt, $^2J_{C,F}$ = 18.8 Hz, C-5), 83.2 (s, C-12), 81.5 (dt, $^1J_{C,F}$ = 171.5 Hz, C-11), 67.4 (dt, $^2J_{C,F}$ = 20.4 Hz, C-10), 53.2 (d, C-2), 36.4 (dt, $^2J_{C,F}$ = 27.4 Hz, C-3), 27.6 (q, C-13). **$^{19}$F NMR** (282 MHz, $CDCl_3$): δ = -96.1 (ddt, $^3J_{H,F}$ = 49.3 Hz, $^3J_{H,F}$ = 20.4 Hz, $^3J_{H,F}$ = 17.2 Hz, 4-CF), -224.3 (tt, $^2J_{H,F}$ = 47.3 Hz, $^3J_{H,F}$ = 27.8 Hz, 11-CH$_2$F). **Elemental analysis:** $C_{17}H_{23}F_2NO_5S$ (M = 391.43 g/mol), alcld. C 52.16, H 5.92, N 3.58; found C 52.64, H 6.24, N 3.55 %. **Exact mass** (ESI$^+$): $C_{17}H_{23}F_2NO_5S$ + Na$^+$: cald. 414.1163, found 414.1160. $(C_{17}H_{23}F_2NO_5S)_2$ + Na$^+$: cald. 805.2428, found 805.2423. **Optical rotation:**

$$[\alpha]_{589}^{20} = +6.8, [\alpha]_{578}^{20} = +7.7, [\alpha]_{546}^{20} = +8.6, [\alpha]_{436}^{20} = +19.7, [\alpha]_{365}^{20} = +44.5 \text{ (c = 1.023, CHCl}_3\text{)}$$

**Example 24b:** Compound MAB 240

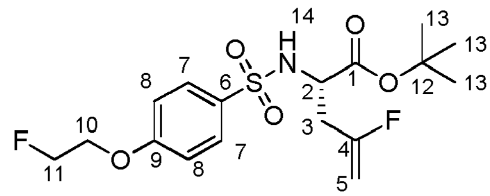

Prepared from *tert*-butyl (*S*)-2-amino-4-fluoropent-4-enecarboxylate (892 mg, 4.7 mmol) with *p*-(2-fluoroethoxy)-phenylsulfonylchloride (1130 mg, 4.7 mmol). **Yield:** 1.18 g (64 %). **M.p.** 102 °C (EtOAc/Cy). **$^1$H NMR** (300 MHz, $CDCl_3$): δ = 7.79 (dm, $^3J_{H,H}$ = 9.0 Hz, 2H, 8-CH), 6.99 (dm, $^3J_{H,H}$ = 9.0 Hz, 2H, 7-CH), 5.37 (d, $^3J_{H,H}$ = 9.0 Hz, 1H, 14-NH), 4.77 (dm, $^2J_{H,F}$ = 47.5 Hz, 2H, 11-$CH_2$), 4.64 (dd, $^3J_{H,F}$ = 17.1 Hz, $^2J_{H,H}$ = 3.1 Hz, 1H, 5-$CH_{cis}$), 4.35 (dd, $^3J_{H,F}$ = 49.3 Hz, $^2J_{H,H}$ = 3.1 Hz, 1H, 5-$CH_{trans}$), 4.26 (dm, $^3J_{H,F}$ = 27.7 Hz, 2H, 10-$CH_2$), 3.98 (dt, $^3J_{H,H}$ = 8.8 Hz, $^3J_{H,H}$ = 5.8 Hz, 1H, 2-CH), 2.65 (d, $^3J_{H,H}$ = 5.9 Hz, 1H, 3-$CH_A$), 2.59 (dd, $^3J_{H,H}$ = 5.7 Hz, $^3J_{H,H}$ = 4.2 Hz, 1H, 3-$CH_B$), 1.30 (s, 9H, 13-$CH_3$). **$^{13}$C NMR** (75 MHz, $CDCl_3$): δ = 169.2 (s, C-1), 161.8 (s, C-9), 160.9 (ds, $^1J_{C,F}$ = 256.9 Hz, C-4), 131.9 (s, C-6), 129.5 (d, C-8), 114.7 (d, C-7), 94.1 (dt, $^2J_{C,F}$ = 18.8 Hz, C-5), 83.2 (s, C-12), 81.5 (dt, $^1J_{C,F}$ = 171.5 Hz, C-11), 67.4 (dt, $^2J_{C,F}$ = 20.4 Hz, C-10), 53.2 (d, $^3J_{C,F}$ = 1.0 Hz, C-2), 36.4 (dt, $^2J_{C,F}$ = 27.6 Hz, C-3), 27.6 (q, C-13). **$^{19}$F NMR** (282 MHz, $CDCl_3$): δ = -96.1 (ddt, $^3J_{H,F}$ = 49.3 Hz, $^3J_{H,F}$ = 20.4 Hz, $^3J_{H,F}$ = 17.2 Hz, 4-CF), -224.3 (tt, $^2J_{H,F}$ = 47.3 Hz, $^3J_{H,F}$ = 27.7 Hz, 11-$CH_2F$). **Elemental analysis:** $C_{17}H_{23}F_2NO_5S$ (M = 391.43 g/mol). Calcd. C 52.16, H 5.92, N 3.58, found C 52.19, H 6.09, N 3.54 %. **Optical rotation:**

$$[\alpha]_{589}^{20} = -4.0, [\alpha]_{578}^{20} = -4.4, [\alpha]_{546}^{20} = -5.3, [\alpha]_{436}^{20} = -13.0, [\alpha]_{365}^{20} = -31.1 \text{ (c = 1.005, CHCl}_3\text{).}$$

**Example 25**

**Example 25a:** Compound MAB 248

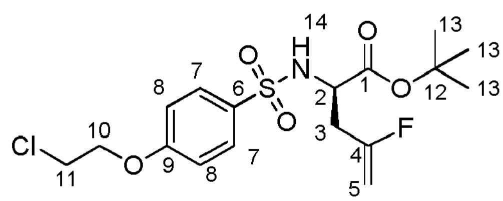

Prepared from *tert*-butyl (*R*)-2-amino-4-fluoropent-4-enoate (1000 mg, 5.28 mmol) and *p*-(2-chlorethoxy)-phenylsulfonylchloride (1350 mg, 5.28 mmol). **Yield:** 1.29 g (61 %). **M.p.** 98-99 °C (EtOAc/Cy). **$^1$H NMR** (300 MHz, $CDCl_3$): δ = 7.80 (dm, $^3J_{H,H}$ = 9.0 Hz, 2H, 8-CH), 6.98 (dm, $^3J_{H,H}$ = 9.0 Hz, 2H, 7-CH), 5.29 (d, $^3J_{H,H}$ = 9.0 Hz, 1H, 14-NH), 4.65 (dd, $^3J_{H,F}$ = 17.0 Hz, $^2J_{H,H}$ = 3.1 Hz, 1 H, 5-$CH_{cis}$), 4.35 (q, $^3J_{H,F}$ = 49.5 Hz, $^2J_{H,H}$ = 2.9 Hz, 2H, 5-$CH_{trans}$), 4.27 (t, $^3J_{H,H}$ = 5.7 Hz, 2H, 10-$CH_2$), 3.98 (dt, $^3J_{H,H}$ = 8.9 Hz, $^3J_{H,H}$ = 5.7 Hz, 1H, 2-CH), 3.84 (t, $^3J_{H,H}$ = 5.7 Hz, 2H, 11-$CH_2Cl$), 2.65 (dd, $^3J_{H,H}$ = 5.8 Hz, $^2J_{H,H}$ = 0.9 Hz, 1H, 3-$CH_A$), 2.59 (t, $^3J_{H,H}$ = 5.3 Hz, 1H, 3-$CH_B$), 1.30 (s, 9H, 13-$CH_3$). **$^{13}$C NMR** (75 MHz, $CDCl_3$): δ = 169.2 (s, C-1), 161.6 (s, C-9), 160.9 (ds, $^1J_{C,F}$ = 257.1 Hz, C-4), 132.0 (s, C-6), 129.5 (d, C-8), 114.7 (d, C-7), 94.2 (dt, $^2J_{C,F}$ = 18.9 Hz, C-5), 83.2 (s, C-12), 68.2 (t, C-10), 53.2 (dd, $^3J_{C,F}$ = 0.9 Hz, C-2), 41.5 (t, C-11), 36.5 (dt, $^2J_{C,F}$ = 27.2 Hz, C-3), 27.7 (q, C-13). **$^{19}$F NMR** (282 MHz, $CDCl_3$): δ = -96.1 (ddt, $^3J_{H,F}$ = 49.2 Hz, $^3J_{H,F}$ = 20.6 Hz, $^3J_{H,F}$ = 17.1 Hz, 4-CF). **Elemental analysis:** $C_{17}H_{23}ClFNO_5S$ (M = 407.88 g/mol); calcd. C 50.06, H 5.68, N 3.43; found C 50.02, H 5.51 N 3.36 %. **Exact mass** (ESI$^+$): $C_{17}H_{23}ClFNO_5S$ + $Na^+$; calcd. 430.0867, found 430.0851;

$(C_{17}H_{23}ClFNO_5S)_2 + Na^+$; calcd. 837.1837, found 837.1824. **Optical rotation:**

$$[\alpha]_{589}^{20} = +6.3, [\alpha]_{578}^{20} = +6.9, [\alpha]_{546}^{20} = +8.1, [\alpha]_{436}^{20} = +18.5, [\alpha]_{365}^{20} = +42.5$$ (c = 0.991, $CHCl_3$).

**Example 25b:** compound MAB 249

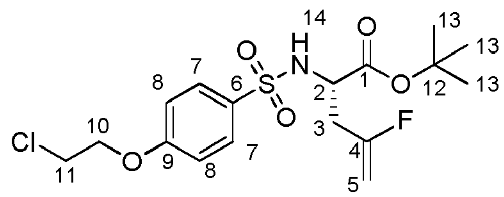

Prepared from *tert*-butyl (*S*)-2-amino-4-fluoropent-4-enoate (1000 mg, 5.28 mmol) and *p*-(2-chlorethoxy)phenylsulfonylchloride (1350 mg 5.28 mmol). **Yield:** 1.58 g (73 **%). $^1$H NMR** (300 MHz, $CDCl_3$): δ = 7.80 (dm, $^3J_{H,H}$ = 9.0 Hz, 2H, 8-CH), 6.98 (dm, $^3J_{H,H}$ = 9.0 Hz, 2H, 7-CH), 5.32 (d, $^3J_{H,H}$ = 8.9 Hz, 1H, 14-NH), 4.64 (dd, $^3J_{H,F}$ = 17.1 Hz, $^2J_{H,H}$ = 3.0 Hz, 1H, 5-$CH_{cis}$), 4.35 (q, $^3J_{H,F}$ = 49.4 Hz, $^2J_{H,H}$ = 3.0 Hz, 2H, 5-$CH_{trans}$), 4.27 (t, $^3J_{H,H}$ = 5.7 Hz, 2H, 10-$CH_2$), 3.98 (dt, $^3J_{H,H}$ = 9.0 Hz, $^3J_{H,H}$ = 5.8 Hz, 1 H, 2-CH), 3.84 (t, $^3J_{H,H}$ = 5.7 Hz, 2H, 11-$CH_2Cl$), 2.65 (dd, $^3J_{H,H}$ = 5.9 Hz, 1H, 3-$CH_A$), 2.59 (m, $^3J_{H,H}$ = 5.4 Hz, 1H, 3-$CH_B$), 1.30 (s, 9H, **13-$CH_3$). $^{13}$C NMR** (75 MHz, $CDCl_3$): δ = 169.2 (s, C-1), 161.6 (s, C-9), 160.9 (ds, $^1J_{C,F}$ = 257.1 Hz, C-4), 132.0 (s, C-6), 129.5 (d, C-8), 114.7 (d, C-7), 94.2 (dt, $^2J_{C,F}$ = 18.8 Hz, C-5), 83.2 (s, C-12), 68.2 (t, C-10), 53.2 (dd, $^3J_{C,F}$ = 0.9 Hz, C-2), 41.5 (t, C-11), 36.5 (dt, $^2J_{C,F}$ = 27.6 Hz, C-3), 27.6 (q, C-13). **$^{19}$F NMR** (282 MHz, $CDCl_3$): δ = -96.1 (ddt, $^3J_{H,F}$ = 49.4 Hz, $^3J_{H,F}$ = 20.5 Hz, $^3J_{H,F}$ = 17.1 Hz, 1 H). **Elemental analysis:** $C_{17}H_{23}ClFNO_5S$ (M = 407.88 g/mol), calcd. C 50.06, H 5.68, N 3.43, found C 49.90, H 5.53, N 3.38 %. **Exact mass** ($ESI^+$): $C_{17}H_{23}ClFNO_5S + Na^+$, calcd. 430.0867, found 430.0856; $(C_{17}H_{23}ClFNO_5S)_2 + Na^+$, calcd. 837.1837, found 837.1835. **Optical rotation:**

$$[\alpha]_{589}^{20} = -2.6, [\alpha]_{578}^{20} = -2.9, [\alpha]_{546}^{20} = -3.5, [\alpha]_{436}^{20} = -8.5, [\alpha]_{365}^{20} = -19.8$$ (c = 1.021, $CHCl_3$).

**General procedure for substitution with benzyl bromide**

The N-substituted *tert*-butyl 2-amino-4-fluoropent-4-enoate dissolved in dimethylformamide is treated under stirring with 10 equivalents of potassium carbonate. After 20 min 1 equivalent of benzyl bromide is added and the mixture is stirred at r.t. for 40 h. Then water is added and the mixture is extracted with ethyl acetate (4 times). The combined organic extracts are washed with water (4 times), saturated sodium chloride solution and dried with magnesium sulfate. After removal of the solvent the crude product is purified chromatographically an (silica gel, Cy/EtOAc, 4:1). The products are obtained as colorless or yellowish viscos oils.

Scheme 3

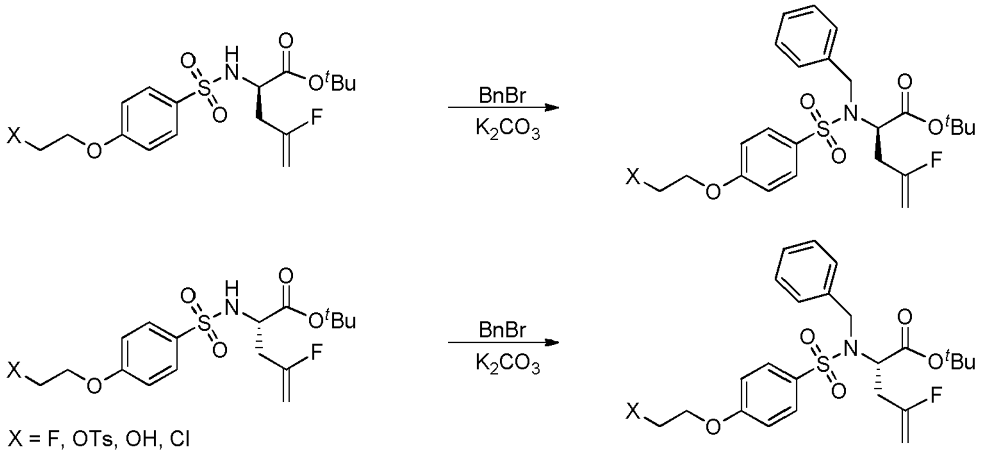


X = F, OTs, OH, Cl

**Example 26**

**Example 26a:** Compound MAB 242

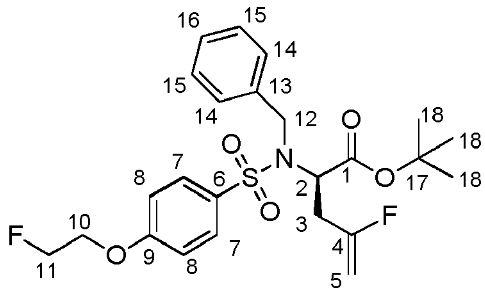

Prepared from *tert*-butyl *N*-[*p*-(2-fluoroethoxy)phenylsulfonyl]aminopent-4-enoate (783 mg, 2 mmol), benzyl bromide (342 mg, 2 mmol) and potassium carbonate 2.8 g (20 mmol). **Yield**: 600 mg (63 %). **$^1$H NMR** (300 MHz, $CDCl_3$): $\delta$ = 7.82 (dm, $^3J_{H,H}$ = 9.0 Hz, 2H, 8-CH), 7.29 (m, 5H, 14-CH, 15-CH, 16-CH), 6.98 (dm, $^3J_{H,H}$ = 9.0 Hz, 2H, 7-CH), 4.80 (dm, $^2J_{H,F}$ = 47.1 Hz, 2H, 11-$CH_2$), 4.66 (d, $^2J_{H,H}$ = 16.3 Hz, 1 H, 12-$CH_A$), 4.56 (t, $^3J_{H,H}$ = 7.3 Hz, 1H, 2-CH), 4.53 (dd, $^3J_{H,F}$ = 17.1 Hz, $^2J_{H,H}$ = 2.9 Hz, 1H, 5-$CH_{cis}$), 4.29 (d, $^2J_{H,H}$ = 15.9 Hz, 2H, 12-$CH_B$),4.27 (dm, $^3J_{H,F}$ = 27.8 Hz, 2H, 10-$CH_2$), 4.11 (dd, $^3J_{H,F}$ = 49.8 Hz, $^2J_{H,H}$ = 3.1 Hz, 1H, 5-$CH_{trans}$), 2.70 (dt, $^3J_{H,F}$ = 15.4 Hz, $^3J_{H,H}$ = 6.9 Hz, 1H, 3-$CH_A$), 2.46 (ddd, $^3J_{H,F}$ = 20.0 Hz, $^2J_{H,H}$ = 15.1 Hz, $^3J_{H,H}$ = 7.6 Hz, 1H, 3-$CH_B$), 1.36 (s, 9H, 18-$CH_3$). **$^{13}$C NMR** (75 MHz, $CDCl_3$): $\delta$ = 168.5 (s, C-1), 161.9 (ds, $^1J_{C,F}$ = 256.5 Hz, C-4), 161.7 (s, C-9), 136.7 (s, C-13), 132.5 (s, C-6), 129.9 (d, C-8), 128.5 (d, C-14 o. C-15), 128.4 (d, C-14 o. C-15), 127.7 (d, C-16), 114.6 (d, C-7), 93.1 (dt, $^2J_{C,F}$ = 19.1 Hz, C-5), 82.6 (s, C-17), 81.5 (dt, $^1J_{C,F}$ = 171.7 Hz, C-11), 67.4 (dt, $^2J_{C,F}$ = 20.5 Hz, C-10), 57.6 (d, C-2), 50.0 (t, C-12), 34.1 (dt, $^2J_{C,F}$ = 27.9 Hz, C-3), 27.8 (q, C-18). **$^{19}$F NMR** (282 MHz, $CDCl_3$): $\delta$ = -97.7 (m, $^3J_{H,F}$ = 49.8 Hz, $^3J_{H,F}$ = 20.0 Hz, $^3J_{H,F}$ = 17.0 Hz, 4-CF), -224.3 (tt, $^2J_{H,F}$ = 47.3 Hz, $^3J_{H,F}$ = 27.6 Hz, 11-$CH_2$F). **Exact mass** (ESI$^+$): $C_{24}H_{29}F_2NO_5S + Na^+$, calcd. 504.1632, found 504.1615; $(C_{24}H_{29}F_2NO_5S)_2 + Na^+$, calcd.985.3367, found 985.3348. **Optical rotation:**

$$[\alpha]_{589}^{20} = -22.8, [\alpha]_{578}^{20} = -23.8, [\alpha]_{546}^{20} = -27.2, [\alpha]_{436}^{20} = -49.6, [\alpha]_{365}^{20} = -80.6 \text{ (c = 1.018 CHCl}_3\text{).}$$

**Example 26b**: compound MAB 243

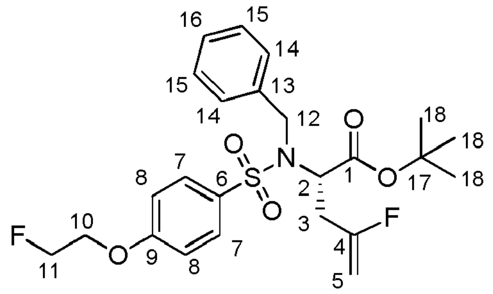

Prepared from *tert*-butyl *N*-[*p*-(-2-fluoroethoxy)phenylsulfonyl]aminopent-4-enoate (830 mg, 2.12 mmol), benzyl bromide (363 mg, 2.12 mmol) and 2.93 g (21.2 mmol) potassium carbonate. **Yield**: 800 mg (1.7 mmol, 78 %). **$^1$H NMR** (300 MHz, $CDCl_3$): δ = 7.82 (dm, $^3J_{H,H}$ = 9.0 Hz, 2H, 8-CH), 7.29 (m, 5H, 14-CH, 15-CH, 16-CH), 6.98 (dm, $^3J_{H,H}$ = 8.9 Hz, 2H, 7-CH), 4.80 (dm, $^2J_{H,F}$ = 47.3 Hz, 2H, 11-$CH_2$), 4.65 (d, $^2J_{H,H}$ = 16.0 Hz, 1H, 12-$CH_A$), 4.56 (t, $^3J_{H,H}$ = 7.3 Hz, 1H, 2-CH), 4.53 (dd, $^3J_{H,F}$ = 17.1 Hz, $^2J_{H,H}$ = 3.0 Hz, 1H, 5-$CH_{cis}$), 4.29 (d, $^2J_{H,H}$ = 15.7 Hz, 2H, 12-$CH_B$),4.25 (dm, $^3J_{H,F}$ = 27.8 Hz, 2H, 10-$CH_2$), 4.11 (dd, $^3J_{H,F}$ = 49.9 Hz, $^2J_{H,H}$ = 3.0 Hz, 1H, 5-$CH_{trans}$), 2.70 (dt, $^3J_{H,F}$ = 15.3 Hz, $^3J_{H,H}$ = 6.9 Hz, 1H, 3-$CH_A$), 2.46 (ddd, $^3J_{H,F}$ = 20.0 Hz, $^2J_{H,H}$ = 15.1 Hz, $^3J_{H,H}$ = 7.6 Hz, 1H, 3-$CH_B$), 1.36 (s, 9H, 18-$CH_3$). **$^{13}$C NMR** (75 MHz, $CDCl_3$): δ = 168.5 (s, C-1), 161.9 (ds, $^1J_{C,F}$ = 256.5 Hz, C-4), 161.7 (s, C-9), 136.7 (s, C-13), 132.5 (s, C-6), 129.9 (d, C-8), 128.5 (d, C-14 o. C-15), 128.4 (d, C-14 o. C-15), 127.7 (d, C-16), 114.6 (d, C-7), 93.1 (dt, $^2J_{C,F}$ = 19.2 Hz, C-5), 82.6 (s, C-17), 81.5 (dt, $^1J_{C,F}$ = 171.6 Hz, C-11), 67.4 (dt, $^2J_{C,F}$ = 20.2 Hz, C-10), 57.6 (d, C-2), 50.0 (t, C-12), 34.1 (dt, $^2J_{C,F}$ = 27.9 Hz, C-3), 27.8 (q, C-18). **$^{19}$F NMR** (282 MHz, $CD_3CN$): δ = -97.7 (m, $^3J_{H,F}$ = 49.8 Hz, $^3J_{H,F}$ = 19.8 Hz, $^3J_{H,F}$ = 16.4.0 Hz, 4-CF), -224.3 (tt, $^2J_{H,F}$ = 47.3 Hz, $^3J_{H,F}$ = 27.6 Hz, 11-$CH_2F$). **Exact mass** ($ESI^+$): $C_{24}H_{29}F_2NO_5S$ + $Na^+$, calcd. 504.1632, found 504.1613. $(C_{24}H_{29}F_2NO_5S)_2$ + $Na^+$, calcd. 985.3367, found 985.3345. **Optical rotation:**

$$[\alpha]_{589}^{20} = +19.9, [\alpha]_{578}^{20} = +20.3, [\alpha]_{546}^{20} = +23.2, [\alpha]_{436}^{20} = +42.5, [\alpha]_{365}^{20} = +74.0 \text{ (c = 1.018, CHCl}_3\text{).}$$

**Example 27**

**Example 27a:** compound MAB 250

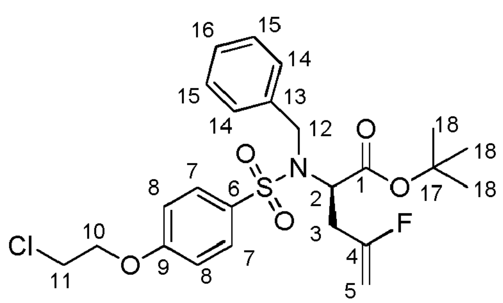

Prepared from *tert*-butyl *N*-[*p*-(2-chloroethoxy)phenylsulfonyl]-2-amino-4-fluoropent-4-enoate (816 mg 2.0 mmol), benzyl bromide (342 mg, 2.0 mmol) and potassium carbonate 2.8 g (20.0 mmol). **Yield:** 580 mg (58 %). **$^1$H NMR** (300 MHz, $CDCl_3$): δ = 7.82 (dm, $^3J_{H,H}$ = 8.9 Hz, 2H, 8-CH), 7.29 (m, 5H, 14-CH, 15-CH, 16-CH), 6.96 (dm, $^3J_{H,H}$ = 9.0 Hz, 2H, 7-CH), 4.65 (d, $^2J_{H,H}$ = 15.8 Hz, 1H, 12-$CH_A$), 4.56 (t, $^3J_{H,H}$ = 7.3 Hz, 1H, 2-CH), 4.53 (dd, $^3J_{H,F}$ = 17.2 Hz, $^2J_{H,H}$ = 2.9 Hz, 1H, 5-$C_{cis}$), 4.29 (d, $^2J_{H,H}$ = 15.7 Hz, 1H, 12-$CH_B$), 4.27 (t, $^3J_{H,H}$ = 5.6 Hz, 1H, 10-$CH_2$), 4.11 (dd, $^3J_{H,F}$ = 49.9 Hz, $^2J_{H,H}$ = 3.0 Hz, 1H, 5-$CH_{trans}$), 3.84 (t, $^3J_{H,H}$ = 5.7 Hz, 2H, 11-$CH_2Cl$), 2.70 (td, $^2J_{H,H}$ = 15.4 Hz, $^3J_{H,H}$ = 7.0 Hz, 1H, 3-$CH_A$), 2.46 (ddd, $^3J_{H,F}$ = 20.0 Hz, $^2J_{H,H}$ = 15.2 Hz, $^3J_{H,H}$ = 7.6 Hz, 1H, 3-$CH_B$), 1.36 (s, 9H, 18-$CH_3$)- **$^{13}$C NMR** (75 MHz, $CDCl_3$): δ = 168.5 (s, C-1), 161.9 (ds, $^1J_{C,F}$ = 256.5 Hz, C-4), 161.5 (s, C-9), 136.7 (s, C-13), 132.6 (s, C-6), 129.9

(d, C-8), 128.5 (d, C-14 o. C-15), 128.4 (d, C-14 o. C-15), 127.7 (d, C-16), 114.6 (d, C-7), 93.1 (dt, $^2J_{C,F}$ = 18.9 Hz, C-5), 82.6 (s, C-17), 68.2 (t, C-10), 57.6 (d, C-2), 49.9 (t, C-12), 41.5 (t, C-11), 34.1 (dt, $^2J_{C,F}$ = 28.2 Hz, C-3), 27.8 (q, C-18). **$^{19}$F NMR** (282 MHz, $CDCl_3$): δ = -97.7 (ddt, $^3J_{H,F}$ = 49.9 Hz, $^3J_{H,F}$ = 19.9 Hz, $^3J_{H,F}$ = 16.4 Hz, 4-CF). **Exact mass** ($ESI^+$): $C_{24}H_{29}ClFNO_5S + Na^+$, calcd. 520.1337, found 520.1314; $(C_{24}H_{29}ClFNO_5S)_2 + Na^+$, calcd. 1017.2776 , found 1017.2708. **Optical rotation:**

$$[\alpha]_{589}^{20} = -24.8, [\alpha]_{578}^{20} = -26.2, [\alpha]_{546}^{20} = -30.0, [\alpha]_{436}^{20} = -54.4, [\alpha]_{365}^{20} = -95.0 \text{ (c = 1.038, CHCl}_3\text{).}$$

**Example 27b:** compound MAB 251

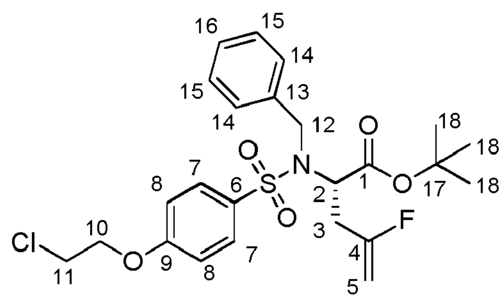

Prepared from *tert*-butyl *N*-[*p*-(2-chloroethoxy)phenylsulfonyl]-2-amino-4-fluoropent-4-enoate (1.50 g, 3.68 mmol), benzyl bromide (630 mg, 3.68 mmol) and potassium carbonate (5.0 g, 36.8 mmol). **Yield:** 1.28 g (70 %). **$^1$H NMR** (300 MHz, $CDCl_3$): δ = 7.82 (dm, $^3J_{H,H}$ = 9.0 Hz, 2H, 8-CH), 7.28 (m, 5H, 14-CH, 15-CH, 16-CH), 6.96 (dm, $^3J_{H,H}$ = 9.0 Hz, 2H, 7-CH), 4.66 (d, *J*=15.8, 1H, 12-$CH_A$), 4.56 (m, 1H, 2-CH), 4.53 (dd, *J*=17.1 Hz, 3.0 Hz, 2H, 5-$CH_{cis}$), 4.29 (d, *J*=15.7 Hz, 1 H, 12-$CH_B$), 4.28 (t, *J*=5.6 Hz, 2H, 10-$CH_2$), 4.11 (dd, *J*=49.9 Hz, 3.0 Hz, 1H, 5-$CH_{trans}$), 3.83 (t, *J*=5.8 Hz, 2H, 11-$CH_2Cl$), 2.70 (td, *J*=15.4 Hz, 6.9 Hz, 1H, 3-$CH_A$), 2.46 (ddd, *J*=20.1 Hz, 15.2 Hz, 7.6 Hz, 1H, 3-$CH_B$), 1.36 (s, 9H, 18-$CH_3$). **$^{13}$C NMR** (75 MHz, $CDCl_3$): δ = 168.6 (s, C-1), 161.9 (ds, $^1J_{C,F}$ = 256.3 Hz, C-4), 161.5 (s, C-9), 136.7 (s, C-13), 132.7 (s, C-6), 129.9 (d, C-8), 128.5 (d, C-14 o. C-15), 128.4 (d, C-14 o. C-15), 127.8 (d, C-16), 114.6 (d, C-7), 93.1 (dt, $^2J_{C,F}$ = 19.1 Hz, C-5), 82.6 (s, C-17), 68.2 (t, C-10), 57.6 (d, C-2), 50.0 (t, C-12), 41.5 (t, C-11), 34.1 (dt, $^2J_{C,F}$ = 27.9 Hz, C-3), 27.8 (q, C-18). **$^{19}$F NMR** (282 MHz, $CDCl_3$): δ = -97.7 (m, $^3J_{H,F}$ = 49.9 Hz, $^3J_{H,F}$ = 19.9 Hz, $^3J_{H,F}$ = 16.7 Hz, 4-CF). **Exact mass** ($ESI^+$): $C_{24}H_{29}ClFNO_5S + Na^+$, calcd. 520.1337, found 520.1326; $(C_{24}H_{29}ClFNO_5S)_2 + Na^+$, calcd. 1017.2776, found 1017.2748. **Optical rotation:**

$$[\alpha]_{589}^{20} = +21.1, [\alpha]_{578}^{20} = +22.2, [\alpha]_{546}^{20} = +25.1, [\alpha]_{436}^{20} = +45.5, [\alpha]_{365}^{20} = \text{n.d. (c = 1.005, CHCl}_3\text{).}$$

**Hydrolysis of the *tert*-butylesters**

In a dried YOUNG-tube the amino acid *tert*-butylester is dissolved in dry dichloromethane (20 mL/mmol) under argon and treated with trifluoroacetic acid (20 mL/mmol). The YOUNG-tube is flushed with argon and sealed. The mixture is stirred at r.t. for 3-4 h. Subsequently the reaction mixture is evaporated to dryness in vacuum. The residue is dissolved in chloroform (100 mL/mmol) and washed with an aqueous solution of citric acid and sodium bicarbonate (25 mL/mmol, pH ≈ 4). The aqueous phase is extracted with chloroform (4 × 30 mL/mmol) and the combined organic phases are dried with magnesium sulfate. After evaporation of the solvent the crude products are obtained as colorless, viscos oils, which in high vacuum solidified. These products were used for the next step without purification.

Scheme 4

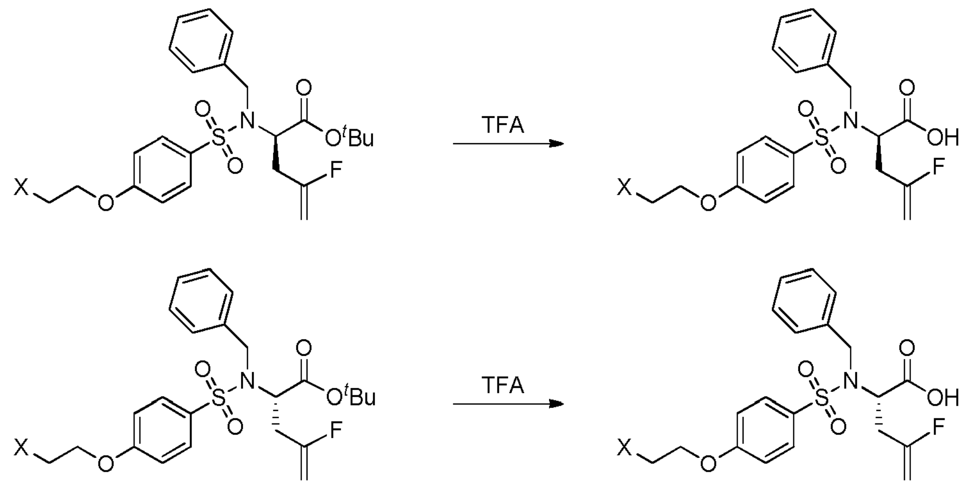


X = F, OTs, OH, Cl

**Example 28**

**Example 28a:** compound MAB 252

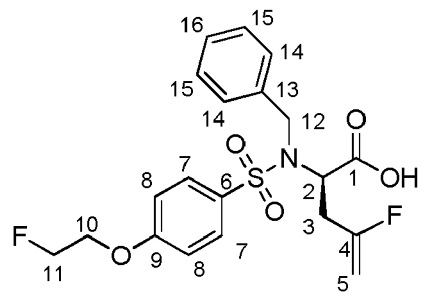

Prepared from *tert*-butyl *N*-benzyl-*N*-[*p*-(2-fluoroethoxy)phenylsulfonyl]-2-amino-4-fluoropent-4-enoate (300 mg 0.7 mmol). **$^{1}$H NMR** (300 MHz, $CD_3CN$): δ = 7.78 (dm, $^{3}J_{H,H}$ = 9.0 Hz, 2H, 8-CH), 7.30 (m, 5H, 14-CH, 15-CH, 16-CH), 7.04 (dm, $^{3}J_{H,H}$ = 9.0 Hz, 2H, 7-CH), 4.76 (dm, $^{2}J_{H,F}$ = 47.7 Hz, 2H, 11-$CH_2$), 4.65 (dd, $^{3}J_{H,H}$ = 8.5 Hz, $^{3}J_{H,H}$ = 6.0 Hz, 1H, 2-CH), 4.57 (d, $^{2}J_{H,H}$ = 16.1 Hz, 1H, 12-$CH_A$), 4.52 (dd, $^{3}J_{H,F}$ = 17.8 Hz, $^{2}J_{H,H}$ = 3.0 Hz, 1H, 5-$CH_{cis}$), 4.33 (d, $^{2}J_{H,H}$ = 16.1 Hz, 1H, 12-$CH_B$),4.29 (dm, $^{3}J_{H,F}$ = 29.5 Hz, 2H, 10-$CH_2$), 4.14 (dd, $^{3}J_{H,F}$ = 51.5 Hz, $^{2}J_{H,H}$ = 3.5 Hz, 1H, 5-$CH_{trans}$), 2.76 (ddd, $^{3}J_{H,F}$ = 14.8 Hz, $^{2}J_{H,H}$ = 13.7 Hz, $^{3}J_{H,H}$ = 6.0 Hz, 1H, 3-$CH_A$), 2.48 (ddd, $^{3}J_{H,F}$ = 22.3 Hz, $^{2}J_{H,H}$ = 15.4 Hz, $^{3}J_{H,H}$ = 8.5 Hz, 1H, 3-$CH_B$). **$^{13}$C NMR** (75 MHz, $CD_3CN$): δ = 171.2 (s, C-1), 163.2 (ds, $^{1}J_{C,F}$ = 254.7 Hz, C-4), 163.1 (s, C-9), 138.2 (s, C-13), 133.0 (s, C-6), 131.0 (d, C-8), 129.6 (d, C-14 o. C-15), 129.3 (d, C-14 o. C-15), 128.7 (d, C-16), 115.7 (d, C-7), 93.8 (dt, $^{2}J_{C,F}$ = 18.9 Hz, C-5), 83.1 (dt, $^{1}J_{C,F}$ = 167.3 Hz, C-11), 68.8 (dt, $^{2}J_{C,F}$ = 19.4 Hz, C-10), 57.9 (d, C-2), 50.8 (t, C-12), 34.1 (dt, $^{2}J_{C,F}$ = 28.2 Hz, C-3). **$^{19}$F NMR** (282 MHz, $CD_3CN$): δ = -97.7 (dddd, $^{3}J_{H,F}$ = 51.0 Hz, $^{3}J_{H,F}$ = 22.1 Hz, $^{3}J_{H,F}$ = 17.7 Hz, $^{3}J_{H,F}$ = 13.4 Hz, 4-CF), -224.0 (tt, $^{2}J_{H,F}$ = 47.7 Hz, $^{3}J_{H,F}$ = 29.5 Hz, 11-$CH_2F$). **Exact mass** ($ESI^+$): $C_{20}H_{31}F_2NO_5S + Na^+$, calcd. 448.1006, found 448.1004, $(C_{20}H_{31}F_2NO_5S)_2 + Na^+$, calcd. 873.2115, found 873.2116. **Exact mass** ($ESI^-$): $C_{20}H_{31}F_2NO_5S - H^+$, calcd. 424.1036, found 424.1057, $(C_{20}H_{31}F_2NO_5S)_2 - H^+$, calcd. 849.2144, found 849.2152.

**Example 28b:** compound MAB 253

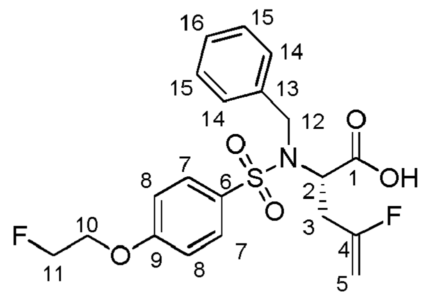

Prepared from *tert*-butyl *N*-benzyl-*N*-[*p*-(2-fluoroethoxy)phenylsulfonyl]-2-amino-4-fluoropent-4-enoate (400 mg, 0.94 mmol). The product is contaminated with some starting material. **$^1$H NMR** (300 MHz, $CD_3CN$): δ = 7.78 (dm, $^3J_{H,H}$ = 9.1, 2H, 8-CH), 7.29 (m, 5H, 14-CH, 15-CH, 16-CH), 7.04 (dm, $^3J_{H,H}$ = 9.0, 2H, 7-CH), 4.75 (dm, $^2J_{H,F}$ = 47.7 Hz, 2H, 11-$CH_2$), 4.65 (dd, $^3J_{H,H}$ = 8.5, $^3J_{H,H}$ = 6.0, 1 H, 2-CH), 4.57 (d, $^2J_{H,H}$ = 16.1, 1H, 12-$CH_A$), 4.52 (dd, $^3J_{H,F}$ = 17.6, $^2J_{H,H}$ = 3.0, 1H, 5-$CH_{cis}$), 4.32 (d, $^2J_{H,H}$ = 16.1, 1 H, 12-$CH_B$), 4.30 (dm, $^3J_{H,F}$ = 29.4 Hz, 2H, 10-$CH_2$), 4.14 (dd, $^3J_{H,F}$ = 51.8, $^2J_{H,H}$ = 3.4, 1 H, 5-$CH_{trans}$), 2.76 (ddd, $^3J_{H,F}$ = 14.7, $^2\mathbf{J}_{H,H}$ = 13.7, $^3J_{H,H}$ = 6.0, 1H, 3-$CH_A$), 2.47 (ddd, $^3J_{H,F}$ = 22.2, $^2J_{H,H}$ = 15.4, $^3J_{H,H}$ = 8.5, 1H, 3-$CH_B$). **$^{13}$C NMR** (75 MHz, $CD_3CN$): δ = 171.2 (s, C-1), 163.2 (ds, $^1J_{C,F}$ = 254.7 Hz, C-4), 163.1 (s, C-9), 138.2 (s, C-13), 133.0 (s, C-6), 131.0 (d, C-8), 129.6 (d, C-14 o. C-15), 129.3 (d, C-14 o. C-15), 128.7 (d, C-16), 115.7 (d, C-7), 93.8 (dt, $^2J_{C,F}$ = 18.9 Hz, C-5), 83.1 (dt, $^1J_{C,F}$ = 167.5 Hz, C-11), 68.8 (dt, $^2J_{C,F}$ = 19.2 Hz, C-10), 57.9 (d, C-2), 50.8 (t, C-12), 34.2 (dt, $^2J_{C,F}$ = 28.0 Hz, C-3). **$^{19}$F NMR** (282 MHz, $CD_3CN$): δ = -97.7 (dddd, $^3J_{H,F}$ = 50.9 Hz, $^3J_{H,F}$ = 22.1 Hz, $^3J_{H,F}$ = 17.7 Hz, $^3J_{H,F}$ = 13.3 Hz, 4-CF), -224.0 (tt, $^2J_{H,F}$ = 47.7 Hz, $^3J_{H,F}$ = 29.5 Hz, 11-$CH_2F$). **Exact mass** (ESI$^+$): $C_{20}H_{31}F_2NO_5S$ + $Na^+$, calcd. 448.1006, found 448.1009; **Exact mass** (ESI$^-$): $C_{20}H_{31}F_2NO_5S$ - $H^+$, calcd. 424.1036, found 424.1057, $(C_{20}H_{31}F_2NO_5S)_2$ - $H^+$, calcd. 849.2144, found 849.2157.

**Example 29**

**Example 29a:** compound MAB 256

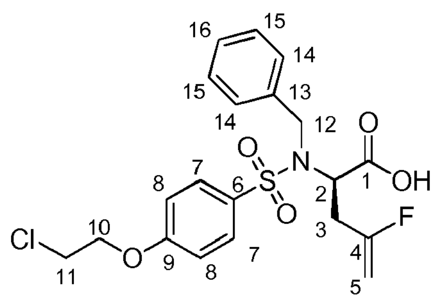

Prepared from *tert*-butyl *N*-benzyl-*N*-[*p*-(2-chloroethoxy)phenylsulfonyl]-2-amino-4-fluoropent-4-enoate (300 mg, 0.68 mmol). **$^1$H NMR** (300 MHz, $CD_3CN$): δ = 7.78 (d, $^3J_{H,H}$ = 9.1 Hz, 2H, 8-CH), 7.29 (m, 5H, 14-CH, 15-CH, 16-CH), 7.03 (d, $^3J_{H,H}$ = 9.1 Hz, 1H, 7-CH), 4.65 (dd, $^3J_{H,H}$ = 8.5 Hz, $^3J_{H,H}$ = 6.0 Hz, 1H, 2-CH), 4.57 (d, $^2J_{H,H}$ = 16.1 Hz, 1H, 12-$CH_A$), 4.52 (dd, $^3J_{H,F}$ = 17.7 Hz, $^2J_{H,H}$ = 3.2 Hz, 1H, 5-$CH_{cis}$), 4.33 (m, 2H, 10-$CH_2$), 4.32 (d, $^2J_{H,H}$ = 16.1 Hz, 1H, 12-$CH_B$), 4.14 (dd, $^3J_{H,F}$ = 50.9 Hz, $^2J_{H,H}$ = 3.1 Hz, 1H, 5-$CH_{trans}$), 3.90 (m, 2H, 11-$CH_2Cl$), 2.76 (ddd, $^3J_{H,F}$ = 16.1 Hz, $^2J_{H,H}$ = 14.2 Hz, $^3J_{H,H}$ = 5.9 Hz, 2H), 2.47 (ddd, $^3J_{H,F}$ = 22.3 Hz, $^2J_{H,H}$ = 15.5 Hz, $^3J_{H,H}$ = 8.6 Hz, 1H). **$^{13}$C NMR** (75 MHz, $CD_3CN$): δ = 171.2 (s, C-1), 163.2 (d, $^1J_{C,F}$ = 254.5 Hz, C-4), 162.9 (s, C-9), 138.2 (s, C-13), 133.1 (s, C-6), 131.0 (s, C-8), 129.6 (s, C-14 or C-15), 129.3 (s, C-14 or C-15), 128.7 (s, C-16), 115.7 (s, C-7), 93.8 (d, $^2J_{C,F}$ = 18.9 Hz, C-5), 69.6 (s, C-10), 57.9 (s, C-2), 50.8 (s, C-12), 43.5 (s, C-11), 34.1 (d, $^2J_{C,F}$ = 28.4 Hz, C-3). **$^{19}$F NMR** (282 MHz, $CD_3CN$): δ = -97.7 (dddd, $^3J_{H,F}$ = 51.0 Hz, $^3J_{H,F}$ = 22.2 Hz, $^3J_{H,F}$ = 17.7 Hz, $^3J_{H,F}$ = 13.3 Hz, 4-CF).

**Example 29b**: compound MAB 257

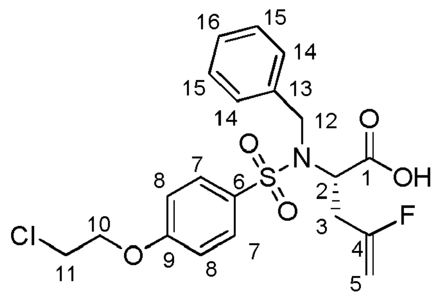

Prepared from *tert*-butyl *N*-benzyl-N-[p-(2-chloroethoxy)phenylsulfonyl]-2-amino-4-fluoropent-4-enoate (430 mg, 0.97 mmol). The crude product was directly used for the next step.

**Synthesis of the *tert*-butyl hydroxamates**

The *N,N*-disubstituted α-amino acid (1 equivalent) is suspended in dichloromethane (15 mL/mmol). 1-Hydroxybenzotriazol (HOBT, 1 equivalent) is added and the mixture is stirred for a short period of time. Then *N*-methylmorpholin (NMM, 5 equivalents) is added under stirring while the carboxylic acid dissolves. Subsequently, O-*tert*-butylhydroxylamine (3 aquivalents) is added and the solution is stirred for 5 min. After addition of 1-(3-dimethylaminopropyl)-3-ethylcarbodiimid-hydrochloride (EDC) (1.3 equivalents) the mixture is stirred at r.t. over night. The solution is washed with water (20 mL), and the aqueous phase is extracted with dichloromethane (3 × 20 mL). The combined organic phase is washed with brine (1 × 20 mL) and dried with magnesium sulfate. The solvent is evaporated and the crude product is purified by column chromatography (silica gel, Cy/EtOAc, 1:3). The silica gel has to be inactivated using the eluent mixture with 2 % of triethylamine. The products are obtained as highly viscose liquids, which on drying in high vacuum give amorphous solids.

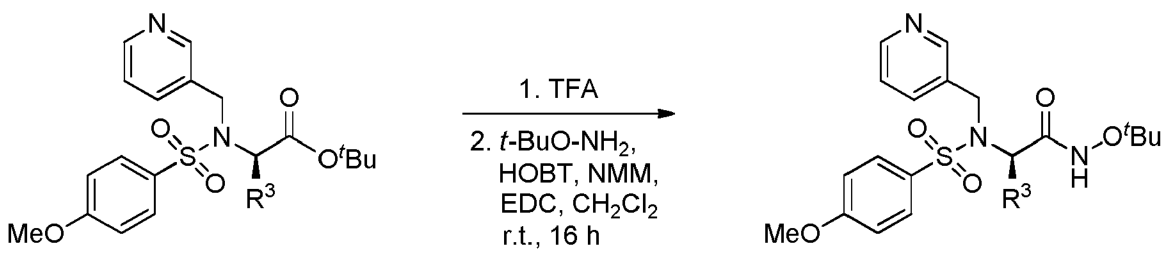


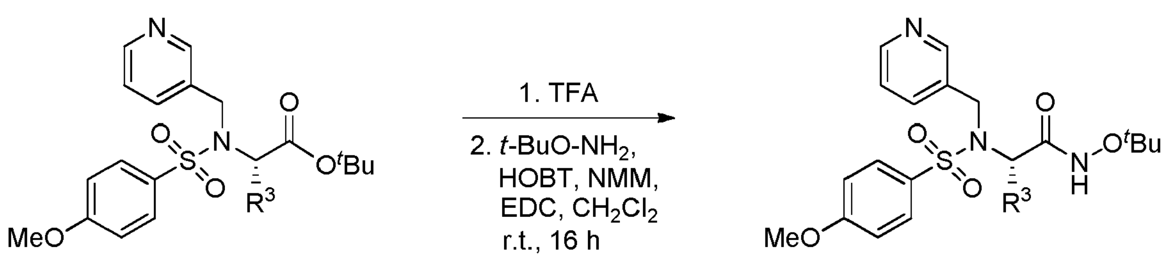


wherein $R^3$ is $CH_2CH_3$, $CH_2CH_2F$, $CH_2CH{=}CH_2$, $CH_2CF{=}CH_2$.

**Example 30**

**Example 30a:** compound MAB 254

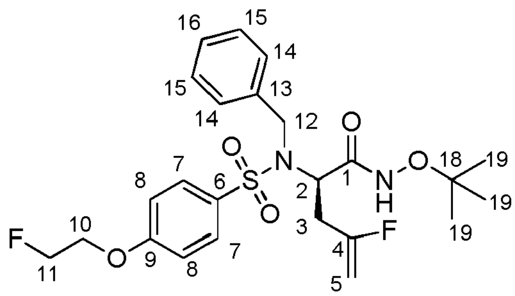

Prepared from *N*-benzyl-*N*-[p-(2-fluoroethoxy)phenylsulfonyl]-2-amino-4-fluoropent-4-ene carboxylic acid (191 mg, 0.45 mmol), HOPT (62 mg, 0.45 mmol), NMM (240 mg, 2.25 mmol), O-tert-butylhydroxylamine (175 mg, 1.35 mmol) and EDC (115 mg, 0.58 mmol). **Yield:** 147 mg (66 %). **$^{1}$H NMR** (300 MHz, $CDCl_3$): δ = 8.54 (s, 1H, 17-NH), 7.69 (dm, $^{3}J_{H,H}$ = 9.0 Hz, 2H, 8-CH), 7.29 (m, 5H, 14-CH, 15-CH, 16-CH), 6.95 (dm, $^{3}J_{H,H}$ = 9.0 Hz, 2H, 7-CH), 4.77 (dm, $^{2}J_{H,F}$ = 47.3 Hz, 2H, 11-$CH_2$), 4.64 (d, $^{2}J_{H,H}$ = 15.7 Hz, 1 H, 12-$CH_A$), 4.54 (t, $^{3}J_{H,H}$ = 7.2 Hz, 1H, 2-CH), 4.42 (dd, $^{3}J_{H,F}$ = 17.1 Hz, $^{2}J_{H,H}$ = 2.7 Hz, 2H, 5-$CH_{cis}$), 4.42 (d, overlapped with dd at 4.42), $^{2}J_{H,H}$ = 15.8 Hz, 1 H, 12-$CH_B$) 4.25 (m, $^{3}J_{H,F}$ = 27.8 Hz, 2H, 10-$CH_2$), 4.05 (dd, $^{3}J_{H,F}$ = 50.0 Hz, $^{2}J_{H,H}$ = 3.0 Hz, 1 H, 5-$CH_{trans}$), 2.81 (ddd, $^{3}J_{H,F}$ = 18.3 Hz, $^{2}J_{H,H}$ = 14.9 Hz, $^{3}J_{H,H}$ = 7.6 Hz, 1H, 3-$CH_A$), 2.32 (ddd, $^{3}J_{H,F}$ = 19.1 Hz, $^{2}J_{H,H}$ = 14.9 Hz, $^{3}J_{H,H}$ = 7.0 Hz, 1 H, 3-$CH_B$), 1.22 (s, 9H, 19-$CH_3$). **$^{13}$C NMR** (75 MHz, $CDCl_3$): δ = 167.0 (s, C-1), 161.9 (s, C-9), 161.3 (ds, $^{1}J_{C,F}$ = 256.0 Hz, C-4), 136.5 (s, C-13), 131.7 (s, C-6), 129.6 (d, C-8), 128.7 (d, C-14 o. C-15), 128.5 (d, C-14 o. C-15), 127.8 (d, C-16), 114.7 (d, C-7), 93.7 (dt, $^{2}J_{C,F}$ = 19.1 Hz, C-5), 82.3 (s, C-18), 81.4 (dt, $^{1}J_{C,F}$ = 171.5 Hz, C-11), 67.3 (dt, $^{2}J_{C,F}$ = 20.4 Hz, C-10), 55.0 (d, C-2), 48.6 (t, C-12), 32.4 (dt, $^{2}J_{C,F}$ = 27.4 Hz, C-3), 26.1 (q, C-19). **$^{19}$F NMR** (282 MHz, $CDCl_3$): δ = - 97.92 (m, $^{3}J_{H,F}$ = 50.1 Hz, $^{3}J_{H,F}$ = 18.4 Hz, 4-CF), -223.79 (tt, $^{2}J_{H,F}$ = 47.3 Hz, $^{3}J_{H,F}$ = 27.8 Hz, 11-$CH_2F$). **Elemental analysis**: $C_{24}H_{30}F_2N_2O_5S$ (M = 496.57 g/mol), calcd. C 58.05, H 6.09, N 5.64, found C 57.94, H 6.24, N: 5.57 %. **Exact mass** (ESI$^+$): $C_{24}H_{30}F_2N_2O_5S + H^+$, calcd. 497.1922, found 497.1908; $C_{24}H_{30}F_2N_2O_5S + Na^+$, calcd. 519.1741, found 519.1733; $(C_{24}H_{30}F_2N_2O_5S)_2 + Na^+$, calcd. 1015.3585, found 1015.3566. **Optical rotation:**

$$[\alpha]_{589}^{20} = -23.9, [\alpha]_{578}^{20} = -25.1, [\alpha]_{546}^{20} = -28.9, [\alpha]_{436}^{20} = -52.6, [\alpha]_{365}^{20} = -92.9 \text{ (c = 0.993, CHCl}_3\text{)}.$$

**Example 30b:** compound MAB 255

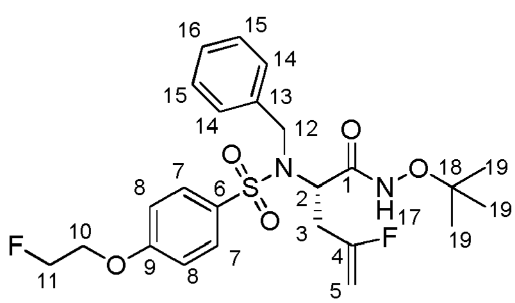

Prepared from *N*-benzyl-*N*-[p-(2-fluorethoxy)phenylsulfonyl]-2-amino-4-fluoropent-4-enoic acid (206 mg, 0.483 mmol), HOPT (65 mg, 0.483 mmol), NMM (245 mg, 2.415 mmol), *O-tert*-butylhydroxylamine (182 mg, 1.450 mmol) and EDC (121 mg, 0.628 mmol). **Yield**: 200 mg (83 %). **$^{1}$H NMR** (300 MHz, $CDCl_3$): δ = 8.46 (s, 1 H, 17-NH), 7.69 (dm, $^{3}J_{H,H}$ = 8.9 Hz, 2H, 8-CH), 7.29 (m, 5H, 14-CH, 15-CH, 16-CH), 6.96 (dm, $^{3}J_{H,H}$ = 9.0 Hz, 2H, 7-CH), 4.78 (dm, $^{2}J_{H,F}$ = 47.4 Hz, 2H, 11-$CH_2$), 4.64 (d, $^{2}J_{H,H}$ = 15.7 Hz, 1H, 12-$CH_A$), 4.54 (t, $^{3}J_{H,H}$ = 7.2 Hz, 1H, 2-CH), 4.42 (dd, $^{3}J_{H,F}$ = 17.1 Hz, $^{2}J_{H,H}$ = 3.0 Hz, 2H, 5-$CH_{cis}$), 4.40 (d, $^{2}J_{H,H}$ = 15.6 Hz, 1H, 12-$CH_B$) 4.27 (m, $^{3}J_{H,F}$ = 27.6 Hz, 2H, 10-$CH_2$), 4.05 (dd, $^{3}J_{H,F}$ = 50.0 Hz, $^{2}J_{H,H}$ = 3.0 Hz, 1H, 5-$CH_{trans}$), 2.821 (ddd, $^{3}J_{H,F}$ = 18.5 Hz, $^{2}J_{H,H}$ = 14.9 Hz, $^{3}J_{H,H}$ = 7.6 Hz, 1H,

3-$CH_A$), 2.31 (ddd, $^3J_{H,F}$ = 19.1 Hz, $^2J_{H,H}$ = 14.9 Hz, $^3J_{H,H}$ = 6.9 Hz, 1H, 3-$CH_B$), 1.23 (s, 9H, 19-$CH_3$). **$^{13}$C NMR** (75 MHz, $CDCl_3$): δ = 167.0 (s, C-1), 161.9 (s, C-9), 161.3 (ds, $^1J_{C,F}$ = 255.8 Hz, C-4), 136.5 (s, C-13), 131.9 (s, C-6), 129.6 (d, C-8), 128.8 (d, C-14 o. C-15), 128.6 (d, C-14 o. C-15), 128.0 (d, C-16), 114.8 (d, C-7), 93.7 (dt, $^2J_{C,F}$ = 19.2 Hz, C-5), 82.4 (s, C-18), 81.4 (dt, $^1J_{C,F}$ = 171.8 Hz, C-11), 67.4 (dt, $^2J_{C,F}$ = 20.5 Hz, C-10), 55.1 (d, C-2), 48.7 (t, C-12), 32.4 (dt, $^2J_{C,F}$ = 27.6 Hz, C-3), 26.2 (q, C-19). **$^{19}$F NMR** (282 MHz, $CDCl_3$): δ = -98.51 (m, $^3J_{H,F}$ = 50.3 Hz, $^3J_{H,F}$ = 18.0 Hz, 4-CF), -224.35 (tt, $^2J_{H,F}$ = 47.3 Hz, $^3J_{H,F}$ = 27.8 Hz, 11-$CH_2F$). **Elemental analysis:** $C_{24}H_{30}F_2N_2O_5S$ (M = 496.57 g/mol), calcd. C 58.05, H 6.09, N 5.64, found C 57.78, H 6.24, N 5.56 %. **Exact mass** (ESI$^+$): $C_{24}H_{30}F_2N_2O_5S$ + $H^+$: calcd. 497.1922, found 497.1917; $C_{24}H_{30}F_2N_2O_5S$ + $Na^+$, calcd. 519.1741, found 519.1733; $(C_{24}H_{30}F_2N_2O_5S)_2$ + $Na^+$, calcd. 1015.3585, found 1015.3564. **Optical rotation:** $[\alpha]_{589}^{20} = +21.7$ (c = 1.039, $CHCl_3$).

**Example 31**

**Example 31a:** compound MAB 258

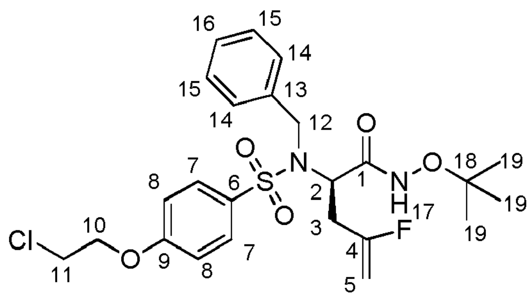

Prepared from *N*-benzyl-*N*-[*p*-(2-chloroethoxy)phenylsulfonyl]-2-amino-4-fluoropent-4-enoic acid (243 mg, 0.55 mmol) HOBT (75 mg, 0.55 mmol), NMM (283 mg, 2.80 mmol) *O-tert*-butylhydroxylamine (213 mg, 1.70 mmol) and EDC(138 mg, 0.72 mmol). **Yield**: 204 mg (72 %). **$^1$H NMR** (300 MHz, $CDCl_3$): δ = 8.48 (s, 1H, 17-NH), 7.69 (dm, $^3J_{H,H}$ = 8.9 Hz, 2H, 8-CH), 7.28 (m, 5H, 14-CH, 15-CH, 16-CH), 6.94 (dm, $^3J_{H,H}$ = 8.8 Hz, 2H, 7-CH), 4.64 (d, $^2J_{H,H}$ = 15.7 Hz, 1H, 12-$CH_A$), 4.53 (t, $^3J_{H,H}$ = 7.2 Hz, 1H, 2-CH), 4.42 (dd, $^3J_{H,F}$ = 17.1 Hz, $^2J_{H,H}$ = 3.0 Hz, 1H, 5-$CH_{cis}$), 4.41 (d, $^2J_{H,H}$ = 15.5 Hz, 1 H, 12-$CH_B$), 4.28 (t, $^3J_{H,H}$ = 5.7 Hz, 2H, 10-$CH_2$), 4.06 (dd, $^3J_{H,F}$ = 49.9 Hz, $^2J_{H,H}$ = 3.1 Hz, 1H, 5-$CH_{trans}$), 3.84 (t, *J*=5.7 Hz, 2H, 11-$CH_2$), 2.82 (ddd, $^3J_{H,F}$ = 18.5 Hz, $^2J_{H,H}$ = 14.9 Hz, $^3J_{H,H}$ = 7.6 Hz, 1H, 3-$CH_A$), 2.31 (ddd, $^3J_{H,F}$ = 18.9 Hz, $^2J_{H,H}$ = 14.9 Hz, $^3J_{H,H}$ = 6.9 Hz, 1H, 3-$CH_B$), 1.23 (s, 9H, 19-$CH_3$). **$^{13}$C NMR** (75 MHz, $CDCl_3$): δ = 167.0 (s, C-1), 161.8 (s, C-9), 161.3 (ds, $^1J_{C,F}$ = 255.8, C-4), 136.4 (s, C-13), 131.9 (s, C-6), 129.7 (d, C-8), 128.8 (d, C-14 o. C-15), 128.6 (d, C-14 o. C-15), 127.9 (d, C-16), 114.8 (d, C-7), 93.7 (dt, $^2J_{C,F}$ = 19.4, C-5), 82.4 (s, C-18), 68.2 (t, C-10), 55.0 (d, C-2), 48.7 (t, C-12), 41.4 (t, C-11), 32.4 (dt, $^2J_{C,F}$ = 27.6 Hz, C-3), 26.1 (q, C-19). **$^{19}$F NMR** (282 MHz, $CDCl_3$): δ = -98.49 (m, $^3J_{H,F}$ = 49.9 Hz, $^3J_{H,F}$ = 18.9 Hz, $^3J_{H,F}$ = 17.2 Hz 4-CF). **Elemental analysis**: $C_{24}H_{30}ClFN_2O_5S$ (M = 513.02 g/mol), calcd. C 56.19, H 5.89, N 5.46, found C 55.55, H 5.89, N 5.34 %. **Exact mass** (ESI$^+$): $C_{24}H_{30}ClFN_2O_5S$ + $Na^+$, calcd. 535.1446, found 535.1434. **Optical rotation**: $[\alpha]_{589}^{20} = -2.2$, $[\alpha]_{365}^{20} = -6.9$ (c = 1.025 $CHCl_3$).

**Example 31b**: compound MAB 259

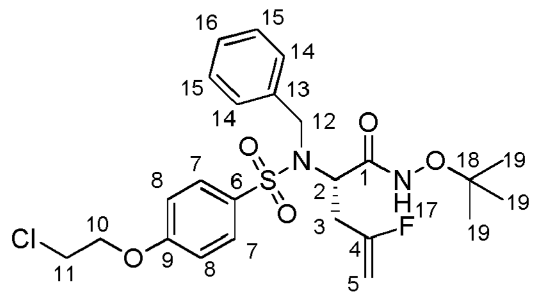

Prepared from *N*-benzyl-*N*-[*p*-(2-chloroethoxy)phenylsulfonyl]-2-amino-4-fluoropent-4-enoic acid (348 mg, 0.79 mmol), HOBT (107 mg, 0.79 mmol), NMM (405 mg, 3.94 mmol), *O-tert*-butylhydroxylamine (305 mg, 2.36 mmol) and EDC (198 mg, 1.20 mmol). **Yield:** 302 mg (75 %). **$^1$H NMR** (300 MHz, $CDCl_3$): δ = 8.48 (s, 1H, 17-NH), 7.69 (dm, $^3J_{H,H}$ = 8.9 Hz, 2H, 8-CH), 7.28 (m, 5H, 14-CH, 15-CH, 16-CH), 6.94 (dm, $^3J_{H,H}$ = 8.9 Hz, 2H, 7-CH), 4.64 (d, $^2J_{H,H}$ = 15.6 Hz, 1H, 12-$CH_A$), 4.53 (t, $^3J_{H,H}$ = 7.2 Hz, 1 H, 2-CH), 4.43 (dd, $^3J_{H,F}$ = 17.1 Hz, $^2J_{H,H}$ = 3.0 Hz, 1H, 5-$CH_{cis}$), 4.40 (d, $^2J_{H,H}$ = 15.6 Hz, 1 H, 12-$CH_B$), 4.28 (t, $^3J_{H,H}$ = 5.7 Hz, 2H, 10-$CH_2$), 4.06 (dd, $^3J_{H,F}$ = 49.6 Hz, $^2J_{H,H}$ = 3.5 Hz, 1 H, 5-$CH_{trans}$), 3.84 (t, *J*=5.7 Hz, 2H, 11-$CH_2$), 2.82 (ddd, $^3J_{H,F}$ = 18.6 Hz, $^2J_{H,H}$ = 14.9 Hz, $^3J_{H,H}$ = 7.6 Hz, 1 H, 3-$CH_A$), 2.31 (ddd, $^3J_{H,F}$ = 18.9 Hz, $^2J_{H,H}$ = 14.9 Hz, $^3J_{H,H}$ = 6.8 Hz, 1 H, 3-$CH_B$), 1.23 (s, 9H, 19-$CH_3$). **$^{13}$C NMR** (75 MHz, $CDCl_3$): δ = 167.0 (s, C-1), 161.8 (s, C-9), 161.3 (ds, $^1J_{C,F}$ = 256.1, C-4), 136.4 (s, C-13), 131.9 (s, C-6), 129.7 (d, C-8), 128.8 (d, C-14 o. C-15), 128.6 (d, C-14 o. C-15), 127.9 (d, C-16), 114.8 (d, C-7), 93.7 (dt, $^2J_{C,F}$ = 19.2, C-5), 82.4 (s, C-18), 68.2 (t, C-10), 55.0 (d, C-2), 48.7 (t, C-12), 41.4 (t, C-11), 32.4 (dt, $^2J_{C,F}$ = 27.1 Hz, C-3), 26.1 (q, C-19). **$^{19}$F NMR** (282 MHz, $CDCl_3$): δ = -98.50 (m, $^3J_{H,F}$ = 49.9 Hz, $^3J_{H,F}$ = 18.5 Hz 4-CF). **Elemental analysis**: $C_{24}H_{30}ClFN_2O_5S$ (M = 513.02 g/mol), calcd. C 56.19, H 5.89, N 5.46, found C 55.20, H 5.88, N 5.36 %. **Exact mass** (ESI$^+$): $C_{24}H_{30}ClFN_2O_5S + Na^+$, calcd. 535.1446, found 535.1434. **Optical rotation**:

$$[\alpha]_{589}^{20} = +21.5, [\alpha]_{578}^{20} = +22.2, [\alpha]_{546}^{20} = +25.5, [\alpha]_{436}^{20} = +46.5, [\alpha]_{365}^{20} = +82.2 \text{ (c = 0.999, CHCl}_3\text{)}.$$

**General procedure for the preparation of tetrahydropyranyl (THP)-protected hydroxamic acids**

To a solution of carboxylic acid (100 mg, 0.235 mmol) in DMF (0.06 mmol/mL, 1 mL) 1-hydroxybenzotriazole hydrate (HOBT, 1.2 eq., 38 mg, 0.282 mmol), 4-methylmorpholine (NMM, 3.0 eq., 78 pL, 249.70 mmol), O-tetrahydro-2*H*-pyran-2-yl-hydroxylamine (3.1 eq., 85 mg, 0.729 mmol and *N*-[(dimethylamino)-propyl]-*N'*-ethylcarbodiimide hydrochloride (EDC, 1.4 eq., 63 mg, 0.329 mmol) were added. After stirring overnight at room temperature the reaction mixture was diluted with water (20 mL) and extracted with ethyl acetate (3 × 5 mL). The combined organic phases were washed successively with water, 5% aqueous $KHSO_4$, saturated aqueous $NaHCO_3$ and brine, and dried over magnesium sulfate. After removing the solvent under reduced pressure column chromatographic purification (silica gel, cyclohexane/ethyl acetate 2:1) yielded the THP-protected hydroxamic acid.

**Example 32**

**Example 32a.** (2*S*)-2-[*N*-benzyl-4-(2-fluoroethoxy)phenylsulfonamido]-4-fluoro-*N*-[(tetrahydro-2*H*-pyran-2-yl)oxy]pent-4-enamide (**HUG 72**)

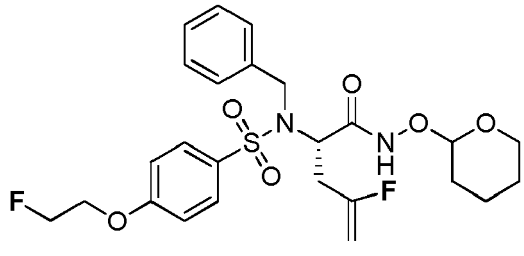

Light brown wax; yield: 92 mg (75%). **$^1$H NMR** (300 MHz, $CDCl_3$); δ 9.19 (s, NH, 1H), 9.15 (s, NH, 1 H), 7.77 (d, $^3J_{H,H}$ = 8.9 Hz, Ph*H*, 2H), 7.72 (d, $^3J_{H,H}$ = 9.0 Hz, Ph*H*, 2H), 7.39 - 7.21 (m, Ph*H*, 5H), 6.98 (d, $^3J_{H,H}$ = 9.0 Hz, Ph*H*, 2H), 6.97 (d, $^3J_{H,H}$ = 9.0 Hz, Ph*H*, 2H), 4.92 - 4.88 (m, NHOC*H*O), 4.79 (dm, $^2J_{H,F}$ = 47.4 Hz, C$H_2$F, 2H), 4.66 (AB, d, $^2J_{H,H}$ = 16.0 Hz, NC$H_2$, 1 H), 4.58 - 4.45 (m, NC*H*, NC$H_2$, 2H), 4.43 (dd, CFC$H_2$, $H_{cis}$, $^3J_{H,F}$ = 17.1 Hz, $^2J_{H,F}$ = 3.0 Hz, 1 H), 4.39 (dd, CFC$H_2$, $H_{cis}$, $^3J_{H,F}$ = 17.1 Hz, $^2J_{H,F}$ = 3.0 Hz, 1 H), 4.27 (dm, $^3J_{H,F}$ = 27.7 Hz, O-C$H_2$C$H_2$F, 2H), 4.10 (dd, CFC$H_2$, $H_{trans}$, $^3J_{H,F}$ = 49.9 Hz, $^2J_{H,F}$ = 3.1 Hz, 1 H), 3.95 - 3.84 (m, NHOCHOC$H_2$, 1 H), 3.70 - 3.55 (m, NHOCHOC$H_2$, 1 H), 2.93 - 2.58 (AB, m, NCHC$H_2$, 1 H), 2.47 - 2.18 (AB, m, NCHC$H_2$, 1 H), 1.88 - 1.50 (m, THP-C$H_2$, 6H). **$^{13}$C NMR** (75 MHz, $CDCl_3$): δ 165.8 (*C*ONH), 165.6 (*C*ONH), 162.0 (q, Ph*C*OC$H_2$C$H_2$F), 161.2 (d, $^1J_{C,F}$ = 256.3 Hz, *C*FC$H_2$), 161.2 (d, $^1J_{C,F}$ = 256.0 Hz, *C*FC$H_2$), 136.6 (qPh*C*C$H_2$N), 136.4 (qPh*C*C$H_2$N), 131.5 (qPh*C*S$O_2$), 129.7 (Ph*C*H), 129.7 (Ph*C*H), 128.7 (Ph*C*H), 128.6 (Ph*C*H), 128.5 (Ph*C*H), 128.4 (Ph*C*H), 127.8 (Ph*C*H), 127.8 (Ph*C*H), 114.7 (Ph*C*H), 114.7 (Ph*C*H), 102.1 (NHO*C*HO), 101.7 (NHO*C*HO), 93.7 (d, $^2J_{C,F}$ = 19.0 Hz, CF*C*$H_2$), 93.6 (d, $^2J_{C,F}$ = 18.9 Hz, CF*C*$H_2$), 81.4 ($^1J_{C,F}$ = 171.6 Hz, OC$H_2$*C*$H_2$F), 67.4 (d, $^2J_{C,F}$ = 20.5 Hz, O*C*$H_2$C$H_2$F), 62.0 (NHOCHO*C*$H_2$), 54.7 (N*C*H), 54.5 (N*C*H), 48.6 (NC$H_2$), 48.5 (N*C*$H_2$), 32.3 (d, $^2J_{C,F}$ = 27.6 Hz, NCH*C*$H_2$CF), 32.0 (d, $^2J_{C,F}$ = 26.9 Hz, NCH*C*$H_2$CF), 27.7 (NHOCH*C*$H_2$), 27.7 (NHOCH*C*$H_2$), 24.9 ($CH_2$), 18.2 (NHOCHC$H_2$*C*$H_2$). **MS-ES-EM:** *m/z* = 547.1683 [$(M+Na)^+$] calcd for $C_{25}H_{30}F_2N_2O_6SNa^+$: 547.1685.

**Example 32b.**(2*R*)-2-[*N*-benzyl-4-(2-fluoroethoxy)phenylsulfonamido]-4-fluoro-*N*-[(tetrahydro-2H-pyran-2-yl)oxy]pent-4-enamide (**HUG 71**)

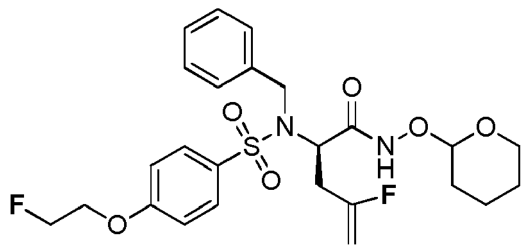

Light brown wax; yield: 101 mg (81%). *$^1$H NMR* (300 MHz, $CDCl_3$): δ 9.08 (s, NH, 1H), 7.77 (d, $^3J_{H,H}$ = 9.0 Hz, Ph*H*, 2H), 7.72 (d, $^3J_{H,H}$ = 9.0 Hz, Ph*H*, 2H), 7.39 - 7.21 (m, Ph*H*, 5H), 6.98 (d, $^3J_{H,H}$ = 9.0 Hz, Ph*H*, 2H), 6.97 (d, $^3J_{H,H}$ = 9.0 Hz, Ph*H*, 2H), 4.92 - 4.88 (m, NHOC*H*O), 4.80 (dm, $^2J_{H,F}$ = 47.3 Hz, C$H_2$F, 2H), 4.66 (AB, m, NC$H_2$, 1H), 4.58 - 4.45 (m, NC*H*, NC$H_2$, 2H), 4.45 - 4.34 (m, CFC$H_2$, $H_{cis}$, 1 H), 4.28 (dm, $^3J_{H,F}$ = 27.7 Hz, O-C$H_2$C$H_2$F, 2H), 4.10 (dd, CFC$H_2$, $H_{trans}$, $^3J_{H,F}$ = 49.9 Hz, $^2J_{H,F}$ = 3.0 Hz, 1H), 3.95 - 3.84 (m, NHOCHOC$H_2$, 1H), 3.70 - 3.58 (m, NHOCHOC$H_2$, 1 H), 2.92 - 2.64 (AB, m, NCHC$H_2$, 1 H), 2.43 - 2.18 (AB, m, NCHC$H_2$, 1 H), 1.88 - 1.50 (m, THP-C$H_2$, 6H). **$^{13}$C NMR** (75 MHz, $CDCl_3$): δ 165.8 (CONH), 165.7 (CONH), 162.0 (qPh*C*OC$H_2$C$H_2$F), 161.3 (d, $^1J_{C,F}$ = 256.1 Hz, *C*FC$H_2$), 136.6 (qPh*C*C$H_2$N), 136.4 (qPh*C*C$H_2$N), 131.6 (qPh*C*S$O_2$), 129.8 (Ph*C*H), 129.8 (Ph*C*H), 128.8 (Ph*C*H), 128.7 (Ph*C*H), 128.5 (Ph*C*H), 128.5 (Ph*C*H), 127.9, (Ph*C*H), 127.9 (Ph*C*H), 114.8 (Ph*C*H), 114.8 (Ph*C*H), 102.2 (NHO*C*HO), 101.8 (NHO *C*HO), 93.8 (d, $^2J_{C,F}$ = 18.9 Hz, CF*C*$H_2$), 81.4 ($^1J_{C,F}$ = 171.9 Hz, OC$H_2$*C*$H_2$F), 67.4 (d, $^2J_{C,F}$ = 20.5 Hz, O*C*$H_2$C$H_2$F), 62.1 (NHOCHO*C*$H_2$), 62.1 (NHOCHO*C*$H_2$), 54.7 (N*C*H), 54.6 (N*C*H), 48.7 (N*C*$H_2$), 48.5 (N**C**$H_2$), 32.2 (d, $^2J_{C,F}$ = 27.6 Hz, NCH*C*$H_2$CF), 27.7 (NHOCH*C*$H_2$), 24.9 ($CH_2$), 18.2 (NHOCHC$H_2$*C*$H_2$). **$^{19}$F NMR** (282 MHz, $CDCl_3$): δ -97.51 and 97.77 (m, CFC$H_2$, 1 F), -223.89 and 223.90 (tt, $^2J_{H,F}$ = 47.3, $^3J_{H,F}$ = 27.6 Hz, OC$H_2$C$H_2$F, 1F). **MS-ES-EM** *m/z* = 547.1695 [$(M+Na)^+$] calcd for $C_{25}H_{30}F_2N_2O_6SNa^+$: 547.1685.

**General procedure for the acidic hydrolysis of the THP-protected hydroxamic acids**

To a solution of THP-protected hydroxamic acid in 1,4-dioxane (0.5 mL/0.18 mmol) 4 N hydrochloric acid in 1,4-dioxane (4 eq.) and methanol (0.5 mL/0.18 mmol) were added and the reaction mixture was stirred at room temperature. The reaction progress was monitored by TLC (ethyl acetate). After complete conversion the reaction mixture was diluted with ethyl acetate (20 mL), washed with water (3 × 10 mL) and dried ($MgSO_4$). The solvent was removed under reduced pressure and the product was purified by column chromatography (silica gel, cyclohexane/ethyl acetate 2:1).

**Example 33**

**Example 33a**. (*S*)-2-[*N*-benzyl-4-(2-fluoroethoxy)phenylsulfonamido]-4-fluoro-*N*-hydroxypent-4-enamide (**HUG 74**)

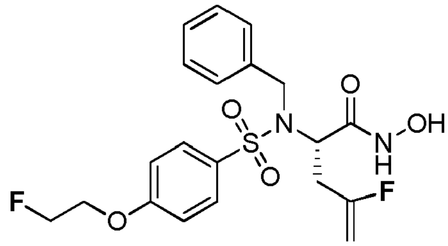

The reaction was carried out in a 0.175 mmol scale with (2S)-2-(*N*-benzyl-4-(2-fluoroethoxy)phenylsulfonamido)-4-fluoro-*N*-[(tetrahydro-2*H*-pyran-2-yl)oxy]pent-4-enamide (**HUG 72**). After column chromatography a light brown wax was obtained. Yield: 33 mg (43%). **$^1$H NMR** (400 MHz, $CDCl_3$): δ 9.19 (s, 1 OH or NH), 7.72 (d, $^3J_{H,H}$= 8.9 Hz, Ph*H*, 2H), 7.35 - 7.25 (m, Ph*H*, 5H), 6.97 (d, $^3J_{H,H}$ = 8.9 Hz, Ph*H*, 2H), 4.79 (dm, $^2J_{H,F}$ = 47.3 Hz, $CH_2F$, 2H), 4.54 (AB, d, $^2J_{H,H}$ = 15.6 Hz, $NCH_2$, 1 H), 4.52 (m, NC*H*, 1 H), 4.46 (AB, d, $^2J_{H,H}$ = 15.7 Hz, $NCH_2$, 1H), 4.38 (dd, $CFCH_2$, $H_{cis}$, $^3J_{H,F}$ = 17.0 Hz, $^2J_{H,F}$ = 2.9 Hz, 1H), 4.26 (dm, $^3J_{H,F}$ = 27.6 Hz, $CH_2FCH_2$, 2H), 4.08 (dd, $CFCH_2$, $H_{trans}$, $^3J_{H,F}$ = 49.7 Hz, $^2J_{H,F}$ = 3.1 Hz, 1H), 2.85 - 2.69 (AB, m, $NCHCH_2$, 1H), 2.38 (AB, m, $NCHCH_2$, 1H). **$^{13}$C NMR** (101 MHz, $CDCl_3$): δ 166.4 (CONH), 162.1 (qPh$COCH_2CH_2F$), 161.1 (d, $^1J_{C,F}$ = 256.3 Hz, $CFCH_2$), 136.1 (qPh$CCH_2N$), 131.3 (qPh$CSO_2$), 129.7 (Ph*C*H), 128.6 (Ph*C*H), 128.6 (Ph*C*H), 128.0 (Ph*C*H), 114.8 (Ph*C*H), 93.8 (d, $^2J_{C,F}$ = 18.9 Hz, $CFCH_2$), 81.4 (d, $^1J_{C,F}$= 171.7 Hz, $OCH_2CH_2F$), 67.4 (d, $^2J_{C,F}$ = 20.4 Hz, $OCH_2CH_2F$), 54.2 (NCH), 48.7 ($NCH_2$), 31.7 (d, $^2J_{C,F}$ = 27.7 Hz, $NCHCH_2CF$). **$^{19}$F NMR** (282 MHz, $CDCl_3$): δ -97.58 (m, $CFCH_2$, 1 F), -223.84 (tt, $^2J_{H,F}$ = 47.3, $^3J_{H,F}$ = 27.6 Hz, $OCH_2CH_2F$, 1 F). **MS-ES-EM:** m/z = 463.1120 [$(M+Na)^+$] calcd for $C_{20}H_{22}F_2N_2O_5SNa^+$: 463.1110. HPLC $t_R$ = 9.05 min (100%).

Example 33b.(R)-2-[N-benzyl-4-(2-fluoroethoxy)phenylsulfonamido]-4-fluoro-N-hydroxypent-4-enamide (HUG 78)

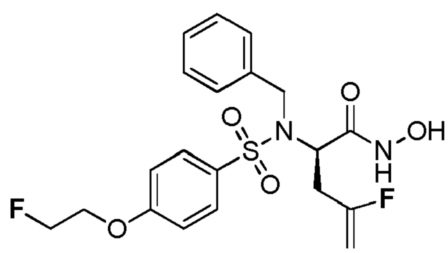

The reaction was carried out in a 0.190 mmol scale with (2R)-2-[N-benzyl-4-(2-fluoroethoxy)phenylsulfonamido]-4-fluoro-N-[(tetrahydro-2H-pyran-2-yl)oxy]pent-4-enamide (HUG 71). After column chromatography a light brown wax was obtained. Yield: 52 mg (62%). $^1$H NMR (400 MHz, $CDCl_3$): δ 9.25 (s, 1 OH or NH), 7.71 (d, $^3J_{H,H}$ = 8.8 Hz, PhH, 2H), 7.38 - 7.18 (m, PhH, 5H), 6.96 (d, $^3J_{H,H}$ = 8.8 Hz, PhH, 2H), 4.79 (dm, $^2J_{H,F}$ = 47.2 Hz, $CH_2F$, 2H), 4.54 (AB, d, $^2J_{H,H}$ = 15.5 Hz, $NCH_2$, 1 H), 4.52 (m, NCH, 1 H), 4.46 (AB, d, $^2J_{H,H}$ = 15.8 Hz, $NCH_2$, 1 H), 4.38 (dd, $CFCH_2$, $H_{cis}$, $^3J_{H,F}$ = 17.0 Hz, $^2J_{H,F}$ = 2.8 Hz, 1 H), 4.26 (dm, $^3J_{H,F}$ = 27.3 Hz, $CH_2FCH_2$, 2H), 4.08 (dd, $CFCH_2$, $H_{trans}$, $^3J_{H,F}$ = 50.2 Hz, $^2J_{H,F}$ = 2.2 Hz, 1 H), 2.86 - 2.63 (AB, m, $NCHCH_2$, 1 H), 2.47 - 2.25 (A$\underline{B}$, m, $NCHCH_2$, 1 H).**$^{13}$C NMR** (101 MHz, $CDCl_3$): δ 166.4 (CONH), 162.1 (qPh$COCH_2CH_2F$), 161.1 (d, $^1J_{C,F}$ = 256.3 Hz, $CFCH_2$), 136.2 (qPh$CCH_2N$), 131.3 (qPh$CSO_2$), 129.7 (PhCH), 128.6 (Ph*C*H), 128.6 (Ph*C*H), 128.0 (Ph*C*H), 114.8 (Ph*C*H), 93.7 (d, $^2J_{C,F}$ = 19.4 Hz, $CFCH_2$), 81.4 (d, $^1J_{C,F}$ = 171.6 Hz, $OCH_2CH_2F$), 67.4 (d, $^2J_{C,F}$ = 20.4 Hz, $OCH_2CH_2F$), 54.2 (NCH), 48.7 ($NCH_2$), 31.8 (d, $^2J_{C,F}$ = 27.8 Hz, $NCHCH_2CF$). **$^{19}$F NMR** (282 MHz, $CDCl_3$): δ -97.54 (ddd, $^3J_{H,F}$ = 49.9 Hz, $^3J_{H,F}$ = 36.9 Hz, $^3J_{H,F}$ = 17.1 Hz, $CFCH_2$, 1 F), -223.83 (tt, $^2J_{H,F}$ = 47.3, $^3J_{H,F}$ = 27.6 Hz, $OCH_2CH_2F$, 1 F). **MS-ES-EM**: *m/z* = 463.11049 [$(M+Na)^+$] calcd for $C_{20}H_{22}F_2N_2O_5SNa^+$: 463.11097. HPLC $t_R$ = 9.10 min (99%).

**Synthesis of (S)-2-{4-[N-benzyl-N-(4-fluoro-1-hydroxyamino-1-oxopent-4-en-2-yl)sulfamoyl]-phenoxy}ethyl-**

**4-methylbenzenesulfonate (HUG 118)**

**Example 34:**

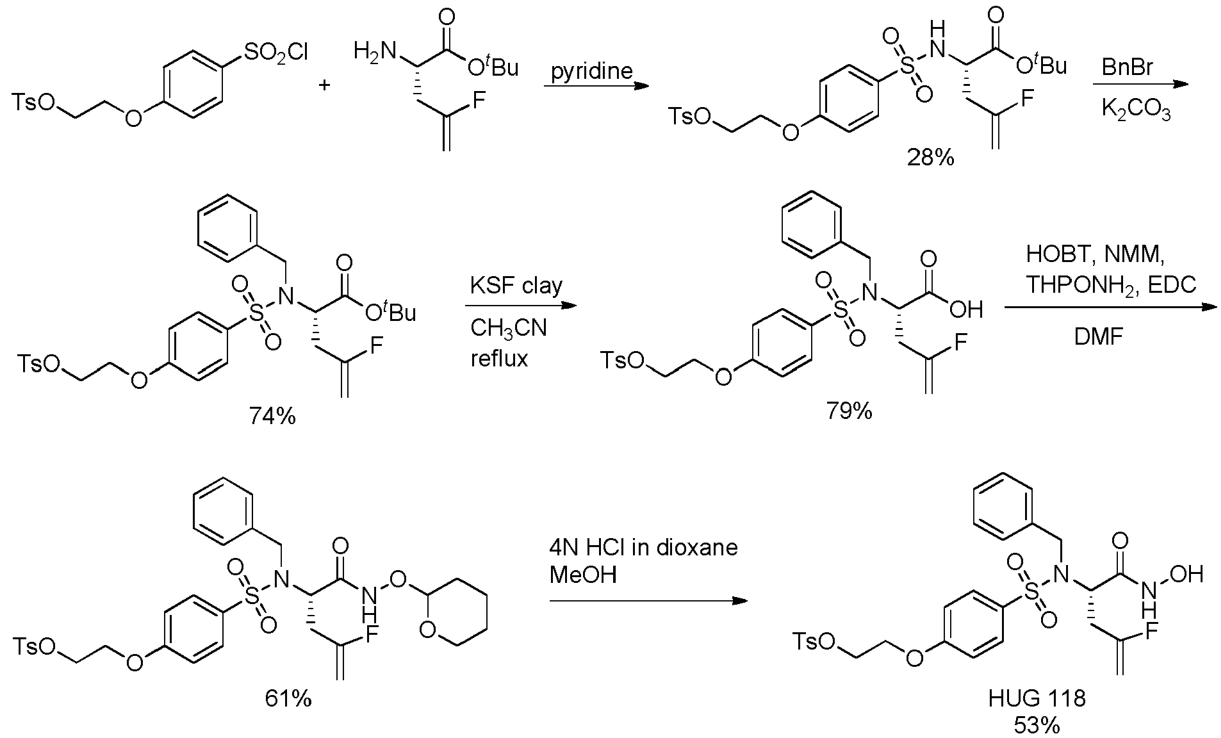


**Example 34a**:2-Phenoxyethyl 4-methylbenzenesulfonate (**KR217**)

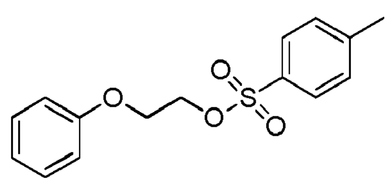

Phenoxyethanol (5.00 g, 36.18 mmol), triethylamine (5.0 eq., 18.20 g, 25 mL, 180.00 mmol) and dimethylaminopyridine (250 mg) were dissolved in dichloromethane (60 mL) and cooled to 0˚C. 4-Methylbenzene-1-sulfonyl chloride (2.5 eq., 17.15 g, 90.00 mmol) in dichloromethane (40 mL) was given dropwise to the solution. After stirring at room temperature overnight the reaction mixture was diluted with water (200 mL) and dichloromethane (100 mL). The organic layer was washed with brine, dried over $MgSO_4$ and the solvent was removed under reduced pressure. Column chromatographic purification (silica gel, cyclohexane/ethyl acetate 9:1) gave the product as a yellow oil. **Yield**: 6.60 g (62%). **$^1$H NMR** (300 MHz, $CDCl_3$) δ 7.97 - 7.76 (m, Ph*H*, 2H), 7.42 - 7.31 (m, Ph*H*, 2H), 7.30 - 7.21 (m, Ph*H*, 2H), 7.07 - 6.91 (m, Ph*H*, 1 H), 6.83 - 6.74 (m, Ph*H*, 2H), 4.41 - 4.33 (m, $OCH_2CH_2$, 2H), 4.18 - 4.11 (m, $OCH_2CH_2$, 2H) 2.45 (s, $SO_2PhCH_3$, 3H). **$^{13}$C NMR** (75 MHz, $CDCl_3$) δ 157.87 (q$PhCOCH_2CH_2OSO_2$), 144.88(q$PhCCH_3$), 132.75 ($OSO_2CPh$), 129.78 (Ph*C*H), 129.40 (Ph*C*H), 121.27 (Ph*C*H), 114.44 (Ph*C*H), 68.13 ($OCH_2CH_2$), 65.22 ($OCH_2CH_2$), 21.63 ($SO_2PhCH_3$). **MS-ES(+)-EM:** *m/z* = 315.0664 [$(M+Na)^+$] calcd for $C_{15}H_{16}NO_4SNa^+$: 315.0662.

**Example 34b**: 2-[4-(chlorosulfonyl)phenoxy]ethyl-4-methylbenzenesulfonate (**KR 220**)

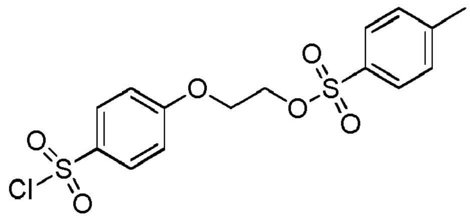

2-Phenoxyethyl 4-methylbenzenesulfonate (6.6 g, 22.6 mmol) was dissolved in dichloromethane (50 mL) and cooled to 0 ˚C. $HClSO_3$ was added dropwise to the solution. After the gas formation has stopped ice-water (200 mL) was given to the reaction mixture. The aqueous phase was extracted with diethyl ether (3 × 50 mL) and the combined organic layer was dried over $MgSO_4$. The solvent was removed under reduced pressure and column chromatographic purification (silica gel, cyclohexane/ethyl acetate, 4:1) gave the product as a yellow oil. **Yield**: 8.0 g (91%). **$^1$H NMR** (300 MHz, $CDCl_3$) δ 7.94 (d, $^3J_{H,H}$= 9.1 Hz, Ph*H*, 2H), 7.34 (d, $^3J_{H,H}$= 8.3 Hz Ph*H*, 2H), 7.36 (d, $^3J_{H,H}$= 8.5 Hz, Ph*H*, 2H), 6.95 (d, $^3J_{H,H}$= 9.1 Hz, Ph*H*, 1 H), 4.45 - 4.38 (m, OC$H_2$C$H_2$, 2H), 4.30 - 4.34 (m, OC$H_2$C$H_2$, 2H) 2.46 (s, S0$_2$PhC$H_3$, 3H). **$^{13}$C NMR** (75 MHz, $CDCl_3$) δ163.11 (qPh*C*OCH$_2$CH$_2$OSO$_2$), 145.25 (qPh*C*CH$_3$), 136.66 (OSO$_2$*C*Ph), 132.54 (qPh*C*SO$_2$), 129.91 (Ph*C*H), 129.47 (Ph*C*H), 127.91 (Ph*C*H), 115.11 (Ph*C*H), 67.36 (O*C*H$_2$CH$_2$), 65.05 (OCH$_2$*C*H$_2$), 21.64 (SO$_2$Ph*C*H$_3$). **MS-ES(+)-EM:** *m*/*z* = 412.9896 [(M+Na)$^+$] calcd for $C_{15}H_{15}ClO_6S_2Na^+$: 412.9891.

**Example 34c**: tert-Butyl (2*S*)-4-fluoro-2-{4-[2-(tosyloxy)ethoxy]phenylsulfonamido}pent-4-enoate (**HUG 109**)

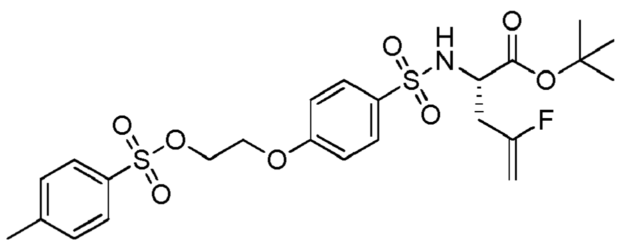

*tert*-Butyl (2*S*)-2-amino-4-fluoropent-4-enoate (2.00 g, 10.57 mmol) was dissolved in pyridine (4 mL). After cooling to 0 ˚C 2-[4-(chlorosulfonyl)phenoxy]ethyl-4-methylbenzenesulfonate (**KR 220**, 1.0 eq., 4.13 g, 10.57 mmol) was added to the stirred solution. The mixture was allowed to warm up to room temperature and was stirred overnight. Subsequently the mixture was diluted with dichloromethane (10 mL) and washed with aqueous 1 N HCl and brine. After drying over magnesium sulfate the solvent was removed under reduced pressure. Column chromatographic purification (silica gel, cyclohexane/ethyl acetate, 6:1) gave the sulfonamide as a colorless solid. **Yield:** 1.61 g (28%). **M.p.** 124.8 ˚C. **$^1$H NMR** (300 MHz, $CDCl_3$) δ 7.81 (d, $^3J_{H,H}$= 8.3 Hz, Ph*H*, 2H), 7.75 (d, $^3J_{H,H}$ = 9.0 Hz, Ph*H*, 2H), 7.36 (d, $^3J_{H,H}$= 8.0 Hz, Ph*H*, 2H), (6.85 (d, $^3J_{H,H}$= 9.0 Hz, Ph*H*, 2H), 5.27 (d, $^3J_{H,H}$= 8.9 Hz, N*H*, 1H), 4.64 (dd, $^3J_{H,F}$ = 17.1 Hz, $^2J_{H,H}$ = 3.1 Hz, CFC*H*$_2$, H$_{cis}$, 1 H), 4.38 (m, OC*H*$_2$CH$_2$, 2H), 4.34 (dd, $^3J_{H,F}$= 49.3 Hz, $^2J_{H,H}$ = 3.0 Hz, CFC*H*$_2$, H$_{trans}$, 1H), 4.19 (m, OCH$_2$C*H*$_2$, 2H), 3.97 (dt, $^3J_{H,H}$= 8.8 Hz, $^3J_{H,H}$ = 5.7 Hz, NHC*H*, 1H), 2.62 (m, $^3J_{H,F}$ = 20.5 Hz, $^3J_{H,H}$ = 5.7 Hz, $^2J_{H,H}$ =1.2 Hz, NHCHC*H*$_2$, 2H), 2.46 (s, SO$_2$PhC*H*$_3$, 3H), 1.30 (s, C(C*H*$_3$)$_3$, 9H). **$^{13}$C NMR** (75 MHz, $CDCl_3$) δ 169.16 (*C*O), 160.91 ($^1J_{C,F}$ = 257.1 Hz, *C*FCH$_2$), 161.38 (qPh*C*OCH$_2$CH$_2$OSO$_2$), 145.19 (qPh*C*CH$_3$), 132.65 (OSO$_2$*C*Ph), 132.10 (qPh*C*SO$_2$), 129.91 (Ph*C*H), 129.44 (Ph*C*H), 127.96 (Ph*C*H), 114.64 (Ph*C*H), 94.21 (d, $^2J_{C,F}$ = 18.8 Hz, CF*C*H$_2$), 83.23 (*C*(CH$_3$)$_3$), 67.53 (O*C*H$_2$CH$_2$), 65.74 (OCH$_2$*C*H$_2$), 53.22 (NH*C*H), 36.45 (d, $^2J_{C,F}$ = 27.4 Hz, NHCH*C*H$_2$CF), 27.66 (C(*C*H$_3$)$_3$), 21.66 (SO$_2$Ph*C*H$_3$). **$^{19}$F NMR** (282 MHz, $CDCl_3$) δ -95.63 (ddt, $^3J_{H,F}$= 49.3 Hz, $^3J_{H,F}$ = 20.6 Hz, $^3J_{H,F}$ = 17.0 Hz, 1F). **MS-ES (+)-EM**: m/z = 566.1281 [(M+Na)$^+$] calcd for $C_{24}H_{30}FNO_8S_2Na^+$: 566.1289.

**Example 34d**: tert-Butyl (2*S*)-2-{*N*-benzyl-4-[2-(tosyloxy)ethoxy]phenylsulfonamido}-4-fluoropent-4-enoate (**HUG 113**)

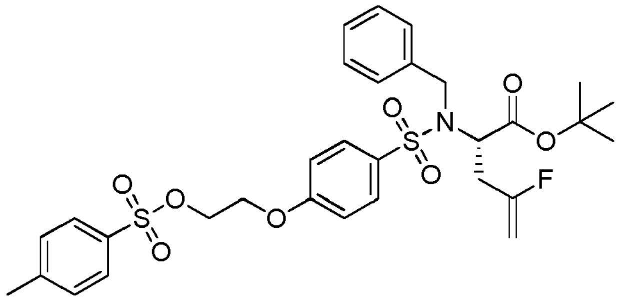

To a stirred solution of *tert*-butyl(2*S*)-4-fluoro-2-{4-[2-(tosyloxy)ethoxy]phenylsulfonamido}-pent-4-enoate (**HUG 109**, 1.60 g, 2.94 mmol) in DMF (50 mL) benzyl bromide (1.1 eq., 3.24 mmol, 0.55 g) and $K_2CO_3$ (10 eq., 4.05 g, 29.4 mmol) were added and the solution was stirred at room temperature over 2 d. The reaction mixture was diluted with water (200 mL) and extracted with ethyl acetate (3 × 100 mL). The combined organic layer was washed with brine and dried over $MgSO_4$. After removing the solvent under reduced pressure column chromatographic purification (silica gel, cyclohexane/ethyl acetate 2:1) gave the carbonic acid ester as a colorless waxy oil. **Yield**: 1.38 g (74%). **$^1$H NMR** (300 MHz, $CDCl_3$) δ 7.81 (d, $^3J_{H,H}$= 8.3 Hz, Ph*H*, 2H), 7.78 (d, $^3J_{H,H}$ = 9.0 Hz, Ph*H*, 2H), 7.35 (d, $^3J_{H,H}$= 8.0 Hz, Ph*H*, 2H), 7.33 - 7.22 (m, Ph*H*, 5H), 6.84 (d, $^3J_{H,H}$= 9.0 Hz, Ph*H*, 2H), 4.63 (d, $^2J_{H,H}$= 15.8 Hz, NC$H_2$, 1H), 4.55 (t, $^3J_{H,H}$ = 7.2 Hz, NC*H*, 1H), 4.53 (dd, $^3J_{H,F}$ = 17.2 Hz, $^2J_{H,H}$ = 3.0 Hz, CFC$H_2$,$H_{cis}$, 1H), 4.39 (m, OC$H_2$C$H_2$, 2H), 4.28 (d, $^2J_{H,H}$= 15.8 Hz, NC$H_2$, 1 H), 4.23 - 4.17 (m, OCH$_2$C$H_2$, 2H), 4.13 (dd, $^3J_{H,F}$= 49.7 Hz, $^2J_{H,H}$ 3.0 Hz, CFC$H_2$, $H_{trans}$, 1H), 2.70 (m, $^3J_{H,F}$ = 15.3 Hz, $^3J_{H,H}$ = 6.9 Hz, NCHC$H_2$, 1H), 2.54- 2.34 (m, NCHC$H_2$, 1H), 2.45 (s, SO$_2$PhC$H_3$, 3H), 1.36 (s, C(C$H_3$)$_3$, 9H). **$^{13}$C NMR** (75 MHz, $CDCl_3$) δ 168.48 (*C*O), 160.91 (d, $^1J_{C,F}$ = 256.4 Hz, *C*FCH$_2$), 161.21 (qPh*C*OCH$_2$CH$_2$OSO$_2$), 145.16 (qPh*C*CH$_3$), 136.62 (OSO$_2$*C*Ph), 132.68 (qPh*C*CH$_2$N), 132.64 (qPh*C*SO$_2$), 129.90 (Ph*C*H), 129.81 (Ph*C*H), 128.51 (Ph*C*H), 128.37 (Ph*C*H), 127.95 (PhCH), 127.76 (Ph*C*H), 114.46 (PhCH), 93.13 (d, $^2J_{C,F}$ = 19.1 Hz, CF*C*H$_2$), 82.60 (*C*(CH$_3$)$_3$), 67.57 (O*C*H$_2$CH$_2$), 65.68 (OCH$_2$*C*H$_2$), 57.57 (N*C*H), 49.99 (N*C*H$_2$), 34.04 (d, $^2J_{C,F}$ = 27.9 Hz, NCH*C*H$_2$CF), 27.78 (C(*C*H$_3$)$_3$), 21.64 (SO$_2$Ph*C*H$_3$). **$^{19}$F NMR** (282 MHz, $CDCl_3$) δ -95.63 (m, $^3J_{H,F}$= 49.3 Hz, $^3J_{H,F}$ = 20.6 Hz, $^3J_{H,F}$ = 17.0 Hz, 1F). **MS-ES(+)-EM**: *m/z* = 566.1281 [(M+Na)$^+$] calcd for $C_{24}H_{30}FNO_8S_2Na^+$: 566.1289.

**Example 34e**: (2*S*)-2-{*N*-benzyl-4-[2-(tosyloxy)ethoxy]phenylsulfonamido}-4-fluoropent-4-enoic acid (**HUG 115**)

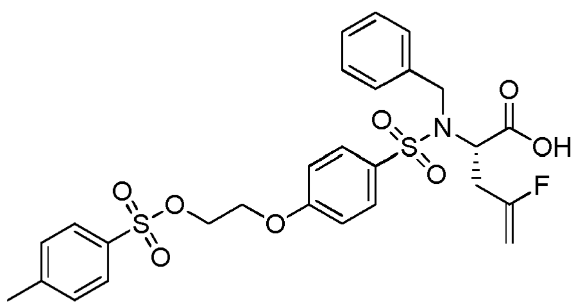

*(S)-tert-Butyl* 2-{*N*-benzyl-4-[2-(tosyloxy)ethoxy]phenylsulfonamido}-4-fluoropent-4-enoate (**HUG 113**, 1.38 g, 2.17 mmol) was dissolved in $CH_3CN$ (10 mL). KSF-clay (450 mg) was added and the mixture was refluxed for 3 h. The mixture was filtrated, washed with ethyl acetate (5 mL) and dried over $Na_2SO_4$. The solvent was removed under reduced pressure to give the colorless waxy product. **Yield**: 0.99 g (79%). **$^1$H NMR** (300 MHz, $CDCl_3$) δ 7.81 (d, $^3J_{H,H}$= 8.9 Hz, Ph*H*, 2H), 7.80 (d, $^3J_{H,H}$ = 8.4 Hz, Ph*H*, 2H), 7.35 (d, $^3J_{H,H}$= 8.0 Hz, Ph*H*, 2H), 7.31 - 7.22 (br s, Ph*H*, 5H), 6.88 (d, $^3J_{H,H}$= 8.9 Hz, Ph*H*, 2H), 4.53 (d, $^2J_{H,H}$= 15.1 Hz, NC$H_2$, 1H), 4.55 (t, $^3J_{H,H}$= 7.2 Hz, NC*H*, 1 H), 4.57 (dd, $^3J_{H,F}$ = 17.1 Hz, $^2J_{H,H}$ = 3.2 Hz, CFC$H_2$, $H_{cis}$, 1H), 4.41-4.32 (m, OC$H_2$CH$_2$, 2H), 4.33 (d, $^2J_{H,H}$= 15.8 Hz, NC$H_2$, 1H), 4.21 (dd, $^3J_{H,F}$= 50.2 Hz, $^2J_{H,H}$ 3.3 Hz, CFC$H_2$, $H_{trans}$, 1H), 4.28 - 4.20 (m, OCH$_2$C$H_2$, 2H), 2.99 - 2.71 (m, NCHC$H_2$, 1 H), 2.62 (ddd, $^3J_{H,F}$= 23.4 Hz, $^2J_{H,H}$ = 15.3 Hz, $^3J_{H,H}$ = 8.4 Hz, NCHC$H_2$, 1 H), 2.45 (s, SO$_2$PhC$H_3$, 3H). **$^{13}$C NMR** (75 MHz, $CDCl_3$) δ 173.47 (*C*OOH), 161.40 (d, $^1J_{C,F}$ = 256.0 Hz, CFCH$_2$), 161.54 (qPh*C*OCH$_2$CH$_2$OSO$_2$), 145.24 (qPh*C*CH$_3$), 135.64 (OSO$_2$*C*Ph), 132.52 (qPh*C*CH$_2$N), 131.77 (qPh*C*SO$_2$), 130.02 (Ph*C*H), 129.94 (Ph*C*H), 128.70 (Ph*C*H), 128.54 (Ph*C*H),128.12 (Ph*C*H), 127.98 (Ph*C*H), 114.67 (Ph*C*H), 93.76 (d, $^2J_{C,F}$ = 18.9 Hz, CF*C*H$_2$), 67.76 (O*C*H$_2$CH$_2$), 65.69 (OCH$_2$*C*H$_2$), 56.31 (N*C*H), 50.47 (N*C*H$_2$), 33.41 (d, $^2J_{C,F}$ = 28.1 Hz, NCHCH$_2$CF), 21.66 (SO$_2$PhCH$_3$).**$^{19}$F NMR** (282 MHz, $CDCl_3$) δ -97.93

(dddd, $^3J_{H,F}$= 49.9 Hz, $^3J_{H,F}$ = 22.6 Hz, $^3J_{H,F}$ = 17.1 Hz, $^4J_{H,F}$ = 13.9 Hz, 1 F). **MS-ES(+)-EM:** *m/z* = 600.1128 [(M+Na)$^+$] calcd for $C_{27}H_{28}FNO_8S_2Na^+$: 600.1133.

**Example 34f**:2-{4-[*N*-benzyl-*N*-((2*S*)-4-fluoro-1-oxo-1-{[(tetrahydro-2*H*-pyran-2-yl)oxy]amino}-pent-4-en-2-yl)sulfamoyl]phenoxy}ethyl 4-methylbenzenesulfonate (HUG 117)

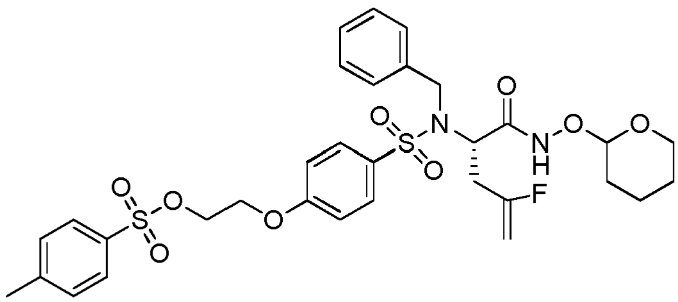

To a solutionof (S)-2-(N-benzyl-4-(2-(tosyloxy)ethoxy)phenylsulfonamido)-4-fluoropent-4-enoic acid (HUG 115, 1.10 g, 1.90 mmol) in DMF (9 mL) HOBT (1.2 eq., 0.31 g, 2.29 mmol), NMM (3 eq., 0.58 g, 0.63 mL), O-tetrahydro-2-H-pyran-2-yl-hydroxylamine (3.1 eq., 0.69 g, 5.89 mmol) and EDC (1.4 eq., 0.51 g, 2.66 mmol) were added. After stirring at room temperature overnight the reaction mixture was diluted with water (100 mL) and extracted with ethyl acetate (3 x 50 mL). The combined organic phases were washed successively with water, 5% aqueous $KHSO_4$, saturated aqueous $NaHCO_3$ and brine (100 mL) and dried over magnesium sulfate. After removing the solvent under reduced pressure column chromatographic purification (silica gel, cyclohexane/ethyl acetate, 2:1) yielded the mixture of diastereomeric THP-protected hydroxamic acids as a colorless waxy oil. **Yield:** 0.78 g (61%). $^1$H **NMR** (300 MHz, $CDCl_3$) δ 9.09 (s, N*H*, 1 H), 9.07 (s, N*H*, 1 H), 7.82 (d, $^3J_{H,H}$= 8.3 Hz, Ph*H*, 4H), 7.72 (d, $^3J_{H,H}$ = 8.9 Hz, Ph*H*,, 2H), 7.68 (d, $^3J_{H,H}$ = 9.0 Hz, Ph*H*, 2H), 7.36 (d, $^3J_{H,H}$= 8.0 Hz, Ph*H*, 4H), 7.33 - 7.21 (m, Ph*H*, 10H), 6.85 (d, $^3J_{H,H}$= 9.0 Hz, Ph*H*, 2H), ), 6.83 (d, $^3J_{H,H}$= 9.0 Hz, Ph*H*, 2H), 4.90 (m, NHOC*H*O, 1 H), 4.68 (m, NHOC*H*O, 1 H), 4.64 (d, $^2J_{H,H}$= 15.9 Hz, NC$H_2$, 1H), 4.52 - 4.43 (m, NC*H*, CFC$H_2$, $H_{cis}$, $^2J_{H,H}$ = 2.9 Hz, 1.5H), 4.52 - 4.43 (m, OC$H_2$CH$_2$, CFC$H_2$, $H_{cis}$, $^2J_{H,H}$ = 3.0 Hz, 2.5H), 4.46 (d, $^2J_{H,H}$= 15.0 Hz, NC$H_2$, 1H), 4.25 - 4.19 (m, OCH$_2$C$H_2$, 2H), 4.10 (dd, $^3J_{H,F}$= 50.0 Hz, $^2J_{H,H}$ 3.1 Hz, CFC$H_2$, $H_{trans}$, 1 H), 3.98 - 3.83 (m, NHOCHOC$H_2$, 2H), 3.74 - 3.58 (m, NHOCHOC$H_2$, 2H), 2.93 - 2.61 (m, NCHC$H_2$, 1 H), 2.46 (s, SO$_2$PhC$H_3$, 3H), 2.44 - 2.23 (m, NCHC$H_2$, 1 H), 1.86 - 1.48 (m, THP-CH$_2$, 6H). **$^{13}$C NMR** (75 MHz, $CDCl_3$) δ 165.75 (*C*ONH), 165.67 (*C*ONH), 161.59 (qPh*C*OCH$_2$CH$_2$OSO$_2$), 161.22 (d, $^1J_{C,F}$ = 255.7 Hz, *C*FCH$_2$), 145.19 (qPh*C*CH$_3$), 136.32 (OSO$_2$*C*Ph), 132.65 (qPh*C*CH$_2$N), 131.73 (qPh*C*SO$_2$), 129.91 (Ph*C*H), 129.76 (Ph*C*H), 128.81 (Ph*C*H), 128.76 (Ph*C*H), 128.57 (Ph*C*H'), 128.52 (Ph*C*H), 127.97 (Ph*C*H), 114.70 (Ph*C*H), 114.67 (Ph*C*H), 101.77 (NHOCHO), 101.76 (NHO*C*HO), 93.84 (d, $^2J_{C,F}$ = 18.9 Hz, CF*C*H$_2$), 67.51 (O*C*H$_2$CH$_2$), 65.75 (OCH$_2$*C*H$_2$), 62.13 (NHOCHO*C*H$_2$), 54.53 (N*C*H), 48.72 (N*C*H$_2$), 48.53 (NCH$_2$), 33.41 (d, $^2J_{C,F}$ = 28.1 Hz, NCH*C*H$_2$CF), 27.73 (NHOCH*C*H$_2$), 24.90 (CH$_2$), 21.66 (SO$_2$Ph*C*H$_3$), 18.21 (NHOCHCH$_2$*C*H$_2$). $^{19}$F NMR (282 MHz, $CDCl_3$) δ -97.56 (m), -97.82 (m,$^3J_{H,F}$ = 50.1 Hz, $^3J_{H,F}$ = 37.1 Hz, $^3J_{H,F}$ = 17.9 Hz, 1 F). **MS-ES(+)-EM:***m/z* = 669.1809 [(M+Na)$^+$] calcd. for $C_{32}H_{37}FN_2O_9S_2Na^+$: 669.1817.

**Example 34g:** (*S*)-2-(4-{*N*-benzyl-*N*-[4-fluoro-1-(hydroxyamino)-1-oxopent-4-en-2-yl]sulfamoyl}phenoxy)ethyl-4-methylbenzenesulfonate(**HUG 118**)

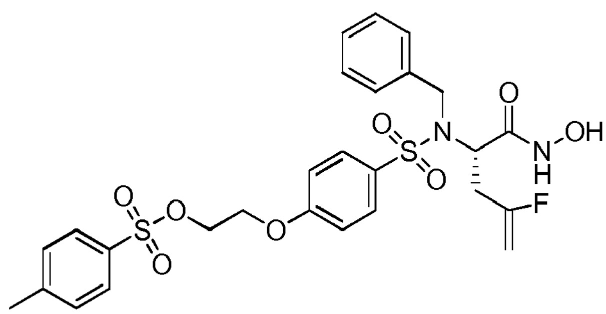

The reaction was carried out in a 0.30 mmol scale with 2-{4-[*N*-benzyl-*N*-((2*S*)-4-fluoro-1-oxo-1-{[(tetrahydro-2*H*-pyran-2-yl)oxy]amino}pent-4-en-2-yl)sulfamoyl]phenoxy}ethyl-4-methylbenzene-sulfonate (**HUG 117**). After column chromatography (silica gel, cyclohexane/ethyl acetate, 1:1) a light brown wax was obtained. **Yield**: 75 mg (53%). **$^1$H NMR** (300 MHz, $CDCl_3$) δ 9.24 (br s, 1-OH), 7.81 (d, $^3J_{H,H}$= 8.3 Hz, PhH, 2H), 7.67 (d, $^3J_{H,H}$ = 8.8 Hz, Ph*H*, 2H), 7.35 (d, $^3J_{H,H}$= 8.0 Hz, Ph*H*, 2H), 7.32 - 7.27 (br s, Ph*H*, 5H), 6.83 (d, $^3J_{H,H}$= 8.8 Hz, Ph*H*, 2H), 4.53 (d, $^2J_{H,H}$= 15.6 Hz, $NCH_2$, 1 H), 4.51 (t, $^3J_{H,H}$ = 8.3 Hz, NCH, 1H), 4.60 - 4.46 (m, $CFCH_2$, $H_{cis}$, 1 H), 4.46 - 4.30 (m, $OCH_2CH_2$, $NCH_2$, 3H), 4.24 - 4.17 (m, $OCH_2CH_2$, 2H), 4.08 (dd, $^3J_{H,F}$= 49.7 Hz, $^2J_{H,H}$ 3.0 Hz, $CFCH_2$, $H_{trans}$, 1 H), 2.85 - 2.64 (m, $NCHCH_2$, 1 H), 2.46 - 2.24 (m, $NCHCH_2$, 1 H), 2.45 (s, $SO_2PhCH_3$, 3H). **$^{13}$C NMR** (75 MHz, $CDCl_3$) δ 166.40 (*C*ONH), 161.63 ($qPhCOCH_2CH_2OSO_2$), 161.02 (d, $^1J_{C,F}$ = 256.3 Hz, $CFCH_2$), 145.19 ($qPhCCH_3$), 136.13 ($OSO_2CPh$), 132.56 ($qPhCCH_2N$), 131.44 ($qPhCSO_2$), 129.90 (Ph*C*H), 129.63 (Ph*C*H), 128.57 (Ph*C*H), 128.56 (Ph*C*H),127.97 (Ph*C*H), 127.93 (Ph*C*H), 114.72 (Ph*C*H), 93.82 (d, $^2J_{C,F}$ = 18.8 Hz, $CFCH_2$), 67.59 ($OCH_2CH_2$), 65.74 ($OCH_2CH_2$), 54.21 (N*C*H), 48.67 ($NCH_2$), 31.86(d, $^2J_{C,F}$ = 27.5 Hz, $NCHCH_2CF$), 21.61 ($SO_2PhCH_3$). **$^{19}$F NMR** (282 MHz, $CDCl_3$) δ -97.59 (ddd, $^3J_{H,F}$= 50.1 Hz, $^3J_{H,F}$ = 37.1 Hz, $^3J_{H,F}$ = 17.4 Hz,1 F). **MS-ES(+)-EM:** *m*/*z* = 615.1253 [$(M+Na)^+$] calcd for $C_{27}H_{29}FN2O_8S_2Na^+$: 615.1242. HPLC: $t_R$ = 9.95 min (100%).

**Example 35: Assay for MMP-2 and MMP-9 inhibitory activity**

The synthetic broad-spectrum fluorogenic substrate (7-methoxycoumarin-4-yl) acetyl pro-Leu-Gly-Leu- (3-(2,4-dinitrophenyl)-L-2,3-diamino-propionyl)-Ala-Arg-$NH_2$ (R & D Systems) was used to assay MMP-2 and MMP-9 activity.

The inhibition of MMP-2 and MMP-9 by the MMP inhibitors of table 1 was assayed by incubating either MMP-2 (2 nM) or MMP-9 (2 nM) and the compounds to be assayed at different concentrations (10 pM - 1 mM) for 30 min. at 37˚ C in 50 mM Tris • HCl (pH = 7.5), 0.2M NaCl, 5 mM $CaCl_2$, 20 μM $ZnSO_4$ and 0.05% Brij35.

An aliquot of substrate (10 μl of a 50 μM solution) was then added to 90 μM of pre-incubated MMP/compound mixture, and activity was determined at 37˚ C by following product release with time. The fluorescence changes were monitored using a Fusion Universal Microplate Analyzer (Packard Bioscience) with excitation and emission wavelengths set to 330 and 390 nm, respectively. Reaction rates were measured from the initial 10 min of the reaction profile where product release was linear with time and plotted as a function of inhibitor dose. From the resulting inhibition curves, the $IC_{50}$ values for each inhibitor were calculated by nonlinear regression analysis, performed using the GRACE 5.1.8 software (Linux).

Table 1: $IC_{50}$ values obtained for the compounds of formula (I) assayed and comparative compounds CGS-27023A and (S)-CGS-27023A.

| Compounds (S) | MMP-2 | MMP-9 | Compounds (R) | MMP-2 | MMP-9 |
|---|---|---|---|---|---|
| 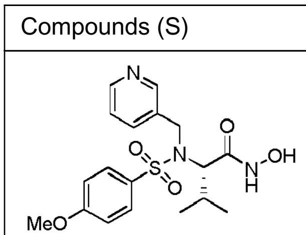 (*S*)-CGS-27023A | 494 nM[1)] | 198 nM[1)] | 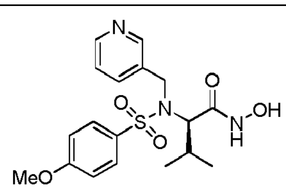 CGS-27023A | 20 nM | 8 nM |
| 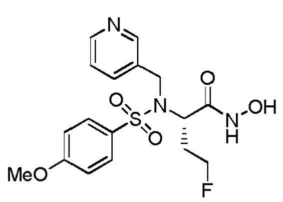 (*S*)-22a | 32.8 nM ± 16.4 nM | 3.0 nM ± 0.7 nM | 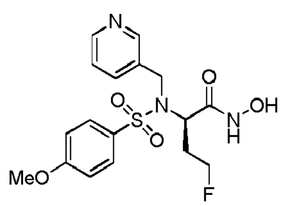 (*R*)-22b | 6.4 nM ± 1.8 nM | 12.3 nM ± 1.0 nM |
| 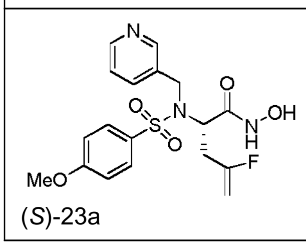 (*S*)-23a | 7.2 nM ± 0.1 nM | 4.9 nM ± 0.4 nM | 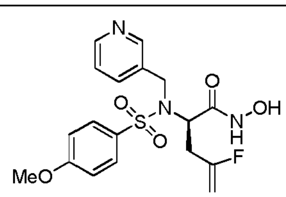 (*R*)-23b | 9.3 nM ± 2.0 nM | 8.3 nM ± 0.1 nM |

(continued)

| Compounds (S) | MMP-2 | MMP-9 | Compounds (R) | MMP-2 | MMP-9 |
|---|---|---|---|---|---|
| 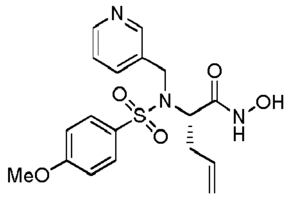 (*S*)-21a | 6.3 nM ± 1.5 nM | 6.4 nM ± 0.4 nM | 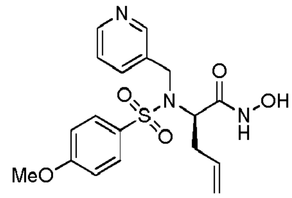 (*R*)-21b | 3.9 nM ± 0.3 nM | 0.54 nM ± 0.13 nM |
| 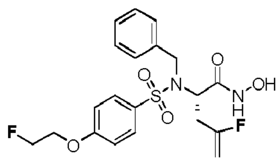 (*S*)-33a (HUG 74) | 22.2 nM | 6.96 nM | 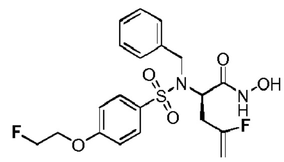 (*R*)-33b (HUG 78) | 10.4 nM | 2.86 nM |
| 1) calculated from the K, values | | | | | |

As can be observed from the data reported in Table 1, the compounds of the invention show an improved activity with respect to the reference compounds CGS-27023A. This effect is remarkable also in the (S)-enantiomer of the compounds of the invention.

**Example 36: Synthesis of [$^{18}$F]HUG 74**

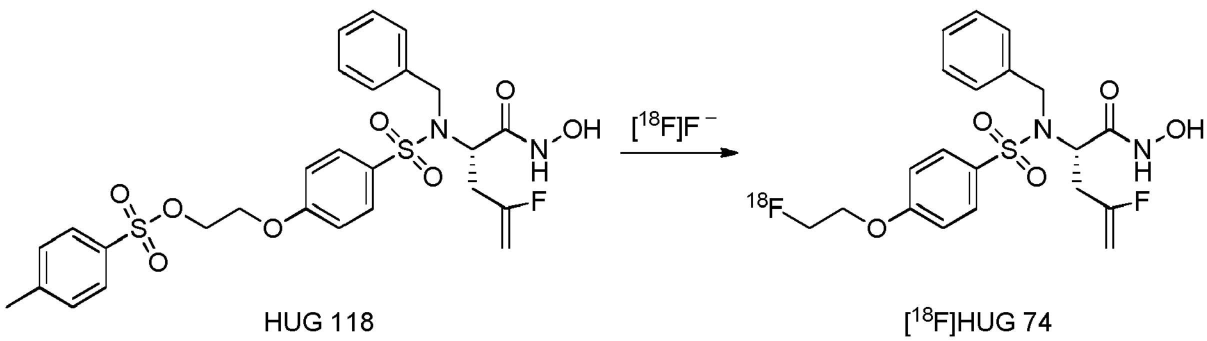


HUG 118 [$^{18}$F]HUG 74

No-carrier-added aqueous [$^{18}$F]fluoride was produced on a RDS 111e cyclotron (CTI-Siemens) by irradiation of a 1.2 mL water target using 10 MeV proton beams on 97.0% enriched [$^{180}$O]water by the $^{18}$O(p,n)$^{18}$F nuclear reaction. To recover the [$^{18}$O]water, the batch of aqueous [$^{18}$F]fluoride was passed through an anion exchange resin (Sep-Pak Light Waters Accell Plus QMA cartridge, preconditioned with 5 mL 1 M $K_2CO_3$ and 10 mL water). [$^{18}$F]Fluoride was eluted from the resin with a mixture of 40 μl 1 M $K_2CO_3$, 200 μl water for injection, and 800 μl DNA-grade $CH_3CN$ containing 18 mg Kryptofix 2.2.2 (K 222). Subsequently, the aqueous [$^{18}$F]K(K 222)F solution was carefully evaporated to dryness in *vacuo.*

An amount of -5.0 mg (-8.4 μmol) of compound **HUG 118** and carefully dried [$^{18}$F]K(K 222)F residue were heated at 84 °C in 1 mL DNA-grade $CH_3CN$ for 20 min. The mixture was cooled to 40 °C, diluted with 10 mL water for injection and passed through a Waters Sep-Pak Light C18 cartridge. The cartridge was washed with additional 10 mL water for injection and eluted with 0.5 mL warm DMF (warmed to 90 °C before elution). The eluate was diluted with 0.5 mL water for injection and purified by HPLC using a gradient radio-HPLC procedure (conditions: Nucleosil 100-10 C18 column (250 x 8 mm$^2$), λ = 254 nm; flow = 4 mL/min; eluents: A: $CH_3CN$/TFA, 1000/1, B: $H_2O$/TFA, 1000/1; time program: eluent B from 70% to 30% in 30 min, halt 5 min, from 30% to 70% in 5 min). The product fraction of compound (**$^{18}$F] HUG 74** (retention time $t_R$ = 15.5-17.1 min) was evaporated to dryness *in vacuo* and redissolved with 1 mL $H_2O$/EtOH (9:1) The radiosynthesis provided (**$^{18}$F]HUG 74** with a radiochemical yield of 31% (decay-corrected), a radiochemical purity of 98% in 95 min from end of radionuclide production and a calculated specific radioactivity of 3.4 GBq/μmol at the EOS (analytical HPLC-conditions: Nucleosil 100-5 C18 column (250 x 4 mm$^2$), λ = 254 nm; flow = 1 mL/min; eluents:

A: $CH_3CN$/TFA, 1000/1, B: $H_2O$/TFA; time program: eluent A: 40%, 0-22 min, from 40% to 90% in 4 min, from 90% to 40% in 4 min; retention time $t_R$ = 16.2 min).

**Example 37: Determination of the partition coefficient (log P)**

A solution of **[$^{18}$F]HUG 74** (25 μL, 10% EtOH/water) was diluted with water (10 mL). The measured activity was 145 μCi in 10 mL. Four aliquots of this solution were put into 4 different Eppendorf tubes containing octanol (250 μL) (1 control vial, 3 parent vials). All samples were vortexed for 5 min and then centrifuged at 3g for 5 min to separate the layers. For the three parent vials, the top layer (octanol, 100 μL) and the bottom layer (water, 100 μL) were removed and separately transferred to new Eppendorf vials. The fourth vial (control vial) was left undisturbed.
All the vials were counted (the control vial, the parent vials, the octanol and water vials) in rapid succession. The P values were calculated by dividing CPMoct/CPMwater. The log P values were calculated by log [CPMoct/CPMwater].
The total activity of the parent vials plus their respective octanol and water vials gave a relative recovery of 113%.
The average log P (outlier removed) is 1.16 (with outlier the log P is 1.08).

Example 38: Biodistribution of **[$^{18}$F]HUG 74**

Small animal PET scanning. PET experiments were carried out using a sub-millimeter high resolution (0.7 mm full width at half maximum) small animal scanner (32 module quadHIDAC, Oxford Positron Systems Ltd., Oxford, UK) with uniform spatial resolution (< 1 mm) over a large cylindrical field (165 mm diameter, 280 mm axial length).
PET list-mode data were acquired for 120 min and co-registered with contrast enhanced CT. Three-dimensional volumes of interest (VOIs) were defined over the respective organs in CT data sets, transferred to the co-registered PET data and analyzed quantitatively. Regional uptake was calculated as percentage of injected dose by dividing counts per milliliter in the VOI by total counts in the mouse multiplied by 100 (%ID/ml).
Overall, **[$^{18}$F]HUG 74** is cleared fast and efficiently from the body through hepatic and renal elimination with no significant tracer remaining in non-excretion organs 90-120 min p.i. Immediately upon injection of **[$^{18}$F]HUG 74** high levels of radioactivity were observed in the liver and the kidneys. While the activity in the kidney decreased ($T_{max}$: 100 sec, $T_{1/2}$: 3 min p.i.) in parallel to the activity in the blood, the liver first showed a further accumulation of **[$^{18}$F]HUG 74** ($T_{max}$: 3 min, $T_{1/2}$: 36 min p.i.) before clearance into the gallbladder and finally into the intestine. (Figure 7).
Defluorination of the radioligand *in vivo* potentially impairing image interpretation (indicated by bone uptake of [$^{18}$F]fluoride ions) was not observed in the entire dynamic imaging study. Furthermore, accumulation of **[$^{18}$F]HUG 74** in organs/tissues such as the brain, myocardium, lung and muscles, indicating unspecific binding, was not observed.

## Claims

1. Compound of formula (I)

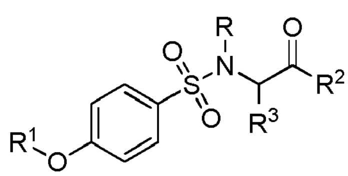

formula (I)

wherein:

R is an optionally substituted arylalkyl or an optionally substituted heteroarylalkyl;
$R^1$ is $C_1$-$C_6$ alkyl, $C_1$-$C_6$ alkenyl, $C_1$-$C_7$ alkynyl wherein the $C_1$-$C_6$ alkyl, $C_1$-$C_6$ alkenyl, $C_1$-$C_7$ alkynyl are optionally substituted with one or more substituents selected from halogen, OH or OTs, or $R^1$ is $(CH_2$-$CH_2$-O-$)_n$-$CH_2$-$CH_2$-$R^5$;
$R^2$ is $C_1$-$C_6$ alkyl, $C_1$-$C_6$ alkoxy or -NH-$OR^4$;
$R^3$ is $C_1$-$C_6$ fluoro-alkyl or an optionally substituted $C_2$-$C_6$ alkenyl wherein the substituent is a F atom;
$R^4$ is H, $C_1$-$C_6$ alkyl, $C_3$-$C_6$ cycloalkyl or $C_5$-$C_6$ heterocycloalkyl;
$R^5$ is F, Cl, OH, OTs; and
n is 0, 1, 2, 3, 4, 5 or 6;

and enantiomers, esters and pharmaceutically acceptable salts thereof.

**2.** Compound of formula (I)

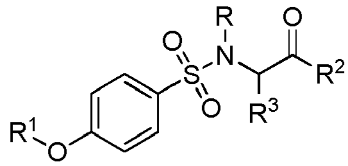

formula (I)

wherein:

R is H or an optionally substituted arylalkyl or an optionally substituted heteroarylalkyl;
$R^1$ is $C_1$-$C_6$ alkyl, $C_1$-$C_6$ alkenyl, $C_1$-$C_7$ alkynyl wherein the $C_1$-$C_6$ alkyl, $C_1$-$C_6$ alkenyl, $C_1$-$C_7$ alkynyl are optionally substituted with one or more substituents selected from halogen, OH or OTs, or $R^1$ is $(CH_2\text{-}CH_2\text{-}O\text{-})_n\text{-}CH_2\text{-}CH_2\text{-}R^5$;
$R^2$ is $C_1$-$C_6$ alkyl, $C_1$-$C_6$ alkoxy or-NH-$OR^4$;
$R^3$ is $C_1$-$C_6$ fluoro-alkyl or an optionally substituted $C_2$-$C_6$ alkenyl wherein the substituent is a F atom;
$R^4$ is H, $C_1$-$C_6$ alkyl, $C_3$-$C_6$ cycloalkyl or $C_5$-$C_6$ heterocycloalkyl;
$R^5$ is F, Cl, OH, OTs; and
n is 0, 1, 2, 3, 4, 5 or 6;

and enantiomers, esters and pharmaceutically acceptable salts thereof.

**3.** The compound according to claim 1 wherein:

R is pyrid-3-yl-$CH_2$- or phenyl-$CH_2$-;
$R^1$ is $-CH_3$, $-CH_2\text{-}CH_2Cl$, $-CH_2\text{-}CH_2F$, $-CH_2\text{-}CH_2\text{-}OTs$ or $-CH_2\text{-}CH_2OH$;
$R^2$ is -NH-$OR^4$;
$R^3$ is $-CH_2\text{-}CH_2F$, $-CH_2\text{-}C(F)\text{=}CH_2$ or $CH_2\text{-}CH\text{=}CH_2$;
$R^4$ is H, methyl, tert-butyl or tetrahydropyranyl; and
enantiomers, esters or pharmaceutically acceptable salts thereof.

**4.** The compound according to claim 1 or 2 or 3 wherein the carbon atom bearing radical $R^3$ has a "S" configuration.

**5.** The compound according to claim 1 or 2 or 3 wherein the carbon atom bearing radical $R^3$ has a "R" configuration.

**6.** Compounds selected from the group consisting of:

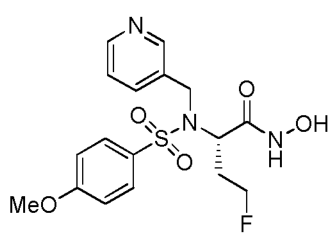

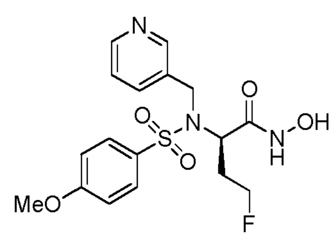

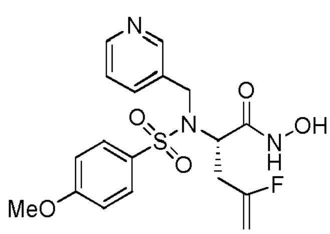

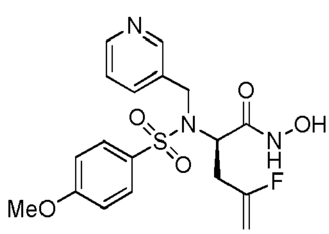

,

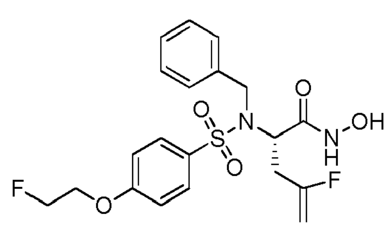

,

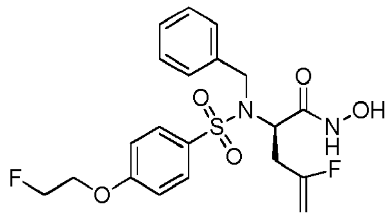

,

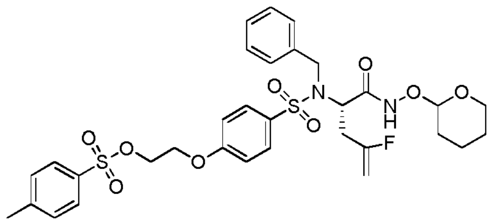

,

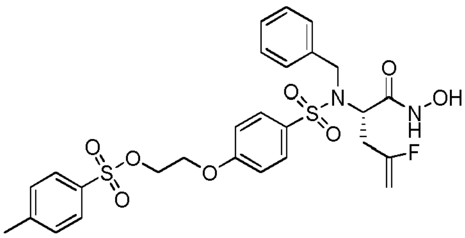

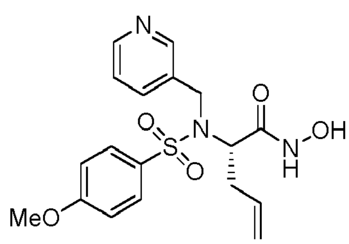

,

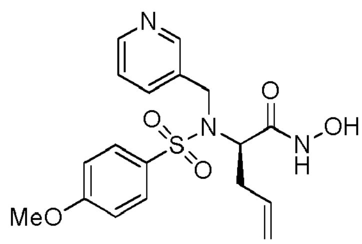

,

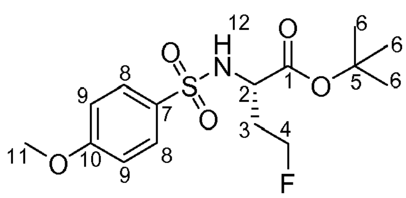

,

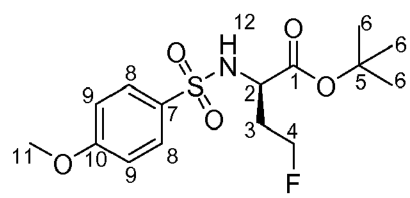

,

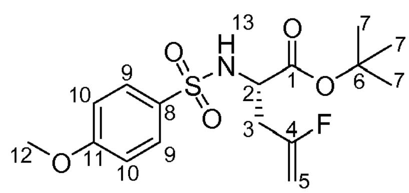

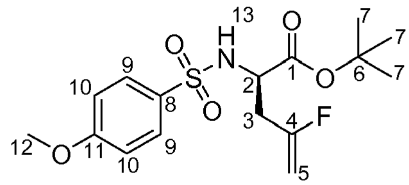

racemates, esters and pharmaceutically acceptable salts thereof.

**7.** A compound of formula (I) as defined in any of claims 1, 2, 3, 4, 5, 6 wherein a carbon atom bears as a substituent a $^{18}F$ in addition or in place to a substituent already present on said carbon atom.

**8.** The compound according to claim 7 wherein the carbon atom is a carbon atom of radical $R^3$.

**9.** The compound according to claim 8 wherein $R^3$ is selected from $C_1$-$C_6$ ($^{18}F$) alkyl or $C_2$-$C_6$ ($^{18}F$)-alkenyl.

**10.** The compound according to claim 9 wherein $R^3$ is selected from $CH_2$-$CH_2{}^{18}F$ or $CH_2$-$C(^{18}F)=CH_2$.

**11.** The compound according to claim 7 wherein the carbon atom is a carbon atom of radical $R^1$.

**12.** The compound according to claim 11, wherein $R^1$ is -$CH_2$-$CH_2{}^{18}F$.

**13.** Compounds of formulae

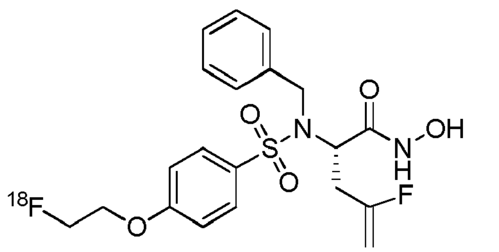

or

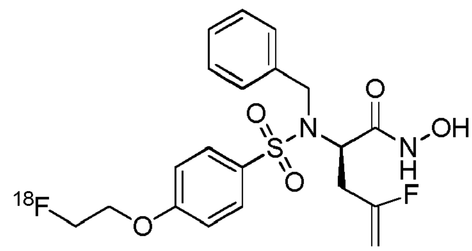

racemate, esters and pharmaceutically acceptable salts thereof.

**14.** A compound according to any of claims 1, 2, 3, 4, 5, 6 for use as a medicament.

**15.** A compound according to any of claims 7, 8, 9, 10, 11, 12, 13 for use as a diagnostic agent.

**16.** A compound according to any of claims 1, 2, 3, 4, 5, 6 for use in the prevention or treatment of pathological conditions associated with an unpaired expression of matrix-metalloprotease in human and animal.

**17.** A compound according to any of claims 7, 8, 9, 10, 11, 12, 13 for use in the diagnosis of pathological conditions associated with an unpaired expression of matrix-metalloprotease in human and animal.

**18.** The compound according to claim 16 or 17 wherein the pathological condition is selected from the group consisting of cardiovascular diseases, inflammatory diseases and malignant diseases.

**19.** The compound according to claim 18 wherein the cardiovascular diseases are selected from atherosclerosis and congestive heart failure and the inflammatory disease is chronic obstructive pulmonary disease.

**20.** A pharmaceutical composition comprising a compound as defined in any of claims 1, 2, 3, 4, 5, 6 and a pharmaceutically acceptable carrier.

**21.** A diagnostic composition comprising a compound as defined in any of claims 7, 8, 9, 10, 11, 12, 13 and a pharmaceutically acceptable carrier.

**22.** Process for preparing a compound as defined in any of claims 7, 8, 9, 10, 11, 12 comprising:

a) reacting a compound of formula (II)

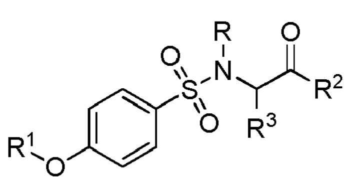

formula (II)

wherein:

R and $R^2$ are as defined in claim 1 or 2 or 3;
$R^1$ is an optionally substituted $C_1$-$C_6$ alkyl wherein the substituent is OH, OTs or a another leaving group for nucleophile substitution;
$R^3$ is an optionally substituted $C_1$-$C_6$ -alkyl or an optionally substituted $C_2$-$C_6$ alkenyl wherein the substituent is a OH, a OTs or another leaving group for nucleophile
substitution

with a $^{18}F$ containing reagent for nucleophile substitution of the group OTs or of the group OH or of the leaving group with $^{18}F$;
with the proviso that least one of $R^1$ and $R^3$ is substituted with a OH, OTs or another leaving group for nucleophile substitution and/or with the proviso that when $R^1$ is substituted with a OH, OTs or another leaving group for nucleophile substitution then $R^3$ is $C_1$-$C_6$ fluoro alkyl or $C_2$-$C_6$ alkenyl optionally substituted with F.

**23.** The process according to claim 22 wherein in formula (II):

$R^1$ is $CH_2$-$CH_2$-OTs and $R^3$is $CH_2$-$CH_2$ F, $CH_2$-C(F)=$CH_2$ or $CH_2$-CH=$CH_2$; or
$R^1$ is $CH_3$ and $R^3$ is $CH_2$-$CH_2$OTs.

**24.** The process according to claim 22 or 23 wherein the $^{18}F$ containing reagent is [$^{18}F$](Kryptofix222)KF.

**25.** Intermediate compound of formula (III)

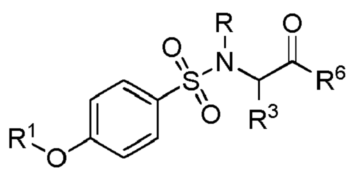

formula (III)

wherein
R, $R^1$ and $R^3$ are as defined in claim 1 or 2 or 3; and $R^6$ is OH.

**26.** Intermediate compound according to claim 25 selected from:

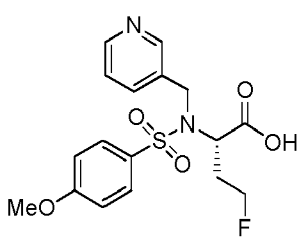 , 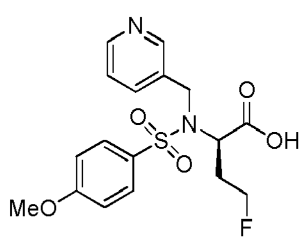 , 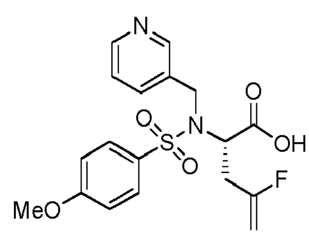 ,

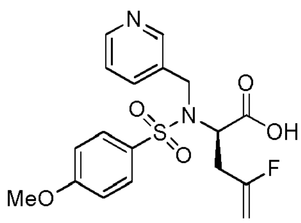 , 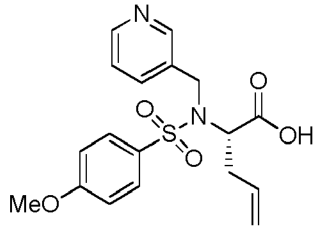 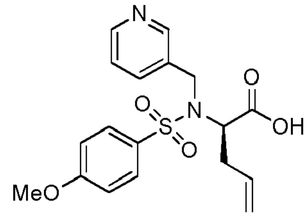 ,

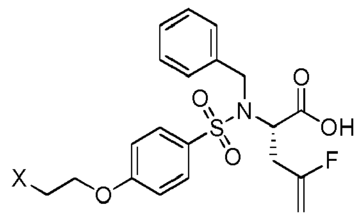,
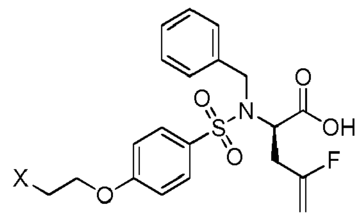

wherein X is F, Cl, OH or OTs or intermediate compound of formula

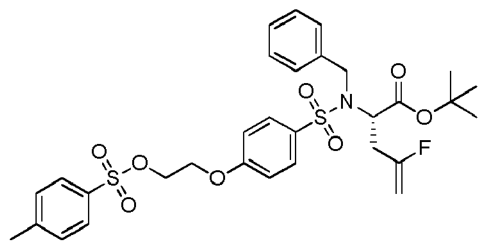.

**27.** Process for preparing a compound of formula (I)

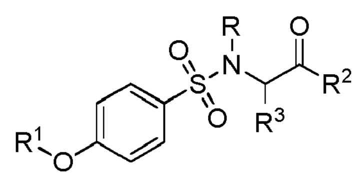

formula (I)

wherein
R, $R^1$ and $R^3$ are as defined in claim 1 or 2 or 3;
$R^2$ is NH-O$R^4$; and
$R^4$ is H, $C_1$-$C_6$ alkyl, $C_3$-$C_6$ cycloalkyl or $C_5$-$C_6$ heterocycloalkyl
comprising

a) reacting a compound of formula (III')

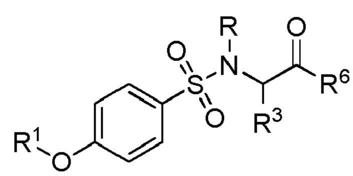

formula (III')

wherein
R, $R^1$ and $R^3$ are as defined in claim 1 or 2 or 3
and $R^6$ is OH or $C_1$-$C_6$ alkoxy
with an hydroxylamine derivative of formula (IV)

$R^4O$-$NH_2$ formula (IV)

wherein $R^4$ is H, $C_1$-$C_6$ alkyl, $C_3$-$C_6$ cycloalkyl or $C_5$-$C_6$ heterocycloalkyl; and
b) when in the compound obtained in step a) $R^4$ is $C_1$-$C_6$ alkyl, $C_3$-$C_6$ cycloalkyl or $C_5$-$C_6$ heterocycloalkyl optionally hydrolyzing it.

**28.** Process for preparing a compound of formula (I) according to claim 27 wherein the compound of formula (III') is prepared by

a') reacting a compound of formula (V)

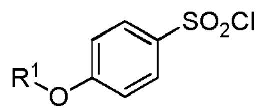

formula (V)

with a compound of formula (VI)

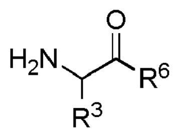

formula (VI)

to obtain a compound of formula (VII)

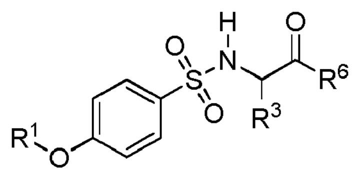

formula (VII)

and

a") reacting the compound of formula (VII) with a compound of formula (VIII)

R-Cl Formula (VIII)

to obtain a compound of formula (III')

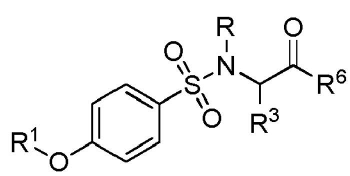

formula (III')

wherein in any of the formula (III'), (V), (VI), and (VII) and (VIII) R, $R^1$, $R^2$, $R^3$, $R^4$ and $R^6$ are as defined in claim 27.

**Figure 1**

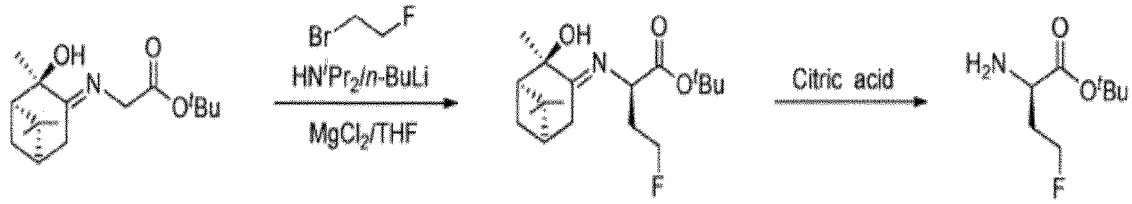

HN$^i$Pr$_2$/*n*-BuLi
MgCl$_2$/THF
Citric acid


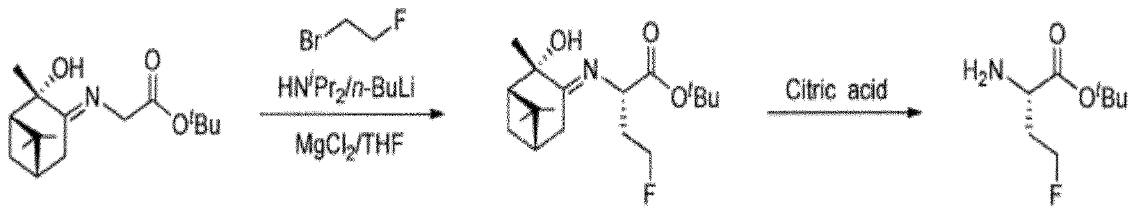

HN$^i$Pr$_2$/*n*-BuLi
MgCl$_2$/THF
Citric acid


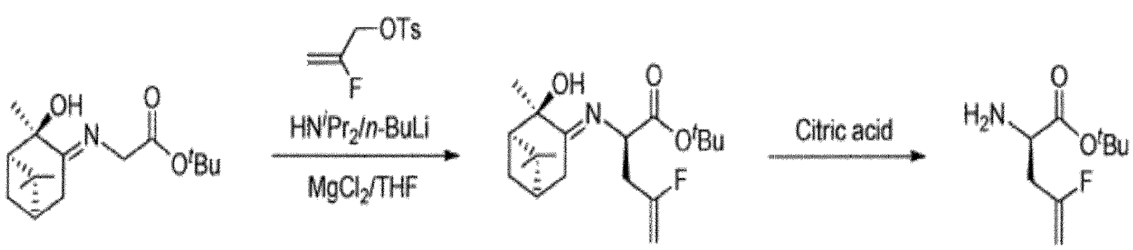

HN$^i$Pr$_2$/*n*-BuLi
MgCl$_2$/THF
Citric acid


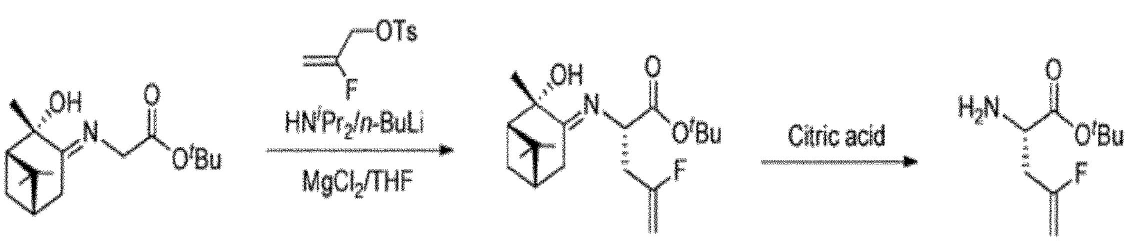

HN$^i$Pr$_2$/*n*-BuLi
MgCl$_2$/THF
Citric acid

**Figure 2 (cont'd)**

**A)**

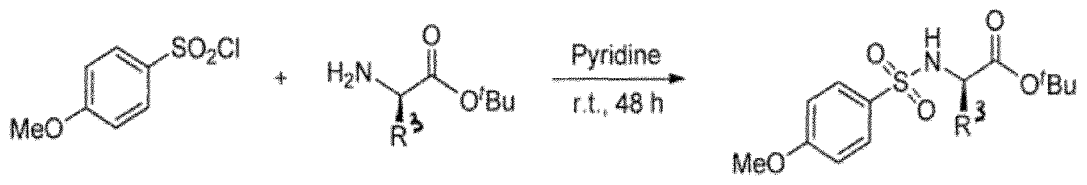


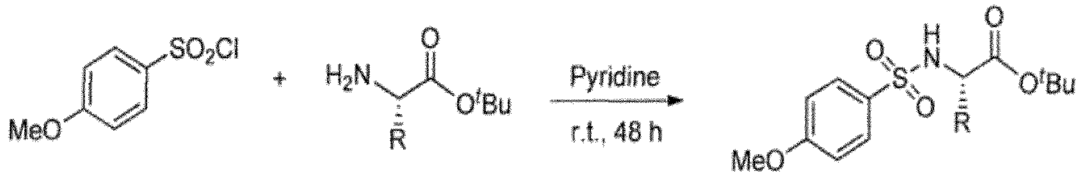


$R^3 = CH_2CH_3$, $CH_2CH_2F$, $CH_2CH{=}CH_2$, $CH_2CF{=}CH_2$

**B)**

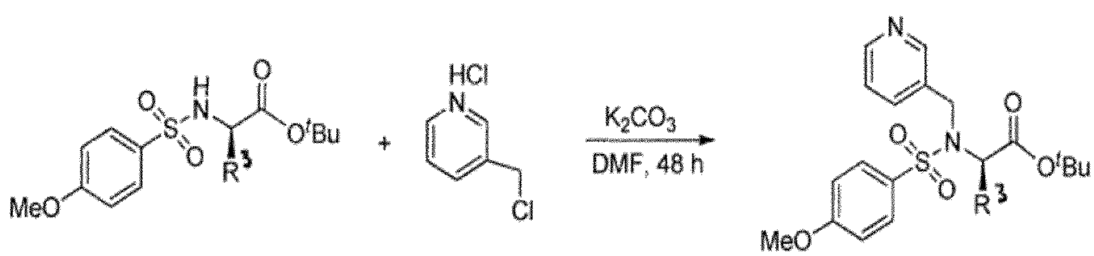


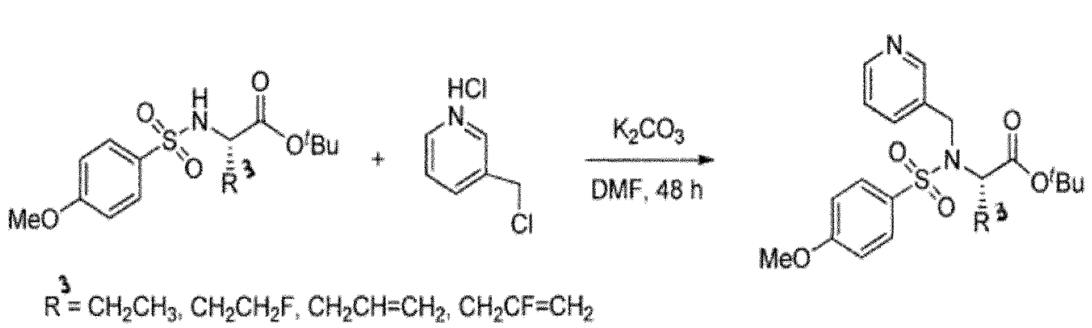


$R^3 = CH_2CH_3$, $CH_2CH_2F$, $CH_2CH{=}CH_2$, $CH_2CF{=}CH_2$

**Figure 2 (cont'd)**

C)

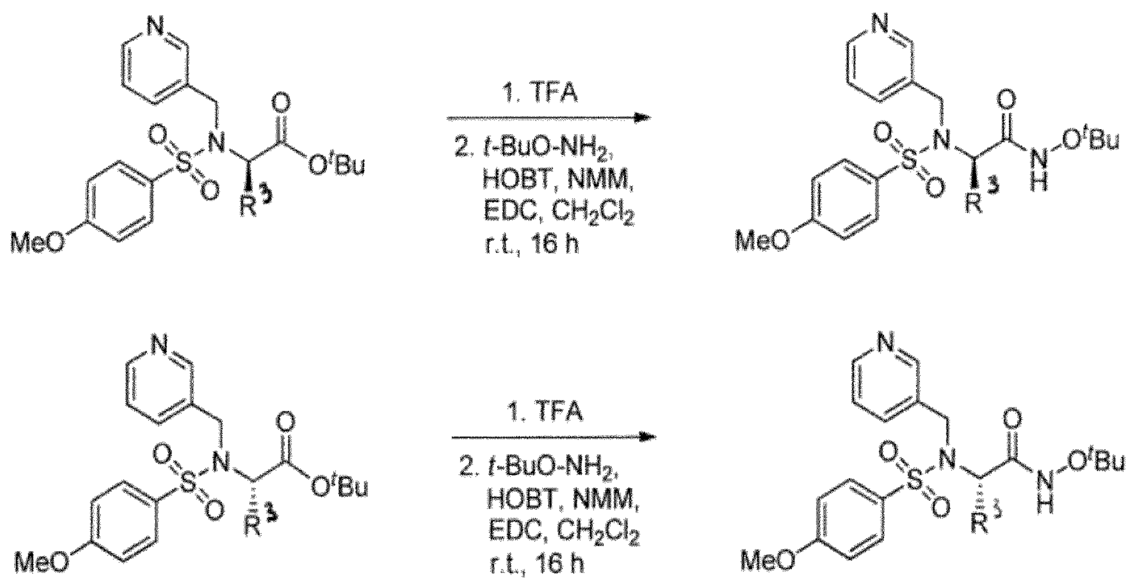


$R^3$= $CH_2CH_3$, $CH_2CH_2F$, $CH_2CH{=}CH_2$, $CH_2CF{=}CH_2$

**Figure 3**

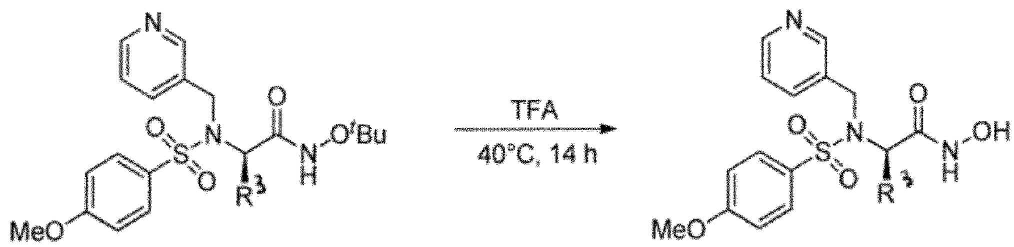


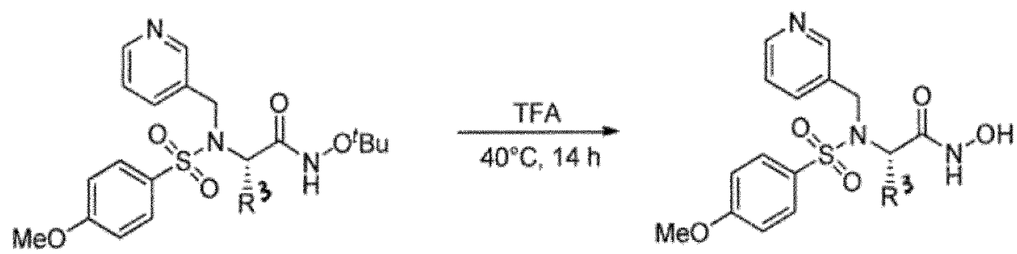


$R^3$= $CH_2CH_3$, $CH_2CH_2F$, $CH_2CH{=}CH_2$, $CH_2CF{=}CH_2$

**Figure 4**

**A)**

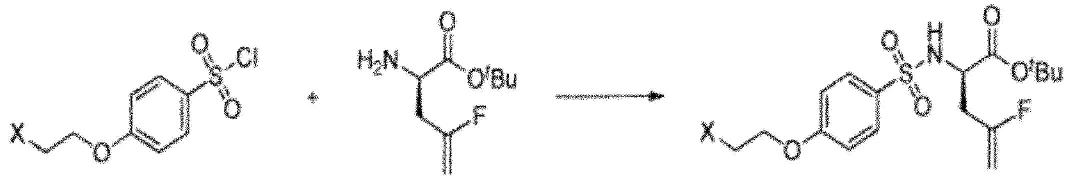

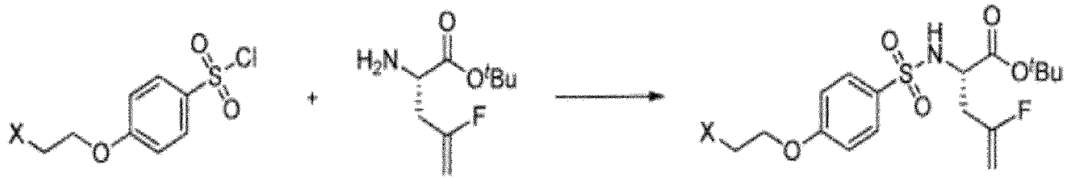

X = F, OTs, OH, Cl

**B)**

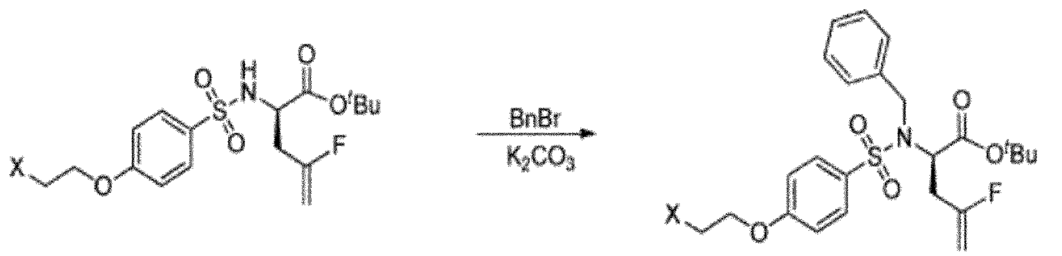


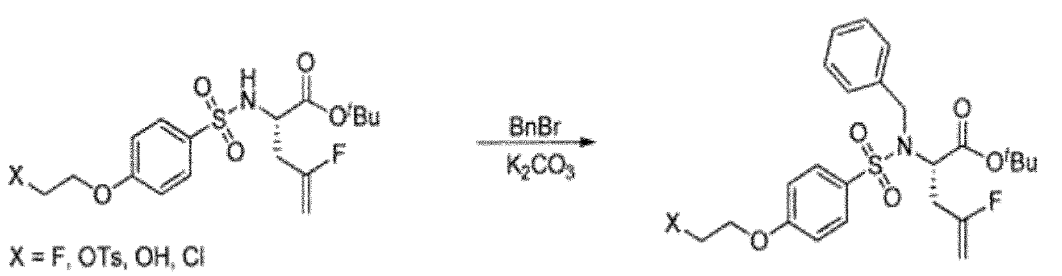


X = F, OTs, OH, Cl

Figure 4 (cont'd)

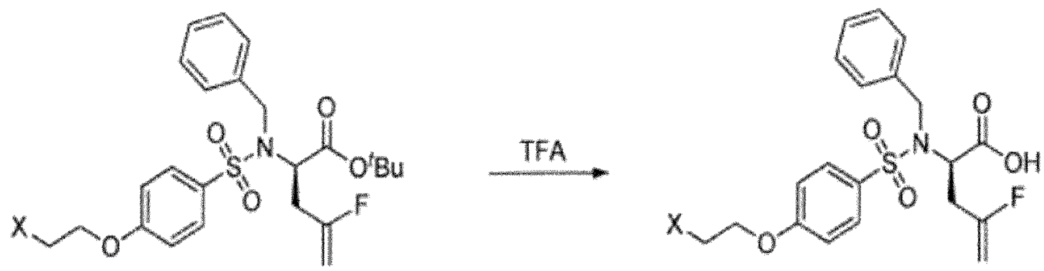


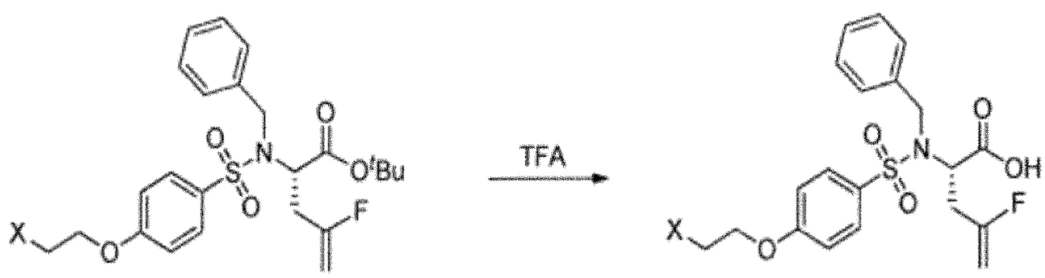


X = F, OTs, OH, Cl

Figure 5

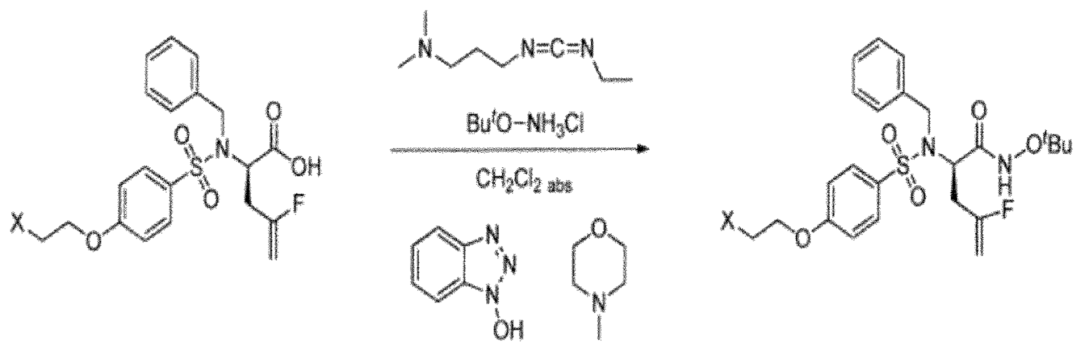


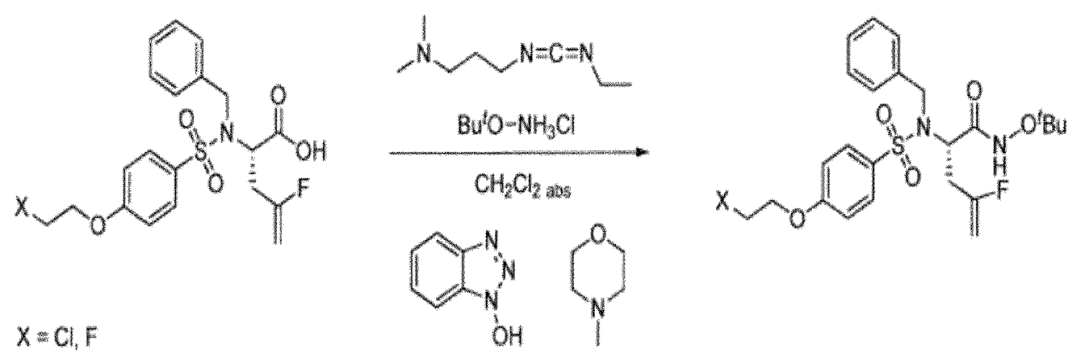


X = Cl, F

**Figure 6**

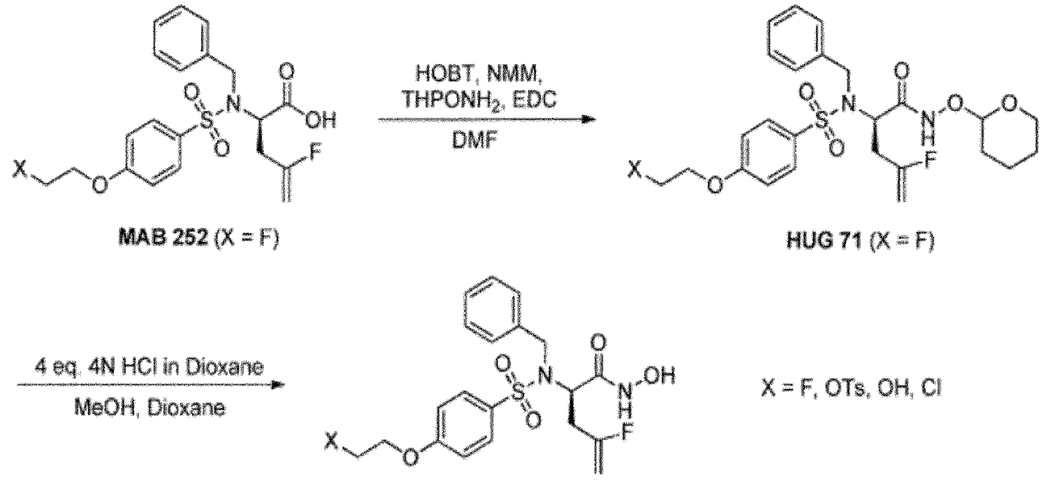


**MAB 252** (X = F)

**HUG 71** (X = F)

X = F, OTs, OH, Cl

**HUG 78** (X = F)

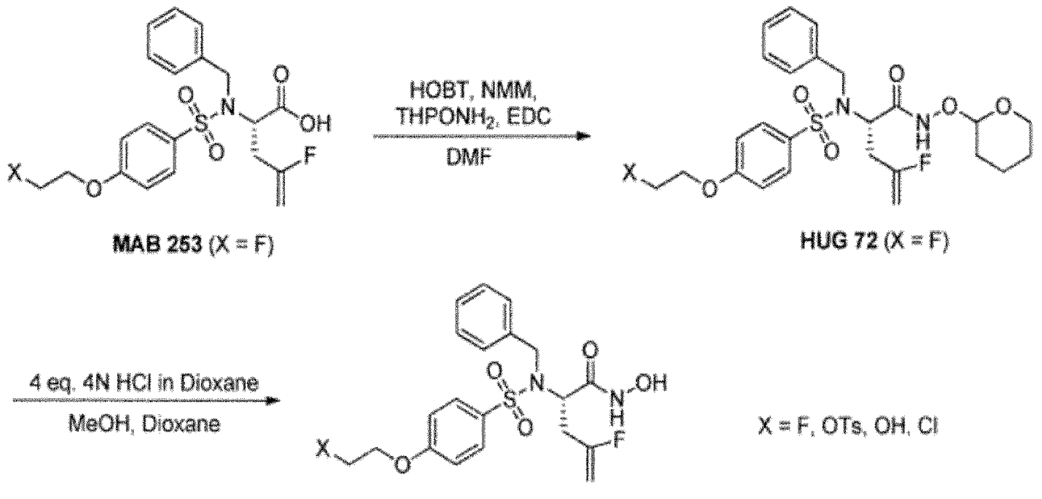


**MAB 253** (X = F)

**HUG 72** (X = F)

X = F, OTs, OH, Cl

**HUG 74** (X = F)

Figure 7

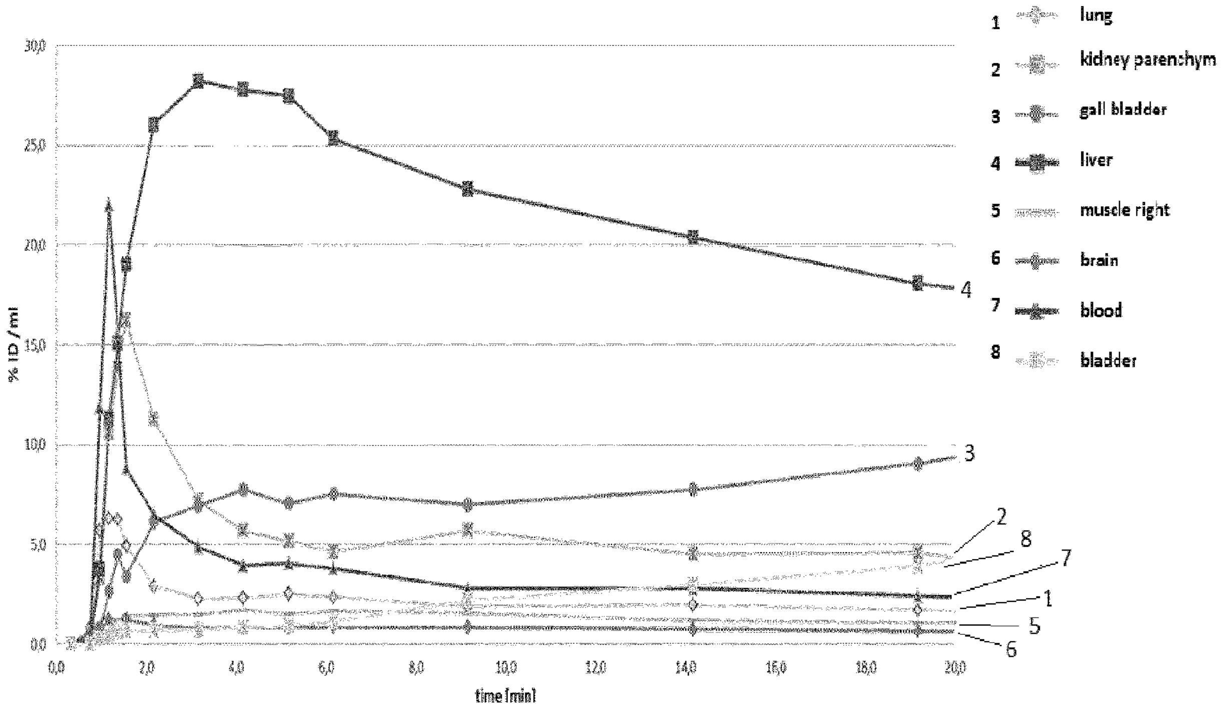
1 lung
2 kidney parenchym
3 gall bladder
4 liver
5 muscle right
6 brain
7 blood
8 bladder
% ID / ml
time [min]
30,0
25,0
20,0
15,0
10,0
5,0
0,0
2,0
4,0
6,0
8,0
10,0
12,0
14,0
16,0
18,0
20,0
4
3
2
8
7
1
5
6

Europäisches Patentamt
European Patent Office
Office européen des brevets

# EUROPEAN SEARCH REPORT

Application Number
EP 12 16 6980

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 5 455 258 A (MACPHERSON LAWRENCE J [US] ET AL) 3 October 1995 (1995-10-03)<br>* page 1, lines 6-15; claim 1; example 1 *<br>----- | 1-28 | INV.<br>C07D309/12<br>C07D213/42<br>C07C311/29<br>C07B59/00<br>A61K31/255<br>A61K31/4409<br>A61P9/00<br>A61P29/00 |
| | | | TECHNICAL FIELDS SEARCHED (IPC) |
| | | | C07D<br>C07C<br>C07B<br>A61P<br>A61K |

The present search report has been drawn up for all claims

| Place of search | Date of completion of the search | Examiner |
|---|---|---|
| The Hague | 4 July 2012 | Matés Valdivielso, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

2

**ANNEX TO THE EUROPEAN SEARCH REPORT ON EUROPEAN PATENT APPLICATION NO.** EP 12 16 6980

This annex lists the patent family members relating to the patent documents cited in the above-mentioned European search report.
The members are as contained in the European Patent Office EDP file on
The European Patent Office is in no way liable for these particulars which are merely given for the purpose of information.

04-07-2012

| Patent document cited in search report | Publication date | Patent family member(s) | Publication date |
|---|---|---|---|
| US 5455258 A | 03-10-1995 | AT 159012 T | 15-10-1997 |
| | | AU 684255 B2 | 11-12-1997 |
| | | BR 1100131 A | 14-03-2000 |
| | | CA 2112779 A1 | 07-07-1994 |
| | | DE 69314456 D1 | 13-11-1997 |
| | | DE 69314456 T2 | 26-02-1998 |
| | | DK 0606046 T3 | 04-05-1998 |
| | | EP 0606046 A1 | 13-07-1994 |
| | | ES 2107648 T3 | 01-12-1997 |
| | | FI 940012 A | 07-07-1994 |
| | | GR 3025611 T3 | 31-03-1998 |
| | | HK 1002633 A1 | 04-09-1998 |
| | | HU 221015 B1 | 29-07-2002 |
| | | IL 108229 A | 30-10-1998 |
| | | JP 2951527 B2 | 20-09-1999 |
| | | JP 6256293 A | 13-09-1994 |
| | | NO 940038 A | 07-07-1994 |
| | | NZ 250517 A | 26-10-1995 |
| | | SG 42933 A1 | 17-10-1997 |
| | | US 5455258 A | 03-10-1995 |
| | | ZA 9400048 A | 11-08-1994 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0160416 A


### Non-patent literature cited in the description


- **MASSOVA et al.** *FASEB J.,* 1998, vol. 12, 1075-1095
- **NAGASE ; WOESSNER.** *J. Biol. Chem.,* 1999, vol. 274, 21491-21494
- **WHITTAKER et al.** *Chem. Rev.,* 1999, vol. 99, 2735-2776
- **ZHENG et al.** *Nuc. Med. Biol.,* 2002, vol. 29, 761-770
- **GEORGE.** *Exp. Opin. Invest. Drugs,* 2000, vol. 9, 993-1007
- **LI et al.** *Am. J. Pathol.,* 1996, vol. 148, 121-128
- **HERMAN et al.** *Circulation,* 2001, vol. 104, 1899-1904
- **PETERSON et al.** Matrix metalloproteinase inhibitor development for the treatment of heart failure. *Drug Dev. Res.,* 2002, vol. 55, 29-44
- **VIHINEN et al.** *Int. J. Cancer,* 2002, vol. 99, 157-186
- **JACSON et al.** Selective matrix metalloproteinase inhibition in rheumatoid arthritis-targeting gelatinase A activation. *Inflamm. Res.,* 2001, vol. 50, 183-186
- **LIM et al.** *J. Neurochem.,* 1996, vol. 67, 251-259
- **SPINALE.** *Circul. Res.,* 2002, vol. 90, 520-530
- **LUKES et al.** *Mol. Neurobiol.,* 1999, vol. 19, 267-284
- **BACKSTROM et al.** *J. Neurochem.,* 1992, vol. 58, 983-992
- **MUN-BRYCE et al.** *Brain. Res.,* 2002, vol. 933, 42-49
- **SEGURA-VALDEZ et al.** *Chest.,* 2000, vol. 117, 684-694
- **KURPAKUS-WHEATER et al.** *Prog. Histo. Cytochem.,* 2001, vol. 36, 179-259
- **HEROUY.** *Int. J. Mol. Med.,* 2001, vol. 7, 3-12
- **D. M. SHENDAGE ; R. FRÖHLICH ; K. BERGANDER ; G. HAUFE.** *Eur. J. Org. Chem.,* 2005, 719-727
- **K.-W. LAUE ; S. KRÖGER ; E. WEGELIUS ; G. HAUFE.** *Eur. J. Org. Chem.,* 2000, 3737-3743
- **K.-W. LAUE ; C. MÜCK-LICHTENFELD ; G. HAUFE.** *Tetrahedron,* 1999, vol. 55, 10413-10424
- **K.-W. LAUE ; G. HAUFE.** *Synthesis,* 1998, 1453-1456
- **S. KRÖGER ; G. HAUFE.** *Amino Acids,* 1997, vol. 12, 363-372